(19) Europäisches Patentamt
European Patent Office
Office européen des brevets

(11) EP 4 458 819 A1

(12) **EUROPEAN PATENT APPLICATION**
published in accordance with Art. 153(4) EPC

(43) Date of publication:
**06.11.2024 Bulletin 2024/45**

(21) Application number: **22915126.1**

(22) Date of filing: **29.12.2022**

(51) International Patent Classification (IPC):
***C07D 401/14*** (2006.01) ***C07D 417/14*** (2006.01)
***C07D 487/04*** (2006.01) ***A61K 31/4725*** (2006.01)
***A61P 35/00*** (2006.01)

(52) Cooperative Patent Classification (CPC):
**A61K 31/4725; A61P 35/00; C07D 401/14; C07D 417/14; C07D 487/04**

(86) International application number:
**PCT/CN2022/143665**

(87) International publication number:
**WO 2023/125877 (06.07.2023 Gazette 2023/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.12.2021 CN 202111655759**
**14.03.2022 CN 202210249052**
**31.08.2022 CN 202211064934**

(71) Applicants:
- **Shanghai Hansoh Biomedical Co., Ltd.**
  **Shanghai 201203 (CN)**
- **Jiangsu Hansoh Pharmaceutical Group Co., Ltd.**
  **Lianyungang, Jiangsu 222047 (CN)**

(72) Inventors:
- **WU, Jianrui**
  **Shanghai 201203 (CN)**
- **DONG, Jiaqiang**
  **Shanghai 201203 (CN)**
- **JIN, Fangfang**
  **Shanghai 201203 (CN)**
- **YU, Wensheng**
  **Shanghai 201203 (CN)**
- **ZHANG, Lipu**
  **Shanghai 201203 (CN)**

(74) Representative: **Cabinet Beau de Loménie**
**158, rue de l'Université**
**75340 Paris Cedex 07 (FR)**

# (54) TRICYCLIC DERIVATIVE INHIBITOR, PREPARATION METHOD THEREFOR, AND APPLICATION THEREOF

(57) The invention relates to a tricyclic derivative inhibitor, a preparation method therefor, and application thereof. In particular, the present invention relates to a compound as shown in general formula (I), a preparation method therefor, a pharmaceutical composition containing the compound, as well as a use thereof in the treatment of cancer and autoimmune diseases, wherein each substituent in general formula (I) is the same as the definition in the description.



Processed by Luminess, 75001 PARIS (FR)

## Description

### TECHNICAL FIELD

The present invention belongs to the field of biomedicine, and specifically relates to a tricyclic derivative inhibitor, and a preparation method therefor and the use thereof.

### BACKGROUND ART

MALT1 (mucosa-associated lymphoid tissue lymphoma translocation protein 1) is a protease and scaffolding protein, which is an intracellular signaling molecule that plays an important role in immunity and inflammation and participates in NF-kB signal transduction downstream of B cells and T cell receptors (BCR, TCR). MALT1, CARM1 and BCL10 form a CBM complex, and mutations in these genes can cause constitutive activation of the NF-kB signaling pathway.

The growth of ABC-DLBCL is strongly dependent on the sustained activation of the NF-kB signaling pathway. It accounts for 16% of the incidence of lymphoma in China and 40% of DLBCL in China. MALT lymphoma is caused by translocation and rearrangement of MALT1 and BCL10 genes, and accounts for 9% of the incidence of lymphoma in China. In addition, MALT inhibitors are also expected to be used in CLL patients who are resistant to BTK inhibitors.

Currently, only Johnson & Johnson's compound JNJ-67856633 has entered clinical phase I in the world. The NCT03900598 trial in April 2019 was a clinical phase I, and the NCT04657224 trial in December 2020 was a clinical phase Ib, using a combination with a BTK inhibitor. Published patent applications for MALT1 inhibitors include: Johnson & Johnson's patented compounds (WO 2018119036,WO 2019243964, WO 2019243965, and WO 2020208222); Novartis; MLT-985 and others (WO 2015181747, and WO 2017081641); Lupin's LND-700110 and others (WO 2018020474); Medvir's cmpd A and others (WO 2018226150); Takeda's patented compounds (WO 2020111087); Cornell University's patented compounds (WO 2014074815, WO 2018165385, and WO 2018085247) and Toray's patented compounds (WO 2017057695, WO 2018021520, and WO 2018159650).

MALT1 inhibitors have good application prospects as drugs in the pharmaceutical industry.

I: It is expected to become a targeted therapy for ABC-DLBCL. The current standard therapy is R-Chop, which has a poor prognosis.

II: It is expected to become a follow-up treatment for CLL and MCL patients with BTK resistance. Main problems with current inhibitors are that: the in vitro activity decreases greatly from enzyme activity itself to the activity in a cell, and the druggability of the compound needs to be improved (shorter half-life, etc.).

### SUMMARY OF THE INVENTION

The objective of the present invention is to provide a compound as represented by general formula (I), or a stereoisomer or pharmaceutically acceptable salt thereof, wherein the compound as represented by general formula (I) has a structure as follows:

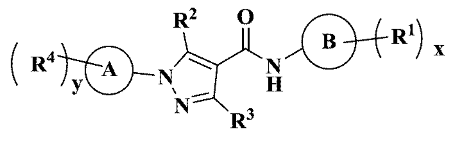

(I)

wherein:

ring A is selected from cycloalkyl, heterocyclyl, aryl or heteroaryl, wherein the cycloalkyl, heterocyclyl, aryl or heteroaryl can be optionally further substituted;

ring B is selected from cycloalkyl, heterocyclyl, aryl or heteroaryl, wherein the cycloalkyl, heterocyclyl, aryl or heteroaryl can be optionally further substituted;

$R^1$ is independently selected from hydrogen, deuterium, halogen, amino, hydroxyl, cyano, nitro, alkyl, alkenyl, alkynyl, deuterated alkyl, haloalkyl, alkoxy, haloalkoxy, hydroxyalkyl, cycloalkyl, cycloalkoxy, heterocyclyl, aryl, heteroaryl, $-CR_{cc}=CR_{dd}(CH_2)_nR_{aa}$, $-CR_{cc}=CR_{dd}(CH_2)_nNR_{aa}R_{bb}$, $-CR_{cc}=CR_{dd}(CH_2)_nNR_{ee}C(O)R_{aa}$, $-CR_{cc}=CR_{dd}(CH_2)_nN$-

$R_{ee}C(O)NR_{aa}R_{bb}$, $-O(CH_2)_nR_{cc}$, $-OC(R_{aa}R_{bb})_n(CH_2)_mR_{cc}$, $-NR_{ee}(CH_2)_nR_{cc}$, $-(CH_2)_n-$, $-(CH_2)_nR_{cc}$, $-(CH_2)_nOR_{cc}$, $-(CH_2)_nSR_{cc}$, $-(CH_2)_nC(O)R_{cc}$, $-(CH_2)_nC(=NR_{ee})R_{cc}$, $-(CH_2)_nC(O)OR_{cc}$, $-(CH_2)_nS(O)_mR_{cc}$, $-(CH_2)_nNR_{aa}R_{bb}$, $-(CH_2)_nC(O)NR_{aa}R_{bb}$, $-(CH_2)_nNR_{ee}C(O)R_{cc}$, $-(CH_2)_nN=S(O)R_{cc}R_{ee}$ or $-(CH_2)_nNR_{ee}S(O)_mR_{cc}$, wherein the alkyl, alkenyl, alkynyl, deuterated alkyl, haloalkyl, alkoxy, haloalkoxy, hydroxyalkyl, cycloalkyl, heterocyclyl, aryl and heteroaryl can be optionally further substituted;

preferably, $R^1$ is independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cycloalkyl, cycloalkoxy, heterocyclyl, aryl, heteroaryl, $-CR_{cc}=CR_{dd}(CH_2)_nR_{aa}$, $-CR_{cc}=CR_{dd}(CH_2)_nNR_{aa}R_{bb}$, $-CR_{cc}=CR_{dd}(CH_2)_nNR_{ee}C(O)R_{aa}$, $-CR_{cc}=CR_{dd}(CH_2)_nNR_{ee}C(O)NR_{aa}R_{bb}$, $-O(CH_2)_nR_{cc}$, $-OC(R_{aa}R_{bb})_n(CH_2)_mR_{cc}$, $-NR_{ee}(CH_2)_nR_{cc}$, $-(CH_2)_n-$, $-(CH_2)_nR_{cc}$, $-(CH_2)_nOR_{cc}$, $-(CH_2)_nSR_{cc}$, $-(CH_2)_nC(O)R_{cc}$, $-(CH_2)_nC(=NR_{ee})R_{cc}$, $-(CH_2)_nC(O)OR_{cc}$, $-(CH_2)_nS(O)_mR_{cc}$, $-(CH_2)_nNR_{aa}R_{bb}$, $-(CH_2)_nC(O)NR_{aa}R_{bb}$, $-(CH_2)_nNR_{ee}C(O)R_{cc}$ or $-(CH_2)_nNR_{ee}S(O)_mR_{cc}$, wherein the alkyl, alkenyl, alkynyl, deuteroalkyl, haloalkyl, alkoxy, haloalkoxy, hydroxyalkyl, cycloalkyl, heterocyclyl, aryl and heteroaryl optionally can be further substituted;

$R^2$ is selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cycloalkyl, cycloalkoxy, heterocyclyl, aryl or heteroaryl, wherein the alkyl, alkenyl, alkynyl, deuteroalkyl, haloalkyl, alkoxy, haloalkoxy, hydroxyalkyl, cycloalkyl, heterocyclyl, aryl and heteroaryl optionally can be further substituted;

$R^3$ is selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cycloalkyl, cycloalkoxy, heterocyclyl, aryl or heteroaryl, wherein the alkyl, alkenyl, alkynyl, deuteroalkyl, haloalkyl, alkoxy, haloalkoxy, hydroxyalkyl, cycloalkyl, heterocyclyl, aryl and heteroaryl optionally can be further substituted;

$R^4$ is independently selected from hydrogen, deuterium, halogen, amino, hydroxyl, cyano, nitro, alkyl, alkenyl, alkynyl, oxo, thio, deuteroalkyl, haloalkyl, alkoxy, haloalkoxy, hydroxyalkyl, cyano-substituted alkyl, cycloalkyl, heterocyclyl, aryl, heteroaryl, $-CR_{33}=CR_{44}(CH_2)_pR_{11}$, $-CR_{33}=CR_{44}(CH_2)_pNR_uR_{22}$, $-CR_{33}=CR_{44}(CH_2)_pNR_{55}C(O)R_{11}$, $-CR_{33}=CR_{44}(CH_2)_pNR_{55}C(O)NR_{11}R_{22}$, $-O(CH_2)_pR_{33}$, $-OC(R_{11}R_{22})_q(CH_2)_pR_{33}$, $-NR_{55}(CH_2)_pR_{33}$, $-(CH_2)_p-$, $-(CH_2)_pR_{33}$, $-(CH_2)_pOR_{33}$, $-(CH_2)_pSR_{33}$, $-(CH_2)_pC(O)R_{33}$, $-(CH_2)_pC(=NR_{55})R_{33}$, $-(CH_2)_pC(O)OR_{33}$, $-(CH_2)_pS(O)_qR_{33}$, $-(CH_2)_pNR_{11}R_{22}$, $-(CH_2)_pC(O)NR_{11}R_{22}$, $-(CH_2)_pNR_{15}C(O)R_{33}$ or $-(CH_2)_pNR_{55}S(O)_qR_{33}$, wherein the alkyl, alkenyl, alkynyl, deuteroalkyl, haloalkyl, alkoxy, haloalkoxy, hydroxyalkyl, cyano-substituted alkyl, cycloalkyl, heterocyclyl, aryl and heteroaryl can be optionally further substituted,

alternatively, any two adjacent or non-adjacent $R^4$ can be connected to form cycloalkyl, heterocyclyl, aryl or heteroaryl, wherein the cycloalkyl, heterocyclyl, aryl or heteroaryl can be optionally further substituted;

$R_{aa}$ and $R_{bb}$ are each independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl and heteroaryl optionally can be further substituted,

alternatively, $R_{aa}$ and $R_{bb}$ together with adjacent atoms form cycloalkyl, heterocyclyl, aryl or heteroaryl, wherein the cycloalkyl, heterocyclyl, aryl and heteroaryl optionally can be further substituted;

$R_{cc}$-$R_{ee}$ are each independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl and heteroaryl optionally can be further substituted;

$R_{11}$ and $R_{22}$ are each independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl and heteroaryl optionally can be further substituted,

alternatively, $R_{11}$ and $R_{22}$ together with adjacent atoms form cycloalkyl, heterocyclyl, aryl or heteroaryl, wherein the cycloalkyl, heterocyclyl, aryl and heteroaryl optionally can be further substituted;

$R_{33}$-$R_{55}$ are each independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl and heteroaryl optionally can be further substituted;

m is 0, 1, 2 or 3;

n is 0, 1, 2 or 3;

p is 0, 1, 2 or 3;

q is 0, 1, 2 or 3;

x is 0, 1, 2, 3 or 4; and

y is an integer from 0 to 6.

In a preferred embodiment of the present invention, the compound, the stereoisomer or pharmaceutically acceptable salt thereof is further represented by general formula (II):

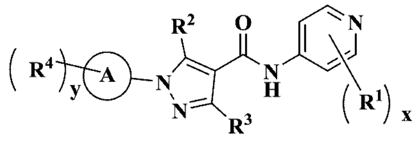

(II)

.

In a preferred embodiment of the present invention, ring A is selected from

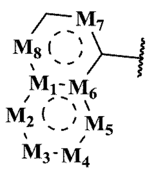

,

wherein, $M_1$ is selected from -N- or -C-, $M_2$ is selected from -C(O)-, -C(S)-, -C(NH)-, - NH-, -N-, - $CH_2$-, -CH-, -O- or -S-, and $M_3$ is selected from -N-, -NH-, -CH -, -$CH_2$-, -C(O)-, -C(S)- or -C(NH)-; $M_4$ is selected from -CH-, -$CH_2$- or -N-, $M_6$ is selected from -N-, -CH- or -CH-, $M_5$ or $M_7$ is -O-, -N-, -CH- , -CH=CH-, -$CH_2$- or absent, and $M_8$ is selected from -N-, -NH-, -O-, -S- or -CH-,

preferably, $M_1$ is selected from -N- or -C-, $M_2$ is selected from -C(O)-, -C(S)-, -C(NH)-, -NH-, -N-, - $CH_2$-, -CH-, -O- or -S-, and $M_3$ is selected from -N-, -NH-, -CH-, -C(O)-, -C(S)- or -C(NH)-; $M_4$ is selected from -CH-, -$CH_2$- or -N-, $M_6$ is selected from -N- or -CH-, $M_5$ or $M_7$ is -CH-, -$CH_2$- or absent, and $M_8$ is selected from -NH-, -O-, -S- or -CH-,

optionally, ring A is further substituted with 1 to 3 $R^4$, and any two $R^4$ and adjacent atoms form $C_{3-8}$ cycloalkyl, 3- to 8-membered heterocyclyl containing 1 to 2 atom(s) selected from oxygen, sulfur or nitrogen, $C_{6-10}$ aryl or 3- to 8-membered heteroaryl containing 1 to 2 atom(s) selected from oxygen, sulfur or nitrogen,

preferably, ring A is further substituted with 1 to 3 $R^4$, and any two $R^4$ and adjacent atoms form $C_{3-8}$ cycloalkyl, 3- to 8-membered heterocyclyl containing 1 to 2 atom(s) selected from oxygen or nitrogen, $C_{6-10}$ aryl or 3- to 8-membered heteroaryl containing 1 to 2 atom(s) selected from oxygen or nitrogen;

preferably,

ring A is

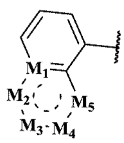,

$M_1$ is selected from -N- or -C-, $M_2$ is selected from -C(O)-, -C(S)-, -C(NH)-, - NH-, -N-, -$CH_2$-, -CH-, - O- or -S-, $M_3$ is selected from -N-, -NH-, -CH -, -$CH_2$-, -C(O)-, -C(S)- or -C(NH)-; $M_4$ is selected from -CH-, -$CH_2$- or -N-, and $M_5$ is -O-, -N-, -CH-, -$CH_2$- or absent,

preferably, $M_1$ is selected from -N- or -C-, $M_2$ is selected from -C(O)-, -C(S)-, -C(NH)-, -NH-, -N-, - $CH_2$-, -CH-, -O- or -S-, and $M_3$ is selected from -N-, -NH-, -CH-, -C(O)-, -C(S)- or -C(NH)-; $M_4$ is selected from -CH-, -$CH_2$- or -N-, and $M_5$ is -CH-, -$CH_2$- or absent,

alternatively, ring A is

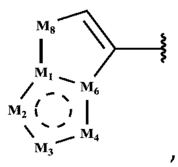,

$M_1$ is selected from -N- or -C-, $M_2$ is selected from -C(O)-, -C(S)-, -C(NH)-, - NH-, -N- or -CH-, $M_3$ is selected from -CH- or -N-, $M_4$ is selected from -NH- or -CH-, $M_6$ is selected from -NH- or -CH-, and $M_8$ is selected from -S- or -CH-,

optionally, ring A is further substituted with 1 to 2 $R^4$, and any two $R^4$ and adjacent atoms form $C_{3-6}$ cycloalkyl or 3- to 6- membered heterocyclyl containing 1 to 2 members selected from oxygen or nitrogen atoms;

more preferably,

ring A is

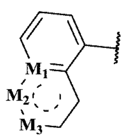,

$M_1$ is selected from -N- or -C-, $M_2$ is selected from -C(O)-, -C(S)-, -C(NH)-, - NH-, -N-, -$CH_2$-, -CH-, - O- or -S-, $M_3$ is selected from -N-, -NH-, -CH -, -C(O)-, -C(S)- or -C(NH)-,

optionally, ring A is further substituted with 1 to 2 $R^4$, and any two $R^4$ and adjacent atoms form $C_{3-6}$ cycloalkyl or 3- to 6- membered heterocyclyl containing 1 to 2 members selected from oxygen or nitrogen atoms.

In a preferred embodiment of the present invention, ring A is selected from the following groups:

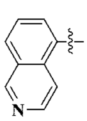, 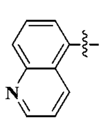, 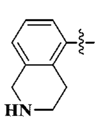, 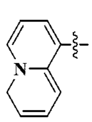, 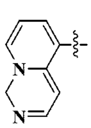, 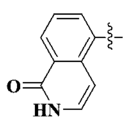, 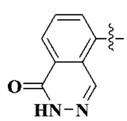, 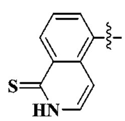, 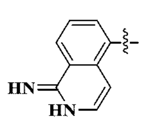,

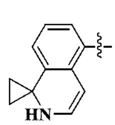, 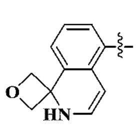, 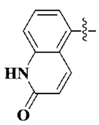, 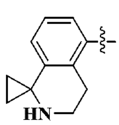, 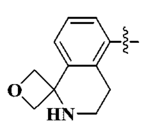, 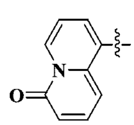, 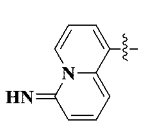, 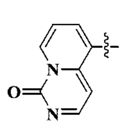,

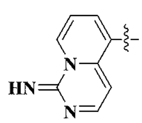, 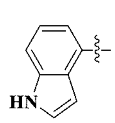, 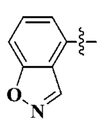, 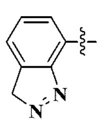, 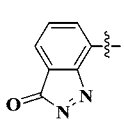, 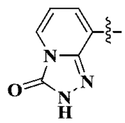, 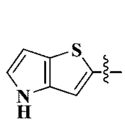, 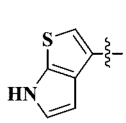,

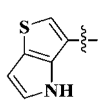, 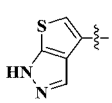, 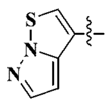, 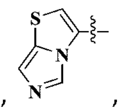, 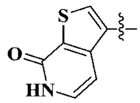, 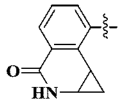, 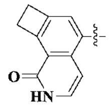, 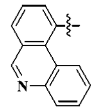, 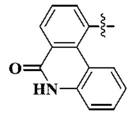,

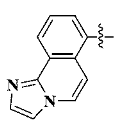, 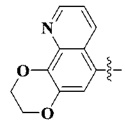, 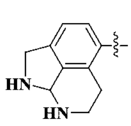, 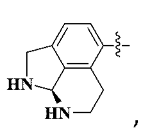, 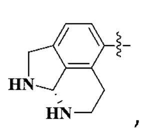, 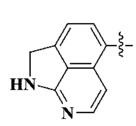, 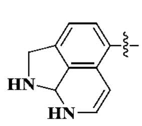 or

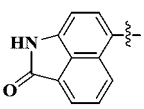.

In a preferred embodiment of the present invention, $R^2$ is selected from hydrogen, deuterium, halogen, amino, hydroxyl, cyano, nitro, $C_{1-6}$alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$alkyl, $C_{3-12}$ cycloalkyl, 3- to 12- membered heterocyclyl, $C_{6-12}$ aryl or 5- to 12-membered heteroaryl, wherein the amino, $C_{1-6}$alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$alkyl, $C_{3-12}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-6}$alkyl, $C_{2-6}$ alkenyl, $C_{2-6}$ alkynyl, oxo, thio, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1_6}$alkyl, $C_{3-12}$ cycloalkyl, 3- to 12- membered heterocyclyl, $C_{6-12}$ aryl and 5- to 12-membered heteroaryl,

preferably, selected from hydrogen, deuterium, halogen, $C_{1-3}$alkyl, $C_{1-3}$ deuteroalkyl, $C_{1-3}$ haloalkyl, $C_{1-3}$ alkoxy, $C_{1-3}$ hydroxyalkyl, 3- to 12- membered heterocyclyl or 5- to 12-membered heteroaryl, wherein the $C_{1-3}$alkyl, $C_{1-3}$ alkoxy, $C_{1-3}$ hydroxyalkyl, 3- to 12- membered heterocyclyl or 5- to 12-membered heteroaryl, which are optionally further substituted with one or more substituents selected from deuterium, halogen, amino, nitro, hydroxyl, cyano, oxo, $C_{1_3}$alkyl, $C_{1-3}$ deuteroalkyl, $C_{1-3}$ haloalkyl, $C_{1-3}$ alkoxy, $C_{1-3}$ hydroxyalkyl, $C_{1-3}$ haloalkoxy, $C_{2-6}$ alkenyl, $C_{2-6}$ alkynyl, $C_{3-8}$ cycloalkyl, 3- to 12- membered heterocyclyl, $C_{6-12}$ aryl and 5- to 12-membered heteroaryl; and further preferably, hydrogen, deuterium, fluorine, chlorine, bromine, methyl, ethyl, trifluoromethyl or cyclopropyl.

In a preferred embodiment of the present invention, $R^3$ is selected from hydrogen, deuterium, halogen, amino, hydroxyl, cyano, nitro, $C_{1-6}$alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{l_6}$ hydroxyalkyl, cyano-substituted $C_{1-6}$alkyl, $C_{3-12}$ cycloalkyl, 3- to 12- membered heterocyclyl, $C_{6-12}$ aryl or 5- to 12-membered heteroaryl, wherein the amino, $C_{1-6}$alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$alkyl, $C_{3-12}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-6}$alkyl, $C_{2-6}$ alkenyl, $C_{2-6}$ alkynyl, oxo, thio, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$alkyl, $C_{3-12}$ cycloalkyl, 3- to 12- membered heterocyclyl, $C_{6-12}$ aryl and 5- to 12-membered heteroaryl,

preferably, selected from hydrogen, deuterium, halogen, $C_{1-3}$alkyl, $C_{1-3}$ deuteroalkyl, $C_{1-3}$ haloalkyl, $C_{1-3}$ alkoxy, $C_{1-3}$ hydroxyalkyl, 3- to 12- membered heterocyclyl or 5- to 12-membered heteroaryl, wherein the $C_{1-3}$alkyl, $C_{1-3}$ alkoxy, $C_{1-3}$ hydroxyalkyl, 3- to 12- membered heterocyclyl or 5- to 12-membered heteroaryl, which are optionally further substituted with one or more substituents selected from deuterium, halogen, amino, nitro, hydroxyl, cyano,

oxo, $C_{1-3}$alkyl, $C_{1-3}$ deuteroalkyl, $C_{1-3}$ haloalkyl, $C_{1-3}$ alkoxy, $C_{1-3}$ hydroxyalkyl, $C_{1-3}$ haloalkoxy, $C_{2-6}$ alkenyl, $C_{2-6}$ alkynyl, $C_{3-8}$ cycloalkyl, 3- to 12- membered heterocyclyl, $C_{6-12}$ aryl and 5- to 12-membered heteroaryl; and further preferably, hydrogen, deuterium, fluorine, chlorine, bromine, methyl, ethyl or trifluoromethyl.

In a preferred embodiment of the present invention, the compound, the stereoisomer or pharmaceutically acceptable salt thereof is characterized that, the compound is further as represented by general formula (I-A):

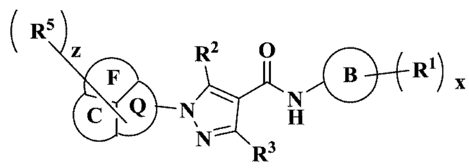

(I-A)

wherein:

ring B is selected from $C_{3-12}$ cycloalkyl, 3- to 14-membered heterocyclyl, $C_{6-14}$ aryl or 5- to 14-membered heteroaryl;

preferably, ring B is selected from $C_{6-12}$ aryl or 5- to 14- membered heteroaryl containing 1 to 3 nitrogen atoms, oxygen atoms and/or sulfur atoms,

more preferably, ring B is selected from phenyl, 5- to 7-membered nitrogen-containing heteroaryl, benzo 5- to 7-membered nitrogen-containing heteroaryl, 5- to 7-membered nitrogen-containing heteroaryl fused phenyl, 5- to 7-membered heteroaryl fused 5- to 7-membered heteroaryl or tricyclic heteroaryl,

more preferably, ring B is selected from phenyl, pyridyl or pyridazinyl;

ring C is selected from $C_{3-6}$ cycloalkyl, 3- to 8-membered heterocyclyl or 5- to 8-membered heteroaryl,

preferably $C_{5-6}$ cycloalkyl or 5- to 6-membered heterocyclyl,

more preferably, cyclopentyl, cyclohexyl, 5-membered heterocyclyl containing 1 to 3 nitrogen atoms, oxygen atoms and/or sulfur atoms, 6-membered heterocyclyl containing 1 to 3 nitrogen atoms, oxygen atoms and/or sulfur atoms;

ring F is selected from $C_{3-6}$ cycloalkyl, 3- to 8-membered heterocyclyl, $C_{6-12}$ aryl or 5- to 12-membered heteroaryl,

preferably $C_{5-6}$ cycloalkyl, 5- to 6-membered heterocyclyl, $C_{6-8}$aryl or 5- to 8-membered heteroaryl;

ring Q is selected from $C_{3-6}$ cycloalkyl, 3- to 8-membered heterocyclyl, $C_{6-12}$ aryl or 5- to 12-membered heteroaryl,

preferably $C_{5-6}$ cycloalkyl, 5- to 6-membered heterocyclyl, $C_{6-8}$aryl or 5- to 8-membered heteroaryl;

$R^1$ is independently selected from hydrogen, deuterium, halogen, amino, hydroxyl, cyano, nitro, $C_{l-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{l-6}$ haloalkoxy, $C_{1-6}$ hydroxyalkyl, cyano-substituted $C_{1-6}$alkyl, $C_{3-12}$ cycloalkyl, 3- to 12- membered heterocyclyl, $C_{6-12}$ aryl, 5- to 12-membered heteroaryl, $-CR_{cc}=CR_{dd}(CH_2)_nR_{aa}$, $-CR_{cc}=CR_{dd}(CH_2)_nNR_{aa}R_{bb}$, $-CR_{cc}=CR_{dd}(CH_2)_nNR_{ee}C(O)R_{aa}$, $-CR_{cc}=CR_{dd}(CH_2)_nNR_{ee}C(O)NR_{aa}R_{bb}$, $-O(CH_2)_nR_{cc}$, $-OC(R_{aa}R_{bb})_n(CH_2)_mR_{cc}$, $-NR_{ee}(CH_2)_nR_{cc}$, $-(CH_2)_n-$, $-(CH_2)_nR_{cc}$, $-(CH_2)_nOR_{cc}$, $-(CH_2)_nSR_{cc}$, $-(CH_2)_nC(O)R_{cc}$, $-(CH_2)_nC(=NR_{ee})R_{cc}$, $-(CH_2)_nC(O)OR_{cc}$, $-(CH_2)_nS(O)_mR_{cc}$, $-(CH_2)_nNR_{aa}R_{bb}$, $-(CH_2)_nC(O)NR_{aa}R_{bb}$, $-(CH_2)_nNR_{ee}C(O)R_{cc}$, $-(CH_2)_nN=S(O)R_{cc}R_{ee}$ or $-(CH_2)_nNR_{ee}S(O)_mR_{cc}$, wherein the amino, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$alkyl, $C_{3-12}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-6}$ alkyl, $C_{2-6}$ alkenyl, $C_{2-6}$ alkynyl, oxo, thio, $C_{1-6}$ deuteroalkyl, $C_{1-6}$ haloalkyl, $C_{1-6}$ alkoxy, $C_{l-6}$ haloalkoxy, $C_{1-6}$ hydroxyalkyl, cyano-substituted $C_{1-6}$alkyl, $C_{3-12}$ cycloalkyl, 3- to 12- membered heterocyclyl, $C_{6-12}$ aryl and 5- to 12-membered heteroaryl;

$R^2$ is selected from hydrogen, deuterium, halogen, amino, hydroxyl, cyano, nitro, $C_{1-6}$alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, $C_{3-8}$ cycloalkyl, 3- to 14-membered heterocyclyl, $C_{6-14}$ aryl or 5- to 14-membered heteroaryl;

$R^3$ is selected from hydrogen, deuterium, halogen, amino, hydroxyl, cyano, nitro, $C_{1-6}$alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, $C_{3-8}$ cycloalkyl, 3- to 14-membered heterocyclyl, $C_{6-14}$ aryl or 5- to 14-membered heteroaryl;

$R^5$ is selected from hydrogen, deuterium, halogen, amino, hydroxyl, cyano, nitro, $C_{1-6}$ alkyl, $C_{2-6}$ alkenyl, $C_{2-6}$ alkynyl, oxo, thio, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-8}$ cycloalkyl, 3- to 14-membered heterocyclyl, $C_{6-14}$ aryl, 5- to 14-membered heteroaryl, $-(CH_2)_{n2}C(O)R_a$, $-(CH_2)_{n2}C(O)OR_a$, $-(CH_2)_{n2}OR_a$, $-(CH_2)_{n2}R_a$ or - $(CH_2)_{n2}S(O)_{m2}R_a$, wherein the amino, $C_{1-6}$alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$alkyl, $C_{3-8}$ cycloalkyl, 3- to 14-membered heterocyclyl, $C_{6-14}$ aryl and 5- to 14-membered heteroaryl are optionally substituted with one or more substituents selected from deuterium, halogen, amino, hydroxyl, cyano, nitro, $C_{1-6}$ alkyl, $C_{2-6}$ alkenyl, $C_{2-6}$ alkynyl, oxo, thio, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$alkyl, $C_{3-8}$ cycloalkyl, 3- to 14-membered heterocyclyl, $C_{6-14}$ aryl and 5- to 14-membered heteroaryl,

preferably, $R^5$ is selected from hydrogen, deuterium, halogen, amino, hydroxyl, cyano, nitro, $C_{1-3}$ alkyl, $C_{2-4}$ alkenyl, $C_{2-4}$ alkynyl, oxo, thio, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl, 5- to 12-membered heteroaryl, $-(CH_2)_{n2}C(O)R_a$, $-(CH_2)_{n2}C(O)OR_a$, - $(CH_2)_{n2}OR_a$, $-(CH_2)_{n2}R_a$ or $-(CH_2)_{n2}S(O)_{m2}R_a$, wherein the amino, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-3}$ alkyl, $C_{2-4}$ alkenyl, $C_{2-4}$ alkynyl, oxo, thio, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl and 5- to 12-membered heteroaryl;

$R_a$ is selected from hydrogen, deuterium, halogen, amino, hydroxyl, cyano, nitro, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl or 5- to 12-membered heteroaryl, wherein the amino, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-3}$ alkyl, $C_{2-4}$ alkenyl, $C_{2-4}$ alkynyl, oxo, thio, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl and 5- to 12-membered heteroaryl;

$R_{aa}$ and $R_{bb}$ are each independently selected from hydrogen, deuterium, halogen, amino, hydroxyl, cyano, nitro, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12-membered heterocyclyl, $C_{6-12}$ aryl or 5- to 12-membered heteroaryl, which are optionally substituted with one or more substituents selected from deuterium, halogen, amino, hydroxyl, cyano, nitro, $C_{1-6}$ alkyl, $C_{3-12}$ cycloalkyl, $-O(CH_2)_{n1}R_{c1}$, $-OC(R_{a1}R_{b1})_{m1}(CH_2)_{n1}R_{c1}$, $-NR_{a1}(CH_2)_{n1}R_{c1}$, $-(CH_2)_{n1}-$, $-(CH_2)_{n1}R_{c1}$, $-(CH_2)_{n1}OR_{c1}$, $-(CH_2)_{n1}SR_{c1}$, $-(CH_2)_{n1}C(O)R_{c1}$, $-(CH_2)_{n1}C(O)OR_{c1}$, $-(CH_2)_{n1}S(O)_{m1}R_{c1}$, $-(CH_2)_{n1}NR_{a1}R_{b1}$, $-(CH_2)_{n1}C(O)NR_{a1}R_{b1}$, $-(CH_2)_{n1}NR_{a1}C(O)R_{c1}$ and $-(CH_2)_{n1}NR_{a1}S(O)_{m1}R_{c1}$,

alternatively, $R_{aa}$ and $R_{bb}$ together with adjacent atoms form $C_{3-12}$ cycloalkyl, 3- to 12- membered heterocyclyl, $C_{6-12}$ aryl or 5- to 12-membered heteroaryl, wherein the $C_{3-12}$ cycloalkyl, 3- to 12-membered heterocyclyl, $C_{6-12}$ aryl and 5- to 12-membered heteroaryl are optionally substituted with one or more substituents selected from deuterium, halogen, amino, hydroxyl, cyano, nitro, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12- membered heterocyclyl, $C_{6-12}$ aryl, 5- to 12-membered heteroaryl, $-O(CH_2)_{n1}R_{c1}$, $-OC(R_{a1}R_{b1})_{n1}(CH_2)_{m1}R_{c1}$, $-NR_{a1}(CH_2)_{n1}R_{c1}$, $-(CH_2)_{n1}-$, $-(CH_2)_{n1}R_{c1}$, $-(CH_2)_{n1}OR_{c1}$, $-(CH_2)_{n1}SR_{c1}$, $-(CH_2)_{n1}C(O)R_{c1}$, $-(CH_2)_{n1}C(O)OR_{c1}$, $-(CH_2)_{n1}S(O)_mR_{c1}$, $-(CH_2)_{n1}NR_{a1}R_{b1}$ $-(CH_2)_{n1}C(O)NR_{a1}R_{b1}$, $-(CH_2)_{n1}NR_{a1}C(O)R_{c1}$ and $-(CH_2)_{n1}NR_{a1}S(O)_{m1}R_{c1}$;

$R_{cc}$ is selected from hydrogen, deuterium, halogen, amino, hydroxyl, cyano, nitro, $C_{1-6}$ alkyl, $C_{2-6}$ alkenyl, $C_{2-6}$

alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12- membered heterocyclyl, $C_{6-12}$ aryl or 5- to 12-membered heteroaryl, which are optionally substituted with one or more substituents selected from deuterium, halogen, amino, hydroxyl, cyano, nitro, $C_{1-6}$ alkyl, $C_{3-12}$ cycloalkyl, - $O(CH_2)_{n1}R_{c1}$, $-OC(R_{a1}R_{b1})_{n1}(CH_2)_mR_{c1}$, $-NR_{a1}(CH_2)_{n1}R_{c1}$, $-(CH_2)_{n1}-$, $-(CH_2)_{n1}R_{c1}$, $-(CH_2)_{n1}OR_{c1}$, - $(CH_2)_{n1}5R_{c1}$, $-(CH_2)_{n1}C(O)R_{c1}$, $-(CH_2)_{n1}C(O)OR_{c1}$, $-(CH_2)_{n1}S(O)_mR_{c1}$, $-(CH_2)_{n1}NR_{a1}R_{b1}$, - $(CH_2)_{n1}C(O)NR_{a1}R_{b1}$, $-(CH_2)_{n1}NR_{a1}C(O)R_{c1}$ and $-(CH_2)_{n1}NR_{a1}5(0)_{m1}R_{c1}$;

$R_{a1}$, $R_{b1}$ and $R_{c1}$ are each independently selected from hydrogen, deuterium, halogen, amino, hydroxyl, cyano, nitro, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12-membered heterocyclyl, $C_{6-12}$ aryl or 5- to 12-membered heteroaryl, which are optionally substituted with one or more substituents selected from deuterium, halogen, amino, hydroxyl, cyano, nitro, $C_{1-6}$ alkyl and $C_{3-12}$ cycloalkyl;

m is 0, 1, 2 or 3;

m1 is 0, 1, 2 or 3;

m2 is selected from 1 or 2;

n is 0, 1, 2 or 3;

n1 is 0, 1, 2 or 3;

n2 is selected from 0, 1, 2 or 3;

x is selected from 0, 1, 2, 3 or 4; and

z is selected from 0, 1, 2 or 3.

In a preferred embodiment of the present invention, the compound, the stereoisomer or pharmaceutically acceptable salt thereof is characterized that, the compound is further as represented by general formula (III):

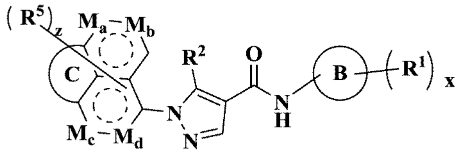

(III)

wherein:

$M_a$, $M_b$, $M_c$ or $M_d$ are each independently selected from -N-, -NH- or -CH-;

ring C is selected from phenyl, $C_{3-6}$ cycloalkyl, 5- to 7-membered heterocyclyl or 5- to 7-membered heteroaryl;

preferably, 5-membered heterocyclyl containing 1 to 3 nitrogen atoms and/or 1 to 2 oxygen atoms, 6-membered heterocyclyl containing 1 to 3 nitrogen atoms and/or 1 to 2 oxygen atoms, 5-membered heteroaryl containing 1 to 3 nitrogen atoms or 6-membered heteroaryl containing 1 to 3 nitrogen atoms;

$R^2$ is selected from hydrogen, deuterium, halogen, amino, hydroxyl, cyano, nitro, $C_{1-3}$alkyl, $C_{2-6}$ alkenyl, $C_{2-6}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, $C_{3-6}$ cycloalkyl, 3- to 12- membered heterocyclyl, $C_{6-12}$ aryl or 5- to 12-membered heteroaryl;

$R^5$ is selected from hydrogen, deuterium, halogen, amino, hydroxyl, cyano, nitro, $C_{1-3}$ alkyl, $C_{2-6}$ alkenyl, $C_{2-6}$ alkynyl, oxo, thio, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl or 5- to 12-membered heteroaryl, wherein the amino, $C_{1-3}$ alkyl, $C_{2-6}$ alkenyl, $C_{2-6}$ alkynyl, $C_{1-3}$ deuteroalkyl, $C_{1-3}$ haloalkyl, $C_{1-3}$ alkoxy, $C_{1-3}$ haloalkoxy, $C_{1-3}$

hydroxyalkyl, cyano-substituted $C_{1-3}$alkyl, $C_{3-6}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-3}$ alkyl, $C_{2-6}$ alkenyl, $C_{2-6}$ alkynyl, oxo, thio, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl and 5- to 12-membered heteroaryl;

$R^5$ is preferably selected from hydrogen, deuterium, fluorine, chlorine, bromine, amino, hydroxyl, cyano, nitro, methyl, ethyl, propyl, isopropyl, oxo, methoxy, ethoxy, difluoromethyl, trifluoromethyl, difluoromethoxy, trifluoromethoxy, cyclopropyl or cyclobutyl;

z is selected from 0, 1, 2 or 3.

In a preferred embodiment of the present invention, ring B is selected from $C_{3-12}$ cycloalkyl, 3- to 14-membered heterocyclyl, $C_{6-14}$ aryl or 5- to 14-membered heteroaryl;

preferably, ring B is selected from $C_{6-12}$ aryl or 5- to 14-membered heteroaryl containing 1 to 3 nitrogen atoms, 1 oxygen atom and/or 1 sulfur atom;

more preferably, ring B is selected from phenyl, 5- to 7-membered nitrogen-containing heteroaryl, benzo 5- to 7-membered nitrogen-containing heteroaryl, 5- to 7-membered nitrogen-containing heteroaryl fused phenyl, 5- to 7-membered heteroaryl fused 5- to 7-membered heteroaryl or tricyclic heteroaryl;

more preferably, ring B is selected from phenyl, pyridyl or pyridazinyl.

In a preferred embodiment of the present invention, the compound, the stereoisomer or pharmaceutically acceptable salt thereof is characterized that, the compound is further as represented by general formula (IV) or (V):

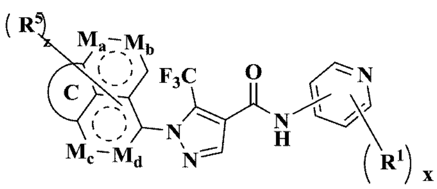

(IV)

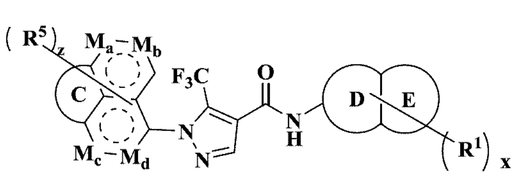

(V)

ring D is selected from phenyl, 5-membered heteroaryl or 6-membered heteroaryl;

preferably, phenyl, 5-membered heteroaryl containing 1 to 3 nitrogen atoms or 6-membered heteroaryl containing 1 to 3 nitrogen atoms;

more preferably, phenyl, pyridyl, pyrimidinyl or pyridazinyl;

ring E is selected from phenyl, $C_{3-6}$ cycloalkyl, 5- to 7-membered heterocyclyl or 5- to 7-membered heteroaryl;

preferably, phenyl, $C_{5-6}$ cycloalkyl, 5-membered heterocyclyl containing 1 to 3 nitrogen atoms and/or 1 to 2 oxygen atoms, 6-membered heterocyclyl containing 1 to 3 nitrogen atoms and/or 1 to 2 oxygen atoms, 5-membered heteroaryl containing 1 to 3 nitrogen atoms or 6-membered heteroaryl containing 1 to 3 nitrogen atoms;

and more preferably, phenyl, cyclopentyl, cyclohexyl, pyrrolyl, pyrazolyl, imidazolyl, triazolyl, pyridyl, pyrimidinyl, pyridazinyl or pyrazinyl.

In a preferred embodiment of the present invention, the compound, the stereoisomer or pharmaceutically acceptable salt thereof is further represented by general formula (IV-A) or (V-A):

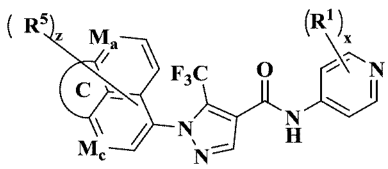

( IV-A )

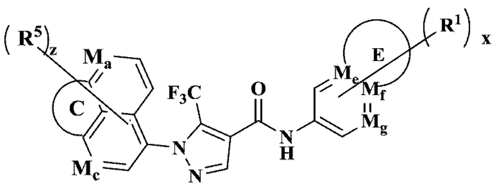

( V-A )

wherein:

$M_a$, $M_c$, $M_e$, $M_f$ or $M_g$ are each independently selected from -N- or -CH-.

In a preferred embodiment of the present invention, the compound, the stereoisomer or pharmaceutically acceptable salt thereof is characterized that, the compound is further as represented by general formula (IV-B):

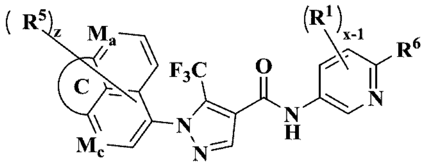

( IV-B )

wherein:

$M_a$ is selected from -N- or -CH-;

$M_c$ is selected from -N- or -CH-;

$R_6$ is selected from 3- to 7-membered heterocyclyl or 5- to 7-membered heteroaryl, wherein the 3- to 7-membered heterocyclyl and 5- to 7-membered heteroaryl are optionally further substituted with one or more substituents selected from deuterium, halogen, amino, hydroxyl, cyano, nitro, $C_{1-6}$ alkyl, $C_{2-6}$ alkenyl, $C_{2-6}$ alkynyl, oxo, thio, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12-membered heterocyclyl, $C_{6-12}$ aryl and 5- to 12-membered heteroaryl,

preferably, $R_6$ is selected from 4-membered heterocyclyl, 5-membered heterocyclyl, 6-membered heterocyclyl or 5-membered heteroaryl, wherein the 4-membered heterocyclyl, 5-membered heterocyclyl, 6-membered heterocyclyl and 5-membered heteroaryl are optionally further substituted with one or more substituents selected from deuterium, halogen, amino, hydroxyl, cyano, nitro, $C_{1-3}$ alkyl, $C_{2-4}$ alkenyl, $C_{2-4}$ alkynyl, oxo, thio, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 8-membered heterocyclyl, $C_{6-10}$ aryl and 5- to 8-membered heteroaryl,

and more preferably, $R_6$ is selected from

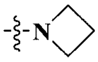 , 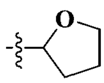 , 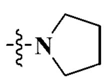 , 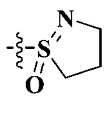 , 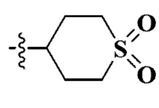 ,

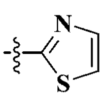 , 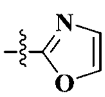 , 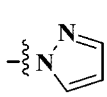 , 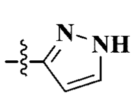 , 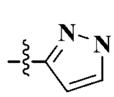 , 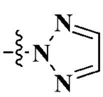 , 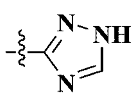 , 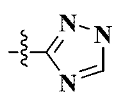 ,

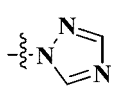 or 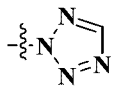,

optionally further substituted with one or more substituents selected from deuterium, fluorine, chlorine, bromine, amino, hydroxyl, cyano, nitro, methyl, ethyl, oxo, thio, difluoromethyl, trifluoromethyl, methoxy, ethoxy and trifluoromethoxy.

In a preferred embodiment of the present invention, the compound, the stereoisomer or pharmaceutically acceptable salt thereof is further represented by general formula (IV-C):

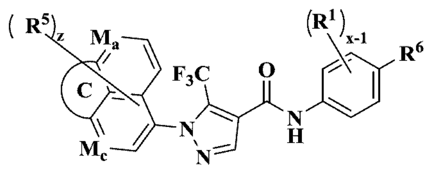

( IV-C )

wherein:

$M_a$ is selected from -N- or -CH-;

$M_c$ is selected from -N- or -CH-;

$R_6$ is selected from 3- to 7-membered heterocyclyl or 5- to 7-membered heteroaryl, wherein the 3- to 7-membered heterocyclyl and 5- to 7-membered heteroaryl are optionally further substituted with one or more substituents selected from deuterium, halogen, amino, hydroxyl, cyano, nitro, $C_{1-6}$ alkyl, $C_{2-6}$ alkenyl, $C_{2-6}$ alkynyl, oxo, thio, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12-membered heterocyclyl, $C_{6-12}$ aryl and 5- to 12-membered heteroaryl,

preferably, $R_6$ is selected from 4-membered heterocyclyl, 5-membered heterocyclyl, 6-membered heterocyclyl or 5-membered heteroaryl, wherein the 4-membered heterocyclyl, 5-membered heterocyclyl, 6-membered heterocyclyl and 5-membered heteroaryl are optionally further substituted with one or more substituents selected from deuterium, halogen, amino, hydroxyl, cyano, nitro, $C_{1-3}$ alkyl, $C_{2-4}$ alkenyl, $C_{2-4}$ alkynyl, oxo, thio, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 8-membered heterocyclyl, $C_{6-10}$ aryl and 5- to 8-membered heteroaryl,

and more preferably, $R_6$ is selected from

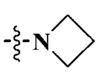, 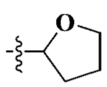, 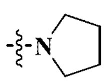, 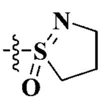, 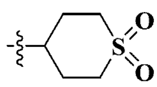,

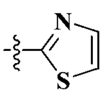, 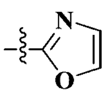, 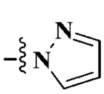, 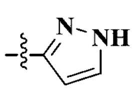, 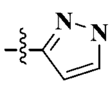, 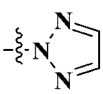, 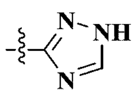, 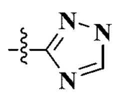,

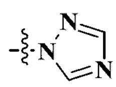 or 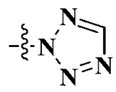,

optionally further substituted with one or more substituents selected from deuterium, fluorine, chlorine, bromine,

amino, hydroxyl, cyano, nitro, methyl, ethyl, oxo, thio, difluoromethyl, trifluoromethyl, methoxy, ethoxy and trifluoromethoxy.

In a preferred embodiment of the present invention,

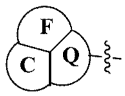

is selected from

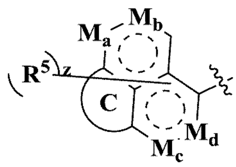

,

the $M_a$, $M_b$, $M_c$, $M_d$, $R^5$, ring C and z are as defined in any of the preceding embodiments;

preferably is selected from

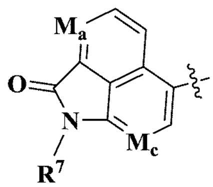

,

$R^7$ is selected from hydrogen, deuterium, halogen, amino, hydroxyl, cyano, nitro, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1_3}$alkyl, $C_{3-6}$ cycloalkyl, 3- to 12- membered heterocyclyl, $C_{6-12}$ aryl, 5- to 12-membered heteroaryl, $-(CH_2)_{n2}C(O)R_a$, $-(CH_2)_{n2}C(O)OR_a$, $-(CH_2)_{n2}OR_a$, $-(CH_2)_{n2}R_a$ or - $(CH_2)_{n2}S(O)_{m2}R_a$, wherein the amino, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$alkyl, $C_{3-6}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-3}$ alkyl, $C_{2-4}$ alkenyl, $C_{2-4}$ alkynyl, oxo, thio, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl and 5- to 12-membered heteroaryl;

$R_a$ is selected from hydrogen, deuterium, halogen, amino, hydroxyl, cyano, nitro, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl or 5- to 12-membered heteroaryl, wherein the amino, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-3}$ alkyl, $C_{2-4}$ alkenyl, $C_{2-4}$ alkynyl, oxo, thio, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl and 5- to 12-membered heteroaryl;

n2 is selected from 0, 1, 2 or 3;

m2 is selected from 1 or 2;

the $M_a$ and $M_c$ are as defined in any of the preceding embodiments.

In a preferred embodiment of the present invention,

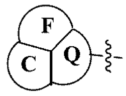

is selected from

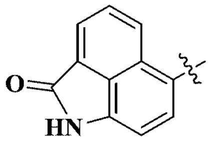 , 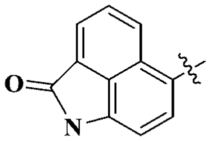 , 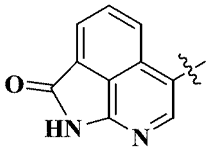 , 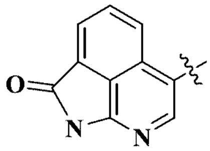 , 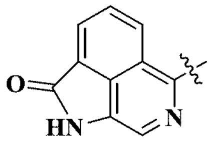 ,

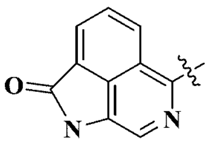 , 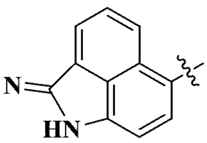 , 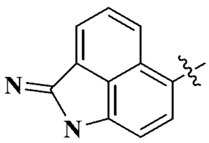 , 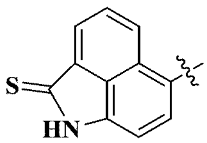 , 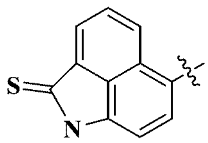 ,

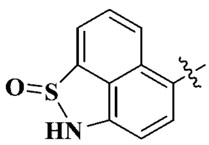 , 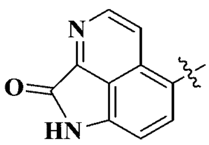 , 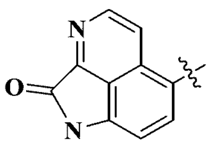 , 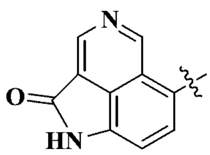 , 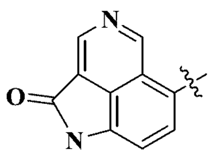 ,

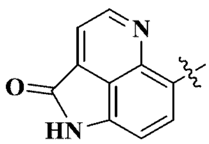 , 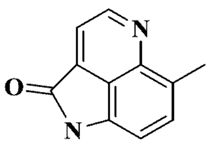 , 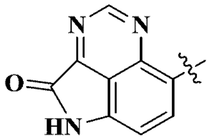 , 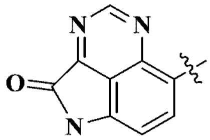 , 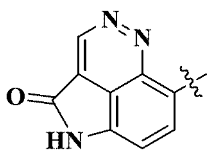 ,

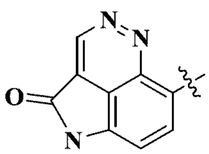 , 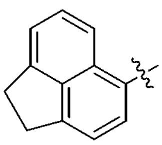 , 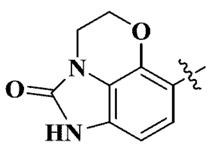 , 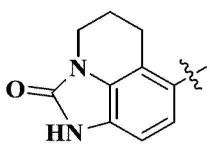 , 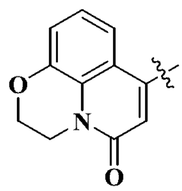 ,

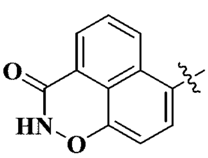 , 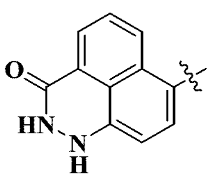 , 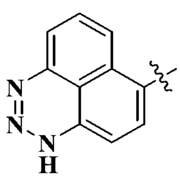 , 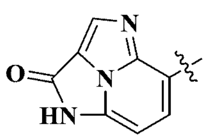 , 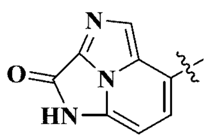 ,

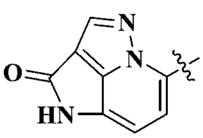 , 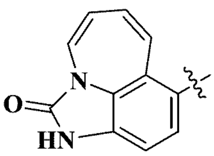 , 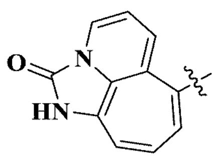 , 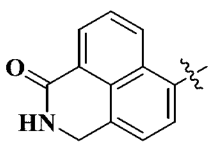 , 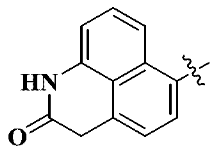

, 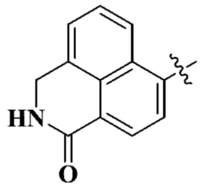 , 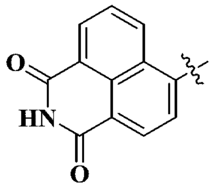 , 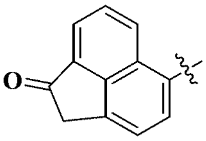 , 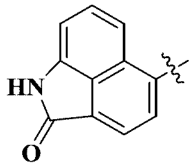 , 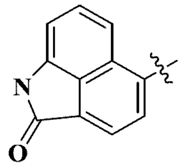

or

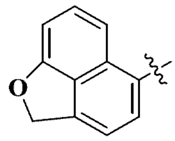 .

In a preferred embodiment of the present invention, ring C is selected from

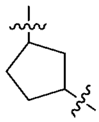 , 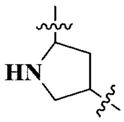

, 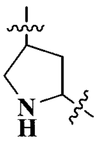 , 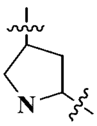 , 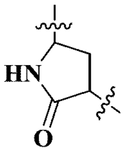 , 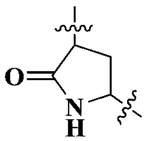 , 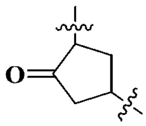 , 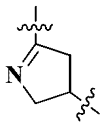 , 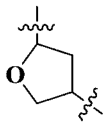 , 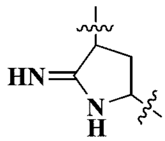 ,

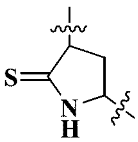 , 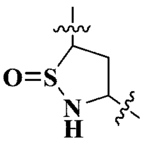 , 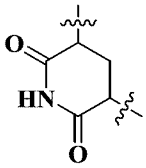 , 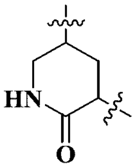 , 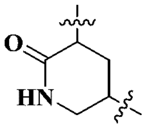 , 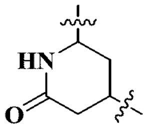 , 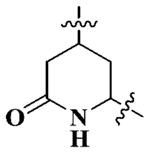 ,

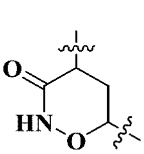 , 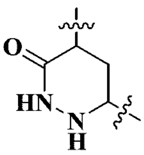 or 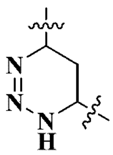 ,

and preferably, ring C is selected from

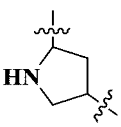 , 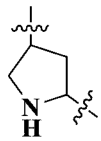 , 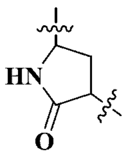 , 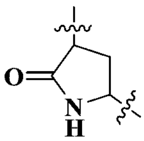 ,

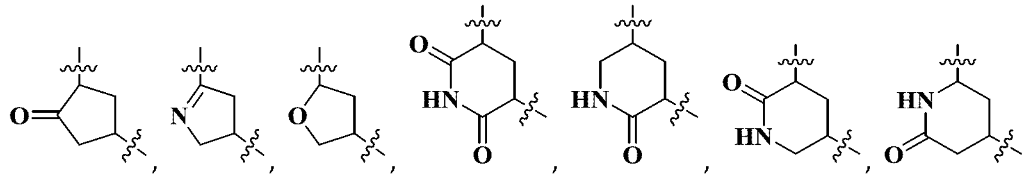

or

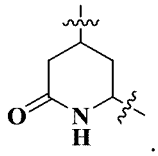

In a preferred embodiment of the present invention, the compound, the stereoisomer or pharmaceutically acceptable salt thereof is further represented by general formula (VI):

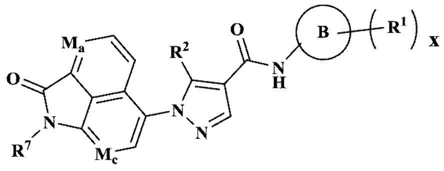

(VI)

$R^7$ is selected from hydrogen, deuterium, halogen, amino, hydroxyl, cyano, nitro, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$alkyl, $C_{3-6}$ cycloalkyl, 3- to 12- membered heterocyclyl, $C_{6-12}$ aryl, 5- to 12-membered heteroaryl, $-(CH_2)_{n2}C(O)R_a$, $-(CH_2)_{n2}C(O)OR_a$, $-(CH_2)_{n2}OR_a$, $-(CH_2)_{n2}R_a$ or $-(CH_2)_{n2}S(O)_{m2}R_a$, wherein the amino, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$alkyl, $C_{3-6}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-3}$ alkyl, $C_{2-4}$ alkenyl, $C_{2-4}$ alkynyl, oxo, thio, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl and 5- to 12-membered heteroaryl;

$R^7$is preferably selected from hydrogen, deuterium, methyl, ethyl, propyl, isopropyl, methoxy, ethoxy, difluoromethyl, trifluoromethyl, difluoromethoxy, trifluoromethoxy, cyclopropyl, cyclobutyl, benzyl or p-methoxybenzyl;

$R_a$ is selected from hydrogen, deuterium, halogen, amino, hydroxyl, cyano, nitro, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl or 5- to 12-membered heteroaryl, wherein the amino, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-3}$ alkyl, $C_{2-4}$ alkenyl, $C_{2-4}$ alkynyl, oxo, thio, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl and 5- to 12-membered heteroaryl;

n2 is selected from 0, 1, 2 or 3;

and m2 is selected from 1 or 2.

In a preferred embodiment of the present invention, the compound, the stereoisomer or pharmaceutically acceptable salt thereof is further represented by general formula (VI-A), (VI-B), (VI-C), (VI-D), (VI-E), (VI-F) or (VI-G):

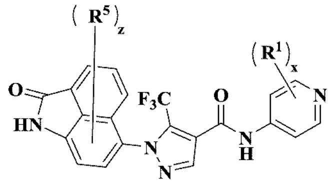

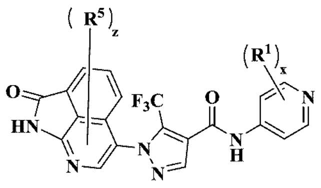

( VI-A ) ( VI-B )

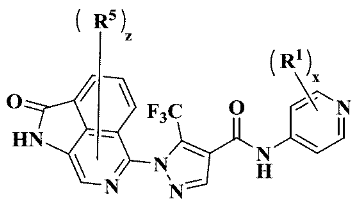

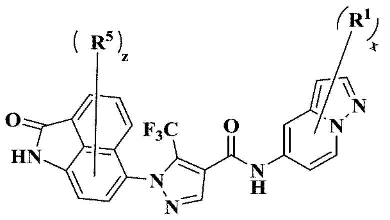

( VI-C ) ( VI-D )

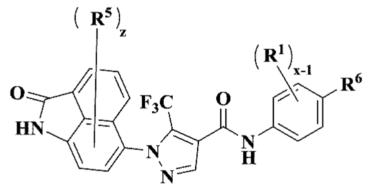

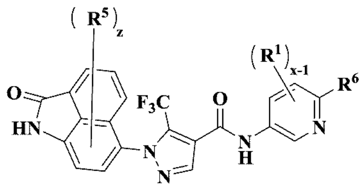

( VI-E ) ( VI-F )

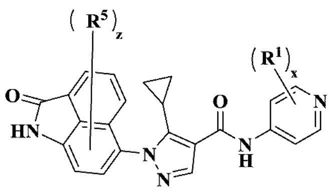

( VI-G ) .

In a preferred embodiment of the present invention, $R^1$ is independently selected from

hydrogen, deuterium, halogen, amino, hydroxyl, cyano, nitro, $C_{1-6}$alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$alkyl, $C_{3-12}$ cycloalkyl, 3- to 12- membered heterocyclyl, $C_{6-12}$ aryl, 5- to 12-membered heteroaryl, $-CR_{cc}=CR_{dd}(CH_2)_nR_{aa}$, $-CR_{cc}=CR_{dd}(CH_2)_nNR_{aa}R_{bb}$, $-CR_{cc}=CR_{dd}(CH_2)_nNR_{ee}C(O)R_{aa}$, $-CR_{cc}=CR_{dd}(CH_2)_nNR_{ee}C(O)NR_{aa}R_{bb}$, $-O(CH_2)_nR_{cc}$, $-OC(R_{aa}R_{bb})_n(CH_2)_mR_{cc}$, $-NR_{ee}(CH_2)_nR_{cc}$, $-(CH_2)_n-$, $-(CH_2)_nR_{cc}$, $-(CH_2)_nOR_{cc}$, $-(CH_2)_nSR_{cc}$, $-(CH_2)_nC(O)R_{cc}$, $-(CH_2)_nC(=NR_{ee})R_{cc}$, $-(CH_2)_nC(O)OR_{cc}$, $-(CH_2)_nS(O)_mR_{cc}$, $-(CH_2)_nNR_{aa}R_{bb}$, $-(CH_2)_nC(O)NR_{aa}R_{bb}$, $-(CH_2)_nNR_{ee}C(O)R_{cc}$, $-(CH_2)_nN=S(O)R_{cc}R_{ee}$ or $-(CH_2)_nNR_{ee}S(O)_mR_{cc}$, wherein the amino, $C_{1-6}$alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$alkyl, $C_{3-12}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-6}$alkyl, $C_{2-6}$ alkenyl, $C_{2-6}$ alkynyl, oxo, thio, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$alkyl, $C_{3-12}$ cycloalkyl, 3- to 12- membered heterocyclyl, $C_{6-12}$ aryl and 5- to 12-membered heteroaryl, $R^1$ is independently preferably selected from hydrogen, deuterium, halogen, amino, hydroxyl, cyano, nitro, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12- membered heterocyclyl, $C_{6-12}$ aryl, 5- to 12-membered heteroaryl, $-CR_{cc}=CR_{dd}(CH_2)_nR_{aa}$, $-CR_{cc}=CR_{dd}(CH_2)_nNR_{aa}R_{bb}$, $-CR_{cc}=CR_{dd}(CH_2)_nNR_{ee}C(O)R_{aa}$, $-CR_{cc}=CR_{dd}(CH_2)_nNR_{ee}C(O)NR_{aa}R_{bb}$, $-O(CH_2)_nR_{cc}$, $-OC(R_{aa}R_{bb})_n(CH_2)_mR_{cc}$, $-NR_{ee}(CH_2)_nR_{cc}$, $-(CH_2)_n-$, $-(CH_2)_nR_{cc}$, $-(CH_2)_nOR_{cc}$, $-(CH_2)_nSR_{cc}$, $-(CH_2)_nC(O)R_{cc}$, $-(CH_2)_nC(=NR_{ee})R_{cc}$, $-(CH_2)_nC(O)OR_{cc}$, $-(CH_2)_nS(O)_mR_{cc}$, $-(CH_2)_nNR_{aa}R_{bb}$, $-(CH_2)_nC(O)NR_{aa}R_{bb}$, $-(CH_2)_nNR_{ee}C(O)R_{cc}$ or $-(CH_2)_nN-$

$R_{ee}S(O)_mR_{cc}$, wherein the amino, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-6}$ alkyl, $C_{2-6}$ alkenyl, $C_{2-6}$ alkynyl, oxo, thio, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12- membered heterocyclyl, $C_{6-12}$ aryl and 5- to 12-membered heteroaryl,

more preferably, $R^1$ is selected from hydrogen, deuterium, halogen, amino, hydroxyl, cyano, nitro, $C_{1-3}$ alkyl, $C_{2-6}$ alkenyl, $C_{2-6}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl, 5- to 12-membered heteroaryl, $-O(CH_2)_nR_{cc}$, $-OC(R_{aa}R_{bb})_n(CH_2)_mR_{cc}$, $-(CH_2)_n-$, $-(CH_2)_nR_{cc}$ or $-(CH_2)_nOR_{cc}$, wherein the amino, $C_{1-3}$ alkyl, $C_{2-6}$ alkenyl, $C_{2-6}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-3}$ alkyl, $C_{2-6}$ alkenyl, $C_{2-6}$ alkynyl, oxo, thio, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl and 5- to 12-membered heteroaryl;

$R_{aa}$ and $R_{bb}$ are each independently selected from hydrogen, deuterium, halogen, amino, hydroxyl, cyano, nitro, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12-membered heterocyclyl, $C_{6-12}$ aryl or 5- to 12-membered heteroaryl, which are optionally substituted with one or more substituents selected from deuterium, halogen, amino, hydroxyl, cyano, nitro, $C_{1-6}$ alkyl, $C_{3-12}$ cycloalkyl, $-O(CH_2)_{n1}R_{c1}$, $-OC(R_{a1}R_{b1})_{m1}(CH_2)_{n1}R_{c1}$, $-NR_{a1}(CH_2)_{n1}R_{c1}$, $-(CH_2)_{n1}-$, $-(CH_2)_{n1}R_{c1,}$ $-(CH_2)_{n1}OR_{c1}$, $-(CH2)_{n1}SR_{c1}$, $-(CH_2)_{n1}C(O)R_{c1}$, $-(CH_2)_{n1}C(O)OR_{c1}$, $-(CH_2)_{n1}S(O)_{m1}R_{c1}$, $-(CH_2)_{n1}NR_{a1}R_{b1}$ $-(CH_2)_{n1}C(O)NR_{a1}R_{b1}$, $-(CH_2)_{n1}NR_{a1}C(O)R_{c1}$ and $-(CH_2)_{n1}NR_{a1}S(O)_{m1}R_{c1}$,

alternatively, $R_{aa}$ and $R_{bb}$ together with adjacent atoms form $C_{3-12}$ cycloalkyl, 3- to 12- membered heterocyclyl, $C_{6-12}$ aryl or 5- to 12-membered heteroaryl, wherein the $C_{3-12}$ cycloalkyl, 3- to 12-membered heterocyclyl, $C_{6-12}$ aryl and 5- to 12-membered heteroaryl are optionally substituted with one or more substituents selected from deuterium, halogen, amino, hydroxyl, cyano, nitro, $C_{1-6}$alkyl, $C_{2-6}$ alkenyl, $C_{2-6}$ alkynyl, $C_{l-6}$ deuteroalkyl, $C_{l-6}$ haloalkyl, $C_{1-6}$ alkoxy, $C_{1-6}$ haloalkoxy, $C_{1-6}$ hydroxyalkyl, cyano-substituted $C_{1-6}$alkyl, $C_{3-12}$ cycloalkyl, 3- to 12- membered heterocyclyl, $C_{6-12}$ aryl, 5- to 12-membered heteroaryl, $-O(CH_2)_{n1}R_{c1}$, $-OC(R_{a1}R_{b1})_{n1}(CH_2)_mR_{e1}$, $-NR_{a1}(CH_2)_{n1}R_{c1}$, $-(CH_2)_{n1}-$, $-(CH_2)_{n1}R_{c1}$, $-(CH_2)_{n1}OR_{c1}$, $-(CH_2)_{n1}SR_{c1}$, $-(CH_2)_{n1}C(O)R_{c1}$, $-(CH_2)_{n1}C(O)OR_{c1}$, $-(CH_2)_{n1}S(O)_mR_{c1}$, $-(CH_2)_{n1}NR_{a1}R_{b1}$, $-(CH_2)_{n1}C(O)NR_{a1}R_{b1}$, $-(CH_2)_{n1}NR_{a1}C(O)R_{c1}$ and $-(CH_2)_{n1}NR_{a1}S(O)_{m1}R_{c1}$;

$R_{cc}$ is selected from hydrogen, deuterium, halogen, amino, hydroxyl, cyano, nitro, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12- membered heterocyclyl, $C_{6-12}$ aryl or 5- to 12-membered heteroaryl, which are optionally substituted with one or more substituents selected from deuterium, halogen, amino, hydroxyl, cyano, nitro, $C_{1-6}$ alkyl, $C_{3-12}$ cycloalkyl, $-O(CH_2)_{n1}R_{c1}$, $-OC(R_{a1}R_{b1})_{n1}(CH_2)_mR_{c1}$, $-NR_{a1}(CH_2)_{n1}R_{c1}$, $-(CH_2)_{n1}-$, $-(CH_2)_{n1}R_{c1}$, $-(CH_2)_{n1}OR_{c1}$, $-(CH_2)_{n1}SR_{c1}$, $-(CH_2)_{n1}C(O)R_{c1}$, $-(CH_2)_{n1}C(O)OR_{c1}$, $-(CH_2)_{n1}S(O)_mR_{c1}$, $-(CH_2)_{n1}NR_{a1}R_{b1}$, $-(CH_2)_{n1}C(O)NR_{a1}R_{b1}$, $-(CH_2)_{n1}NR_{a1}C(O)R_{c1}$ and $-(CH_2)_{n1}NR_{a1}S(O)_{m1}R_{c1}$;

$R_{a1}$-$R_{c1}$ are each independently selected from hydrogen, deuterium, halogen, amino, hydroxyl, cyano, nitro, $C_{1-6}$alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$alkyl, $C_{3-12}$ cycloalkyl, 3- to 12- membered heterocyclyl, $C_{6-12}$ aryl or 5- to 12-membered heteroaryl, which are optionally substituted with one or more substituents selected from deuterium, halogen, amino, hydroxyl, cyano, nitro, $C_{1-6}$ alkyl and $C_{3-12}$ cycloalkyl;

$m_1$ is 0, 1, 2 or 3; and

$n_1$ is 0, 1, 2 or 3.

In a further preferred embodiment of the present invention, $R^1$ is selected from hydrogen, deuterium, fluorine, chlorine, bromine, amino, hydroxyl, cyano, nitro, methyl, ethyl, propyl, isopropyl, oxo, methoxy, ethoxy, difluoromethyl, trifluoromethyl, difluoromethoxy, trifluoromethoxy, cyclopropyl, cyclobutyl, oxetanyl, azetidinyl, triazolyl, oxazolyl, isoxazolyl, pyrazolyl, pyrrolyl or thiazolyl, wherein the cyclopropyl, cyclobutyl, oxetanyl, azetidinyl, triazolyl, oxazolyl, isoxazolyl, pyrazolyl, pyrrolyl and thiazolyl are optionally substituted with one or more substituents selected from deuterium,

fluorine, chlorine, bromine, amino, hydroxyl, cyano, nitro, $C_{1-3}$alkyl, oxo, thio, $C_{1-3}$ deuteroalkyl, $C_{1-3}$ haloalkyl, $C_{1-3}$ alkoxy, $C_{1-3}$ haloalkoxy, $C_{1-3}$hydroxyalkyl and cyano-substituted $C_{1-3}$ alkyl.

The present invention further provides a compound represented by general formula (X-A), a stereoisomer or pharmaceutically acceptable salt thereof:

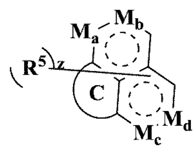

( X-A )

wherein,

$M_a$, $M_b$, $M_c$, $M_d$, $R^5$, ring C and z are as follows as defined in any of the preceding embodiments.

The present invention further provides a compound represented by general formula (X-B), a stereoisomer or pharmaceutically acceptable salt thereof:

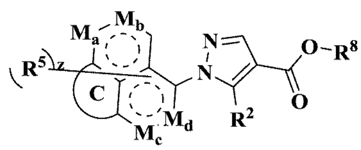

( X-B )

wherein,

$R^8$ is selected from hydrogen, deuterium, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl or 5- to 12-membered heteroaryl, wherein the $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-3}$ alkyl, $C_{2-4}$ alkenyl, $C_{2-4}$ alkynyl, oxo, thio, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl and 5- to 12-membered heteroaryl,

preferably, $R^8$ is selected from hydrogen, deuterium, or $C_{1-3}$ alkyl;

$M_a$, $M_b$, $M_c$, $M_d$, $R^2$, $R^5$, ring C and z are as defined in any of the preceding embodiments.

The present invention further provides a compound represented by general formula (X-C), a stereoisomer or pharmaceutically acceptable salt thereof:

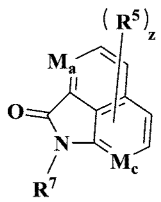

( X-C )

wherein,

$M_a$, $M_c$, $R^5$, $R^7$, and z are as defined in any of the preceding embodiments.

The present invention further provides a compound represented by general formula (X-D), a stereoisomer or pharmaceutically acceptable salt thereof:

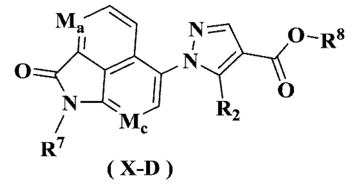

( X-D )

wherein,

$R^8$ is selected from hydrogen, deuterium, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl or 5- to 12-membered heteroaryl, wherein the $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-3}$ alkyl, $C_{2-4}$ alkenyl, $C_{2-4}$ alkynyl, oxo, thio, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl and 5- to 12-membered heteroaryl,

preferably, $R^8$ is selected from hydrogen, deuterium, or $C_{1-3}$ alkyl;

$M_a$, $M_b$ and $R^7$ are as defined in any of the preceding embodiments.

The present invention further relates to a method for preparing the compound of general formula (X-B), a stereoisomer or pharmaceutically acceptable salt thereof, comprising the following steps:

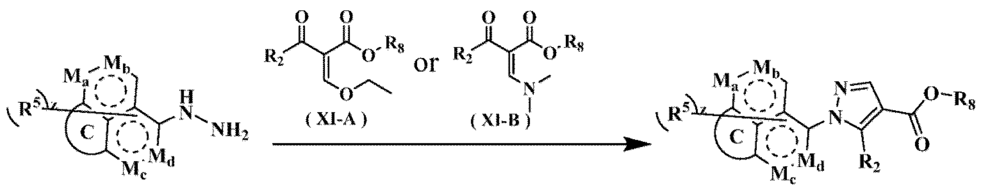

( X-E ) ( X-B )

reacting the compound of general formula (X-E) with

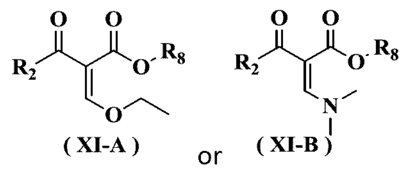

( XI-A ) or ( XI-B ) to

obtain the compound of general formula (X-B);

$M_a$, $M_b$, $M_c$, $M_d$, $R^2$, $R^5$, $R^8$, ring C and z are as defined in any of the preceding embodiments.

The present invention further relates to a method for preparing the compound of general formula (III), a stereoisomer or pharmaceutically acceptable salt thereof, comprising the following steps:

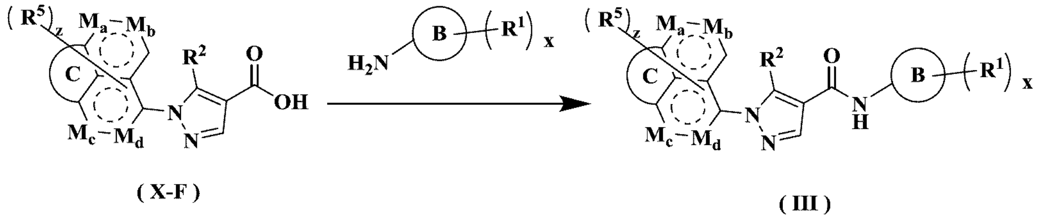

( X-F ) ( III )

subjecting the compound of general formula (X-F) to amide condensation in the presence of a condensing agent to

obtain a compound of general formula (III);

the condensing agent is selected from phosphorus oxychloride, thionyl chloride, oxalyl chloride, diisopropyl azodicarboxylate, diethyl azodicarbonate, carbodiimide hydrochloride, N,N'-diisopropylcarbodiimide, dicyclohexylcarbodiimide, HATU, HBTU, HOBT, TCFH, PyBop or PyClock;

$M_a$, $M_b$, $M_c$, $M_d$, $R^2$, $R^5$, $R^1$, ring B, ring C, x and z are as defined in any of the preceding embodiments.

The present invention further relates to a method for preparing the compound of general formula (X-D), a stereoisomer or pharmaceutically acceptable salt thereof, comprising the following steps:

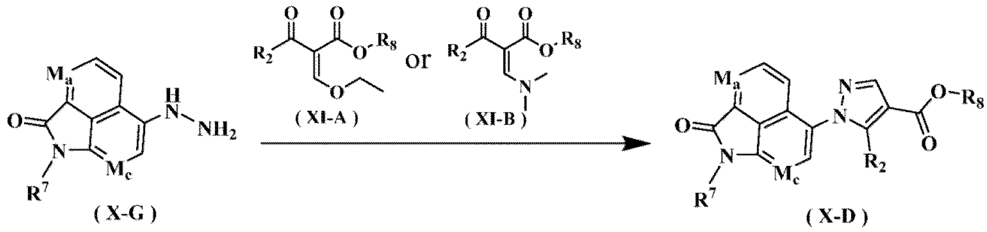

(XI-A) or (XI-B)

(X-G) (X-D)

reacting the compound of general formula (X-G) with

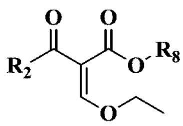

(XI-A)

or

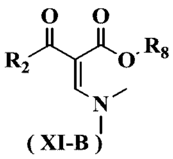

(XI-B)

to obtain the compound of general formula (X-D);
$M_a$, $M_c$, $R^2$, $R^5$, $R^8$and $R^7$ are as defined in any of the preceding embodiments.

The present invention further relates to a method for preparing the compound of general formula (VI), a stereoisomer or pharmaceutically acceptable salt thereof, comprising the following steps:

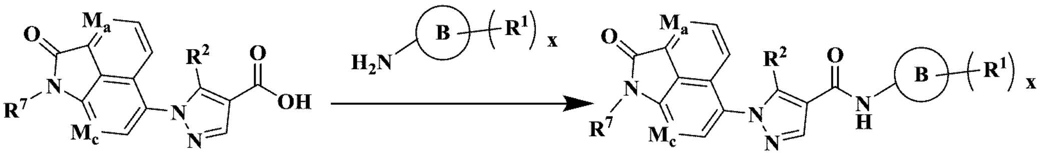

(X-H) (VI)

subjecting the compound of general formula (X-H) to amide condensation in the presence of a condensing agent to obtain a compound of general formula (VI);

the condensing agent is selected from phosphorus oxychloride, thionyl chloride, oxalyl chloride, diisopropyl azodicarboxylate, diethyl azodicarbonate, carbodiimide hydrochloride, N,N'-diisopropylcarbodiimide, dicyclohexylcarbodiimide, HATU, HBTU, HOBT, TCFH, PyBop or PyClock;

$M_a$, $M_c$, $R^2$, $R^7$, $R^1$, ring B and x are as defined in any of the preceding embodiments.

The present invention further relates to a pharmaceutical composition, which comprises a therapeutically effective dose of a compound of general formula (I), a stereoisomer or pharmaceutically acceptable salt thereof and one or more pharmaceutically acceptable carriers, diluents or excipients.

The present invention further relates to the compound of general formula (I), the stereoisomer or pharmaceutically acceptable salt thereof, or the use of the pharmaceutical composition in the preparation of MALT1 inhibitor drugs.

The present invention further relates to the use of the compound represented by the general formula (I), the

stereoisomer or pharmaceutically acceptable salt thereof, or the pharmaceutical composition thereof in the preparation of a drug for the treatment of a cancer or an autoimmune disease.

The present invention further comprises a method of treating a cancer or an autoimmune disease.

The cancer or the autoimmune disease of the present invention is selected from B-cell lymphoma, non-Hodgkin lymphoma, mantle cell lymphoma, follicular lymphoma, mucosa-associated lymphoid tissue lymphoma, chronic lymphocytic leukemia, rheumatoid arthritis, psoriatic arthritis, psoriasis, ulcerative colitis, Crohn's disease, systemic lupus erythematosus, asthma and chronic obstructive pulmonary disease.

The treatment method provided herein includes administering to a subject a therapeutically effective amount of the compound of the present invention. In one embodiment, the present invention provides a method of treating a disease including a cancer or an autoimmune disease in a mammal. The method comprises administering to the mammal a therapeutically effective amount of the compound of the present invention, or the pharmaceutically acceptable salt, ester, prodrug, solvate, hydrate or derivative thereof.

## Detailed Description of The Present Invention

Unless stated to the contrary, the terms used in the specification and claims have the following meanings.

The term "alkyl" refers to a saturated aliphatic hydrocarbon group, which is a straight or branched group containing 1 to 20 carbon atoms, preferably alkyl containing 1 to 8 carbon atoms, more preferably alkyl containing 1 to 6 carbon atoms, and most preferably alkyl containing 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc. More preferably, the alkyl is a lower alkyl group containing 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl group may be substituted or unsubstituted, and when substituted, the substituents may be at any available point of attachment. The substituents are preferably one or more of the following groups independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, heterocycloalkoxy, cycloalkylthio, heterocycloalkylthio, oxo, carboxyl or carboxylate group, and methyl, ethyl, isopropyl, tert-butyl, haloalkyl, deuteroalkyl, alkoxy-substituted alkyl and hydroxy-substituted alkyl are preferred in the present invention.

The term "alkylene" refers to one hydrogen atom of alkyl being further substituted, for example: "methylene" refers to $-CH_2-$, "ethylene" refers to $-(CH_2)_2-$, "propylene" refers to $-(CH_2)_3-$, and "butylene" refers to $-(CH_2)_4-$, etc. The term "alkenyl" refers to an alkyl group as defined above consisting of at least two carbon atoms and at least one carbon-carbon double bond, such as ethenyl, 1-propenyl, 2-propenyl, 1-, 2- or 3 -butenyl, etc. Alkenyl can be substituted or unsubstituted. When the alkenyl is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, or heterocycloalkylthio.

The term "cycloalkyl" refers to a saturated or partially unsaturated monocyclic or polycyclic cyclic hydrocarbon substituent. The cycloalkyl ring contains 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc. Polycyclic cycloalkyl includes spiro, fused and bridged cycloalkyl, preferably cyclopropyl, cyclobutyl, cyclohexyl, cyclopentyl and cycloheptyl.

The term "spirocycloalkyl" refers to a polycyclic group with 5- to 20-membered monocyclic rings sharing one carbon atom (called a spiro atom). It may contain one or more double bonds, but no ring has a completely conjugated $\pi$ electron system. Preferably, the bridged heterocyclyl is 6- to 14-membered, and more preferably 7- to 10-membered. According to the number of shared spiro atoms between the rings, the spirocycloalkyl is divided into monospirocycloalkyl, bispirocycloalkyl or polyspirocycloalkyl, preferably monospirocycloalkyl and bispirocycloalkyl. More preferably, it is a 3-membered/6-membered, 3-membered/5-membered, 4-membered/4-membered, 4-membered/5-membered, 4-membered/6-membered, 5-membered/5-membered or 5-membered/6-membered monospirocycloalkyl. Non-limiting examples of spirocycloalkyl include:

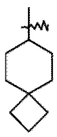 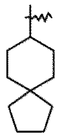 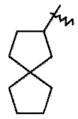 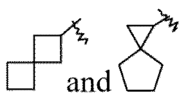

, etc.;

Also included are spirocycloalkyl groups in which monospirocycloalkyl and heterocycloalkyl share a spiro atom. Non-limiting examples include:

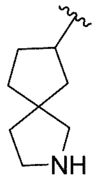 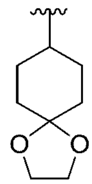 and 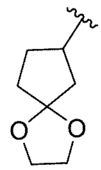 ,

etc.

The term "fused cycloalkyl" refers to a 5- to 20-membered all-carbon polycyclic group in which each ring in the system shares an adjacent pair of carbon atoms with other rings in the system, one or more ring of which may contain one or more double bonds, but no ring has a fully conjugated $\pi$ electron system. Preferably, the bridged heterocyclyl is 6- to 14-membered, and more preferably 7- to 10-membered. According to the number of constituent rings, it can be divided into bicyclic, tricyclic, tetracyclic or polycyclic fused cycloalkyl, preferably bicyclic or tricyclic, and more preferably 5-membered/5-membered or 5-membered/6-membered bicyclic cycloalkyl. Non-limiting examples of fused cycloalkyl include:

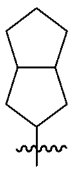 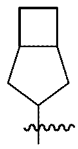 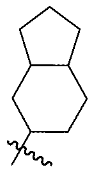 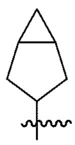 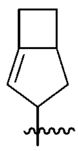 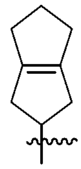 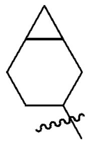 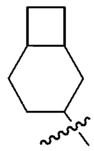 and 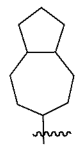 ,

etc.

The term "bridged cycloalkyl" refers to a 5- to 20-membered all-carbon polycyclic group with any two rings sharing two carbon atoms that are not directly connected. It may contain one or more double bonds, but no ring has a fully conjugated $\pi$ electron system. Preferably, the bridged heterocyclyl is 6- to 14-membered, and more preferably 7- to 10-membered. According to the number of constituent rings, it can be divided into bicyclic, tricyclic, tetracyclic or polycyclic bridged cycloalkyl, preferably bicyclic, tricyclic or tetracyclic, and more preferably bicyclic or tricyclic. Non-limiting examples of bridged cycloalkyl include:

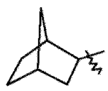 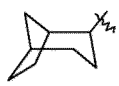 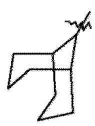 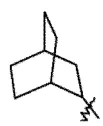 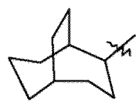

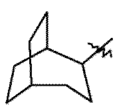 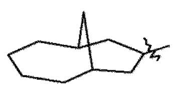 and 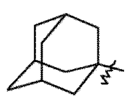 .

The cycloalkyl ring can be fused to an aryl, heteroaryl or heterocycloalkyl ring, where the ring connected to the parent structure is cycloalkyl, non-limiting examples include indanyl, tetrahydronaphthyl, benzocycloheptyl, and the like. Cycloalkyl may be optionally substituted or unsubstituted, and when substituted, the substituent is preferably one or more of the following groups, which are independently selected from alkyl, alkenyl, alkynyl, alkoxy, alkylthio, alkylamino,

halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl or carboxylate group.

The term "heterocyclyl" refers to a saturated or partially unsaturated monocyclic or polycyclic cyclic hydrocarbon substituent, which comprises 3 to 20 ring atoms, wherein one or more ring atoms are heteroatoms selected from nitrogen, oxygen or $S(O)_m$ (wherein m is an integer of 0 to 2), but excluding ring moieties of -O-O-, -O-S- or -S-S-, and the remaining ring atoms are carbon. Preferably it contains 3 to 12 ring atoms, of which 1-4 are heteroatoms; more preferably it contains 3 to 8 ring atoms; further preferably, it is a 3- to 8-membered heterocyclyl containing 1-3 nitrogen atoms, optionally substituted with 1 to 2 oxygen atoms, sulfur atoms, or oxo, including nitrogen-containing monocyclic heterocyclyl, nitrogen-containing spirocyclic heterocyclyl or nitrogen-containing fused heterocyclyl; more preferably, it contains 3 to 7 ring atoms, of which 1 to 4 are heteroatoms. In one embodiment, heterocyclyl contains 3, 4, 5, 6, 7 or 8 ring atoms.

Non-limiting examples of monocyclic heterocyclyl include pyrrolidyl, imidazolidinyl, tetrahydrofuryl, tetrahydrothiophenyl, dihydroimidazolyl, dihydrofuryl, dihydropyrazolyl, dihydropyrrolyl, piperidyl, piperazinyl, morpholinyl, thiomorpholinyl, homopiperazinyl, azepinyl, 1,4-diazacycloheptyl, pyranyl, etc., preferably pyrrolidyl, morpholinyl, piperidyl, azepinyl, 1,4-diazacycloheptyl and piperazinyl. Polycyclic heterocyclyl includes spiro, fused and bridged heterocyclyl; the involved spiro, fused and bridged heterocyclyl are optionally connected to other groups through a single bond, or further fused to other cycloalkyl, heterocyclyl, aryl and heteroaryl through any two or more atoms on the ring.

The term "spiroheterocyclyl" refers to a 5- to 20-membered polycyclic heterocyclic group with monocyclic rings sharing one atom (called a spiro atom), wherein one or more ring atoms are heteroatoms selected from nitrogen, oxygen or $S(O)_m$ (wherein mm is an integer of 0 to 2), and the remaining ring atoms are carbon. Spiroheterocyclyl may contain one or more double bonds, but no ring has a fully conjugated π electron system. Preferably, the bridged heterocyclyl is 6- to 14-membered, and more preferably 7- to 10-membered. According to the number of shared spiro atoms between the rings, the spiro heterocyclyl is divided into monospiroheterocyclyl, bispiroheterocyclyl or polyspiroheterocyclyl, preferably monospiroheterocyclyl and bispiroheterocyclyl. More preferably, it is a 3-membered/5-membered, 3-membered/6-membered, 4-membered/4-membered, 4-membered/5-membered, 4-membered/6-membered, 5-membered/5-membered or 5-membered/6-membered monospiro heterocyclyl. Non-limiting examples of spiro heterocyclyl include:

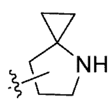 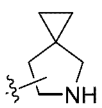 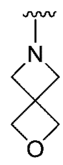 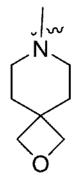 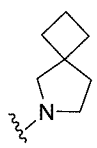 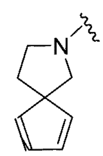 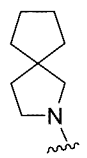 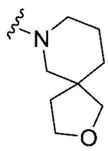

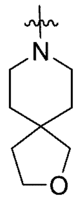 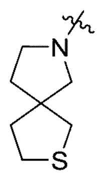 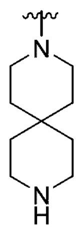 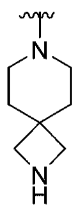 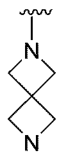 ,

etc.

The term "fused heterocyclyl" refers to a 5- to 20-membered polycyclic heterocyclic group with each ring in the system sharing an adjacent pair of atoms with other rings in the system, and one or more rings may contain one or more double bonds, but no ring has a fully conjugated π electron system, wherein one or more ring atoms are heteroatoms selected from nitrogen, oxygen, or $S(O)_m$ (where m is an integer of 0 to 2), and the remaining ring atoms are carbon. Preferably, the bridged heterocyclyl is 6- to 14-membered, and more preferably 7- to 10-membered. According to the number of constituent rings, it can be divided into bicyclic, tricyclic, tetracyclic or polycyclic fused heterocyclyl, preferably bicyclic or tricyclic, more preferably 5-membered/5-membered or 5-membered/6-membered bicyclic fused heterocyclyl. Non-limiting examples of fused heterocyclyl include:

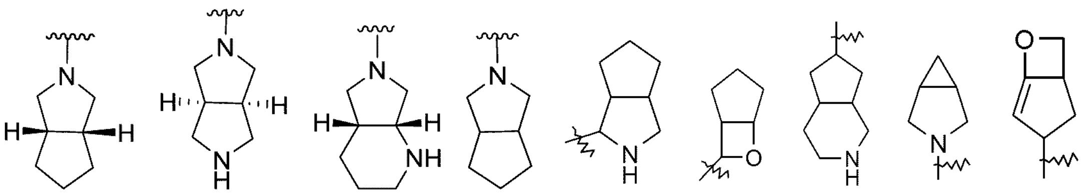

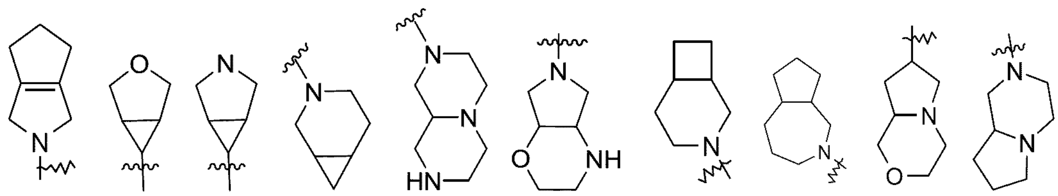

and

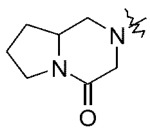

,

etc.

The term "bridged heterocyclyl" refers to a 5- to 14-membered polycyclic heterocyclic group with any two rings sharing two atoms that are not directly connected. It may contain one or more double bonds, but no ring has a fully conjugated π electron system, wherein one or more ring atoms are heteroatoms selected from nitrogen, oxygen, or $S(O)_m$ (where m is an integer of 0 to 2), and the remaining ring atoms are carbon. Preferably, the bridged heterocyclyl is 6- to 14-membered, and more preferably 7- to 10-membered. According to the number of constituent rings, it can be divided into bicyclic, tricyclic, tetracyclic or polycyclic bridged heterocyclyl, preferably bicyclic, tricyclic or tetracyclic, and more preferably bicyclic or tricyclic. Non-limiting examples of bridged heterocyclyl include:

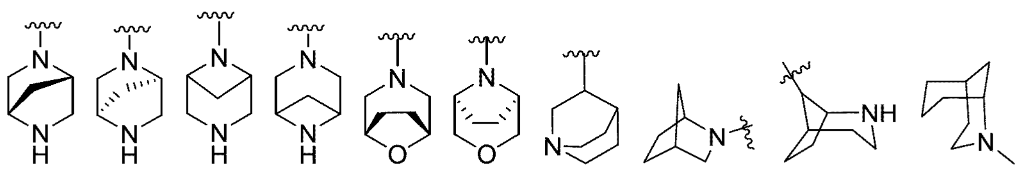

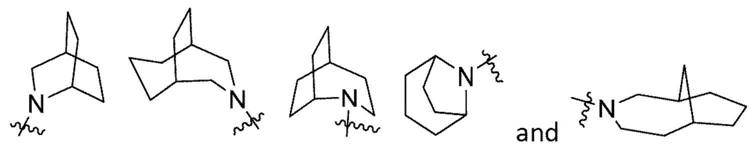

and ,

etc.

The heterocyclyl ring can be fused to an aryl, heteroaryl or cycloalkyl ring, wherein the ring connected to the parent structure is heterocyclyl, and the non-limiting examples thereof include:

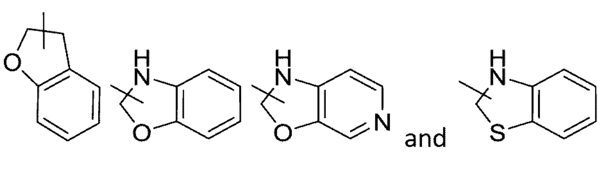

and ,

etc.

Heterocyclyl may be optionally substituted or unsubstituted, and when substituted, the substituent is preferably

one or more of the following groups, which are independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl or carboxylate group.

The term "aryl" refers to a 6- to 14-membered all-carbon monocyclic or fused polycyclic (i.e., rings sharing an adjacent pair of carbon atoms) group having a conjugated π electron system, preferably 6- to 12-membered aryl, such as phenyl and naphthyl, and more preferably phenyl.

The aryl ring can be fused to heteroaryl, heterocyclyl or cycloalkyl ring, including benzo 5- to 10-membered heteroaryl, benzo 3- to 8-membered cycloalkyl and benzo 3- to 8-membered heteroalkyl, preferably benzo 5- to 6-membered heteroaryl, benzo 3- to 6-membered cycloalkyl and benzo 3- to 6-membered heteroalkyl, wherein the heterocyclyl is heterocyclyl containing 1-3 nitrogen atoms, oxygen atoms, or sulfur atoms; or further including a three-membered nitrogen-containing fused ring containing a benzene ring,
wherein the ring connected to the parent structure is aryl ring, and the non-limiting examples thereof include:

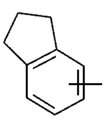
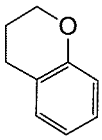
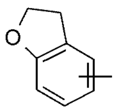
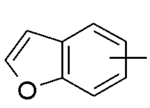
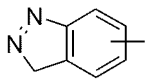
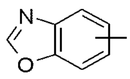
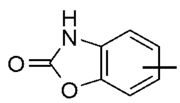
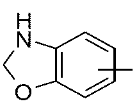
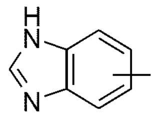
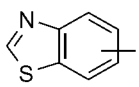
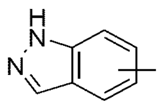
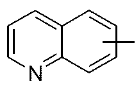
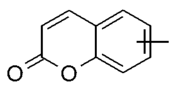
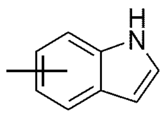
and
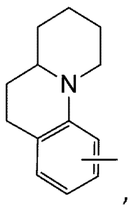
,

etc.

Aryl may be substituted or unsubstituted, and when substituted, the substituent is preferably one or more of the following groups, which are independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, carboxyl or carboxylate group.

The term "heteroaryl" refers to a heteroaromatic system containing 1 to 4 heteroatoms and 5 to 14 ring atoms, where the heteroatoms are selected from oxygen, sulfur and nitrogen. The heteroaryl is preferably 5- to 12-membered, more preferably 5- to 8-membered, further preferably 5-to 7-membered, even more preferably 5- or 6-membered, such as imidazolyl, furyl, thienyl, thiazolyl, pyrazolyl, oxazolyl, pyrrolyl, triazolyl, tetrazolyl, pyridyl, pyrimidinyl, thiadiazole, pyrazinyl, etc., preferably triazolyl, thienyl, imidazolyl, pyrazolyl, oxazolyl, pyrimidinyl or thiazolyl; more preferred pyrazolyl, pyrrolyl, imidazolyl, triazolyl, oxazolyl or pyridinyl. The heteroaryl ring can be fused to an aryl, heterocyclyl or cycloalkyl ring, wherein the ring connected to the parent structure is heteroaryl ring, and the non-limiting examples thereof include:

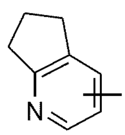
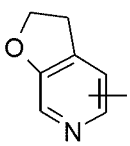
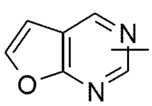
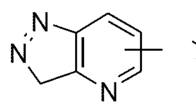
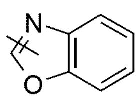
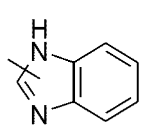
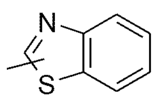
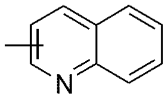
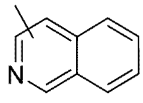
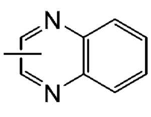
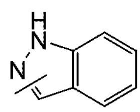
and
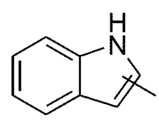
,

etc.

Heteroaryl may be optionally substituted or unsubstituted, and when substituted, the substituent is preferably one or more of the following groups, which are independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, carboxyl or carboxylate group.

The term "alkoxy" refers to -O-(alkyl) and -O-(unsubstituted cycloalkyl), where alkyl is as defined above. Non-limiting examples of alkoxy include: methoxy, ethoxy, propoxy, butoxy, cyclopropoxy, cyclobutoxy, cyclopentoxy, and cyclohexoxy. Alkoxy may be optionally substituted or unsubstituted, and when substituted, the substituent is preferably one or more of the following groups, which are independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, carboxyl or carboxylate group.

"Haloalkyl" refers to an alkyl substituted with one or more halogens, where alkyl is as defined above.

"Haloalkoxy" refers to an alkoxy substituted with one or more halogens, where alkoxy is as defined above.

"Hydroxyalkyl" refers to alkyl substituted with hydroxy, wherein alkyl is defined as above. "Alkenyl" is also known as alkylene, wherein the alkenyl can be further substituted with other related groups, such as: alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or a carboxylate group. "Alkynyl" refers to (CH=C-), wherein the alkynyl can be further substituted with other related groups, such as: alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or a carboxylate group.

The term "alkenylcarbonyl" refers to -C(O)-(alkenyl), wherein alkenyl is defined as above. Non-limiting examples of alkenylcarbonyl include: ethenylcarbonyl, propenylcarbonyl, and butenylcarbonyl. Alkenylcarbonyl can be optionally substituted or unsubstituted. When the alkenylcarbonyl is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or a carboxylate group. "Hydroxyl" refers to the -OH group.

"Halogen" refers to fluorine, chlorine, bromine or iodine.

"Amino" refers to $-NH_2$.

"Cyano" refers to -CN.

"Nitro" refers to $-NO_2$.

"Carbonyl" refers to -C(O)-.

"Carboxy" refers to -C(O)OH.

"THF" refers to tetrahydrofuran.

"EtOAc" refers to ethyl acetate.

"MeOH" refers to methanol.

"DMF" refers to N,N-dimethylformamide.

"DIPEA" refers to diisopropylethylamine.

"TFA" refers to trifluoroacetic acid.

"MeCN" refers to acetonitrile.

"DMA" refers to N,N-dimethylacetamide.

"$Et_2O$" refers to diethyl ether.

"DCE" refers to 1,2 dichloroethane.

"DIPEA" refers to N,N-diisopropylethylamine.

"NBS" refers to N-bromosuccinimide.

"NIS" refers to N-iodosuccinimide.

"Cbz-Cl" refers to benzyl chloroformate.

"$Pd_2(dba)_3$" refers to tris(dibenzylideneacetone)dipalladium.

"Dppf" refers to 1,1'-bisdiphenylphosphine ferrocene.

"HATU" refers to 2-(7-azabenzotriazol-1-yl)-N,N,N',N'-tetramethyluronium hexafluorophosphate.

"KHMDS" refers to potassium bis(trimethylsilyl)amide.

"LiHMDS" refers to lithium hexamethyldisilazide.

"MeLi" refers to methyllithium.

"n-BuLi" refers to n-butyllithium.

"$NaBH(OAc)_3$" refers to sodium triacetoxyborohydride.

"NaOH" refers to sodium hydroxide.

"Boc" refers to tert-butoxycarbonyl.

Different terms such as "X is selected from A, B, or C", "X is selected from A, B and C", "X is A, B or C", "X is A, Band C" all express the same meaning, *i.e.,* X can be any one or more of A, B, and C.

The hydrogen atoms described in the present invention can be replaced by its isotope deuterium, and any

hydrogen atom in the example compounds involved in the present invention can also be replaced by deuterium atom.

"Optional" or "optionally" means that the event or circumstance subsequently described may but need not to occur, and the description includes the occasions where the events or circumstances occur or do not occur. For example, "heterocyclic group optionally substituted with alkyl" means the alkyl may but need not be present, the description includes the case where the heterocyclic group is substituted with alkyl and the case where the heterocyclic group is not substituted with alkyl.

"Substituted" refers to one or more hydrogen atoms in the group, preferably at most 5, more preferably 1-3 hydrogen atoms each independently substituted with a corresponding number of substituents. It goes without saying, the substituents may be only in their possible chemical positions, a person skilled in the art can determine the possible or impossible substitutions (by experiment or theory) without paying too much effort. For example, the amino group having a free hydrogen or a hydroxy group may be unstable when combined the carbon atoms having an unsaturated (e.g., olefinic) bond.

"Pharmaceutical composition" denotes a mixture containing one or more of the compounds as stated herein or physiologically/pharmaceutically acceptable salts or prodrug thereof and other chemical components, as well as other components, such as a physiologically/pharmaceutically acceptable carrier and an excipient. The purpose of pharmaceutical compositions is to facilitate administration to living organisms and facilitate the absorption of active ingredients to exert biological activity.

"Pharmaceutically acceptable salt" refers to a salt of the compound of the present invention, which are safe and effective when used in mammals, and have appropriate biological activity.

DETAILED DESCRIPTION OF EMBODIMENTS

The present invention will be further described below with reference to examples, but these examples do not limit the scope of the present invention.

Examples

The structures of the compounds of the present invention are determined by nuclear magnetic resonance (NMR) and/or mass spectrometry (MS). The NMR shift (δ) is given in the unit of $10^{-6}$ (ppm). NMR was determined using a Bruker AVANCE-400 nuclear magnetic instrument. The solvents for determination were deuterated dimethyl sulfoxide (DMSO-*d6*), deuterated chloroform ($CDCl_3$), and deuterated methanol ($CD_3OD$), and the internal standard was tetramethylsilane (TMS).

MS was measured using a FINNIGAN LCQAd (ESI) mass spectrometer (manufacturer: Thermo, model: Finnigan LCQ advantage MAX).

HPLC determination used Agilent 1200DAD high-pressure liquid chromatograph instrument (Sunfire C18 150 × 4.6 mm chromatographic column) and Waters 2695-2996 high-pressure liquid chromatograph instrument (Gimini C18 150 × 4.6 mm chromatographic column). The average kinase inhibition rate and $IC_{50}$ value were measured using NovoStar microplate reader (BMG Company, Germany).

Yantai Huanghai HSGF254 or Qingdao GF254 silica gel plate is used as a thin layer chromatography silica plate, and the silica gel plate for the thin layer chromatography (TLC) is of the specification of 0.15 mm-0.2 mm, and the specification when separating and purifying a product by thin layer chromatography is 0.4 mm-0.5 mm.

For the column chromatography, Yantai Huanghai silica gel of 200-300 mesh silica gel was generally used as a carrier.

The known starting materials of the present invention can be synthesized by methods known in the art, or can be purchased from ABCR GmbH & Co. KG, Acros Organics, Aldrich Chemical Company, Accela ChemBio Inc., Chembee Chemicals Inc. and other companies.

If there is no special instruction in the examples, reactions can be carried out under an argon atmosphere or a nitrogen atmosphere.

Argon atmosphere or nitrogen atmosphere means that the reaction bottle is connected to an argon or nitrogen balloon with a volume of about 1 L.

The hydrogen atmosphere means that the reaction bottle is connected to a hydrogen balloon with a volume of about 1 L.

The pressurized hydrogenation reaction uses Parr 3916EKX hydrogenator and Qinglan QL-500 hydrogen generator or HC2-SS hydrogenator.

The hydrogenation reaction is usually performed under the condition that vacuumizing and filling hydrogen is repeated three times.

The microwave reaction uses CEM Discover-S 908860 microwave reactor.

If there is no special instruction in the examples, the solution refers to an aqueous solution.

If there is no special instruction in the examples, the reaction temperature is room temperature, which is 20°C to 30°C.

The progress of the reaction in the examples is monitored by thin layer chromatography (TLC). The developing agent system used in the reaction is: A: dichloromethane and methanol system, B: n-hexane and ethyl acetate system, C: petroleum ether and ethyl acetate system, D: acetone, the volume ratio of the solvents is adjusted according to the polarity of the compounds.

The eluent system of column chromatography and the developing agent system of thin layer chromatography used to purify compounds include: A: n-hexane and ethyl acetate system, B: n-hexane and tetrahydrofuran system, the volume ratio of the solvents is adjusted according to the polarity of the compounds, and a small amount of alkaline or acidic reagents such as triethylamine and acetic acid can also be added for adjustment.

**Intermediate 1** 1-(2-carbonyl-1,2-dihydrobenzo[*cd*]indol-6-yl)-5-trifluoromethyl-1H-pyrazole-4-carboxylic acid

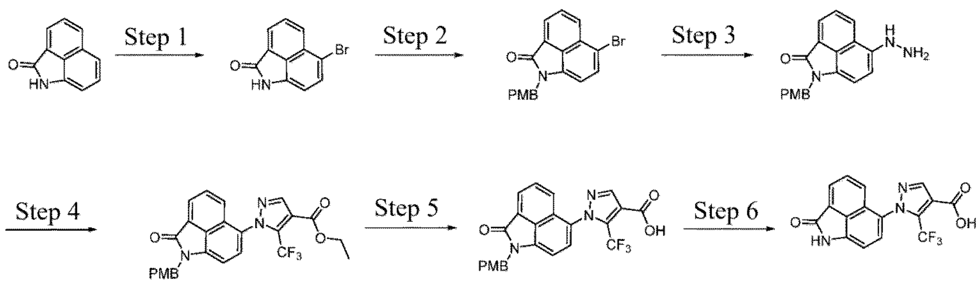


Step 1 6-bromobenzo[*cd*]indol-2(1H)-one

Benzo[*cd*]indol-2(1H)-one (20 g, 118.2 mmol) was dissolved in 150 mL of glacial acetic acid, and liquid bromine (37.8 g, 236.5 mmol) was added dropwise under an ice bath to the reaction liquid, and the mixture was reacted under stirring at room temperature for 16 h. The reaction liquid was concentrated under reduced pressure to obtain the title product 6-bromobenzo[*cd*]indol-2(1H)-one (18.7 g), yield: 63.8%.

MS m/z (ESI): 248 [M+1].

Step 2 6-bromo-1-(4-methoxybenzyl)benzo[*cd*]indol-2(1H)-one

6-bromobenzo[cd]indol-2(1H)-one (10 g, 40.3 mmol) was dissolved in 200 mL of dimethylformamide, 4-methoxybenzyl bromine (12.2 g, 61.5 mmol) and potassium carbonate (11.3 g, 82 mmol) were added and the mixture was reacted under stirring at room temperature for 16 h. The reaction liquid was filtered, and the filtrate was concentrated under reduced pressure. The obtained residue was purified by silica gel column chromatography with eluent system A to obtain the title product 6-bromo-1-(4-methoxybenzyl)benzo[cd]indol-2(1H)-one (10.7 g), yield: 72.1%.

MS m/z (ESI): 368 [M+1].

Step 3 6-hydrazino-1-(4-methoxybenzyl)benzo[*cd*]indol-2(1H)-one

Palladium chloride (π-cinnamyl) dimer (229.4 mg, 0.44 mmol) was mixed with *N*-[2-bis(1-adamantane)phosphine phenyl]morpholine (415.5 mg, 0.89 mmol) was dissolved in 5 mL of dioxane, and the mixture was reacted under stirring at room temperature for 15 min. 6-bromo-1-(4-methoxybenzyl)benzo[*cd*]indol-2(1H)-one (3.3 g, 8.96 mmol) and sodium tert-butoxide (1.72 g, 17.90 mmol) was dissolved in 50 mL of dioxane, added to the reaction liquid, and the mixture was reacted under stirring at room temperature for 5 min. Hydrazine hydrate (897.3 mg, 17.92 mmol) was added to the reaction liquid, and the mixture was reacted under stirring at 50°C for 2 h. The reaction liquid was filtered, and the filter residue was washed with 20 mL of ethyl acetate. The filtrate was concentrated under reduced pressure to obtain the title product 6-hydrazino-1-(4-methoxybenzyl)benzo[*cd*]indol-2(1H)-one (2.66 g), yield: 93.0%.

MS m/z (ESI): 319 [M+1].

Step 4 Ethyl 1-(1-(4-methoxybenzyl)-2-carbonyl-1,2-dihydrobenzo[*cd*]indol-6-yl)-5-trifluoromethyl-1H-pyrazole-4-carboxylate

6-hydrazino-1-[(4-methoxybenzyl]benzo[*cd*]indol-2(1H)-one (2.66 g, 8.33 mmol), potassium carbonate (2.3 g,

16.7 mmol) and ethyl ethoxy-2-methylene trifluoroacetoacetate (4 g, 16.7 mmol) were dissolved in 50 mL of ethanol, and the mixture was reacted under stirring at 80°C for 3 h. The reaction liquid was concentrated under reduced pressure, and the obtained residue was purified by silica gel column chromatography with eluent system A to obtain the title product ethyl 1-(1-(4-methoxybenzyl)-2-carbonyl-1,2-dihydrobenzo[cd]indol-6-yl)-5-(trifluoromethyl)-1H-pyrazole-4-carboxylate (880 mg), yield: 21.3%.

MS m/z (ESI): 496 [M+1].

Step 5 1-(1-(4-methoxybenzyl)-2-carbonyl-1,2-dihydrobenzo[*cd*]indol-6-yl)-5-trifluoromethyl-1H-pyrazole-4-carboxylic acid

Ethyl 1-(1-(4-methoxybenzyl)-2-carbonyl-1,2-dihydrobenzo[*cd*]indol-6-yl)-5-(trifluoromethyl)-1H-pyrazole-4-carboxylate (880 mg, 1.78 mmol) and sodium hydroxide (350 mg, 8.9 mmol) were dissolved in 2 mL of tetrahydrofuran and 1 mL of water, and the mixture was reacted under stirring at 60°C for 16 h. 2M hydrochloric acid was added to the reaction liquid to adjust the pH to less than 7, the obtained mixture was extracted with ethyl acetate (50 mL×3), the organic phases were combined, and washed with water (50 mL×1) and saturated sodium chloride solution (50 mL×1) in sequence, dried over anhydrous sodium sulfated, and filtered, and the filtrate was concentrated under reduced pressure to obtain the title product 1-(1-(4-methoxybenzyl)-2-carbonyl-1,2-dihydrobenzo[*cd*]indol-6-yl)-5-trifluoromethyl-1H-pyrazole-4-carboxylic acid (770 mg), yield: 93%.
MS m/z (ESI): 468 [M+1]

Step 6 1-(2-carbonyl-1,2-dihydrobenzo[*cd*]indol-6-yl)-5-trifluoromethyl-1H-pyrazole-4-carboxylic acid

1-(1-(4-methoxybenzyl)-2-carbonyl-1,2-dihydrobenzo[cd]indol-6-yl)-5-trifluoromethyl-1H-pyrazole-4-carboxylic acid (770 mg, 1.65 mmol) was dissolved in 20 mL of trifluoroacetic acid,

and the mixture was reacted under stirring at 90°C for 16 h. The reaction liquid was filtered, and the filtrate was concentrated under reduced pressure. The obtained residue was purified by silica gel column chromatography with eluent system A to obtain the title intermediate 1-(2-carbonyl-1,2-dihydrobenzo[cd]indol-6-yl)-5-trifluoromethyl-1H-pyrazole-4-carboxylic acid (450 mg), yield: 78.5%.
MS m/z (ESI): 348 [M+1]

$^1$H NMR (400 MHz, DMSO-$d_6$) δ 11.09 (s, 1H), 8.33 (s, 1H), 8.12 (d, 1H), 7.86 (dd, 1H), 7.69 (d, 1H), 7.61 (d, 1H), 7.08 (d, 1H).

**Example 1**

1-(2-carbonyl-1,2-dihydropyrrolo[2,3,4-*ij*]isoquinolin-5-yl)-5-trifluoromethyl-*N*-(2-trifluoromethylpyridin-4-yl)-1*H*-pyrazole-4-carboxamide

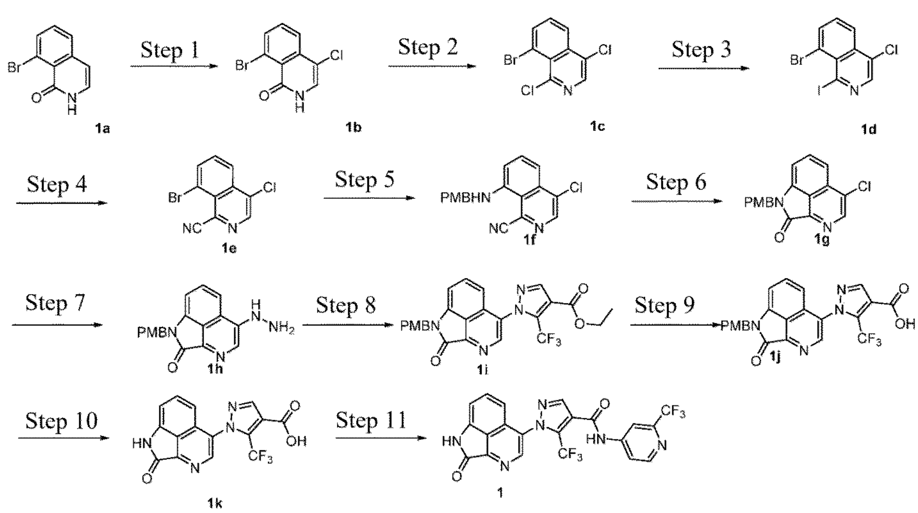

Step 1

8-bromo-4-chloroisoquinolin-1(2*H*)-one

8-bromoisoquinolin-1(2*H*)-one **1a** (5.00 g, 22.32 mmol) was dissolved in 50 mL of N,N-dimethylformamide, *N*-chlorosuccinimide (3.15 g, 23.59 mmol) was added to the reaction liquid under ice bath, and , the mixture was reacted under stirring at room temperature for 12 h. The obtained reaction liquid was extracted with ethyl acetate (50 mL×3), the organic phases were combined, washed with water (50 mL×1) and saturated sodium chloride solution (50 mL×1) in sequence, dried over anhydrous sodium sulfate, and filtered, and the filtrate was concentrated under reduced pressure and the obtained residue was purified by silica gel column chromatography with eluent system A to obtain 8-bromo-4-chloroisoquinolin-1(2*H*)-one **1b** (4.70 g), yield: 81.5%.
MS m/z (ESI): 258 [M+1]

Step 2

8-bromo-1,4-dichloroisoquinoline

8-bromo-4-chloroisoquinolin-1(2*H*)-one **1b** (4.70 g, 18.18 mmol) was dissolved in 50 mL of phosphorus oxychloride, the mixture was reacted under stirring at 100°C for 16 h. The reaction liquid was filtered, and the filtrate was concentrated under reduced pressure. The obtained residue was purified by silica gel column chromatography with eluent system A to obtain the title product 8-bromo-1,4-dichloroisoquinoline **1c** (4.50 g), yield: 89.3%.
MS m/z (ESI): 276 [M+1]

Step 3

8-bromo-4-chloro-1-iodoisoquinoline

8-bromo-1,4-dichloroisoquinoline **1c** (4.50 g, 16.25 mmol) and sodium iodide (4.90 g, 32.69 mmol) were dissolved in 50 mL of acetonitrile and added to the reaction liquid, and the mixture was reacted under stirring stir at room temperature for 5 min. Trismethylchlorosilane (3.30 mg, 30.37 mmol) was added to the reaction liquid, and the mixture was reacted under stirring at 80°C for 12 h. The reaction liquid was concentrated under reduced pressure, and the obtained residue was purified by silica gel column chromatography with eluent system A to obtain the title product 8-bromo-4-chloro-1-iodoisoquinoline **1d** (3.50 g), yield: 58.6%.
MS m/z (ESI): 368 [M+1]

Step 4

8-bromo-4-chloro-1-cyanoisoquinoline

8-bromo-4-chloro-1-iodoisoquinoline **1d** (3.50 g, 9.50 mmol), zinc cyanide (700 mg, 5.96 mmol) and tetrakis(triphenylphosphine)palladium (550 mg, 0.48 mmol) was dissolved in 50 mL of dimethylformamide, and the mixture was reacted under stirring at 80°C for 13 h. The obtained reaction liquid was extracted with ethyl acetate (50 mL×3), the organic phases were combined, washed with water (50 mL×1) and saturated sodium chloride solution (50 mL×1) in sequence, dried over anhydrous sodium sulfate, and filtered, and the filtrate was concentrated under reduced pressure and the obtained residue was purified by silica gel column chromatography with eluent system A to obtain the title product 8-bromo-4-chloro-1-cyanoisoquinoline **1e** (2.00 g), yield: 79.1%.
MS m/z (ESI): 267 [M+1]

Step 5

4-chloro-8-(4-methoxybenzyl)amino-1-cyanoisoquinoline

8-bromo-4-chloro-1-cyanoisoquinoline **1e** (2.00 g, 7.48 mmol) and 4-methoxybenzylamine (1.10 g, 8.02 mmol) were dissolved in 10 mL of dioxane, cesium carbonate (4.9 g, 15.04 mmol) and bis(dibenzylideneacetone)palladium (430 mg, 0.76 mmol) were added, and then the mixture was reacted under stirring at 90°C for 16 h. The obtained reaction liquid was extracted with ethyl acetate (50 mL×3), the organic phases were combined, washed with water (50 mL×1) and saturated sodium chloride solution (50 mL×1) in sequence, dried over anhydrous sodium sulfate, and filtered, and the filtrate was concentrated under reduced pressure and the obtained residue was purified by silica gel column chromatography with eluent system A to obtain the title product 4-chloro-8-(4-methoxybenzyl)amino-1-cyanoisoquinoline **1f** (2.10 g), yield: 86.2%.
MS m/z (ESI): 324 [M+1]

Step 6

5-chloro-1-(4-methoxybenzyl)pyrrolo[2,3,4-*ij*]isoquinolin-2(1*H*)-one

4-chloro-8-((4-methoxybenzyl)amino)isoquinolin-1-carbonitrile **1f** (2.10 g, 6.48 mmol) was dissolved in 20 mL of concentrated sulfuric acid, and the mixture was reacted under stirring at 100°C for 16 h. The reaction liquid was added to ice water and filtered to obtain the title product 5-chloro-1-(4-methoxybenzyl)pyrrolo[2,3,4-*ij*]isoquinolin-2(1*H*)-one **1g** (450 mg), yield: 21.3%.
MS m/z (ESI): 325 [M+1]

Step 7

5-hydrazino-1-(4-methoxybenzyl)pyrrolo[2,3,4-*ij*]isoquinolin-2(1*H*)-one

Palladium chloride (π-cinnamyl) dimer (35 mg, 0.068 mmol) was mixed with *N*-[2-bis(1-adamantane)phosphine phenyl]morpholine (64 mg, 0.14 mmol) was dissolved in 5 mL of dioxane, and the mixture was reacted under stirring at room temperature for 15 min. 5-chloro-1-(4-methoxybenzyl)pyrrolo[2,3,4-*ij*]isoquinolin-2(1H)-one **1g** (450 mg, 1.38 mmol) and sodium tert-butoxide (265 mg, 2.76 mmol) was dissolved in 50 mL of dioxane, added to the reaction liquid, and the mixture was reacted under stirring at room temperature for 5 min. Hydrazine hydrate (897 mg, 17.92 mmol) was added to the reaction liquid, and the mixture was reacted under stirring at 50°C for 2 h. The reaction liquid was filtered and the filter residue was washed with ethyl acetate (20 mL). The filtrate was concentrated under reduced pressure to obtain the title product 5-hydrazino-1-(4-methoxybenzyl)pyrrolo[2,3,4-*ij*]isoquinolin-2(1*H*)-one **1h** (500 mg) as a crude product.
MS m/z (ESI): 320 [M+1]

Step 8

Ethyl 1-(1-(4-methoxybenzyl)-2-carbonyl-1,2-dihydropyrrolo[2,3,4-*ij*]isoquinolin-5-yl)-2-trifluoromethyl-1*H*-pyrrol-3-carboxylate

5-hydrazino-1-(4-methoxybenzyl)pyrrolo[2,3,4-*ij*]isoquinolin-2(1*H*)-one **1h** (500 mg, 1.56 mmol) and ethyl ethoxy-2-methylene trifluoroacetoacetate (750 mg, 3.12 mmol) were dissolved in 10 mL of ethanol and the mixture was reacted under stirring at 80°C for 3 h. The reaction liquid was concentrated under reduced pressure, and the obtained residue was purified by silica gel column chromatography with eluent system A to obtain the title product ethyl 1-(1-(4-methoxybenzyl)-2-carbonyl-1,2-dihydropyrrolo[2,3,4-*ij*]isoquinolin-5-yl)-2-trifluoromethyl-1*H*-pyrrol-3-carboxylate **1i** (200 mg), yield: 25.9%.
MS m/z (ESI): 496 [M+1]

Step 9

1-(1-(4-methoxybenzyl)-2-carbonyl-1,2-dihydropyrrolo[2,3,4-*ij*]isoquinolin-5-yl)-2-trifluoromethyl-1*H*-pyrrol-3-carboxylic acid

Ethyl 1-(1-(4-methoxybenzyl)-2-carbonyl-1,2-dihydropyrrolo[2,3,4-*ij*]isoquinolin-5-yl)-2-trifluoromethyl-1*H*-pyrrol-3-carboxylate **1i**(200 mg, 0.40 mmol) and sodium hydroxide (350 mg, 8.75 mmol) were dissolved in 4 mL of tetrahydrofuran and 2 mL of water, and the mixture was reacted under stirring at 30°C for 16 h. 2M hydrochloric acid was added to the reaction liquid to adjust the pH to less than 7, the obtained mixture was extracted with ethyl acetate (50 mL×3), the organic phases were combined, and washed with water (50 mL×1) and saturated sodium chloride solution (50 mL×1) in sequence, dried over anhydrous sodium sulfated, and filtered, and the filtrate was concentrated under reduced pressure to obtain the title product 1-(1-(4-methoxybenzyl)-2-carbonyl-1,2-dihydropyrrolo[2,3,4-*ij*]isoquinolin-5-yl)-2-trifluoromethyl-1*H*-pyrrol-3-carboxylic acid **1j** (100 mg), yield: 53.0%.
MS m/z (ESI): 468 [M+1]

Step 10

1-(2-oxo-1,2-dihydropyrrolo[2,3,4-*ij*]isoquinolin-5-yl)-2-trifluoromethyl-1*H*-pyrrol-3-carboxylic acid

1-(1-(4-methoxybenzyl)-2-carbonyl-1,2-dihydropyrrolo[2,3,4-*ij*]isoquinolin-5-yl)-2-trifluoromethyl-1*H*-pyrrol-3-carboxylic acid **1j** (100 mg, 0.21 mmol) was dissolved in 10 mL of trifluoroacetic acid, and the mixture was reacted under stirring at 90°C for 16 h. The reaction liquid was concentrated under reduced pressure, and the obtained residue was purified by silica gel column chromatography using eluent system C to obtain the title compound 1-(2-oxo-1,2-dihydropyrrolo[2,3,4-*ij*]isoquinolin-5-yl)-2-trifluoromethyl-1*H*-pyrrol-3-carboxylic acid **1k** (75 mg), yield: 65.7%.
MS m/z (ESI): 348 [M+1]

Step 11

1-(2-carbonyl-1,2-dihydropyrrolo[2,3,4-*ij*]isoquinolin-5-yl)-2-trifluoromethyl-*N*-(2-trifluoromethylpyridin-4-yl)-1*H*-pyrrol-3-carboxamide

1-(2-oxo-1,2-dihydropyrrolo[2,3,4-*ij*]isoquinolin-5-yl)-2-trifluoromethyl-1*H*-pyrrol- 3-carboxylic acid **1k** (70 mg, 0.20 mmol), 2-trifluoromethyl-4-aminopyridine (31 mg, 0.19 mmol), pyridine (76 mg, 0.96 mmol) and phosphorus oxychloride (73 mg, 0.48 mmol) were dissolved in 5 mL of dichloromethane, and the mixture was reacted under stirring at room temperature for 2 h. Dichloromethane (50 mL) was added to the reaction liquid, and the obtained product was washed with water (50 mL×1) and saturated sodium chloride solution (50 mL×1) in sequence, dried over anhydrous sodium sulfate, filtered, and the filtrate was concentrated under reduced pressure, and the obtained residue was purified by silica gel column chromatography with eluent system A to obtain the title product 1-(1-(4-methoxybenzyl)-2-carbonyl-1,2-dihydropyrrolo[2,3,4-*ij*]isoquinolin-5-yl)-2-trifluoromethyl-*N*-(2-trifluoromethylpyridin-4-yl)-1*H*-pyrrol-3-carboxamide **1** (80 mg), yield: 81.24%.
MS m/z (ESI): 492 [M+1].

**Example 2**

1-(2-oxo-1,2-dihydropyrrolo[2,3,4-*ij*]isoquinolin-6-yl)-5-trifluoromethyl-N-(2-trifluoromethylpyridin-4-yl)-1*H*-pyrazole-4-carboxamide

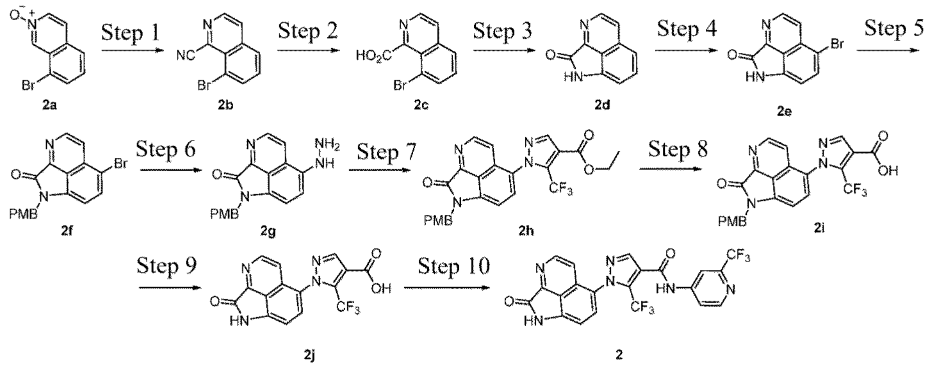


Step 1

1-cyano-8-bromoisoquinoline

8-bromoisoquinoline oxide **2a** (22.50 g, 0.10 mol, prepared by the well-known method "Patent WO 2004002992") was dissolved in 500 mL of 1,4-dioxane, 1,8-diazabicyclo[5.4.0]-7-undecene (33.0 mL, 0.22 mol) and trismethylsilane cyanide (22.5 mL, 0.18 mol) were added, and the mixture was reacted under stirring at 120°C for 18 h. The reaction liquid was concentrated under reduced pressure, and the obtained residue was purified by silica gel column chromatography with eluent system E (PE/THF=1:1) to obtain the title product 1-cyano-8-bromoisoquinoline **2b** (9.90 g), yield: 42.3%.
MS m/z (ESI): 233, 235 [M+1]

Step 2

8-bromoisoquinolin-1-carboxylic acid

1-cyano-8-bromoisoquinoline **2b** (9.90 g, 42.48 mmol) was dispersed in 50 mL of water, sodium hydroxide (10 g, 0.25 mol) was added, and the mixture was reacted under stirring at 85°C for 6 h. 15 mL of concentrated hydrochloric acid was added to the reaction liquid to quench the reaction, the obtained product was extracted with ethyl acetate (100 mL×2), the organic phases were combined, washed with saturated sodium bicarbonate solution (100 mL×2), washed with saturated sodium chloride solution (100 mL×2), and dried over anhydrous sodium sulfate. The obtained product was filtered, and the filtrate was concentrated under reduced pressure to obtain the crude title product 8-bromoisoquinolin-1-carboxylic acid **2c** (10.50 g). The product was directly used in the next reaction without purification.
MS m/z (ESI): 252, 254 [M+1]
Referring to the synthesis method of "Patent US2016/0240789A1", the title product pyrrolo[2,3,4-*ij*]isoquinolin-2(1H)-one **2d** (4.00 g) was obtained through crude 8-bromoisoquinolin-1-carboxylic acid **2c** (10.50 g, 41.66 mmol), yield: 56.4%.
MS m/z (ESI): 171 [M+1]
Referring to the synthesis method of the step 1 in Example 1, 6-bromopyrrolo[2,3,4-*ij*]isoquinolin-2(1*H*)-one **2e** (5.40 g) was obtained through pyrrolo[2,3,4-*ij*]isoquinolin-2(1H)-one **2d** (4.00 g, 23.51 mmol), yield: 92.3%.
MS m/z (ESI): 249, 251 [M+1]

Step 5

6-bromo-1-(4-methoxybenzyl)pyrrolo[2,3,4-*ij*]isoquinolin-2(1*H*)-one

6-bromopyrrolo[2,3,4-*ij*]isoquinolin-2(1*H*)-one **2e** (5.40 g, 21.68 mmol) was dissolved in 200 mL of dimethylformamide, 4-methoxybenzyl bromine (12.90 g, 64.16 mmol) and potassium carbonate (8.96 g, 64.83 mmol) were added and the mixture was reacted under stirring at 80°C for 16 h. The reaction liquid was concentrated under reduced pressure, and the obtained residue was purified by silica gel column chromatography with eluent system A to obtain the title product 6-bromo-1-(4-methoxybenzyl)pyrrolo[2,3,4-*ij*]isoquinolin-2(1*H*)-one **2f** (6.49 g), yield: 81.0%.
MS m/z (ESI): 369, 371 [M+1]

Step 6 to Step 10

1-(2-oxo-1,2-dihydropyrrolo[2,3,4-*ij*]isoquinolin-6-yl)-5-trifluoromethyl-*N*-(2-trifluoromethylpyridin-4-yl)-1*H*-pyrazole-4-carboxamide

Referring to the synthesis method of the step 7 to the step 11 in Example **1,,** the title product 1-(2-oxo-1,2-dihydropyrrolo[2,3,4-*ij*]isoquinolin-6-yl)-5-trifluoromethyl-*N*-(2-trifluoromethylpyridin-4-yl)-1*H*-pyrazole-4-carboxamide **2** (32 mg) was obtained through 1-(2-oxo-1,2-dihydropyrrolo[2,3,4-*ij*]isoquinolin-6-yl)-5-trifluoromethyl-1*H*-pyrazole-4-carboxylic acid **2f** (1.00 g, 2.71 mmol), yield: 2.4%.

MS m/z (ESI): 493.1 [M+1]
$^1$H NMR (400 MHz, DMSO-$d_6$) δ 11.22 (broad s, 1H), 10.98 (broad s, 1H), 8.86 (d, 1H), 8.79 (d, 1H), 8.28 (s, 1H), 8.11 (d, 1H), 8.05 (d, 1H), 7.97 (d, 1H), 7.84 (d, 1H), 6.52 (s, 1H).

**Example 3**

1-(2-carbonyl-1,2-dihydropyrrolo[4,3,2-*ij*]isoquinolin-6-yl)-5-trifluoromethyl-*N*-(2-trifluoromethylpyridin-4-yl)-1*H*-pyrazole-4-carboxamide

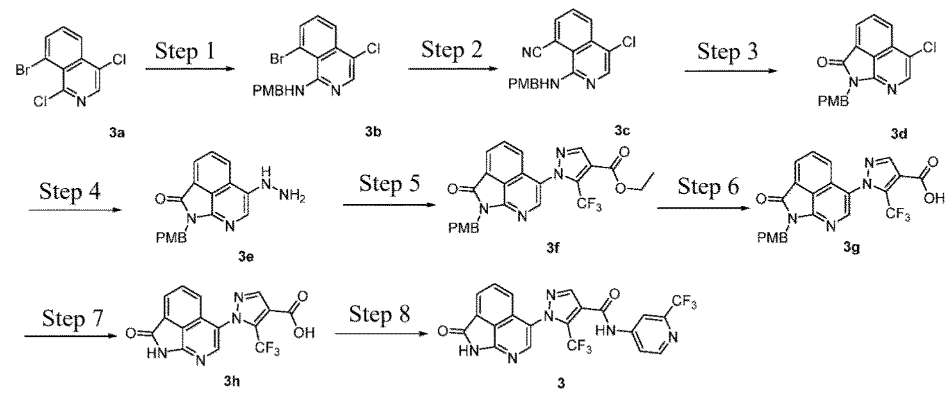


3a 3b 3c 3d 3e 3f 3g 3h 3

Step 1

8-bromo-4-chloro-*N*-(4-methoxybenzyl)isoquinolin-1-amine

8-bromo-1,4-dichloroisoquinoline **3a** (5.00 g, 18.05 mmol) was dissolved in 50 mL of acetonitrile, then 4-methoxybenzylamine (4.90 g, 35.72 mol) was added to the reaction liquid, and the mixture was reacted under stirring at 80°C for 12 h. The obtained reaction liquid was extracted with ethyl acetate (50 mL×3), the organic phases were combined, washed with water (50 mL×1) and saturated sodium chloride solution (50 mL×1) in sequence, dried over anhydrous sodium sulfate, and filtered, and the filtrate was concentrated under reduced pressure and the obtained residue was purified by silica gel column chromatography with eluent system A to obtain 8-bromo-4-chloro-*N*-(4-methoxybenzyl)isoquinolin-1-amine **3b** (4.7 g), yield: 68.9%.
MS m/z (ESI): 377 [M+1]

Referring to the synthesis method of the step 4, the step 6 to the step 11 in Example **1** in sequence,1-(2-carbonyl-1,2-dihydropyrrolo[4,3,2-*ij*]isoquinolin-6-yl)-5-trifluoromethyl-*N*-(2-trifluoromethylpyridin-4-yl)-1*H*-pyrazole-4-carboxamide **3** (10 mg) was obtained from 8-bromo-4-chloro-*N*-(4-methoxybenzyl)isoquinolin-1-amine **3b** (4.70 g, 12.44 mmol), yield: 0.2%.

MS m/z (ESI): 492 [M+1].

$^1$H NMR (400 MHz, MeOD) δ 8.63 (d, *J* = 5.6 Hz, 1H), 8.37 (s, 1H), 8.30 (s, 1H), 8.23 (d, *J* = 2.0 Hz, 1H), 8.16 (d, *J* = 7.0 Hz, 1H), 8.05 (dd, *J* = 8.2, 7.0 Hz, 1H), 7.97 (dd, *J* = 5.6, 2.1 Hz, 1H), 7.71 (d, *J* = 8.2 Hz, 1H).

## Example 4

1-(2,2-dimethyl-1-carbonyl-1,2-dihydroacenaphthylen-5-yl)-5-trifluoromethyl-*N*-(2-trifluoromethylpyridin-4-yl)-1H-pyrazole-4-carboxamide

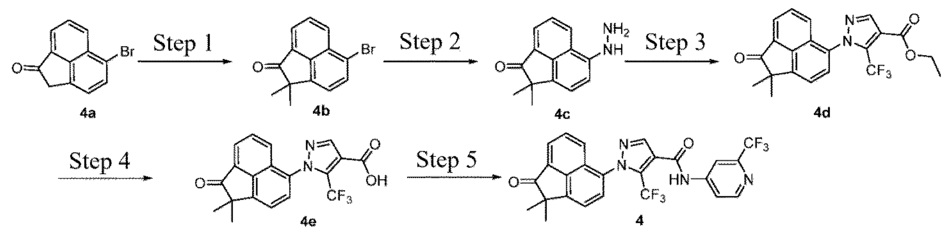


4a 4b 4c 4d 4e 4

Step 1

5-bromo-2,2-dimethylacenaphthylen-1(2H)-one

5-bromo-acenaphthylen-1(2H)-one **4a** (200 mg, 0.81 mmol, synthesized by the well-known method "Patent US6667303 B1") was dissolved in 4 mL of tetrahydrofuran and 1 mL of dimethylformamide, sodium hydride (97.12 mg, 60% purity, 2.43 mmol) was added in small amount for several times under ice bath. Methyl iodide (230 mg, 1.62 mmol) was weighted and the mixture was reacted under stirring at 60°C for 4 h. 50 mL of water was added to the reaction liquid

under ice bath to quench sodium hydrogen, the obtained product was extracted with ethyl acetate (50 mL×2), the organic phases were combined, washed with saturated sodium chloride solution (50 mL×1), dried over anhydrous sodium sulfate, and filtered, and the filtrate was concentrated under reduced pressure and the obtained residue was purified by silica gel column chromatography with eluent system A to obtain the title product 5-bromo-2,2-dimethylacenaphthylen-1(2H)-one **4b** (210 mg), yield: 94.3%.
MS m/z (ESI): 276 [M+1]

Referring to the synthesis method of the step 7 to the step 9 and the step 11 in Example **1** in sequence, 1-(2,2-dimethyl-1-carbonyl-1,2-dihydroacenaphthylen-5-yl)-5-trifluoromethyl-*N*-(2-trifluoromethylpyridin-4-yl)-1H-pyrazole-4-carboxamide **4** (8.3 mg) was obtained, yield: 2.1%.

MS m/z (ESI): 518 [M+1]
$^1$H NMR (400 MHz, DMSO-$d_6$) δ 11.37 (s, 1H), 8.72 (d, 1H), 8.61 (s, 1H), 8.28 (d, 1H), 8.11 (d, 1H), 8.02 (dd, 1H), 7.97 (dd, 2H), 7.84 (d, 1H), 7.69 (d, 1H), 1.48 (s, 6H).

## Example 5

*N*-(6-(2*H*-1,2,3-triazol-2-yl)-5-chloropyridin-3-yl)-1-(2-oxo-1,2-dihydrobenzo[*cd*]indol-6-yl)-5-trifluoromethyl-1*H*-pyrazole-4-carboxamide

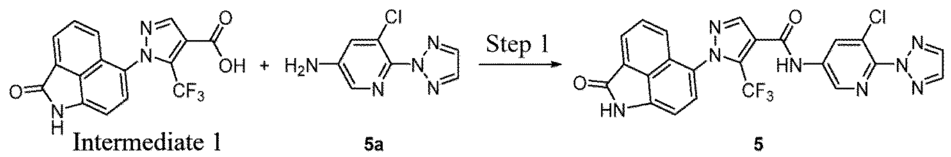


Referring to the synthesis method in the step 11 in Example 1, the title product *N*-(6-(2H-1,2,3-triazol-2-yl)-5-chloropyridin-3-yl)-1-(2-oxo-1,2-dihydrobenzo[cd]indol-6-yl)-5-trifluoromethyl-1*H*-pyrazole-4-carboxamide **5** (16 mg) was obtained through the intermediate **1** (18 mg, 0.052 mmol) and 6-(2*H*-1,2,3-triazol-2-yl)-3-amino-5-chloropyridine **5a** (10 mg, 0.052 mmol, synthesized using the well-known method "Patent US20180170909A1"), yield 58.6%.

MS m/z (ESI): 525, 527 [M+1]
$^1$H NMR (400 MHz, DMSO-$d_6$) δ 11.3 (broad s, 1H), 11.0 (s, 1H), 9.11 (d, 1H), 8.93 (d, 1H), 8.59 (s, 1H), 8.22 (s, 2H), 8.10 (d, 1H), 7.84 (dd, 1H), 7.77 (d, 1H), 7.62 (d, 1H), 7.13 (d, 1H).

## Example 6

1-(2H-naphthaleno[1,8-*bc*]furan-5-yl)-5-trifluoromethyl-*N*-(2-trifluoromethylpyridin-4-yl)-1*H*-pyrazole-4-carboxamide

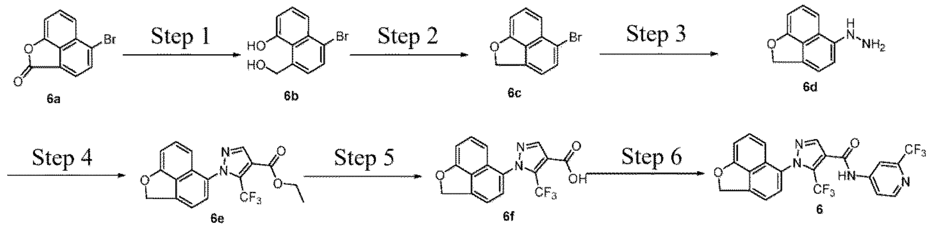


Step 1

5-bromo-8-hydroxymethyl-naphthalen-1-phenol

5-bromo-2*H*-naphthaleno[1,8-*bc*]furan-2-one **6a** (2.48 g, 10 mmol) was dissolved in 30 mL of tetrahydrofuran. At 0°C, lithium aluminum hydride (760 mg, 20 mmol) was added. The mixture was reacted at room temperature for 2 h. 0.76 mL of water, 0.76 mL of 15% sodium hydroxide solution and 2.28 mL of water were added in sequence. The obtained

mixture was filtered, and the filtrate was dried over anhydrous sodium sulfate, filtered, and concentrated to obtain a crude product, which was purified by silica gel column chromatography with eluent system A to obtain the title product 5-bromo-8-hydroxymethyl-naphthalen-1-phenol **6b** (2.05 g), yield: 81.3%.

Step 2

5-bromo-2*H*-naphthaleno[1,8-*bc*]furan

5-bromo-8-hydroxymethyl-naphthalen-1-phenol **6b** (1.50 g, 5.93 mmol) was dissolved in 30 mL of tetrahydrofuran. At 0°C, trisphenylphosphine (1.57 g, 6 mmol) and diisopropyl azodicarboxylate (1.21 g, 6 mmol) were added, and the mixture was reacted at room temperature for 2 h. The reaction liquid was concentrated to obtain a crude product, which was purified by silica gel column chromatography with eluent system A to obtain the title product 5-bromo-2*H*-naphthaleno[1,8-bc]furan **6c** (1.05 g), yield: 70.0%.

Referring to the synthesis method of step 7, step 9 to step 11 in Example 1 in sequence, 1-(2*H*-naphthaleno[1,8-*bc*]furan-5-yl)-5-trifluoromethyl-*N*-(2-trifluoromethylpyridin-4-yl)-1*H*-pyrazole-4-carboxamide **6** (50 mg) was obtained through 5-bromo-2H-naphthaleno[1,8-*bc*]furan **6c** (200 mg, 0.85 mmol), yield: 12.3%.

MS m/z (ESI): 479 [M+1]

$^1$H NMR (400 MHz, DMSO-$d_6$) δ 8.71 (s, 1H), 8.26 (s, 1H), 8.31 (s, 1H), 8.10 - 8.01 (m, 2H), 7.58 - 7.51 (m, 1H), 7.18 (s, 1H), 6.98 (t, 1H), 6.34 - 6.27 (m, 1H), 4.98 (d, 1H), 3.12 (s, 2H).

**Example 7**

1-(2-carbonyl-1,2-dihydrobenzo[*cd*]indol-5-yl)-5-trifluoromethyl-*N*-(2-trifluoromethylpyridin-4-yl)-1H-pyrazole-4-carboxamide

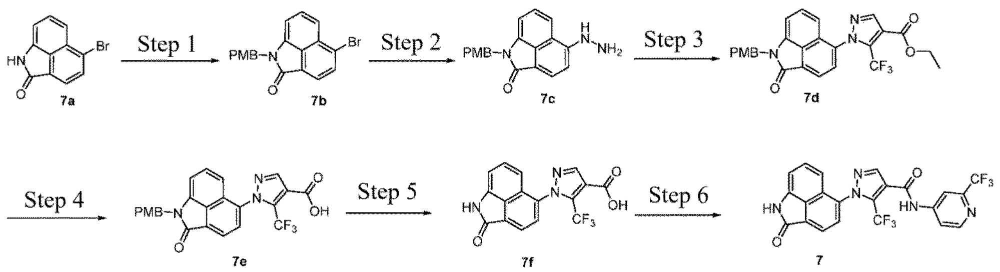


Referring to the synthesis method of step 5 in Example **2** and step 7 to step 11 in Example **1** in sequence, 1-(2-carbonyl-1,2-dihydrobenzo[cd]indol-5-yl)-5-trifluoromethyl-*N*-(2-trifluoromethylpyridin-4-yl)-1H-pyrazole-4-carboxamide **7** (23 mg) was obtained through 5-bromo-benzo[cd]indol-2(1H)-one **7a** (400 mg, 1.61 mmol, prepared by the well-known method in "Patent US2016/0240789A1"), yield: 2.9%.

MS m/z (ESI): 492 [M+1]

$^1$H NMR (400 MHz, DMSO-$d_6$) δ 11.28 (s, 1H), 11.08 (s, 1H), 8.72 (d, 1H), 8.60 (s, 1H), 8.25 (d, 1H), 8.19 (d, 1H), 8.03 - 7.97 (m, 2H), 7.60 (dd, 1H), 7.10 (d, 1H), 7.01 (d, 1H).

**Example 8**

1-(2-oxo-1,2-dihydropyrrolo[2,3,4-*de*]isoquinolin-6-yl)-5-trifluoromethyl-*N*-(2-trifluoromethylpyridin-4-yl)-1H-pyrazole-4-carboxamide

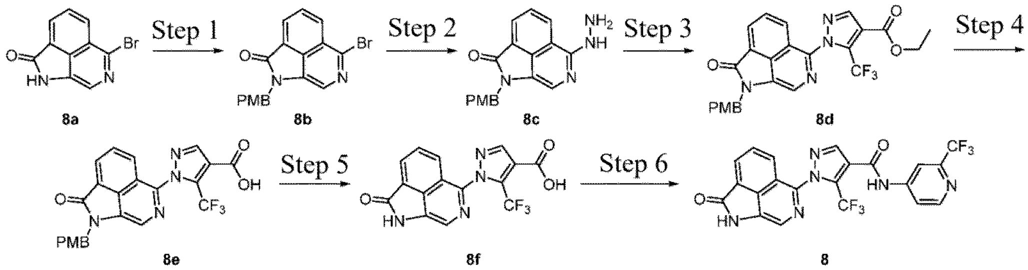

8a 8b 8c 8d 8e 8f 8

Referring to the synthesis method of step 5 in Example **2** and step 7 to step 11 in Example **1** in sequence, 1-(2-oxo-1,2-dihydropyrrolo[2,3,4-*ij*]isoquinolin-6-yl)-5-trifluoromethyl-*N*-(2-trifluoromethylpyridin-4-yl)-1H-pyrazole-4-carboxamide **8** (18 mg) was obtained through 6-bromopyrrolo[2,3,4-*de*]isoquinolin-2(1H)-one **8a** (1.0 g, 4.01 mmol), yield: 0.9%.

MS m/z (ESI): 493.1 [M+1]
$^1$H NMR (400 MHz, DMSO-$d_6$) δ 11.30 (broad s, 1H), 10.91 (broad s, 1H), 8.82 (d, 1H), 8.76 (t, 1H), 8.37 (s, 1H), 8.16 (s, 1H), 8.05 (d, 1H), 7.91 (d, 1H), 7.70 (d, 1H), 6.66 (s, 1H).

## Example 9

1-(1,3-dicarbonyl-2,3-dihydro-1*H*-benzo[de]isoquinolin-6-yl)-2-trifluoromethyl-*N*-(2-trifluoromethylpyridin-4-yl)-1*H*-pyrazol-3-carboxamide

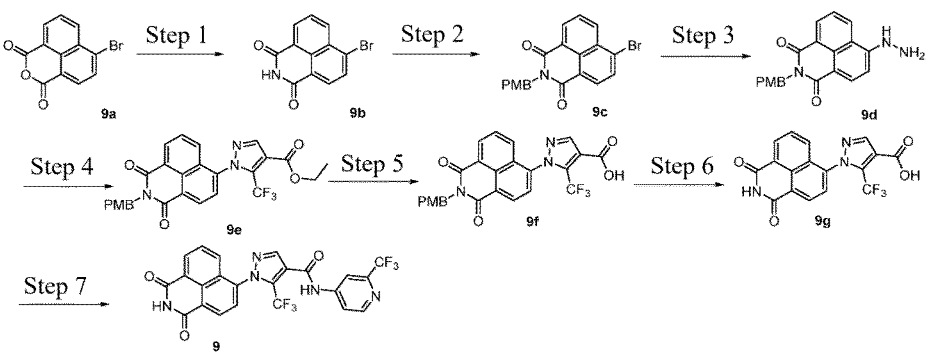

9a 9b 9c 9d 9e 9f 9g 9

Step 1

6-bromo-1*H*-benzo[de]isoquinolin-1,3(2*H*)-dione

6-bromo-1*H*,3*H*-benzo[*de*]isochromene-1,3-dione **9a** (5.00 g, 18.04 mmol) was dissolved in 50 mL of ammonia water, and the mixture was reacted under stirring at 100°C for 12 h. The obtained reaction liquid was extracted with ethyl acetate (50 mL×3), the organic phases were combined, washed with water (50 mL×1) and saturated sodium chloride solution (50 mL×1) in sequence, dried over anhydrous sodium sulfate, and filtered, and the filtrate was concentrated under reduced pressure to obtain 6-bromo-1*H*-benzo[*de*]isoquinolin-1,3(2*H*)-dione **9b** (4.50 g), yield: 90.6%. MS m/z (ESI): 276 [M+1]
Referring to the synthesis method of step 5 in Example **2** and step 7 to step 11 in Example 1 in sequence, 1-(1,3-dicarbonyl-2,3-dihydro-1H-benzo[de]isoquinolin-6-yl)-2-trifluoromethyl-N-(2-trifluoromethylpyridin-4-yl)-1H-pyrazol-3-carboxamide **9** (18 mg) was obtained through 6-bromo-1H-benzo[*de*]isoquinolin-1,3(2*H*)-dione **9b** (4.50 g, 16.30 mmol), yield: 0.2%.
MS m/z (ESI): 519 [M+1].
$^1$H NMR (400 MHz, DMSO) δ 11.32 (s, 1H), 8.72 (d, *J* = 5.5 Hz, 1H), 8.57 (s, 1H), 8.46 (s, 1H), 8.35 - 8.19 (m, 2H), 8.06 - 7.93 (m, 1H), 7.86 (d, *J* = 7.6 Hz, 1H), 7.77 (t, *J* = 7.9 Hz, 1H), 7.69 (d, *J* = 7.7 Hz, 1H), 7.29 (d, *J* = 8.5 Hz, 1H).

**Example 10**

1-(1-carbonyl-2,3-dihydro-1*H*-benzo[*de*]isoquinolin-6-yl)-2-trifluoromethyl-*N*-(2-trifluoromethylpyridin-4-yl)-1*H*-pyrazol-3-carboxamide

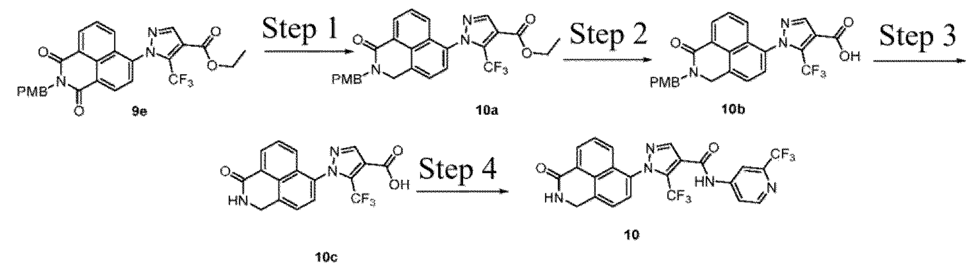


Step 1

Ethyl 1-(2-(4-methoxybenzyl)-1-carbonyl-2,3-dihydro-1*H*-benzo[*de*]isoquinolin-6-yl)-2-trifluoromethyl-1*H*-pyrrol-3-carboxylate

Ethyl 1-(2-(4-methoxybenzyl)-1,3-dioxo-2,3-dihydro-1*H*-benzo[*de*]isoquinolin-6-yl)-2-(trifluoromethyl)-1*H*-pyrrol-3-carboxylate **9e** (500 mg, 0.96 mmol) was dissolved in tetrahydrofuran solution (20 mL), a solution of borane in tetrahydrofuran (1 M. 1.5 mL) was added under stirring at 0°C, and then the mixture was reacted at 20°C for 2 h. The obtained reaction liquid was extracted with ethyl acetate (50 mL×3), the organic phases were combined, washed with water (50 mL×1) and saturated sodium chloride solution (50 mL×1) in sequence, dried over anhydrous sodium sulfate, and filtered, and the filtrate was concentrated under reduced pressure to obtain ethyl 1-(2-(4-methoxybenzyl)-1-carbonyl-2,3-dihydro-1*H*-benzo[de]isoquinolin-6-yl)-2-trifluoromethyl-1*H*-pyrrol-3-carboxylate **10a** (300 mg), yield: 61.6%.
MS m/z (ESI): 509 [M+1]

Referring to the synthesis method of step 9 to the step 11 in Example **1,** 1-(1-carbonyl-2,3-dihydro-1H-benzo[de]isoquinolin-6-yl)-2-trifluoromethyl-*N*-(2-trifluoromethylpyridin-4-yl)-1H-pyrazol-3-carboxamide **10** (4 mg) was obtained through Ethyl 1-(2-(4-methoxybenzyl)-1-carbonyl-2,3-dihydro-1*H*-benzo[de]isoquinolin-6-yl)-2-trifluoromethyl-1H-pyrrol-3-carboxylate **10a** (300 mg, 0.59 mmol), yield: 1.3%.

MS m/z (ESI): 505 [M+1].

$^1$H NMR (400 MHz, DMSO) δ 11.32 (s, 1H), 8.72 (d, *J* = 5.5 Hz, 1H), 8.57 (s, 1H), 8.46 (s, 1H), 8.30 - 8.16 (m, 1H), 8.05 - 7.95 (m, 1H), 7.86 (d, *J* = 7.6 Hz, 1H), 7.77 (t, *J* = 7.9 Hz, 1H), 7.69 (d, *J* = 7.7 Hz, 1H), 7.32 - 6.98 (m, 2H), 5.06 (s, 2H).

**Example 11**

1-(2-carbonyl-2,3-dihydro-1*H*-benzo[de]quinolin-6-yl)-2-trifluoromethyl-*N*-(2-trifluoromethylpyridin-4-yl)-1*H*-pyrazol-3-carboxamide

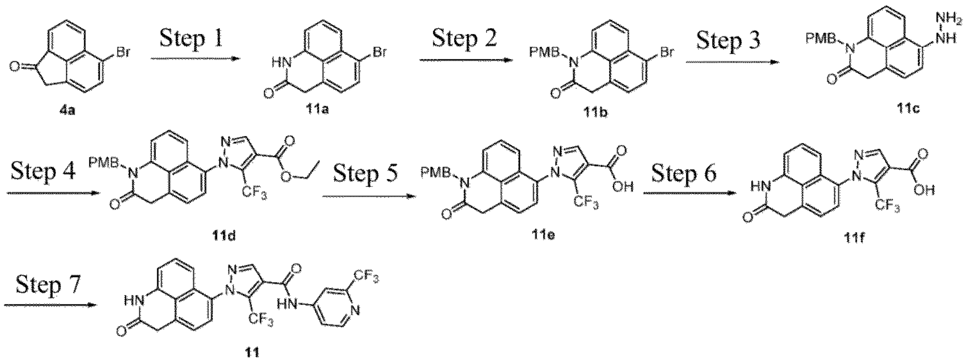

Step 1

6-bromo-1*H*-benzo[*de*]quinolin-2(3*H*)-one

5-bromoacenaphthylen-1(2H)-one **4a** (5.00 g, 20.23 mmol, synthesized by the well-known method in "Patent US6667303 B1") was dissolved in 20 mL of polyphosphoric acid solution, sodium azide (1.90 g, 29.23 mmol) was added under stirring at 55°C, and the mixture was reacted at 55°C for 2 h. The obtained reaction liquid was extracted with ethyl acetate (50 mL×3), the organic phases were combined, washed with water (50 mL×1) and saturated sodium chloride solution (50 mL×1) in sequence, dried over anhydrous sodium sulfate, and filtered, and the filtrate was concentrated under reduced pressure to obtain 6-bromo-1*H*-benzo[*de*]quinolin-2(3*H*)-one **11a** (3 g), yield: 56.7%.
MS m/z (ESI): 262 [M+1]

Referring to the synthesis method of step 5 in Example **2** and step 7 to step 11 in Example **1** in sequence, 1-(2-carbonyl-2, 3-dihydro-1*H*-benzo[de]quinolin-6-yl)-2-trifluoromethyl-N-(2-trifluoromethylpyridin-4-yl)-1*H*-pyrazol-3-carboxamide **11** (20 mg) was obtained through 6-bromo-1*H*-benzo[de]quinolin-2(3H)-one **11a** (1.05 g, 4.01 mmol), yield: 1.0%.

MS m/z (ESI): 505 [M+1].

**Example 12**

1-(3-carbonyl-2,3-dihydro-1*H*-benzo[*de*]isoquinolin-6-yl)-2-trifluoromethyl-*N*-(2-trifluoromethylpyridin-4-yl)-1*H*-pyrazol-3-carboxamide

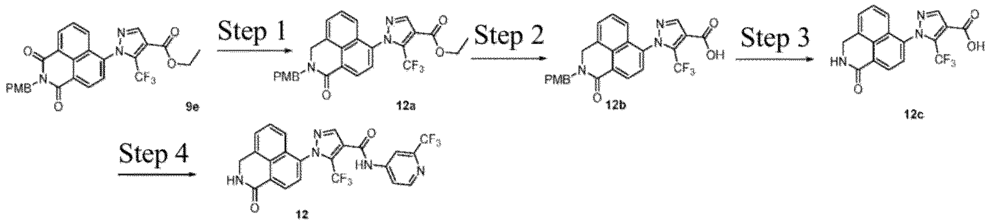


Referring to the synthesis method of step 1 in Examples **10** and step 9 to step 11 in Example **1** in sequence, 1-(3-carbonyl-2,3-dihydro-1*H*-benzo[de]isoquinolin-6-yl)-2-trifluoromethyl-N-(2-trifluoromethylpyridin-4-yl)-1*H*-pyrazol-3-carboxamide **12** (4 mg) was obtained through ethyl 1-(2-(4-methoxybenzyl)-1,3-dicarbonyl-2,3-dihydro-1*H*-benzo[*de*]isoquinolin-6-yl)-2-(trifluoromethyl)-1*H*-pyrrol-3-carboxylate **9e** (500 mg, 0.96 mmol), yield: 0.8%.

MS m/z (ESI): 505 [M+1]
$^1$H NMR (400 MHz, MeOD) δ 8.64 (d, *J* = 5.6 Hz, 1H), 8.45 - 8.31 (m, 2H), 8.25 (d, *J* = 2.0 Hz, 1H), 7.99 (dd, *J* = 5.6, 2.0 Hz, 1H), 7.80 (d, *J* = 7.6 Hz, 1H), 7.71- 7.58 (m, 2H), 7.18 (d, *J* = 7.8 Hz, 1H), 5.12 (s, 2H).

**Example 13**

*N*-(3,7-dichloropyrazolo[1,5-a]pyridin-5-yl)-1-(2-oxo-1,2-dihydrobenzo[cd]indol-6-yl)-5-trifluoromethyl-1*H*-pyrazole-4-carboxamide

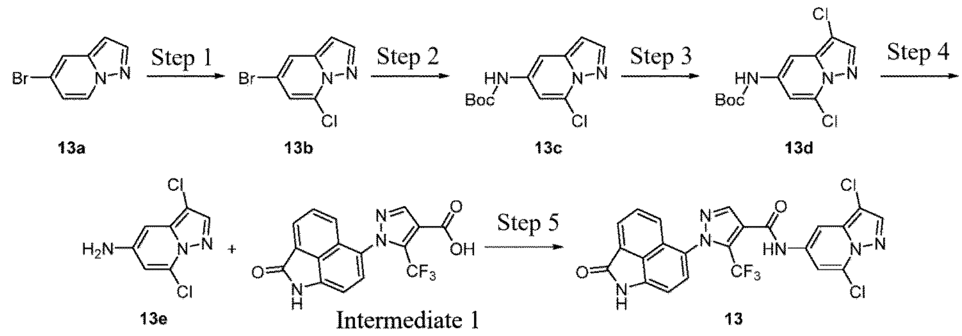

13a 13b 13c 13d 13e 13

Step 1

5-bromo-7-chloropyrazolo[1,5-*a*]pyridine

5-bromo-pyrazolo[1,5-a]pyridine **13a** (1.0 g, 5.08 mmol) was dissolved in 15 mL of dry tetrahydrofuran, 2 M solution of diisopropylaminolithium in tetrahydrofuran (3 mL, 6 mmol) was added dropwise at -78°C, then the mixture was reacted under stirring for 1 h at -78°C, 1 M solution of hexachloroethane in anhydrous tetrahydrofuran (6 mL, 6 mmol) was added dropwise at -78°C, and the mixture was reacted under stirring at -78°C for 1 h. 200 mL of saturated ammonium chloride solution was added to the reaction liquid, the obtained product was extracted with ethyl acetate (50 mL×3), the organic phases were combined, washed with saturated sodium chloride solution (30 mL×2), and dried over anhydrous sodium sulfate. The reaction liquid was filtered, the filtrate was concentrated under reduced pressure, and the obtained residue was purified by silica gel column chromatography with eluent system B to obtain the title product 5-bromo-7-chloropyrazolo[1,5-*a*]pyridine **13b** (750 mg), yield: 63.5%.
MS m/z (ESI): 231 [M+1]

Step 2

Tert-butyl N-(7-chloropyrazolo[1,5-*a*]pyridin-5-yl)-carbamate

5-bromo-7-chloropyrazolo[1,5-a]pyridine **13b** (750 mg, 3.24 mmol) was dissolved in 10 mL of 1,4-dioxane, tris(dibenzylideneacetone)dipalladium (149 mg, 0.16 mmol), 4,5-bisdiphenylphosphine-9,9-dimethylxanthene (184 mg, 0.32 mmol), cesium carbonate (3.16 g, 9.70 mmol) and tert-butyl carbamate (756 mg, 6.45 mmol) were added, and the mixture was reacted under stirring at 100°C for 1 h. The reaction liquid was filtered, and the filtrate was concentrated under reduced pressure. The obtained residue was purified by silica gel column chromatography with eluent system B to obtain the title product tert-butyl (7-chloropyrazolo[1,5-*a*]pyridin-5-yl)-carbamate **13c** (750 mg), yield: 86.5%.
MS m/z (ESI): 268 [M+1]

Step 3

Tert-butyl N-(3,7-dichloropyrazolo[1,5-*a*]pyridin-5-yl)-carbamate

Referring to the synthesis method of step 1 in Example 1, tert-butyl (3,7-dichloropyrazolo[1,5-*a*]pyridin-5-yl)-carbamate **13d** (400 mg) was obtained through tert-butyl (7-chloropyrazolo[1,5-*a*]pyridin-5-yl)-carbamate **13c**(750 mg, 2.79 mmol), yield: 47.5%.
MS m/z (ESI): 302, 304 [M+1]

Step 4

5-amino-3,7-dichloropyrazolo[1,5-*a*]pyridine

Tert-butyl (3,7-dichloropyrazolo[1,5-a]pyridin-5-yl)-carbamate **13d** (400 mg, 1.32 mmol) was dissolved in 5 mL a solution of 4 M chloride hydrogen in 1,4-dioxane, and the mixture was reacted under stirring at 60°C for 3 h. The reaction liquid was concentrated under reduced pressure to obtain the crude title product 5-amino-3,7-dichloropyrazolo[1,5-*a*]pyridine **13e** (366 mg). The product was directly used in the next reaction without purification.

MS m/z (ESI): 202, 204 [M+1].

Referring to the synthesis method of step 11 in Example 1, *N*-(3,7-dichloropyrazolo[1,5-*a*]pyridin-5-yl)-1-(2-oxo-1,2-dihydrobenzo[*cd*]indol-6-yl)-5-trifluoromethyl-1*H*-pyrazole-4-carboxamide **13** (16 mg) was obtained through intermediate **1** (31 mg, 0.089 mmol) and crude product 5-amino-3,7-dichloropyrazolo[ 1,5-*a*]pyridine **13e** (18 mg, 0.089 mmol), yield: 33.8%.

MS m/z (ESI): 531, 533 [M+1].

$^1$H NMR (400 MHz, DMSO-$d_6$) δ 11.12 (broad s, 1H), 11.03 (broad s, 1H), 8.52 (s,1H), 8.31 (s, 1H), 8.23 (d, 1H), 8.15 (d, 1H), 7.91 (t, 1H), 7.72 (d, 1H), 7.60 (d, 1H), 7.49 (d, 1H), 7.12 (d, 1H).

**Example 14**

*N*-(7-methyl-3-chloropyrazolo[1,5-*a*]pyridin-5-yl)-1-(2-oxo-1,2-dihydrobenzo[*cd*]indol-6-yl)-5-trifluoromethyl-1*H*-pyrazole-4-carboxamide

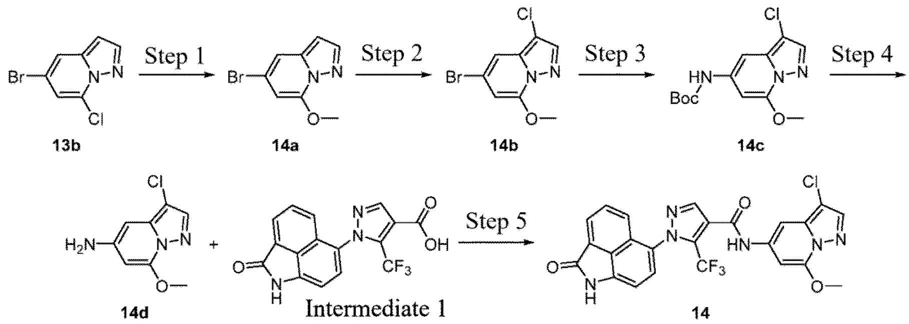


13b 14a 14b 14c

14d 14

Step 1

5-bromo-7-methoxypyrazolo[1,5-a]pyridine

5-bromo-7-chloropyrazolo[1,5-a]pyridine **13b** (320 mg, 1.38 mmol) was dissolved in 10 mL of methanol, and sodium methoxide (375 mg, 6.95 mmol) was added, the mixture was reacted under stirring at reflux for 5 h, the reaction liquid was concentrated under reduced pressure, and purified by silica gel column chromatography and the residue obtained was purified with eluent system B to obtain the title product 5-bromo-7-methoxy pyrazolo[1,5-*a*]pyridine **14a** (292 mg), yield: 93.2%.
MS m/z (ESI): 227 [M+1]

Referring to the synthesis method of step 1 in Example 1, step 2 and step 4 in Example 13, and the step 11 in Example 1 in sequence, *N*-(3,7-dichloropyrazolo[1,5-a]pyridin-5-yl)-1-(2-oxo-1,2-dihydrobenzo[cd]indol-6-yl)-5-trifluoromethyl-1*H*-pyrazole-4-carboxamide **14** (30 mg) was obtained through 5-bromo-7-methoxypyrazolo[1,5-*a*]pyridine **14a** (292 mg, 1.29 mmol), yield 4.4%.

MS m/z (ESI): 527, 529 [M+1].

$^1$H NMR (400 MHz, DMSO-$d_6$) δ 11.13 (broad s, 1H), 11.06 (broad s, 1H), 8.52 (s,1H), 8.31 (s, 1H), 8.23 (d, 1H), 8.15 (d, 1H), 7.91 (t, 1H), 7.72 (d, 1H), 7.60 (d, 1H), 7.49 (s, 1H), 7.12 (s, 1H), 4.35 (s, 3H).

**Example 15**

1-(2*H*-naphthalene [1,8-*bc*]furan-5-yl)-5-trifluoromethyl-*N*-(2-trifluoromethyl-pyridin-4-yl)-1*H*-pyrazole-4-carboxamide

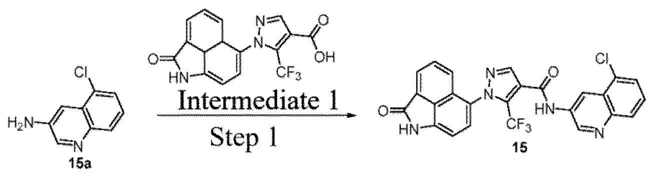


15a 15

Referring to the synthesis method of step 11 in Example 1, 1-(2H-naphthaleno[1,8-*bc*]furan-5-yl)-5-trifluoromethyl-*N*-(2-trifluoromethyl-pyridin-4-yl)-1*H*-pyrazole-4-carboxamide **15** (23 mg) was obtained through the reaction of 5-chloroquinolin-3-amine **15a** (20 mg, 0.11 mmol), yield: 41.0%.

MS m/z (ESI): 508 $[M+1]^+$.

$^1H$ NMR (400 MHz, DMSO-$d_6$) δ 11.21 (s, 1H), 11.12 (s, 1H), 9.23 (d, 1H), 9.16 (s, 1H), 8.58 (s, 1H), 8.16 (d, 1H), 8.03 (d, 1H), 7.95 - 7.89 (m, 1H), 7.83 (d, 1H), 7.75 - 7.67 (m, 2H), 7.62 (d, 1H), 7.13 (d, 1H).

**Example 16** *N*-(5-chloro-7-(trifluoromethyl)pyrrolo[1,2-*b*]pyridazin-3-yl)-1-(2-carbonyl-1,2-dihydrobenzo[*cd*]indol-6-yl)-5-trifluoromethyl-1*H*-pyrazole-4-carboxamide

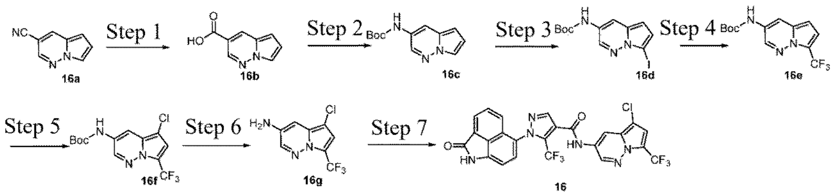


Step 1

Pyrrolo[1,2-*b*]pyridazin-3-carboxylic acid

**16a** (2.00 g, 14.0 mmol, synthesized using the well-known method in "Patent US6667303B1") was dissolved in 10 mL of concentrated sulfuric acid, and the mixture was reacted under stirring at 60°C for 12 h. 50 mL of water was added to the reaction liquid under ice bath to quench sodium hydrogen, and the obtained product was filtered to obtain the title product pyrrolo[1,2-*b*]pyridazin-3-carboxylic acid **16b** (1.20 g), yield: 53.0%.
MS m/z (ESI): 163 [M+1]

Step 2

Tert-butyl pyrrolo[1,2-*b*]pyridazine-3-carbamate

Pyrrolo[1,2-*b*]pyridazin-3-carboxylic acid **16b** (1.20 g, 7.40 mmol) was dissolved in 10 mL of tert-butanol, then triethylamine (2.20 g, 21.74 mmol) and diphenylphosphate azide (2.20 g, 7.99 mmol) were added and the mixture was reacted under stirring at 100°C for 2 h. The obtained reaction liquid was extracted with ethyl acetate (50 mL×3), the organic phases were combined, washed with water (50 mL×1) and saturated sodium chloride solution (50 mL×1) in sequence, dried over anhydrous sodium sulfate, and filtered, and the filtrate was concentrated under reduced pressure and the obtained residue was purified by silica gel column chromatography with eluent system A to obtain the title product tert-butyl pyrrolo[1,2-*b*]pyridazin-3-yl carbamate **16c** (800 mg), yield: 46.3%.
MS m/z (ESI): 234 [M+1]

Step 3

Tert-butyl 7-iodopyrrolo[1,2-*b*]pyridazine-3-carbamate

Tert-butyl pyrrolo[1,2-*b*]pyridazin-3-yl carbamate **16c** (800 mg, 3.43 mmol) was dissolved in 10 mL of tetrahydrofuran, *N*-iododibutyrimide (1.67 g, 7.42 mmol) was added, and the mixture was reacted under stirring at 100°C for 2 h. The obtained reaction liquid was extracted with ethyl acetate (50 mL×3), the organic phases were combined, washed with water (50 mL×1) and saturated sodium chloride solution (50 mL×1) in sequence, dried over anhydrous sodium sulfate, and filtered, and the filtrate was concentrated under reduced pressure and the obtained residue was purified by silica gel column chromatography with eluent system A to obtain the title product tert-butyl 7-iodopyrrolo[1,2-*b*]pyridazine-3-carbamate **16d** (800 mg), yield: 65.0%.
MS m/z (ESI): 360 [M+1]

Step 4

Tert-butyl 7-trifluoromethylpyrrolo[1,2-*b*]pyridazine-3-carbamate

Tert-butyl 7-iodopyrrolo[1,2-*b*]pyridazine-3-carbamate **16d** (800 mg, 2.23 mmol), copper iodide (850 mg, 4.46 mmol), methyl fluorosulfonyldifluoroacetate (865 mg, 4.50 mmol), 10 mLof N,N-dimethyl formamide were added to a three-necked flask, and the mixture was heated at 80°C and reacted for 12 h under nitrogen protection. The obtained residue was purified by silica gel column chromatography with eluent system A to obtain tert-butyl 5-trifluoromethylpyrazolo[1,2-*b*]pyridazine-3-carbamate **16e** (100 mg), yield: 14.9%.
MS m/z (ESI): 302 [M+1].
Referring to the synthesis method of step 2 and step 4 in Example 13 and the step 11 in Example 1 in sequence, *N*-(5-chloro-7-(trifluoromethyl)pyrrolo[1,2-*b*]pyridazin-3-yl)-1-(2-carbonyl-1,2-dihydrobenzo[*cd*]indol-6-yl)-5-trifluoromethyl-1H-pyrazole-4-carboxamide **16** (5 mg) was obtained through tert-butyl 5-trifluoromethylpyrazolo[1,2-*b*]pyridazine-3-carbamate **16e** (100 mg, 0.33 mmol), yield: 2.7%.
MS m/z (ESI): 565 [M+1].

**Example 17**

*N*-(7-chloro-5-(trifluoromethyl)pyrrolo[1,2-b]pyridazin-3-yl)-1-(2-carbonyl-1,2,2a1,5a-tetrahydrobenzo[*cd*]indol-6-yl)-5-(trifluoromethyl)-1H-pyrazole-4-carboxamide

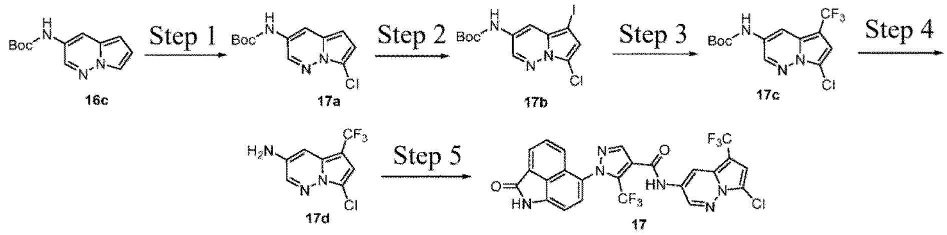

16c 17a 17b 17c 17d 17

Referring to the synthesis method of step 1 in Example 1, step 3 to step 4 in Example 16, step 4 in Example 13, and step 11 in Example 1, *N*-(7-chloro-5-(trifluoromethyl)pyrrolo[1,2-b]pyridazin-3-yl)-1-(2-carbonyl-1,2,2a$^1$,5a-tetrahydro benzo[*cd*]indol-6-yl)-5-(trifluoromethyl)-1H-pyrazole-4-carboxamide **17** (11 mg) was obtained through tert-butyl (7-chloropyrazolo[1,5-a]pyridin-5-yl)-carbamate **16c** (750 mg, 3.22 mmol), yield: 6.0%.
MS m/z (ESI): 565 [M+1].

**Example 18**

*N*-(6-(2*H*-1,2,3-triazol-2-yl)-5-trifluoromethylpyridin-3-yl)-1-(2-oxo-1,2-dihydrobenzo[*cd*]indol-6-yl)-5-trifluoromethyl-1*H*-pyrazole-4-carboxamide

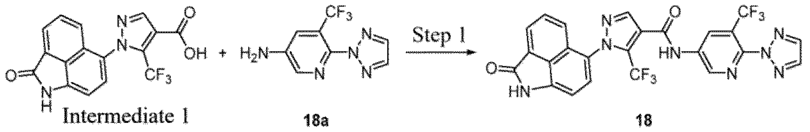

Intermediate 1 18a 18

Referring to the synthesis method in the step 11 in Example 1, the title product *N*-(6-(2H-1,2,3-triazol-2-yl)-5-trifluoromethylpyridin-3-yl)-1-(2-oxo-1,2-dihydrobenzo[*cd*]indol-6-yl)-5-trifluoromethyl-1*H*-pyrazole-4-carboxamide **18** (16 mg) was obtained through the intermediate **1** (18 mg, 0.052 mmol) and 6-(2*H*-1,2,3-triazol-2-yl)-3-amino-5-trifluoromethylpyridine **18a** (12 mg, 0.052 mmol, synthesized using the well-known method "Patent US20180170909A1"), yield 55.1%.

MS m/z (ESI): 559 [M+1]

$^1H$ NMR (400 MHz, DMSO-$d_6$) δ 11.4 (broad s, 1H), 11.1 (s, 1H), 9.18 (d, 1H), 8.90 (d, 1H), 8.57 (s, 1H), 8.21 (s, 2H), 8.15 (d, 1H), 7.91 (dd, 1H), 7.74 (d, 1H), 7.61 (d, 1H), 7.12 (d, 1H).

**Example 19**

1-(2-carbonyl-1,2,2a1,5a-tetrahydrobenzo[*cd*]indol-6-yl)-5-(trifluoromethyl)-*N*-(1-(trifluoromethyl)isoquinolin- 7-yl)-1H-pyrazole-4-carboxamide

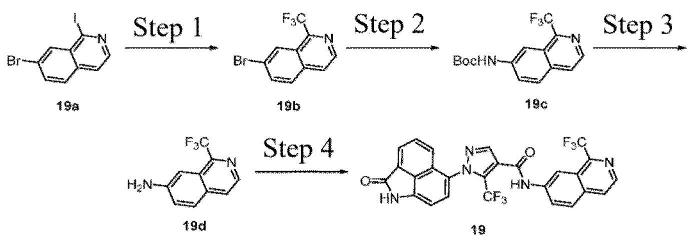


Step 1

7-bromo-1-(trifluoromethyl)isoquinoline

Referring to step 4 of example 16, step 2 and step 4 of example 13, and the step 11 of example 1 in sequence,

the title product 1-(2-carbonyl-1,2,2a1,5a-tetrahydrobenzo[*cd*]indol-6-yl)-5-(trifluoromethyl)-N-(1-(trifluoromethyl)isoquinolin- 7-yl)-1H-pyrazole-4-carboxamide **19** (0.11 g) was obtained through 7-bromo-1-iodoisoquinoline **19a** (1.00 g, 2.99 mmol), yield: 6.8%.
MS m/z (ESI): 544.4 [M+1]
$^1H$ NMR (400 MHz, DMSO-$d_6$): δ 11.33 (s, 1H), 10.82 (s, 1H), 8.32 (s, 1H),8.45 (d, 1H), 8.21 (d, 1H), 8.12 (d, 1H), 7.92 (d, 1H), 7.87 (d, 1H), 7.71 (d, 1H), 7.52 (d, 2H), 7.25 (d, 1H), 7.13(s, 1H), 6.83(m, 1H), 6.23 (m, 1H).

**Example 20**

*N*-(5-chloro-1-trifluoromethylisoquinolin- 7-yl)-1-(2-oxo-1,2-dihydrobenzo[*cd*]indol-6-yl)-5-trifluoromethyl-1*H*-pyrazole-4-carboxamide

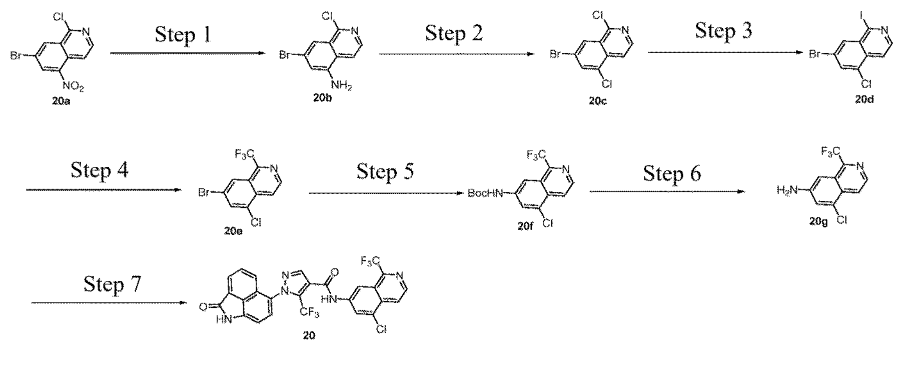


Step 1

7-bromo-1-chloroisoquinolin-5-amine

7-Bromo-1-chloro-5-nitroisoquinoline **20a** (3.50 g, 12.2 mmol) was dissolved in 30 mL of ethanol and 10 mL of water. Iron powder (6.83 g, 122 mmol) and ammonium chloride (6.81 g, 127.31 mmol) were added and the mixture was reacted at 60°C for 2 h. The reaction liquid was cooled to room temperature and filtered. The filtrate was extracted with

dichloromethane (50 mL×3), the organic phases were combined, washed with saturated sodium chloride solution (50 mL×2), dried over anhydrous sodium sulfate, filtered, and concentrated to obtain crude product 7-bromo-1-chloroisoquinolin-5-amine **20b** (2.51 g), yield, 80.1%.
MS m/z (ESI): 257 [M+1]

Step 2

7-bromo-1,5-dichloroisoquinoline

7-bromo-1-chloroisoquinolin-5-amine **20b** (2.51 g, 9.81 mmol) was dissolved in 30 mL of concentrated hydrochloric acid. Sodium nitrite (1.35 g, 19.62 mmol) was added at 0 °C. The mixture was reacted at 0°C for 1 h. Cuprous chloride (1.94 g, 19.62 mmol) was added. The mixture was reacted at room temperature for 2 h. The obtained mixture was filtered, and the filtrate was adjusted to pH 7 with 10 M sodium hydroxide solution. The obtained product was extracted with dichloromethane (50 mL×3). The organic phases were combined, washed with saturated sodium chloride solution (50 mL×2), dried over anhydrous sodium sulfate, filtered and concentrated to obtain a crude product, and the obtained residue was purified by silica gel column chromatography with eluent system A to obtain the title product 7-bromo-1,5-dichloroisoquinoline **20c** (1.1g), yield, 40.7%.
MS m/z (ESI): 276 [M+1]
Referring to the synthesis method of step 3 in Example 1, step 4 in Example 16, step 3 to step 4 in Example 14, and step 11 in Example 1 in sequence, *N*-(5-chloro-1-trifluoromethylisoquinolin-7-yl)-1-(2-oxo-1,2-dihydrobenzo[*cd*]indol-6-yl)-5-trifluoromethyl-1*H*-pyrazole-4-carboxamide **20** (45 mg) was obtained through the reaction of 7-bromo-1,5-dichloroisoquinoline **20c** (500 mg, 1.81 mmol), yield: 4.3%.

MS m/z (ESI): 576 [M+1]
$^1$H NMR (400 MHz, DMSO-$d_6$) δ11.41 (s, 1H), 11.15 (s, 1H), 9.27 (d, 1H), 9.19 (s, 1H), 8.60 (s, 1H), 8.16 (d, 1H), 7.95 - 7.89 (m, 1H), 7.82 (dd, 1H), 7.74 (d, 1H), 7.64 - 7.53 (m, 2H), 7.13 (d, 1H).

**Example 21** 1-(2-carbonyl-1,2,2a1,5a-tetrahydrobenzo[*cd*]indol-6-yl)-5-(trifluoromethyl)-*N*-(5-(trifluoromethyl)-4a,8a-dihydro-1,6-diazanaphthalen-3-yl)-1H-pyrazole-4-carboxamide

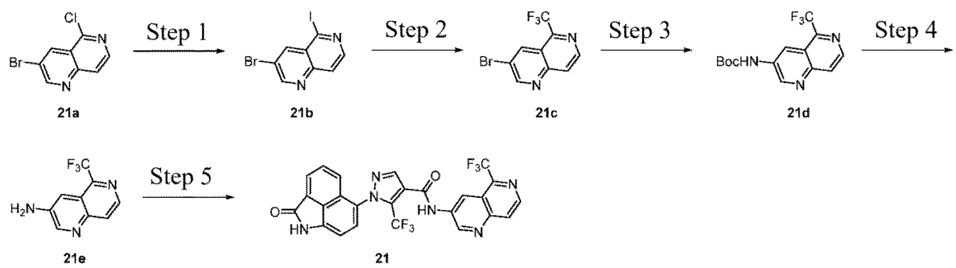

21a
21b
21c
21d
21e
21

Referring to the synthesis method of step 3 in Example 1, step 4 in Example 16, step 3 to step 4 in Example 14, and step 11 in Example 1 in sequence, 1-(2-carbonyl-1,2,2a1,5a-tetrahydrobenzo[cd]indol-6-yl)-5-(trifluoromethyl)-*N*-(5-(trifluoromethyl)-4a,8a-dihydro-1,6-diazanaphthalen-3-yl)-1H-pyrazole-4-carboxamide **21** (101 mg) was obtained through the reaction of 3-bromo-5-chloro-1,6-naphthyridine **21a** (1.00 g, 4.11 mmol), yield: 4.5%.
MS m/z (ESI): 547 [M+1].

**Example 22**

1-(2-carbonyl-1,2,2a1,5a-tetrahydrobenzo[*cd*]indol-6-yl)-5-(trifluoromethyl)-*N*-(4-(trifluoromethyl)quinazolin-6-yl)-1H-pyrazole-4-carboxamide

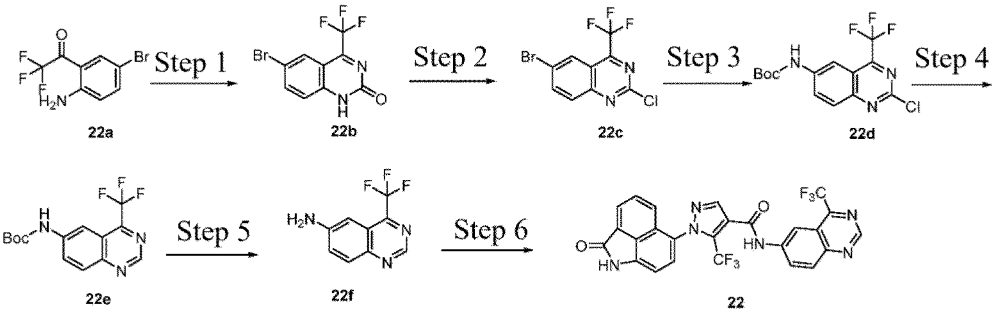


22a 22b 22c 22d 22e 22f 22

Step 1

6-bromo-4-(trifluoromethyl)quinazolin-2(1H)-one

4-bromo-2-trifluoroacetylanilide **22a** (1 g, 3.73 mmol, synthesized using the well-known method in "Patent US20140107096") and potassium cyanate (605 mg, 7.46 mmol) were dissolved in the mixed solvent of 1 mL of acetic acid and 10 mL of water., and the mixture was reacted under stirring at room temperature for 16 h. The reaction liquid was poured into 100 mL of water, the mixture was filtered and the filter residue was dried. The filter residue was dissolved in toluene, the mixture was heated at reflux for 4 h, and a water separator was used to remove water from the reaction liquid. After the reaction liquid was spun to dryness, the obtained residue was purified by silica gel column chromatography with eluent system A to obtain the title product 6-bromo-4-(trifluoromethyl)quinazolin-2(1H)-one **22b** (566 mg), yield: 51.7%.
MS m/z (ESI): 293 [M+1]

Step 2

6-bromo-2-chloro-4-trifluoromethylquinazoline

6-bromo-4-(trifluoromethyl)quinazolin-2(1H)-one **22b** (566 mg, 1.93 mmol) was dissolved in 10 mL of phosphorus oxychloride, the mixture was reacted at 105°C for 16 h. The reaction liquid was concentrated under reduced pressure in an anhydrous environment, and the obtained residue was purified by silica gel column chromatography with eluent system A to obtain the title product 6-bromo-2-chloro-4-(trifluoromethyl)quinazoline **22c** (573 mg), yield: 95.3%.
MS m/z (ESI): 311 [M+1]

Referring to the synthesis method of step 3 in Example 14, tert-butyl 2-chloro-4-trifluoromethyl-6-carbamate **22d** (321 mg) was obtained through 6-bromo-2-chloro-4-(trifluoromethyl)quinazoline **22c** (573 mg, 1.84 mmol), yield 50.2%: .

MS m/z (ESI): 348 [M+1].

Step 4

Tert-butyl 4-trifluoromethylquinazolin-6-carbamate

Tert-butyl 2-chloro-4-trifluoromethyl-6-carbamate **22d** (321 mg, 0.92 mmol) was dissolved in 10 mL of tetrahydrofuran, and palladium carbon (approximately 1 g, 10% purity) was added under nitrogen protection and the mixture was reacted at room temperature for 1 h. The reaction liquid was filtered through diatomaceous earth, and the filtrate was concentrated under reduced pressure. The obtained residue was purified by silica gel column chromatography with eluent system A to obtain the title product tert-butyl 4-trifluoromethyl-6-carbamate **22e** (220 mg), yield: 76.1%.

MS m/z (ESI): 314 [M+1].

Referring to the synthesis method of step 4 in Example 13 and the step 11 in Example 1 in sequence, 1-(2-carbonyl-1,2,2a1,5a-tetrahydrobenzo[*cd*]indol-6-yl)-5-(trifluoromethyl)-N-(4-(trifluoromethyl)quinazolin-6-yl)-1H-pyrazole-4-carboxamide **22** (8.3 mg) was obtained through tert-butyl 4-trifluoromethylquinazolin-6-carbamate **22e** (220 mg, 0.70 mmol), yield: 2.2%.

MS m/z (ESI): 545 [M+1].

**Example 23**

*N*-(3-chloro-1-(trifluoromethyl)isoquinolin- 7-yl)-1-(2-carbonyl-1,2,2a1,5a-tetrahydro benzo[cd]indol-6-yl)-5-(trifluoromethyl)-1H-pyrazole-4-carboxamide

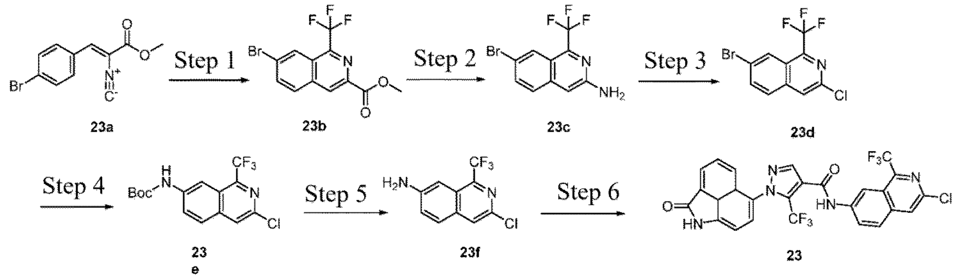


23a 23b 23c 23d 23e 23f 23

Step 1

Methyl 7-bromo-1-(trifluoromethyl)isoquinolin-3-carboxylate

Methyl (Z)-3-(4-bromophenyl)-2-isocyanoacrylate **23a** (1 g, 3.77 mmol, synthesized using the well-known method in "Wang, Hao; et al Chemical Communications (Cambridge, United Kingdom) (2014), 50(88), 13485-13488"), S-(trifluoromethyl)dibenzothiophenium tetrafluoroborate (1.92 g, 5.66 mmol), (4,4'-di-tert-butyl-2,2'-bipyridyl)bis[(2-pyridyl)phenyl]iridium(III) hexafluorophosphate (36 mg, 0.04 mmol) and disodium hydrogen phosphate (0.8 g, 5.66 mmol) were dissolved in 10 mL of ethanol and the mixture was reacted at room temperature for 3 h under irradiation with a 13W white LED lamp. The reaction liquid was concentrated under reduced pressure, and the obtained residue was purified by silica gel column chromatography with eluent system B to obtain the title product 7-bromo-3-(trifluoromethyl)isoquinolin-1(2H)-one **23b** (463 mg), yield: 36.9%.
MS m/z (ESI): 334 [M+1]
Referring to the synthesis method of step 2 in Example 16, 7-bromo-1-(trifluoromethyl)isoquinolin- 3-amine **23c** (238 mg) was obtained through methyl 7-bromo-1-(trifluoromethyl)isoquinolin- 3-carboxylate **23b** (463 mg, 1.39 mmol), yield: 59.1%.
MS m/z (ESI): 291 [M+1].

Step 3

7-bromo-3-chloro-1-(trifluoromethyl)isoquinoline

7-bromo-1-(trifluoromethyl)isoquinolin-3-amine **23c** (238 mg, 0.82 mmol) was dissolved in 3 mL of 2 M hydrochloric acid under ice bath, and sodium nitrite (67 mg, 0.98 mmol) was dissolved in 2 mL of water and added to the reaction liquid, and the mixture was reacted under stirring at room temperature for 1 h. Cuprous chloride (162 mg, 1.64 mmol) was dissolved in 2 mL of water and was added to the reaction liquid, and the mixture was heated at 65°C and reacted under stirring for 1 h. The reaction liquid was concentrated under reduced pressure, and the obtained residue was purified by silica gel column chromatography with eluent system B to obtain the title product 7-bromo-3-chloro-1-(trifluoromethyl)isoquinoline **23d** (114 mg), yield: 36.9%.
MS m/z (ESI): 310 [M+1]
Referring to the synthesis method of step 2 and step 4 in Example 13 and the step 11 in Example 1 in sequence, *N*-(3-chloro-1-(trifluoromethyl)isoquinolin-7-yl)-1-(2-carbonyl-1,2,2a1,5a-tetrahydrobenzo[cd]indol-6-yl)-5-(trifluoromethyl)-1H-pyrazole-4-carboxamide **23** (12 mg) was obtained through 7-bromo-3-chloro-1-(trifluoromethyl)isoquinoline **23d** (114 mg, 0.37 mmol), yield: 5.6%.
MS m/z (ESI): 578 [M+1].

**Example 24**

*N*-(4-methoxy-1-trifluoromethylisoquinolin-7-yl)-1-(2-oxo-1,2-dihydrobenzo[*cd*]indol-6-yl)-5-trifluoromethyl-1*H*-pyrazole-4-carboxamide

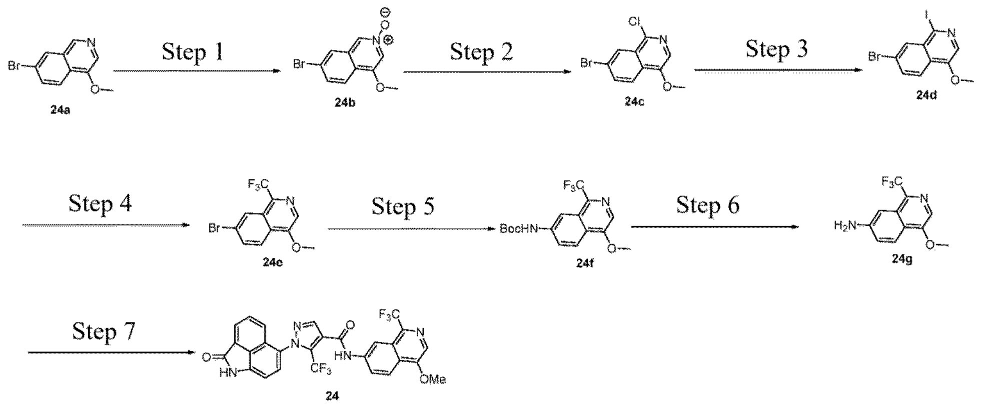


Step 1

7-bromo-4-methoxyisoquinolin-*N*-oxide

7-bromo-4-methoxyisoquinoline **24a** (1 g, 4.20 mmol) was dissolved in 30 mL of dichloromethane. M-chloroperoxybenzoic acid (860 mg, 5 mmol) was added and the mixture was reacted at room temperature for 2 h. The reaction liquid was washed with saturated sodium carbonate solution (50 mL×2) and sodium chloride solution (50 mL×2), dried over anhydrous sodium sulfate, filtered, and concentrated to obtain a crude product, and the obtained residue was purified by silica gel column chromatography with eluent system A to obtain the title product 7-bromo-4-methoxyisoquinolin-*N*-oxide ***24b*** (900 mg), yield: 84.3%. MS m/z (ESI): 254 [M+1]

Step 2

7-bromo-1-chloro-4-methoxyisoquinoline

7-bromo-4-methoxyisoquinolin-*N*-oxide **24b** (900 mg, 3.60 mmol) was dissolved in 10 mLof phosphorus oxychloride, and the mixture was reacted at 100°C for 2 h. The reaction liquid was concentrated to dryness, and the residue was poured into 50 mL of ice water. 10 *N* sodium hydroxide solution was used to adjust the pH to 7, the obtained mixture was filtered to obtain a crude product, and the obtained residue was purified by silica gel column chromatography with eluent system A to obtain the title product 7-bromo-1-chloro-4-methoxyisoquinoline **24c** (650 mg), yield: 67.3%.
MS m/z (ESI): 272 [M+1]

Referring to the synthesis method of step 3 in Example 1, step 4 in Example 16, step 3 to step 4 in Example 14, and step 11 in Example 1 in sequence, *N*-(5-chloro-1-trifluoromethylisoquinolin-7-yl)-1-(2-oxo-1,2-dihydrobenzo[*cd*]indol-6-yl)-5-trifluoromethyl-1H-pyrazole-4-carboxamide **24** (55 mg) was obtained through the reaction of 7-bromo-1-chloro-4-methoxyisoquinoline **24c** (500 mg, 1.83 mmol), yield: 5.3%.

MS m/z (ESI): 572 [M+1]
$^1$H NMR (400 MHz, DMSO-$d_6$) δ 11.11 (s, 1H), 10.87 (s, 1H), 9.27 (d, 1H), 9.19 (s, 1H), 8.60 (s, 1H), 8.46 (d, 1H), 8.01- 7.85 (m, 1H), 7.82 (s, 1H), 7.69 (d, 1H), 7.52 - 7.33 (m, 2H), 7.03 (d, 1H), 4.13 (s, 3H).

**Example 25**

*N*-(5,8-bis(trifluoromethyl)-1,6-diazanaphthalen-3-yl)-1-(2-carbonyl-1,2,2*a*$^1$,5*a*-tetrahydrobenzo[*cd*]indol-6-yl)-5-trifluoromethyl-1H-pyrazole-4-carboxamide

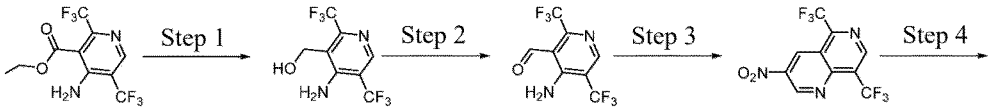


25a 25b 25c 25d

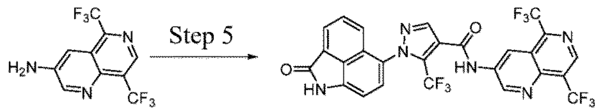


25e 25

Step 1

(4-amino-2,5-bis(trifluoromethyl)pyridin-3-yl)methanol

Ethyl 4-amino-2,5-bis(trifluoromethyl) nicotinate **25a** (1.00 g, 3.31 mmol) was dissolved in 10 mL of tetrahydrofuran, nitrogen replacement was performed three times, and the system was cooled to 0°C in ice water bath, a solution of lithium tetrahydroaluminum in tetrahydrofuran (6.62 mL, 6.62 mmol) was added dropwise to the reaction system, and the reaction system was reacted under stirring at 0°C for 1 h. 0.5 mL of 20% aqueous sodium hydroxide solution was added to quench the reaction, the reaction liquid was filtered, the filtrate was spun to dryness, the filter residue was washed with water (20 mL×2), the filter residue filtrate was concentrated under reduced pressure, and the obtained residue was purified silica gel column chromatography with eluent system A to obtain the title product (4-amino-2,5-bis(trifluoromethyl)pyridin-3-yl)methanol **25b** (0.45 g), yield: 52.3%.
MS m/z (ESI): 261.1 [M+1]

Step 2

4-amino-2,5-bis(trifluoromethyl)nicotinaldehyde

(4-Amino-2,5-bis(trifluoromethyl)pyridin-3-yl)methanol **25b** (0.45 g, 1.73 mmol) was dissolved in 10 mL of dichloromethane, manganese dioxide (1.50 g, 17.30 mmol) was added, and the mixture was reacted under stirring for 2 h. The reaction liquid was filtered, and the filtrate was concentrated under reduced pressure to obtain the title product 4-amino-2,5-bis(trifluoromethyl)nicotinaldehyde **25c** (0.33g), yield: 73.9%.
MS m/z (ESI): 259.2 [M+1]

Step 3

3-nitro-5,8-bis(trifluoromethyl)-1,6-diazanaphthalene

10 M sodium hydroxide solution (3.0 mL, 30 mmol) was added to a reaction flask which was cooled to 0°C in an ice-water bath. Nitromethane (0.5 mL, 9.25 mmol) was added dropwise, and the system was reacted under stirring at 0°C for 15 min, and then reacted at room temperature for 20 min. The reaction system was placed in a room temperature water bath. Nitromethane (0.5 mL, 9.25 mmol) was slowly added dropwise to the system. The system was stirred and reacted at room temperature for 25 min and then poured into 50 mL of ice water. 3 mL of concentrated hydrochloric acid and 50 mL diethyl ether was added, the system was stirred until the ice cubes melt, liquid separation was performed, and then drying over anhydrous sodium sulfate and filtration were performed. The filtrate, 25 mL of ice water and 5 mL of 5 M hydrochloric acid were mixed, and 4-amino-2,5-bis(trifluoromethyl)nicotinaldehyde 25c (0.32 g, 1.24 mmol) was dissolved in a mixed solvent of 35 mL of ethanol, 30 mL of water and 1 mL of 5 M hydrochloric acid, and then the mixture was added dropwise to the reaction system. After the addition, the reaction temperature was raised to 50°C and the mixture was reacted under stirring for 16 h. The reaction liquid was concentrated under reduced pressure to 20 mL, extracted with ethyl acetate (30 mL×3), the organic phases were combined, washed with saturated sodium chloride solution (20 mL×1), dried over anhydrous sodium sulfate, and filtered, and the filtrate was concentrated under reduced pressure and the obtained residue was purified by silica gel column chromatography with eluent system A to obtain the title product 3-nitro-5,8-bis(trifluoromethyl)-1,6-diazanaphthalene **25d** (0.18 g), yield: 46.7%.
MS m/z (ESI): 312.4 [M+1]

Step 4

5,8-bis(trifluoromethyl)-1,6-diazanaphthalen-3-amine

3-nitro-5,8-bis(trifluoromethyl)-1,6-diazanaphthalene **25d** (0.18 g, 0.58 mmol), iron powder (0.16 g, 2.89 mmol) and ammonium chloride (0.15 g, 2.89 mmol) were dissolved in a mixed solvent of 3 mL of ethanol, 3 mL of tetrahydrofuran and 2 mL of water, and the mixture was reacted under stirring at 80°C for 2 h. The reaction liquid was filtered and concentrated to obtain the title product 5,8-bis(trifluoromethyl)-1,6-diazanaphthalen-3-amine **25e** (110 mg), yield: 67.5%.
MS m/z (ESI): 282.1 [M+1]

Referring to the synthesis method of step 11 in Example 1, *N*-(5,8-bis(trifluoromethyl)-1,6-diazanaphthalen-3-yl)-1-(2-carbonyl-1,2,2*a*$^1$,5*a*-tetrahydrobenzo[*cd*]indol-6-yl)-5-trifluoromethyl-1H-pyrazole-4-carboxamide **25** (36 mg) was obtained through 5,8-bis(trifluoromethyl)-1,6-diazanaphthalen-3-amine **25e** (60 mg, 0.21 mmol), yield: 28.0%.

MS m/z (ESI): 613.1 [M+1]
$^1$H NMR (400 MHz, DMSO-$d_6$): $\delta$ 11.28 (s, 1H), 11.12 (s, 1H), 9.47 (d, 1H), 8.82 (s, 1H), 8.53 (s, 1H),8.47 (d, 1H), 8.25 (d, 1H), 8.15 (d, 1H), 7.98 (d, 1H), 7.92 (d, 1H), 7.72 (d, 1H), 7.60 (d, 2H).

**Example 26**

*N*-(4-trifluoromethoxy-1-trifluoromethylisoquinolin-7-yl)-1-(2-oxo-1,2-dihydrobenzo[*cd*]indol-6-yl)-5-trifluoromethyl-1*H*-pyrazole-4-carboxamide

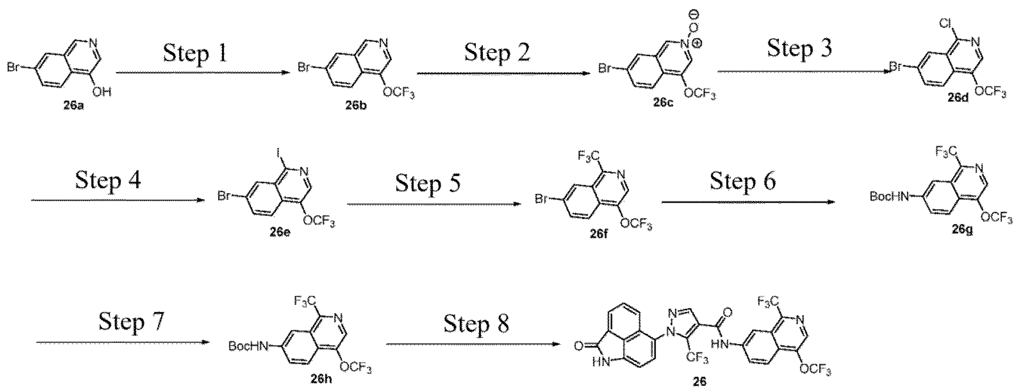

26a 26b 26c 26d 26e 26f 26g 26h 26

Step 1

7-bromo-4-trifluoromethoxyisoquinoline

7-bromo-4-hydroxylisoquinoline **26a** (2 g, 8.91 mmol) was dissolved in 20 mL of *N,N*-dimethylformamide. Potassium fluoride (1.09 g, 18.82 mmol), copper iodide (3.97 g, 20.89 mmol) and 1-trifluoromethyl-1,2-phenyliodoyl-3(1*H*)-one (11.3 g, 35.60 mmol) were added, and the mixture was reacted at 90°C for 16 h. The reaction liquid was cooled to room temperature and poured into 200 mL of water. The obtained mixture was filtered, the filtrate was extracted with ethyl acetate (50 mL×3), the organic phases were combined, washed with saturated sodium chloride solution (50 mL×2), dried over anhydrous sodium sulfate, filtered and concentrated to obtain a crude product, and the obtained residue was purified by silica gel column chromatography with eluent system A to obtain the title product 7-bromo-4-trifluoromethoxyisoquinoline **26b** (1.20 g), yield: 46.2%.
MS m/z (ESI): 292 [M+1]

Referring to the synthesis method of step 1 to step 2 in Example 24, step 3 in Example 1, step 4 in Example 16, step 3 to step 4 in Example 14, and step 11 in Example 1 in sequence, *N*-(4-trifluoromethoxy-1-trifluoromethylisoquinolin-7-yl)-1-(2-oxo-1,2-dihydrobenzo[*cd*]indol-6-yl)-5-trifluoromethyl-1*H*-pyrazole-4-carboxamide **26** (45 mg) was obtained through 7-bromo-4-trifluoromethoxyisoquinoline **26b** (1.20 g, 4.11 mmol), yield: 1.8%.

MS m/z (ESI): 626 [M+1]
$^1$H NMR (400 MHz, DMSO-$d_6$) $\delta$ 11.31 (s, 1H), 11.17 (s, 1H), 9.47 (d, 1H), 9.39 (s, 1H), 8.80 (s, 1H), 8.66 (d, 1H),

8.21 - 7.95 (m, 1H), 7.92 (s, 1H), 7.75 (d, 1H), 7.52 - 7.33 (m, 2H), 7.03 (d, 1H).

**Example 27**

1-(2-carbonyl-1,2-dihydrobenzo[cd]indol-6-yl)-5-(trifluoromethyl)-N-(2-(trifluoromethyl)pyridin-4-yl)-1H-pyrazole-4-carboxamide

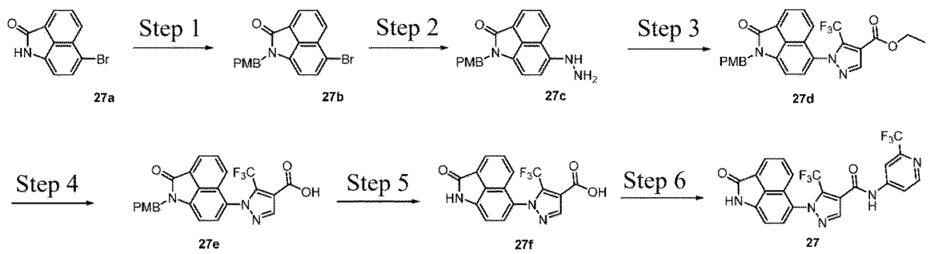


Step 1

6-bromo-1-(4-methoxybenzyl)benzo[cd]indol-2(1H)-one

6-bromobenzo[cd]indol-2(1H)-one (1 g, 4.10 mmol) was dissolved in 20 ml of acetonitrile, 4-methoxybenzylbromine (1.22 g, 6.15 mmol) and potassium carbonate (1.13 g, 8.2 mmol) were added and the mixture was reacted under stirring at room temperature for 16 h. The reaction liquid was filtered, and the filtrate was concentrated under reduced pressure. The obtained residue was purified by silica gel column chromatography with eluent system A to obtain the title product 6-bromo-1-(4-methoxybenzyl)benzo[cd]indol-2(1H)-one **27b** (1.07 g), yield: 71.0%.
MS m/z (ESI): 368 [M+1].
Referring to the synthesis method of step 7 to step 11 in Example 1, 1-(2-carbonyl-1,2-dihydrobenzo[cd]indol-6-yl)-5-(trifluoromethyl)-N-(2-(trifluoromethyl)pyridin-4-yl)-1H-pyrazole-4-carboxamide **27** (20 mg) was obtained.

MS m/z (ESI): 492 [M+1]
1H NMR (400 MHz, DMSO-$d_6$) δ 11.27 (s, 1H), 11.12 (s, 1H), 8.72 (d, 1H), 8.54 (s, 1H), 8.25 (d, 1H), 8.15 (d, 1H), 7.99 (dd, 1H), 7.91 (dd, 1H), 7.72 (d, 1H), 7.60 (d, 1H), 7.12 (d, 1H).

**Example 28**

N-(2-(difluoromethyl)pyridin-4-yl)-1-(2-carbonyl-1,2-dihydrobenzo[cd]indol-6-yl)-5-(trifluoromethyl)-1H-pyrazole-4-carboxamide

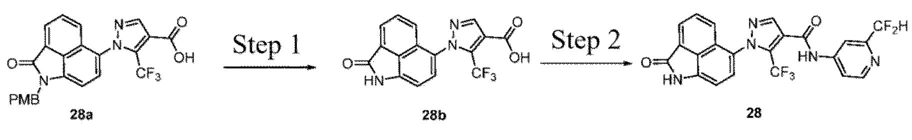


Referring to the synthesis method of step 5 to step 6 in Example 27, N-(2-(difluoromethyl)pyridin-4-yl)-1-(2-carbonyl-1,2-dihydrobenzo[cd]indol-6-yl)-5-(trifluoromethyl)-1H-pyrazole-4-carboxamide **28** (5 mg) was obtained from 1-(1-(4-methoxybenzyl)-2-carbonyl-1,2-dihydrobenzo[cd]indol-6-yl)-5-(trifluoromethyl)-1H-pyrazole-4-carboxylic acid **28a** (50 mg, 0.107 mmol), yield: 9.9%.
MS m/z (ESI): 474[M+1].

**Example 29**

1-(2-carbonyl-1,2-dihydrobenzo[cd]indol-6-yl)-5-(trifluoromethyl)-N-(6-(trifluoromethyl)pyridazin-4-yl)-1H-pyrazole-4-carboxamide

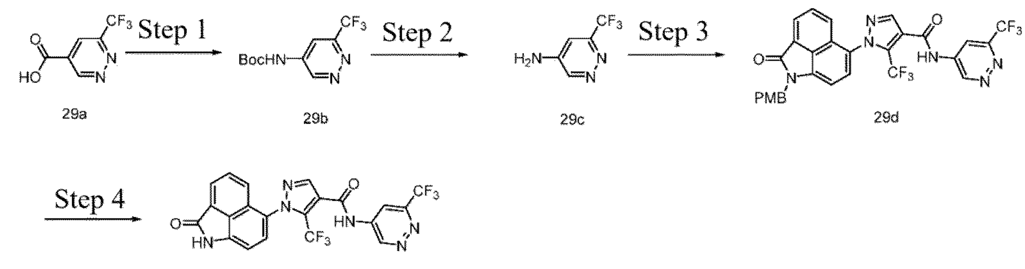


29a 29b 29c 29d

29

Step 1

Tert-butyl 6-(trifluoromethyl)pyridazin-4-carbamate

Methyl 6-(trifluoromethyl)pyridazin-4-carboxylic acid 29a (200 mg, 1.04 mmol, obtained by the well-known method patent "US20200347052") was dissolved in 10 mL of tert-butanol. Diphenylphosphate azide (573 mg, 2.08 mmol) and triethylamine (210 mg, 2.08 mmol) were added and the reaction was carried out at 100°C for 2 h. The reaction liquid was cooled to room temperature and the solvent was spun to dryness. 20 mL was added and extracted with ethyl acetate (20 mL×3), the organic phases were combined, washed with saturated sodium chloride solution (10 mL×2), dried over anhydrous sodium sulfate, filtered, and concentrated to obtain a crude product, and the obtained residue was purified by silica gel column chromatography with eluent system A to obtain the title tert-butyl 6-(trifluoromethyl)pyridazin-4-carbamate 29b (40 mg), yield: 14.6%.
MS m/z (ESI): 264 [M+1]

Step 2

6-(trifluoromethyl)pyridazin-4-amine

Tert-butyl 6-(trifluoromethyl)pyridazin-4-carbamate **29b**(40 mg, 0.15 mmol) was dissolved in 2 mL of ethanol and 1 mL (4M) hydrochloric acid, and the mixture was reacted at 20°C for 2 h. Concentration was performed to give the title product 6-(trifluoromethyl)pyridazin-4-amine **29c** (20 mg), yield: 80.7%.
MS m/z (ESI): 164 [M+1]

Step 3

1-(1-(4-methoxybenzyl)-2-carbonyl-1,2-dihydrobenzo[cd]indol-6-yl)-5-(trifluoromethyl)-N-(6-(trifluoromethyl)pyridazin-4-yl)-1H-pyrazole-4-carboxamide

6-(Trifluoromethyl)pyridazin-4-amine **29c** (20 mg, 0.123 mmol) and 1-(1-(4-methoxybenzyl)-2-carbonyl-1,2-dihydrobenzo[cd]indol-6-yl)-5-(trifluoromethyl)-1H-pyrazole-4-carboxylic acid (60 mg, 0.13 mmol) were dissolved in 10 mL of dichloromethane. Phosphorus oxychloride (60 mg, 0.391 mmol) and pyridine (30 mg, 0.379 mmol) were added and the reaction was carried out at 20°C for 2 h. The reaction liquid was cooled to room temperature and the solvent was spun to dryness. 20 mL was added and extracted with ethyl acetate (20 mL×3), the organic phases were combined, washed with saturated sodium chloride solution (10 mL×2), dried over anhydrous sodium sulfate, filtered, and concentrated to obtain a crude product, and the obtained residue was purified by silica gel column chromatography with eluent system A to obtain the title 1-(1-(4-methoxybenzyl)-2-carbonyl-1,2-dihydrobenzo[cd]indol-6-yl)-5-(trifluoromethyl)-N-(6-(trifluoromethyl)pyridazin-4-yl)-1H-pyrazole-4-carboxamide **29d** (4 mg), yield: 5.3%.
MS m/z (ESI): 613 [M+1].

Step 4

1-(2-carbonyl-1,2-dihydrobenzo[cd]indol-6-yl)-5-(trifluoromethyl)-N-(6-(trifluoromethyl)pyridazin-4-yl)-1H-pyrazole-4-carboxamide

1-(1-(4-methoxybenzyl)-2-carbonyl-1,2-dihydrobenzo[cd]indol-6-yl)-5-(trifluoromethyl)-N-(6-(trifluoromethyl)pyridazin-4-yl)-1H-pyrazole-4-carboxamide **29d** (4 mg, 0.0065 mmol) was dissolved in 3 mL of trifluoroacetic acid and the mixture was reacted at 100°C for 12 h. The reaction liquid was cooled to room temperature and the solvent was spun to dryness. 20 mL was added and extracted with ethyl acetate (20 mL×3), the organic phases were combined, washed with saturated sodium chloride solution (10 mL×2), dried over anhydrous sodium sulfate, filtered, and concentrated to obtain a crude product, and the obtained residue was purified by silica gel column chromatography with eluent system A to obtain the title 1-(1-(4-methoxybenzyl)-2-carbonyl-1,2-dihydrobenzo[cd]indol-6-yl)-5-(trifluoromethyl)-N-(6-(trifluoromethyl)pyridazin-4-yl)-1H-pyrazole-4-carboxamide **29** (2 mg), yield: 62.2%.

MS m/z (ESI): 493 [M+1]
1H NMR (400 MHz, DMSO-$d_6$) δ 11.27 (s, 1H), 11.12 (s, 1H), 8.72 (s, 1H), 8.54 (s, 1H), 8.15 (d, 1H), 7.99 (dd, 1H), 7.91 (dd, 1H), 7.72 (d, 1H), 7.60 (d, 1H), 7.12 (d, 1H).

**Example 30**

N-(6-((dimethyl(carbonyl)-6-sulfanylidene)amino)-5-(trifluoromethyl)pyridin-3-yl)-1-(2-carbonyl-1,2-dihydrobenzo[cd]indol-6-yl)-5-(trifluoromethyl)-1H-pyrazole-4-carboxamide

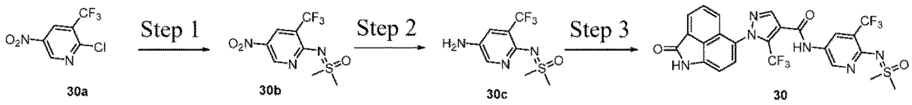


30a 30b 30c 30

Step 1

Dimethyl((5-nitro-3-(trifluoromethyl)pyridin-2-yl)imino)-6-sulfanone

2-chloro-5-nitro-3-(trifluoromethyl)pyridine 30a (150 mg, 0.66 mmol), dimethylsulfoximine (67 mg, 0.73 mmol), tris(dibenzylideneacetone)dipalladium (60 mg, 0.06 mmol), 4,5-bisdiphenylphosphine-9,9-dimethylxanthene (38 mg, 0.06 mmol) and cesium carbonate (323 mg, 0.99 mmol) were dissolved in 5 mL of tetrahydrofuran, and the mixture was heated and reacted at 60°C for 16 h under nitrogen protection. The reaction liquid was concentrated under reduced pressure, and the obtained residue was purified by silica gel column chromatography with eluent system A to obtain the title product dimethyl((5-nitro-3-(trifluoromethyl)pyridin-2-yl)imino)-6-sulfanone **30b**(100 mg), yield: 53.3%.
MS m/z (ESI): 284 [M+1]

Step 2

Dimethyl((5-amino-3-(trifluoromethyl)pyridin-2-yl)imino)-6-sulfanone

Dimethyl((5-nitro-3-(trifluoromethyl)pyridin-2-yl) imino)-6-sulfane **30b** (100 mg, 0.35 mmol) was dissolved in 5 mL of tetrahydrofuran, 5% palladium on carbon (375 mg) was added under nitrogen protection, and the mixture was reacted under stirring at room temperature for 1 h in a hydrogen atmosphere. The reaction liquid was filtered, and the filtrate was concentrated under reduced pressure to obtain the title product dimethyl((5-amino-3-(trifluoromethyl)pyridin-2-yl) imino)-6-sulfanone **30c** (79 mg), yield: 88.9%.
MS m/z (ESI): 254 [M+1]
Referring to the synthesis method of step 11 in Example 1, N-(6-((dimethyl(carbonyl)-6-sulfanylidene)amino)-5-(trifluoromethyl)pyridin-3-yl)-1-(2-carbonyl-1,2-dihydrobenzo[cd]indol-6-yl)-5-(trifluoromethyl)-1H-pyrazole-4-carboxamide **30** (27 mg) was obtained through dimethyl((5-amino-3-(trifluoromethyl)pyridin-2-yl)imino)-6-sulfanone **30c** (38 mg, 0.15 mmol), yield: 31.1%.

MS m/z (ESI): 583.1 [M+1]

1H NMR (400 MHz, DMSO) δ 11.11 (s, 1H), 10.71 (s, 1H), 8.64 (d, J = 2.6 Hz, 1H), 8.46 (s, 1H), 8.32 (d, J = 2.6 Hz, 1H), 8.15 (d, J = 7.0 Hz, 1H), 7.90 (dd, J = 8.3, 7.0 Hz, 1H), 7.70 (d, J = 7.5 Hz, 1H), 7.59 (d, J = 8.3 Hz, 1H), 7.11 (d, J = 7.5 Hz, 1H), 3.44 (s, 6H).

**Example 31**

N-(6-(1H-pyrazol-1-yl)-5-(trifluoromethyl)pyridin-3-yl)-1-(2-carbonyl-1,2-dihydrobenzo[cd]indol-6-yl)-5-(trifluoromethyl)-1H-pyrazole-4-carboxamide

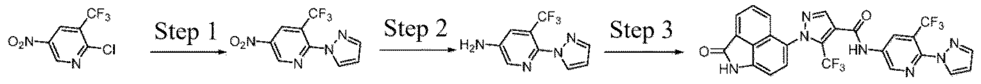


31a 31b 31c 31

Referring to the synthesis method of step 1 to step 2 in Example 30 and step 11 in Example 1 in sequence, N-(6-(1H-pyrazol-1-yl)-5-(trifluoromethyl)pyridin-3-yl)-1-(2-carbonyl-1,2-dihydrobenzo[cd]indol-6-yl)-5-(trifluoromethyl)-1H-pyrazole-4-carboxamide **31** (27 mg) was obtained through the reaction of 2-chloro-5-nitro-3-(trifluoromethyl)pyridine **31a** (150 mg, 0.66 mmol), yield: 7.3%.

MS m/z (ESI): 558 [M+1].

1H NMR (400 MHz, DMSO) δ 11.64 (d, J = 5.8 Hz, 1H), 11.26 (s, 1H), 8.72 (d, J = 5.5 Hz, 1H), 8.53 (s, 1H), 8.45 (dt, J = 8.0, 1.1 Hz, 1H), 8.24 (d, J = 2.0 Hz, 1H), 7.96 (ddd, J = 9.0, 6.5, 1.6 Hz, 2H), 7.68 (t, J = 7.9 Hz, 1H), 7.30 (dd, J = 7.3, 5.8 Hz, 1H), 5.65 (d, J = 7.3 Hz, 1H).

**Example 32**

*N*-(6-(5-cyano-1*H*-pyrazol-1-yl)-(5-trifluoromethylpyridin-3-yl)-1-(2-oxo-1,2-dihydrobenzo[*cd*]indol-6-yl)-5-trifluoromethyl-1*H*-pyrazole-4-carboxamide

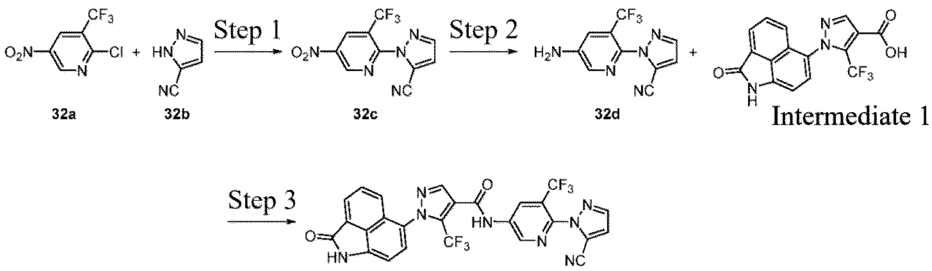


32a 32b 32c 32d Intermediate 1

32

Step 1

1-(5-nitro-3-trifluoromethylpyridin-2-yl)-5-cyano-1*H*-pyrazole

2-chloro-5-nitro3-trifluoromethylpyridine **32a** (2.26 g, 10 mmol) was dissolved in 25 mLof acetonitrile, 5-cyano-1*H*-pyrazole **32b**(0.8 mL, 11 mmol) and potassium carbonate (1.79 g, 13 mmol) were added, and the mixture was reacted under stirring at 40°C for 12 h. The reaction liquid was concentrated under reduced pressure, and the obtained residue was purified by silica gel column chromatography with eluent system A (PE/EA=2:1) to obtain the title product 1-(5-nitro-3-trifluoromethylpyridin-2-yl)-5-cyano-1*H*-pyrazole **32c**(1.15 g), yield: 40.6%.
MS m/z (ESI): 284 [M+1]

Step 2

1-(5-amino-3-trifluoromethylpyridin-2-yl)-5-cyano-1*H*-pyrazole

1-(5-nitro-3-trifluoromethylpyridin-2-yl)-5-cyano-1*H*-pyrazole **32c** (1.15 g, 4.06 mmol) was dispersed in a mixed solvent of 18 mL of ethanol and 3 mL of water, iron powder (1.36 g, 24.36 mmol) and ammonium chloride (1.30 g, 24.36 mmol) were added, and the mixture was reacted under stirring at 80°C for 1 h. The obtained product was cooled to room temperature, the reaction liquid was concentrated under reduced pressure, 30 mL of ethyl acetate was added to the obtained residue to fully dissolve the residue, and the obtained mixture was filtered, and the filtrate was concentrated under reduced pressure to obtain the title product crude 1-(5-amino-3-trifluoromethylpyridin-2-yl)-5-cyano-1*H*-pyrazole **32d** (0.88 g), the product was directly used in the next reaction without purification.
MS m/z (ESI): 254 [M+1]

Step 3

*N*-(6-(5-cyano-1*H*-pyrazol-1-yl)-(5-trifluoromethylpyridin-3-yl)-1-(2-oxo-1,2-dihydrobenzo[*cd*]indol-6-yl)-5-trifluoromethyl-1*H*-pyrazole-4-carboxamide

Referring to the synthesis method of the step 11 in Example 1, the title product *N*-(6-(5-cyano-1*H*-pyrazol-1-yl)-(5-trifluoromethylpyridin-3-yl)-1-(2-oxo-1,2-dihydrobenzo[cd]indol-6-yl)-5-trifluoromethyl-1*H*-pyrazole-4-carboxamide (10 mg) was obtained through the crude 1-(5-amino-3-trifluoromethylpyridin-2-yl)-5-cyano-1*H*-pyrazole **32d** (40 mg, 0.16 mmol) and intermediate **1** (56 mg, 0.16 mmol), yield: 10.7%.

MS m/z (ESI): 583 [M+1]
$^1$H NMR (400 MHz, DMSO-$d_6$) δ 11.44 (br. s, 1H), 11.14 (s, 1H), 9.16 (d, 1H), 8.88 (d, 1H), 8.61 (d, 1H), 8.57 (s, 1H), 8.16 (d, 1H), 7.92 (t, 1H), 7.74 (d, 1H), 7.61 (t, 1H), 7.32 (t, 1H), 7.13 (t, 1H).

**Example 33**

1-(2-oxo-1,2-1,2-dihydrobenzo[*cd*]indol-6-yl)-*N*-(6-(thiazol-2-yl)-5-(trifluoromethyl)pyridin-3-yl)-5-(trifluoromethyl)-1*H*-pyrazole-4-carboxamide

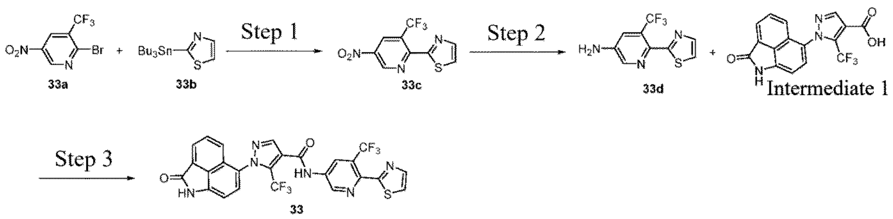


Step 1

2-(5-nitro-3-(trifluoromethyl)pyridin-2-yl)thiazole

2-Bromo-5-nitro-3-(trifluoromethyl)pyridine **33a** (1 g, 3.70 mmol) and 2-(tri-n-butylstannyl)thiazole **33b** (1.87 g, 4.99 mmol) was dissolved in 10 mL of toluene, tetrakis(trisphenylphosphine)palladium (577 mg, 0.50 mmol) was added, and the mixture was reacted under stirring at 100°C for 2 h. The reaction liquid was filtered, and the filtrate was concentrated under reduced pressure. The obtained residue was purified by silica gel column chromatography with eluent system A to obtain the title product 2-(5-nitro-3-(trifluoromethyl)pyridin-2-yl)thiazole **33c** (510 mg), yield: 49.8%.
MS m/z(ESI): 276 [M+1].
Referring to the synthesis method of step 4 in Example **25** and step 11 in Example 1, 1-(2-oxo-1,2-1,2-dihydrobenzo[*cd*]indol-6-yl)-*N*-(6-(thiazol-2-yl)-5-(trifluoromethyl)pyridin-3-yl)-5-(trifluoromethyl)-1*H*-pyrazole-4-carboxamide **33** (31 mg) was obtained through 2-(5-nitro-3-(trifluoromethyl)pyridin-2-yl)thiazole **33c** (510 mg, 1.85 mmol), yield: 2.9%.

MS m/z(ESI): 575 [M+1]

$^1H$ NMR(400 MHz, DMSO-$d_6$)δ 11.31(s, 1H), 11.13(s, 1H), 9.21(d, *J* = 2.1 Hz, 1H), 8.80(d, *J* = 2.1 Hz, 1H), 8.55(s, 1H), 8.15(d, *J* = 6.9 Hz, 1H), 8.07(d, *J* = 3.2 Hz, 1H), 7.98(d, *J* = 3.2 Hz, 1H), 7.94 - 7.86(m, 1H), 7.73(d, *J* = 7.5 Hz, 1H), 7.61(d, *J* = 8.3 Hz, 1H), 7.12(d, *J* = 7.5 Hz, 1H).

**Example 34**

*N*-(6-(oxazol-2-yl)-5-(trifluoromethyl)pyridin-3-yl)-1-(2-carbonyl-1,2-dihydrobenzo[*cd*]indol-6-yl)-5-(trifluoromethyl)-1*H*-pyrazole-4-carboxamide

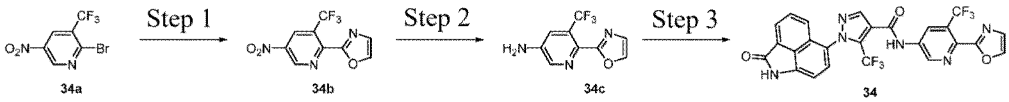


34a 34b 34c 34

Step 1

2-(5-nitro-3-(trifluoromethyl)pyridin-2-yl)oxazole

2-bromo-5-nitro-3-(trifluoromethyl)pyridine **34a** (302 mg, 1.12 mmol) was dissolved in 10 mL toluene, tributyl (oxazol-2-yl)stannane (0.20 g, 0.56 mmol, 0.1 mL) and tetratrisphenylphosphine palladium (64 mg, 0.056 mmol) were added, nitrogen replacement was performed three times, and the reaction mixture was heated to 110°C and reacted for 15 h. The reaction was cooled to room temperature and concentrated. 10 mL of water was added to the residue, and the obtained mixture was extracted with ethyl acetate (30 mL×3), the organic phases were combined, washed with saturated sodium chloride aqueous solution (30 mL×1), dried over anhydrous sodium sulfate, filtered and concentrated. The obtained residue was purified by silica gel column chromatography with eluent system C to obtain the title product 2-(5-nitro-3-(trifluoromethyl)pyridin-2-yl)oxazole **34b** (20 mg), yield: 13.8%.
MS m/z (ESI): 260 [M+1]

Step 2

6-(oxazol-2-yl)-5-(trifluoromethyl)pyridin-3-amine

2-(5-nitro-3-(trifluoromethyl)pyridin-2-yl) oxazole **34b** (0.20 g, 0.77 mmol) was dissolved in (5 mL) ethanol and (2 mL) water, iron powder (215 mg, 3.85 mmol) and ammonium chloride (199 mg, 3.85 mmol) were added, nitrogen replacement was performed three times, and the reaction mixture was heated to 70°C and reacted for 2 h. The reaction liquid was cooled to room temperature, filtered, the filter cake was washed with ethanol (10 mL×3), and the filtrate was concentrated. The residue was dissolved in (DCM/MeOH = 10/1, 30 mL), stirred for 1 h, filtered, and concentrated to obtain the title product 6-(oxazol-2-yl)-5-(trifluoromethyl)pyridin-3-amine **34c** (50 mg), yield: 28.3%.
MS m/z (ESI): 230 [M+1]

Step 3

*N*-(6-(oxazol-2-yl)-5-(trifluoromethyl)pyridin-3-yl)-1-(2-carbonyl-1,2-dihydrobenzo[*cd*]indol-6-yl)-5-(trifluoromethyl)-1*H*-pyrazole-4-carboxamide

1-(2-carbonyl-1,2-dihydrobenzo[*cd*]indol-6-yl)-5-trifluoromethyl-1H-pyrazole-4-carboxylic acid (20 mg, 0.058 mmol), 6-(oxazol-2-yl)-5-(trifluoromethyl)pyridin-3-amine **34c** (16 mg, 0.069 mmol) and DMAP (3 mg, 0.024 mmol) were dissolved in 5 mL of dichloromethane, nitrogen replacement was performed three times, the mixture was cooled to 0°C, and pyridine (14 mg, 0.17 mmol) and phosphorus oxychloride (26 mg, 0.17 mmol) were added. The reaction temperature was raised to 25°C for reaction for 2 h. The reaction was quenched by adding water and concentrated. The obtained residue was purified by silica gel column chromatography with eluent system A to obtain the title product *N*-(6-(oxazol-2-yl)-5-(trifluoromethyl)pyridin-3-yl)-1-(2-carbonyl-1,2-dihydrobenzo[cd]indol-6-yl)-5-(trifluoromethyl)-1*H*-pyrazole-4-carboxamide **34** (1.8 mg), yield: 5.2%.

MS m/z (ESI): 559 [M+1]
$^1H$ NMR (400 MHz, DMSO): δ 11.34 (s, 1H), 11.13 (s, 1H), 9.27 (d, *J* = 2.3 Hz, 1H), 8.81 (d, *J* = 2.3 Hz, 1H), 8.56

(s, 1H), 8.39 (s, 1H), 8.15 (d, *J* = 7.0 Hz, 1H), 7.92 (dd, *J* = 8.3, 7.0 Hz, 1H), 7.73 (d, *J* = 7.5 Hz, 1H), 7.61 (d, *J* = 8.2 Hz, 1H), 7.53 (s, 1H), 7.12 (d, *J* = 7.5 Hz, 1H).

**Example 35**

*N*-(6-(4,5-dimethyloxazol-2-yl)-5-(trifluoromethyl)pyridin-3-yl)-1-(2-carbonyl-1,2-dihydrobenzo[*cd*]indol-6-yl)-5-(trifluoromethyl)-1*H*-pyrazole-4-carboxamide

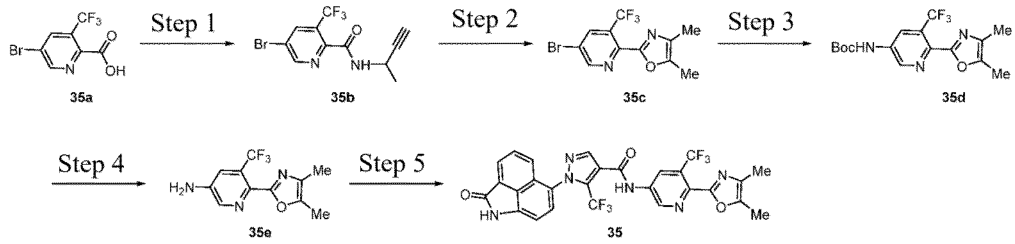


Step 1

5-bromo-*N*-(but-3-yn-2-yl)-3-(trifluoromethyl)methylpicolinamide

5-bromo-3-(trifluoromethyl)pyridine-2-carboxylic acid **35a** (0.60 g, 2.22 mmol), HATU (1.68 g, 4.44 mmol), but-3-yn-2-amine (307 mg, 4.44 mmol) were dissolved in 10 mL of acetonitrile, nitrogen replacement was performed three times, triethylamine (675 mg, 6.67 mmol, 0.93 mL) was added, and the reaction was carried out at 25°C for 1 h. The reaction was quenched by adding water, extracted with ethyl acetate (50 mL×3), the organic phases were combined, washed with saturated sodium chloride aqueous solution (30 mL×1), dried over anhydrous sodium sulfate, filtered and concentrated. The obtained residue was purified by silica gel column chromatography with eluent system A to obtain the title product 5-bromo-*N*-(but-3-yn-2-yl)-3-(trifluoromethyl) methylpicolinamide **35b** (0.65 g), yield: 91.1%.
MS m/z (ESI): 322 [M+1]

Step 2

2-(5-bromo-3-(trifluoromethyl)pyridin-2-yl)-4,5-dimethyloxazole

5-bromo-*N*-(but-3-yn-2-yl)-3-(trifluoromethyl)methylpicolinamide **35b** (0.65 g, 2.02 mmol), acetonitrile (5 mL) and triethylamine (205 mg, 2.02 mmol) were added into a microwave reaction tube, nitrogen was purged for 3 min, then gold trichloride (184 mg, 0.61 mmol) was quickly added and the tube was sealed. The reaction was carried out in a microwave reactor at 80°C for 4 h. The reaction was quenched by adding water, extracted with ethyl acetate (50 mL×3), the organic phases were combined, washed with saturated sodium chloride aqueous solution (30 mL×1), dried over anhydrous sodium sulfate, filtered and concentrated. The obtained residue was purified by silica gel column chromatography using eluent system C to obtain the title product 2-(5-bromo-3-(trifluoromethyl)pyridin-2-yl)-4,5-dimethyloxazole **35c** (0.32 g), yield: 49.2%.
MS m/z (ESI): 322 [M+1]

Step 3

Butyl (6-(4,5-dimethyloxazol-2-yl-5-(trifluoromethyl)pyridin-3-yl)carbamate

2-(5-bromo-3-(trifluoromethyl)pyridin-2-yl)-4,5-dimethyloxazole **35c** (0.32 g, 1.00 mmol) and 5-bisdiphenylphosphine-9,9-dimethylxanthene (58 mg, 0.10 mmol) were dissolved in 1,4-dioxane 10 mL, nitrogen replacement was performed three times, and (dibenzylideneacetone)palladium (57 mg, 0.10 mmol) was added, the reaction temperature was raised to 100°C for reaction for 2 h. The system was lowered to room temperature, the reaction was quenched by introducing air, the reaction liquid was filtered, concentrated and spun to dryness. The obtained residue was purified by silica gel column chromatography with eluent system C to obtain the title product butyl (6-(4,5-dimethyloxazol-2-yl-5-(trifluoromethyl)pyridin- 3-yl)carbamate **35d** (0.14 g), yield: 39.3%.
MS m/z (ESI): 358 [M+1]

Step 4

6-(4,5-dimethyloxazol-2-yl)-5-(trifluoromethyl)pyridin-3-amine

Butyl (6-(4,5-dimethyloxazol-2-yl-5-(trifluoromethyl)pyridin-3-yl)carbamate **35d** (130 mg, 0.36 mmol) was dissolved in 5 mL of a solution of 4 M chloride hydrogen in 1,4-dioxane, and the reaction system was allowed to react at 25°C for 1 h. The reaction liquid was concentrated to obtain the title product 6-(4,5-dimethyloxazol-2-yl)-5-(trifluoromethyl)pyridin-3-amine **35e** (80 mg), yield: 85.1%.
MS m/z (ESI):258 [M+1]

Step 5

*N*-(6-(4,5-dimethyloxazol-2-yl)-5-(trifluoromethyl)pyridin-3-yl)-1-(2-carbonyl-1,2-dihydrobenzo[*cd*]indol-6-yl)-5-(trifluoromethyl)-1*H*-pyrazole-4-carboxamide

Referring to the synthesis method of step 11 in Example 1, the title product *N*-(6-(4,5-dimethyloxazol-2-yl)-5-(trifluoromethyl)pyridin-3-yl)-1-(2-carbonyl-1,2-dihydrobenzo[cd]indol-6-yl)-5-(trifluoromethyl)-1*H*-pyrazole-4-carboxamide **35** (24 mg) was obtained through intermediate **1** (35 mg, 0.10 mmol) and 6-(4,5-dimethyloxazol-2-yl)-5-(trifluoromethyl)pyridin-3-amine 35e (52 mg, 0.20 mmol, synthesized using the well-known method "Patent US20180170909A1"), yield 40.1%.

MS m/z (ESI): 587 [M+1]
$^1$H NMR (400 MHz, DMSO): δ 11.29 (s, 1H), 11.12 (s, 1H), 9.23 (d, *J* = 2.3 Hz, 1H), 8.77 (d, *J* = 2.3 Hz, 1H), 8.55 (s, 1H), 8.15 (d, *J* = 6.9 Hz, 1H), 7.91 (dd, *J* = 8.3, 7.0 Hz, 1H), 7.73 (d, *J* = 7.6 Hz, 1H), 7.60 (d, *J* = 8.2 Hz, 1H), 7.12 (d, *J* = 7.5 Hz, 1H), 2.36 (s, 3H), 2.14 (s, 3H).

**Example 36**

*N*-(6-(1*H*-1,2,3-triazol-1-yl)-5-(trifluoromethyl)pyridin-3-yl)-1-(2-carbonyl-1,2-dihydrobenzo[*cd*]indol-6-yl)-5-(trifluoromethyl)-1*H*-pyrazole-4-carboxamide

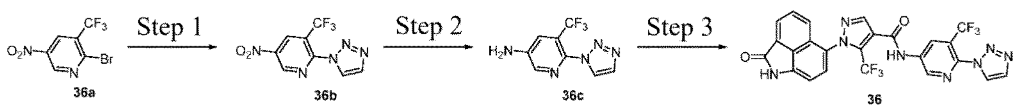


Step 1

5-nitro-2-(1*H*-1,2,3-triazol-1-yl)-3-(trifluoromethyl)pyridine

2-bromo-3-trifluoromethyl-5-nitro-pyridine **36a** (1.40 g, 5.18 mmol) and potassium carbonate (1.43 g, 10.36 mmol) were added to a single-neck bottle and dissolved in 10 mL of acetonitrile, 1,2,3-triazole (537 mg, 7.77 mmol) was added, the system was subjected nitrogen replacement three times, and the reaction mixture was heated to 40°C and reacted under stirring for 2 h. The reaction mixture was cooled to room temperature, filtered, and concentrated. The obtained residue was purified by silica gel column chromatography with eluent system C to obtain the title product 5-nitro-2-(1*H*-1,2,3-triazol-1-yl)-3-(trifluoromethyl)pyridine **36b** (0.50 g), yield: 42.7%.
MS m/z (ESI): 226 [M+1]

Step 2

6-(1*H*-1,2,3-triazol-1-yl)-5-(trifluoromethyl)pyridin-3-amine

5-nitro-2-(1H-1,2,3-triazol-1-yl)-3-(trifluoromethyl)pyridine **36b** (0.25 g, 1.11 mmol), reduced iron powder (309 mg, 5.54 mmol) and ammonium chloride (285 mg, 5.54 mmol) were dissolved in 10 mL of ethanol and 5 mL of water. Nitrogen replacement was performed three times, and the reaction mixture was heated to 70°C and reacted for 1 h. The reaction liquid was cooled to room temperature, filtered, and the filter cake was washed with ethanol (10 mL×3) and

concentrated. The residue was dissolved in (DCM/MeOH = 10/1 30 mL), stirred for 1 h, filtered, and concentrated to obtain the title product 6-(1*H*-1,2,3-triazol-1-yl)-5-(trifluoromethyl)pyridin-3-amine **36c** (150 mg) yield: 69.4%.
MS m/z (ESI): 196 [M+1]

Step 3

*N*-(6-(1*H*-1,2,3-triazol-1-yl)-5-(trifluoromethyl)pyridin-3-yl)-1-(2-carbonyl-1,2-dihydrobenzo[*cd*]indol-6-yl)-5-(trifluoromethyl)-1*H*-pyrazole-4-carboxamide

Referring to the synthesis method of the step 11 in Example 1, the title product 6-(1*H*-1,2,3-triazol-1-yl)-5-(trifluoromethyl)pyridin-3-yl)-1-(2-carbonyl-1,2-dihydrobenzo[*cd*]indol-6-yl)-5-(trifluoromethyl)-1*H*-pyrazole-4-carboxamide **36** (21 mg) was obtained through the intermediate **1** (80 mg, 0.23 mmol) and 6-(1*H*-1,2,3-triazol-1-yl)-5-(trifluoromethyl)pyridin-3-amine **36c** (64 mg, 0.27 mmol, synthesized using the well-known method "Patent US20180170909A1"), yield: 16.4%.

MS m/z (ESI): 559 [M+1]
$^1$H NMR (400 MHz, DMSO): δ 11.40 (s, 1H), 11.12 (s, 1H), 9.20 (d, *J* = 2.4 Hz, 1H), 8.90 (d, *J* = 2.4 Hz, 1H), 8.68 (d, *J* = 1.2 Hz, 1H), 8.57 (s, 1H), 8.15 (d, *J* = 6.9 Hz, 1H), 8.02 (d, *J* = 1.2 Hz, 1H), 7.92 (dd, *J* = 8.3, 7.0 Hz, 1H), 7.73 (d, *J* = 7.5 Hz, 1H), 7.61 (d, *J* = 8.2 Hz, 1H), 7.12 (d, *J* = 7.5 Hz, 1H).

**Example 37**

*N*-(6-(1-methyl-1*H*-pyrazol-3-yl)-5-(trifluoromethyl)pyridin-3-yl)-1-(2-oxo-1,2-dihydrobenzo[*cd*]indol-6-yl)-5-(trifluoromethyl)-1*H*-pyrazole-4-carboxamide

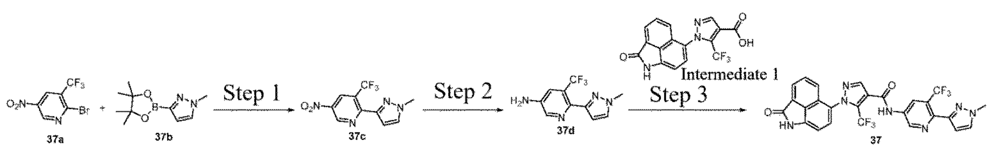


Step 1

2-(1-methyl-1*H*-pyrazol-3-yl)-5-nitro-3-(trifluoromethyl)pyridine

2-bromo-5-nitro-3-(trifluoromethyl)pyridine **37a** (1 g, 3.70 mmol) and 1-methyl-1H-pyrazole-5-boronic acid pinacol ester **37b** (1.04 g, 5 mmol) were dissolved in 10 mL of toluene and 2 mL of water, and tetrakis(triphenylphosphine)palladium (577 mg, 0.50 mmol) and potassium carbonate (1.38 g, 10 mmol) were added, and the mixture was reacted under stirring at 100°C for 1 h. The reaction liquid was filtered, and the filtrate was concentrated under reduced pressure. The obtained residue was purified by silica gel column chromatography with eluent system A to obtain the title product 2-(1-methyl-1*H*-pyrazol-3-yl)-5-nitro-3-(trifluoromethyl)pyridine **37c** (620 mg), yield: 61.1%.
MS m/z(ESI): 273 [M+1].
Referring to the synthesis method of step 4 in Example 25 and step 11 in Example 1, *N*-(6-(1-methyl-1*H*-pyrazol-3-yl)-5-(trifluoromethyl)pyridin-3-yl)-1-(2-oxo-1,2-dihydrobenzo[cd]indol-6-yl)-5-(trifluoromethyl)-1*H*-pyrazole-4-carboxamide **3 7**(30 mg) was obtained through 2-(1-methyl-1*H*-pyrazol-3-yl)-5-nitro-3-(trifluoromethyl)pyridine **37c** (620 mg, 2.27 mmol), yield: 2.3%.

MS m/z(ESI): 572 [M+1]
$^1$H NMR(400 MHz, DMSO-$d_6$)δ 11.13(d, *J* = 5.1 Hz, 2H), 9.15(d, *J* = 2.1 Hz, 1H), 8.68(d, *J* = 2.2 Hz, 1H), 8.54(s, 1H), 8.15(d, *J* = 6.9 Hz, 1H), 7.91(dd, *J* = 8.2, 7.1 Hz, 1H), 7.79(d, *J* = 2.2 Hz, 1H), 7.72(d, *J* = 7.5 Hz, 1H), 7.61(d, *J* = 8.3 Hz, 1H), 7.12(d, *J* = 7.5 Hz, 1H), 6.65(d, *J* = 2.2 Hz, 1H), 3.92(s, 3H).

**Example 38**

*N*-(6-*N*-methyl-1,1-dioxotetrahydro-2*H*-thiapyran-4-formamido)-5-(trifluoromethylpyridin-3-yl)-1-(2-oxo-1,2-dihydrobenzo[*cd*]indol-6-yl)-5-trifluoromethyl-1*H*-pyrazole-4-carboxamide

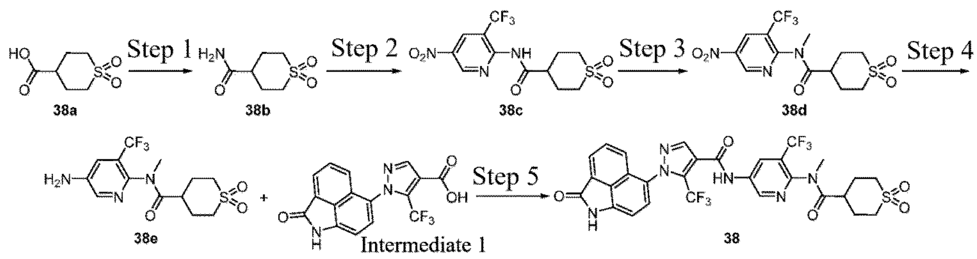


Step 1

Tetrahydro-2*H*-thiapyran-4-carboxamide 1,1-dioxide

Tetrahydro-2H-thiapyran-4-carboxylic acid 1,1-dioxide **38a** (1.59 g, 8.92 mmol) was dissolved in 25 mL of dichloromethane, 0.1 mL of *N*,*N*-dimethylformamide and oxalyl chloride (4.5 mL, 53.53 mmol) were added, and the mixture was reacted under stirring for 0.5 h at room temperature. The reaction liquid was concentrated under reduced pressure, 5 mL of ammonia water was added to the residue, the obtained mixture was filtered, and the filtrate was washed twice with water (5 mL×2) and dried to obtain the title product tetrahydro-2*H*-thiapyran-4-carboxamide 1,1-dioxide **38b** (900 mg), yield: 56.9%.
MS m/z (ESI): 178 [M+1]

Step 2

*N*-(5-nitro-3-trifluoromethylpyridin-2-yl)-tetrahydro-2*H*-thiapyran-4-carboxamide 1,1-dioxide

Sodium hydride (320 mg, 13.33 mmol) was dispersed in 15 mL of N,N-dimethylformamide, and tetrahydro-2*H*-thiapyran-4-carboxamide 1,1-dioxide **38b** (704 mg, 3.97 mmol) was added, the mixture was reacted at 0°C under stirring for 1 h, then 2-chloro-5-nitro-3-trifluoromethylpyridine (900 mg, 3.97 mmol) was added and the mixture was reacted under stirring at room temperature for 12 h. 5 mL of saturated ammonium chloride solution was added to the reaction liquid, the mixture was extracted with ethyl acetate (10 mL×3), the organic phases were combined, washed with saturated sodium chloride aqueous solution (25 mL×2), dried over anhydrous sodium sulfate, and filtered, the filtrate was concentrated under reduced pressure, and the obtained residue was purified by silica gel column chromatography with eluent system A to obtain the title product *N*-(5-nitro-3-trifluoromethylpyridin-2-yl)-tetrahydro-2*H*-thiapyran-4-carboxamide 1,1-dioxide **38c** (219 mg), yield: 15.0%.
MS m/z (ESI): 368 [M+1]

Step 3

*N*-methyl-*N*-(5-nitro-3-trifluoromethylpyridin-2-yl)-tetrahydro-2*H*-thiapyran-4-carboxamide 1,1-dioxide

*N*-(5-nitro-3-trifluoromethylpyridin-2-yl)-tetrahydro-2*H*-thiapyran-4-carboxamide 1,1-dioxide **38c** (219 mg, 0.60 mmol) was dissolved in 5 mL of *N,N*-dimethylformamide, and potassium carbonate (248 mg, 1.80 mmol) and methyl iodide (256 mg, 1.80 mmol) were added and the mixture was reacted under stirring at room temperature for 3 h. 5 mL of saturated ammonium chloride solution was added to the reaction liquid, the mixture was extracted with ethyl acetate (10 mL×3), the organic phases were combined, washed with saturated sodium chloride aqueous solution (25 mL×2), dried over anhydrous sodium sulfate, and filtered, the filtrate was concentrated under reduced pressure, and the obtained residue was purified by silica gel column chromatography with eluent system A to obtain the title product *N*-methyl-*N*-(5-nitro-3-trifluoromethylpyridin-2-yl)-tetrahydro-2*H*-thiapyran-4-carboxamide 1,1-dioxide **38d** (66 mg), yield: 29.0%.
MS m/z (ESI): 382 [M+1]

Step 4 to step 5

*N*-(6-*N*-methyl-1,1-dioxotetrahydro-2*H*-thiapyran-4-formamido)-5-(trifluoromethylpyridin-3-yl)-1-(2-oxo-1,2-dihydrobenzo[cd]indol-6-yl)-5-trifluoromethyl-1H-pyrazole-4-carboxamide

Referring to the synthesis method of step 2 in Example **32** and step 11 in Example 1 in sequence, the title product *N*-(6-*N*-methyl-1,1-dioxotetrahydro-2*H*-thiapyran-4-formamido)-5-(trifluoromethylpyridin-3-yl)-1-(2-oxo-1,2-dihydrobenzo[*cd*]indol-6-yl)-5-trifluoromethyl-1H-pyrazole-4-carboxamide **38** (20 mg) was obtained through *N*-methyl-*N*-(5-nitro-3-trifluoromethylpyridin-2-yl)-tetrahydro-2*H*-thiapyran-4-carboxamide 1,1-dioxide **38d** (66 mg, 0.17 mmol), yield: 17.0%.

MS m/z (ESI): 681 [M+1]

$^1$H NMR (400 MHz, DMSO-$d_6$) $\delta$ 11.47-11.02 (br. s, 1H), 11.13 (s, 1H), 9.06 (s, 1H), 8.86 (d, 1H), 8.51 (s, 1H), 8.16 (d, 1H), 7.92 (t, 1H), 7.73 (d, 1H), 7.61 (d, 1H), 7.12 (t, 1H), 3.08 (s, 3H), 3.05-2.87 (m, 3H), 2.40-1.73 (m, 6H).

**Example 39**

*N*-(6-(2*H*-tetrazol-2-yl)-5-(trifluoromethyl)pyridin-3-yl)-1-(2-oxo-1,2-1,2-dihydrobenzo[*cd*]indol-6-yl)-5-(trifluoromethyl)-1*H*-pyrazole-4-carboxamide

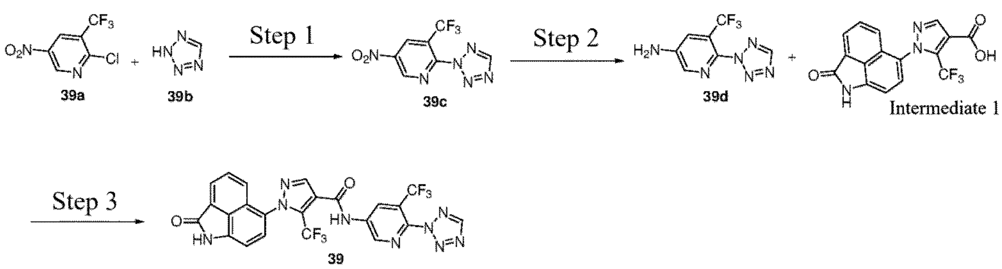


Step 1

5-nitro-2-(2*H*-tetrazol-2-yl)-3-(trifluoromethyl)pyridine

2-chloro-5-nitro-3-(trifluoromethyl)pyridine **39a** (1 g, 4.42 mmol) and tetrazole (350 mg, 5 mmol) were dissolved in 20 mL of acetonitrile, triethylamine (1 g, 10 mmol) was added, and the mixture was reacted under stirring for 16 h at room temperature. The reaction liquid was filtered, and the filtrate was concentrated under reduced pressure. The obtained residue was purified by silica gel column chromatography with eluent system A to obtain the title product 5-nitro-2-(2*H*-tetrazol-2-yl)-3-(trifluoromethyl)pyridine **39b** (420 mg), yield: 36.0%.

MS m/z(ESI): 261 [M+1].

Referring to the synthesis method of step 4 in Example 25 and step 11 in Example 1, *N*-(6-(2*H*-tetrazol-2-yl)-5-(trifluoromethyl)pyridin-3-yl)-1-(2-oxo-1,2-1,2-dihydrobenzo[*cd*]indol-6-yl)-5-(trifluoromethyl)-1*H*-pyrazole-4-carboxamide **39** (20 mg) was obtained through 5-nitro-2-(2*H*-tetrazol-2-yl)-3-(trifluoromethyl)pyridine **39b** (420 mg, 1.62 mmol), yield: 2.3%.

MS m/z(ESI): 560 [M+1]

**Example 40**

1-(2-carbonyl-1,2-dihydrobenzo[cd]indol-6-yl)-N-(6-(tetrahydrofuran-2-yl)-5-(trifluoromethyl)pyridin-3-yl)-5-(trifluoromethyl)-1*H*-pyrazole-4-carboxamide

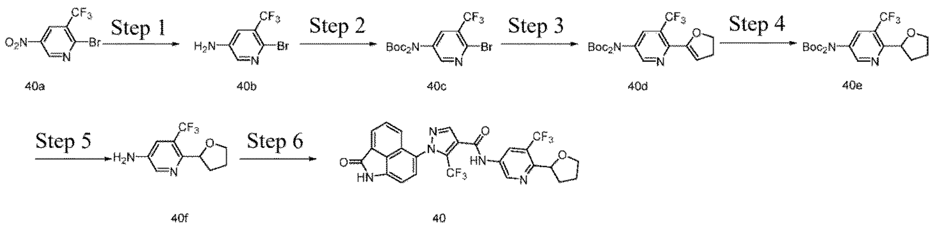


40a 40b 40c 40d 40e

40f 40

Step 1

6-bromo-5-(trifluoromethyl)pyridin-3-amine

2-bromo-5-nitro-3-(trifluoromethyl)pyridine **40a** (2 g, 7.38 mmol) was dissolved in 20 mL of ethanol and 5 ml of water. Ammonium chloride (1 g, 18.7 mmol) and reduced iron powder (1 g, 17.9 mmol) were added and the reaction was carried out at 80°C for 2 h. The reaction liquid was cooled to room temperature and poured into 50 mL of water. The obtained mixture was filtered, the filtrate was extracted with ethyl acetate (50 mL×3), the organic phases were combined, washed with saturated sodium chloride solution (50 mL×2), dried over anhydrous sodium sulfate, filtered and concentrated to obtain a crude product, and the obtained residue was purified by silica gel column chromatography with eluent system A to obtain the title product 6-bromo-5-(trifluoromethyl)pyridin-3-amine 40b (1.4 g), yield: 78.7%.
MS m/z (ESI): 242 [M+1]

Step 2

Tert-butyl N-[6-bromo-5-(trifluoromethyl)-3-pyridyl]-N-tert-butoxycarbonyl-carbamate

6-bromo-5-(trifluoromethyl)pyridin-3-amine 40b (1.4 g, 5.8 mmol) and di-tert-butyl dicarbonate (3 g, 13.7 mmol) were dissolved in 20 ml of dichloromethane. Triethylamine (2 g, 19.7 mmol) and 4-dimethylaminopyridine (100 mg, 0.82 mmol) were added and the reaction was carried out at 20°C for 12 h. The reaction liquid was cooled to room temperature and poured into 200 mL of water. The obtained mixture was filtered, the filtrate was extracted with ethyl acetate (50 mL×3), the organic phases were combined, washed with saturated sodium chloride solution (50 mL×2), dried over anhydrous sodium sulfate, filtered and concentrated to obtain a crude product, and the obtained residue was purified by silica gel column chromatography with eluent system A to obtain the title product tert-butyl N-[6-bromo-5-(trifluoromethyl)-3-pyridyl]-N-tert-butoxycarbonyl-carbamate 40c(2.4 g), yield: 93.6%.
MS m/z (ESI): 442 [M+1]

Step 3

Tert-butyl N-tert-butoxycarbonyl-N-[6-(2,3-dihydrofuran-5-yl)-5-(trifluoromethyl)-3-pyridyl]carbamate

Tert-butyl N-[6-bromo-5-(trifluoromethyl)-3-pyridyl]-N-tert-butoxycarbonyl-carbamate **40c** (2.4 0 g, 5.40 mmol), potassium carbonate (1.5 g, 10.8 mmol), palladium acetate (122 mg, 0.54 mmol), 2,3-dihydrofuran (760 mg, 10.8 mmol) were dissolved in 15 mL of N,N-dimethylformamide. Trishenylphosphine (283 mg, 1.08 mmol) was added, and the reaction was carried out at 110°C for 2 h. The reaction liquid was cooled to room temperature, and 20 mL of water was added. The obtained mixture was filtered, the filtrate was extracted with ethyl acetate (20 mL×3), the organic phases were combined, washed with saturated sodium chloride solution (10 mL×2), dried over anhydrous sodium sulfate, filtered and concentrated to obtain a crude product, and the obtained residue was purified by silica gel column chromatography with eluent system A to obtain the title product tert-butyl N-tert-butoxycarbonyl-N-[6-(2,3-dihydrofuran-5-yl)-5-(trifluoromethyl)-3-pyridyl]carbamate **40d** (200 mg), yield: 8.5%.
MS m/z (ESI): 431 [M+1].

Step 4

Tert-butyl N-tert-butoxycarbonyl-N-[6-tetrahydrofuran-2-yl-5-(trifluoromethyl)-3-pyridyl]carbamate

Tert-butyl N-tert-butoxycarbonyl-N-[6-(2,3-dihydrofuran-5-yl)-5-(trifluoromethyl)-3-pyridyl]carbamate 40d (200 mg, 0.47 mmol) was dissolved in 10 mL of tetrahydrofuran. Pd/C (200 mg) was added and the mixture was reacted at 20°C for 12 h in hydrogen (15 psi) atmosphere. The reaction liquid was cooled to room temperature, filtered, and

concentrated to obtain a crude product. The obtained residue was purified by silica gel column chromatography with eluent system A to obtain the title tert-butyl N-tert-butoxycarbonyl-N-[6-tetrahydrofuran-2-yl-5-(trifluoromethyl)-3-pyridyl]carbamate **40e** (60 mg), yield: 29.8%.

MS m/z (ESI): 433 [M+1].

Referring to the synthesis method of step 5 and step 6 in Example 29, 1-(2-carbonyl-1,2-dihydrobenzo[cd]indol-6-yl)-N-(6-(tetrahydrofuran-2-yl)-5-(trifluoromethyl)pyridin-3-yl)-5-(trifluoromethyl)-1H-pyrazole-4-carboxamide 40 (10 mg) was obtained through tert-butyl N-tert-butoxycarbonyl-N-[6-tetrahydrofuran-2-yl-5-(trifluoromethyl)-3-pyridyl]carbamate 40e (60 mg, 0.139 mmol), yield: 12.8%.

MS m/z (ESI): 562[M+1].

1H NMR (400 MHz, $CD_3OD$) δ 9.09 (d, J = 2.4 Hz, 1H), 8.62 (d, J = 2.5 Hz, 1H), 8.34 (s, 1H), 8.15 (d, J = 7.0 Hz, 1H), 7.87 (dd, J = 8.3, 7.0 Hz, 1H), 7.66 (dd, J = 7.9, 6.3 Hz, 2H), 7.13 (d, J = 7.5 Hz, 1H), 5.38 - 5.23 (m, 1H), 4.20 (q, J = 7.0 Hz, 1H), 3.97 (td, J = 7.6, 5.5 Hz, 1H), 2.32 (td, J = 7.5, 4.0 Hz, 1H), 2.26 -1.96 (m, 3H).

**Example 41**

1-(2-oxo-1,2-dihydrobenzo[*cd*]indol-6-yl)-5-trifluoromethyl-*N*-(5-trifluoromethyl-6-(4-trifluoromethyl-2*H*-1,2,3-triazol-2-yl)pyridin-3-yl)-1*H*-pyrazole-4-carboxamide

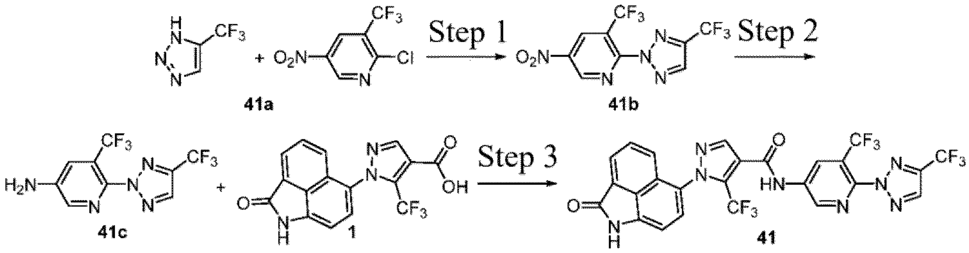


Step 1 to step 3

1-(2-oxo-1,2-dihydrobenzo[*cd*]indol-6-yl)-5-trifluoromethyl-*N*-(5-trifluoromethyl-6-(4-trifluoromethyl-2*H*-1,2,3-triazol-2-yl)pyridin-3-yl)-1*H*-pyrazole-4-carboxamide

Referring to the synthesis method of step 1 to step 2 in Example **32** and step 11 in Example 1 in sequence, the title product 1-(2-oxo-1,2-dihydrobenzo[*cd*]indol-6-yl)-5-trifluoromethyl-N-(5-trifluoromethyl-6-(4-trifluoromethyl-2*H*-1,2,3-triazol-2-yl)pyridin-3-yl)-1*H*-pyrazole-4-carboxamide (11 mg) was obtained through 5-trifluoromethyl-1*H*-1,2,3-triazole (137 mg, 1 mmol, prepared by the well-known method "Patent US2020/102311A1") and 2-chloro-5-nitro-3-trifluoromethylpyridine (230 mg, 1 mmol), yield: 1.8%.
MS m/z (ESI): 627 [M+1]

**Example 42**

*N*-(6-(1-oxo-4,5-dihydro-3*H*-1$\lambda^6$-isothiazol-1-yl)-5-trifluoromethylpyridin-3-yl)-1-(2-oxo-1,2-dihydrobenzo[*cd*]indol-6-yl)-5-trifluoromethyl-1*H*-pyrazole-4-carboxamide

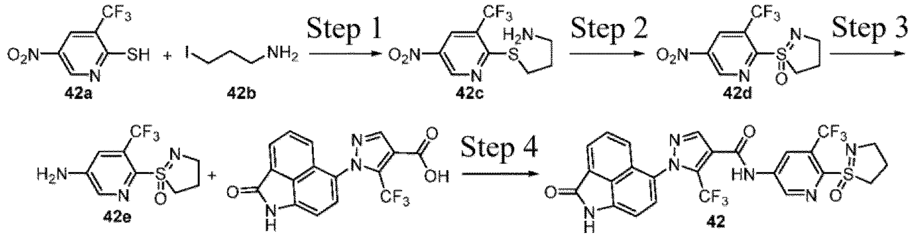

Step 1

3-((5-nitro-3-trifluoromethylpyridin-2-yl)thio)-1-propylamine

5-nitro-3-trifluoromethylpyridin-2-thiophenol **42a** (2.24 g, 10 mmol) was dissolved in 25 mL of acetonitrile, and potassium carbonate (1.52 g, 11.02 mmol) and 3-iodo-1-propylamine **42b**(2.41 g, 12.11 mmol, prepared by a well-known method "Chemistry -A European Journal, 2021, 27(63), 15716-15721") were added and the mixture was reacted under stirring at room temperature for 12 h. The reaction liquid was filtered, and the filtrate was concentrated under reduced pressure. The obtained residue was purified by silica gel column chromatography with eluent system A to obtain the title product 3-((5-nitro-3-trifluoromethylpyridin-2-yl)thio)-1-propylamine **42c** (1.81 g), yield: 64.3%.
MS m/z (ESI): 282 [M+1]

Step 2

1-(5-nitro-3-trifluoromethylpyridin-2-yl)-1-oxo-3*H*-4,5-dihydroisothiazole

3-((5-nitro-3-trifluoromethylpyridin-2-yl)thio)-1-propylamine **42c** (1.81 g, 6.44 mmol) was dissolved in 25 mL of methanol, ammonium formate (1.22 g, 19.30 mmol) and iodobenzene acetate (6.21 g, 19.30 mmol) were added, and the mixture was reacted under stirring at room temperature for 12 h. The reaction liquid was concentrated under reduced pressure, and the obtained residue was purified by silica gel column chromatography with eluent system A to obtain the title product 1-(5-nitro-3-trifluoromethylpyridin-2-yl)-1-oxo-3*H*-4,5-dihydroisothiazole **42d** (0.45 g), yield: 23.7%.
MS m/z (ESI): 296 [M+1]

Step 3 to step 4

Referring to the synthesis method of step 2 in Example **32** and step 11 in Example **1** in sequence, the title product *N*-(6-(1-oxo-4,5-dihydro-3H-1$\lambda^6$-isothiazol-1-yl)-5-trifluoromethylpyridin-3-yl)-1-(2-oxo-1,2-dihydrobenzo[*cd*]indol-6-yl)-5-trifluoromethyl-1*H*-pyrazole-4-carboxamide **42** (31 mg) was obtained through 1-(5-nitro-3-trifluoromethylpyridin-2-yl)-1-oxo-3*H*-4,5-dihydroisothiazole **42d** (0.15 g, 0.51 mmol), yield: 10.0%.
MS m/z (ESI): 595 [M+1]

**Example 43**

1-(2-oxo-1,2-dihydrobenzo[*cd*]indol-6-yl)-*N*-(6-(4-trifluoromethoxymethyl-2*H*-1,2,3-triazol-2-yl)-5-trifluoromethylpyridin-3-yl)-5-trifluoromethyl-1*H*-pyrazole-4-carboxamide

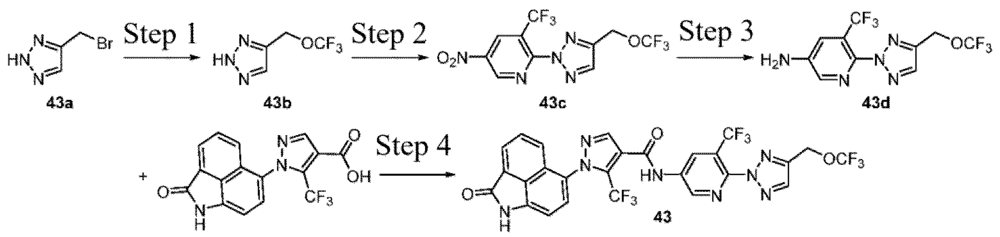

43a
43b
43c
43d
43

Step 1

4-trifluoromethoxymethyl-2*H*-1,2,3-triazole

4-bromomethyl-2*H*-1,2,3-triazole **43a** (1.62 g, 10 mmol, prepared by a well-known method "Tetrahedron, 2005, 61(21), 4983-4987") was dissolved in 25 mL of acetonitrile, and trifluoromethyl trifluoromethanesulfonate (4.4 g, 20 mmol) and silver trifluoromethanesulfonate (5.14 g, 20 mmol) were added and the mixture was reacted under stirring at room temperature for 12 h. The reaction liquid was filtered and concentrated under reduced pressure to obtain the crude title product 4-trifluoromethoxymethyl-2*H*-1,2,3-triazole **43b** (1.81 g). The product was directly used in the next reaction without purification.
MS m/z (ESI): 167 [M+1]

Step 2 to step 4

Referring to the synthesis method of step 1 to step 2 in Example **32** and step 11 in Example 1 in sequence, the title product 1-(2-oxo-1,2-dihydrobenzo[*cd*]indol-6-yl)-*N*-(6-(4-trifluoromethoxymethyl-2*H*-1,2,3-triazol-2-yl)-5-trifluoromethylpyridin-3-yl)-5-trifluoromethyl-1*H*-pyrazole-4-carboxamide (15 mg) was obtained through crude product 4-trifluoromethoxymethyl-2*H*-1,2,3-triazole **43b** (0.49 g, 3 mmol), yield: 0.8%.
MS m/z (ESI): 657 [M+1]

**Example 44**

N-(5-chloro-6-(1H-pyrazol-1-yl)pyridin-3-yl)-1-(2-carbonyl-1,2-dihydrobenzo[cd]indol-6-yl)-5-(trifluoromethyl)-1H-pyrazole-4-carboxamide

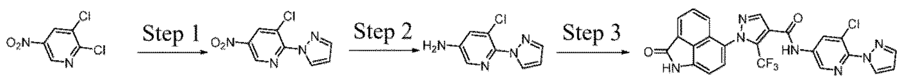


**44a** **44b** **44c** **44**

Referring to the synthesis method of step 1 in Example 30, 3-chloro-5-nitro-2-(1H-pyrazol-1-yl)pyridine **44b** (110 mg) was obtained through the reaction of 2,3-dichloro-5-nitropyridine **44a** (150 mg, 0.79 mmol), yield: 62.0%.
MS m/z (ESI): 225 [M+1].

Step 2

3-chloro-5-amino-2-(1H-pyrazol-1-yl)pyridine

3-chloro-5-nitro-2-(1H-pyrazol-1-yl)pyridine **44b** (110 mg, 0.49 mmol) was dissolved in 5 mL of ethanol and 5 mL of water, iron powder (82 mg, 1.47 mmol) and ammonium chloride (261 mg, 4.90 mmol) were added, and the mixture was heated at 85°C and reacted under stirring for 1 h. The reaction liquid was filtered, the filtrate was concentrated under reduced pressure, and the obtained residue was purified by silica gel column chromatography with eluent system B to obtain the title product 3-chloro-5-amino-2-(1H-pyrazol-1-yl)pyridine **44c** (89 mg), yield: 93.4%.
MS m/z (ESI): 195 [M+1]
Referring to the synthesis method of step 11 in Example 1,
N-(5-chloro-6-(1H-pyrazol-1-yl)pyridin-3-yl)-1-(2-carbonyl-1,2-dihydrobenzo[cd]indol-6-yl)-5-(trifluoromethyl)-1H-pyrazole-4-carboxamide **44** (21 mg) was obtained through the reaction of 3-chloro-5-amino-2-(1H-pyrazol-1-yl)pyridine **44c** (89 mg, 0.45 mmol), yield: 8.9%.
MS m/z (ESI): 524 [M+1].
1H NMR (400 MHz, DMSO) $\delta$ 11.15 (d, J = 11.2 Hz, 2H), 8.80 (d, J = 2.2 Hz, 1H), 8.60 (d, J = 2.3 Hz, 1H), 8.54 (s, 1H), 8.26 (d, J = 2.5 Hz, 1H), 8.16 (d, J = 6.9 Hz, 1H), 7.92 (dd, J = 8.3, 7.0 Hz, 1H), 7.82 (d, J = 1.7 Hz, 1H), 7.73 (d, J = 7.5 Hz, 1H), 7.61 (d, J = 8.3 Hz, 1H), 7.13 (d, J = 7.5 Hz, 1H), 6.57 (t, J = 2.2 Hz, 1H).

**Example 45**

N-(5-chloro-6-(1-methyl-1H-1,2,4-triazol-3-yl)pyridin-3-yl)-1-(2-carbonyl-1,2-dihydrobenzo[cd]indol-6-yl)-5-(trifluoromethyl)-1H-pyrazole-4-carboxamide

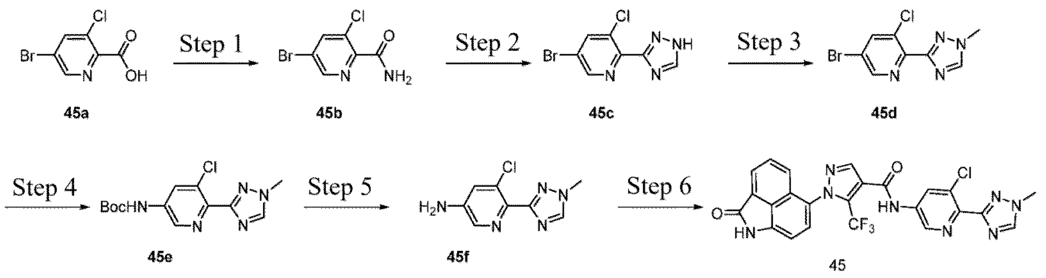


45a 45b 45c 45d 45e 45f 45

Step 1

5-bromo-3-chloromethylpicolinamide

5-bromo-3-chloro-ortho-picolinic acid **45a** (5 g, 21.15 mmol), ammonium chloride (2 g, 37.4 mmol), 2-(7-azobenzotriazole)-N,N,N',N'-tetrakismethylurea hexafluorophosphate (8.40 g, 22.08 mmol) was dissolved in 30 mL of N,N-dimethylformamide and then triethylamine (4 g, 39.53 mmol) was added for reaction at 20°C for 2 h. The reaction liquid was cooled to room temperature and poured into 50 mL of water. The obtained mixture was filtered, the filtrate was extracted with ethyl acetate (50 mL×3), the organic phases were combined, washed with saturated sodium chloride solution (50 mL×2), dried over anhydrous sodium sulfate, filtered and concentrated to obtain a crude product, and the obtained residue was purified by silica gel column chromatography with eluent system A to obtain the title product 5-bromo-3-chloromethylpicolinamide **45b** (3 g), yield: 60.2%.
MS m/z (ESI): 236 [M+1]

Step 2

5-bromo-3-chloro-2-(1H-1,2,4-triazol-3-yl)pyridine

5-bromo-3-chloromethylpicolinamide **45b** (3 g, 12.74 mmol) was dissolved in 20 mL of N,N-dimethylformamidedimethyl acetal for reaction at 100°C for 2 h. The obtained product was spun to dryness, hydrazine hydrate (1 g, 20 mmol) and 50 mL of acetic acid were added, and the mixture was reacted at 90°C for 12 h. The reaction liquid was cooled to room temperature and poured into 100 mL of water. The obtained mixture was filtered, the filtrate was extracted with ethyl acetate (50 mL×3), the organic phases were combined, washed with saturated sodium chloride solution (50 mL×2), dried over anhydrous sodium sulfate, filtered and concentrated to obtain a crude product, and the obtained residue was purified by silica gel column chromatography with eluent system A to obtain the title product 5-bromo-3-chloro-2-(1H-1,2,4-triazol-3-yl)pyridine **45c** (1 g), yield: 30.2%.
MS m/z (ESI): 260 [M+1].

Step 3

5-bromo-3-chloro-2-(1-methyl-1H-1,2,4-triazol-3-yl)pyridine

5-bromo-3-chloro-2-(1H-1,2,4-triazol-3-yl)pyridine **45c** (1 g, 3.85 mmol) was dissolved in 20 mL of N,N- dimethylformamide, then cesium carbonate (1.40 g, 4.30 mmol) and methyl iodide (540 mg, 3.80 mmol) were added, and the reaction was carried out at 20°C for 2 h. The reaction liquid was cooled to room temperature and poured into 30 mL of water. The obtained mixture was filtered, the filtrate was extracted with ethyl acetate (50 mL×3), the organic phases were combined, washed with saturated sodium chloride solution (50 mL×2), dried over anhydrous sodium sulfate, filtered and concentrated to obtain a crude product, and the obtained residue was purified by silica gel column chromatography with eluent system A to obtain the title product 5-bromo-3-chloro-2-(1-methyl-1H-1,2,4-triazol-3-yl)pyridine **45d** (500 mg), yield: 47.4%. MS m/z (ESI): 274 [M+1].
Referring to the synthesis method of step 2 in Example 13 and step 5 and step 6 in Example 29, N-(5-chloro-6-(1-methyl-1H-1,2,4-triazol-3-yl)pyridin-3-yl)-1-(2-carbonyl-1,2-dihydrobenzo[cd]indol-6-yl)-5-(trifluoromethyl)-1H-pyrazole-4-carboxamide 45 (20 mg) was obtained through 5-bromo-3-chloro-2-(1-methyl-1H-1,2,4-triazol-3-yl)pyridine **45d** (500 mg, 1.83 mmol), yield: 2.0%.
MS m/z (ESI): 539 [M+1].
1H NMR (400 MHz, $CD_3OD$) δ 8.96 (d, J = 2.2 Hz, 1H), 8.65 (d, J = 2.2 Hz, 1H), 8.35 (s, 1H), 8.16 (d, J = 7.0 Hz, 1H), 8.09 (s, 1H), 7.88 (dd, J = 8.3, 7.0 Hz, 1H), 7.67 (dd, J = 7.9, 5.8 Hz, 2H), 7.13 (d, J = 7.5 Hz, 1H), 3.94 (s, 3H).

**Examples 46 and 47**

N-(5-chloro-6-(3-methyl-1H-1,2,4-triazol-1-yl)pyridin-3-yl)-1-(2-carbonyl-1,2-dihydrobenzo[cd]indol-6-yl)-5-(trifluoromethyl)-1H-pyrazole-4-carboxamide and N-(5-chloro-6-(5-methyl-1H-1,2,4-triazol-1-yl)pyridin-3-yl)-1-(2-carbonyl-1,2-dihydrobenzo[cd]indol-6-yl)-5-(trifluoromethyl)-1H-pyrazole-4-carboxamide

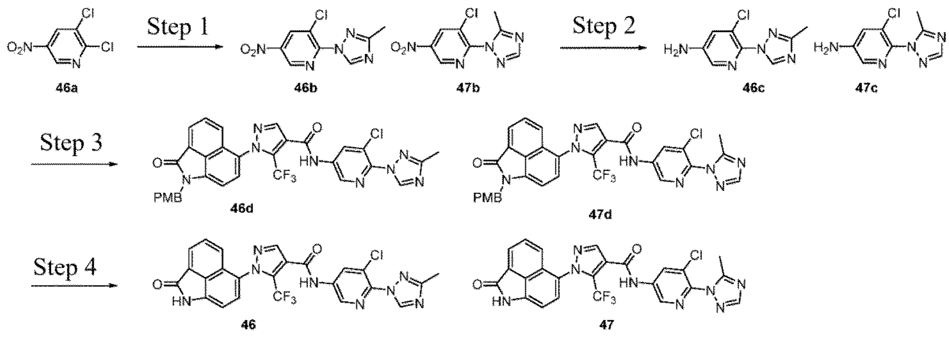

46a 46b 47b 46c 47c 46d 47d 46 47

Step 1

3-chloro-2-(3-methyl-1H-1,2,4-triazol-1-yl)-5-nitropyridine and 3-chloro-2-(5-methyl-1H-1,2,4-triazol-1-yl)-5-nitropyridine

2,3-dichloro-5-nitropyridine **46a** (3 g, 15.54 mmol), cesium carbonate (5 g, 15.34 mmol), 3-methyl-1H-1,2,4-triazole (1.3 g, 15.65 mmol) were dissolved in 30 ml of N,N-dimethylformamide and the mixture was reacted at 20°C for 12 h. The reaction liquid was cooled to room temperature and poured into 50 mL of water. The obtained mixture was filtered, the filtrate was extracted with ethyl acetate (50 mL×3), the organic phases were combined, washed with saturated sodium chloride solution (50 mL×2), dried over anhydrous sodium sulfate, filtered and concentrated to obtain a crude product, and the obtained residue was purified by silica gel column chromatography with eluent system A to obtain the title product 3-chloro-2-(3-methyl-1H-1,2,4-triazol-1-yl)-5-nitropyridine **46b** and 3-chloro-2-(5-methyl-1H-1,2,4-triazol-1-yl)-5-nitropyridine **47b** (2 g), yield: 53.7%.
MS m/z (ESI): 240 [M+1]

Referring to the synthesis method of step 1 in Example 40 and step 5 and step 6 in Example 29, N-(5-chloro-6-(3-methyl-1H-1,2,4-triazol-1-yl)pyridin-3-yl)-1-(2-carbonyl-1,2-dihydrobenzo[cd]indol-6-yl)-5-(trifluoromethyl)-1H-pyrazole-4-carboxamide **46** (20 mg) (yield:0.4%) and N-(5-chloro-6-(5-methyl-1H-1,2,4-triazol-1-yl)pyridin-3-yl)-1-(2-carbonyl-1,2-dihydrobenzo[cd]indol-6-yl)-5-(trifluoromethyl)-1H-pyrazole-4-carboxamide **47** (10 mg) (yield: 0.2%) were obtained through 3-chloro-2-(3-methyl-1H-1,2,4-triazol-1-yl)-5-nitropyridine **46b** and 3-chloro-2-(5-methyl-1H-1,2,4-triazol-1-yl)-5-nitropyridine **47b** (2 g, 8.35 mmol).

MS m/z (ESI): 539 [M+1].

Example 46: 1H NMR (400 MHz, DMSO) δ11.30 (s, 1H), 11.13 (s, 1H), 8.88 (d, J = 2.3 Hz, 1H), 8.68 (d, J = 2.3 Hz, 1H), 8.54 (s, 1H), 8.16 (d, J = 6.9 Hz, 1H), 8.12 (s, 1H), 7.92 (dd, J = 8.3, 7.0 Hz, 1H), 7.73 (d, J = 7.5 Hz, 1H), 7.61 (d, J = 8.3 Hz, 1H), 7.13 (d, J = 7.5 Hz, 1H), 2.37 (s, 3H).

Example 47: 1H NMR (400 MHz, DMSO) δ 11.15 (s, 2H), 8.91 (s, 1H), 8.80 (d, J = 2.3 Hz, 1H), 8.64 (d, J = 2.2 Hz, 1H), 8.53 (s, 1H), 8.15 (d, J = 6.9 Hz, 1H), 7.91 (dd, J = 8.3, 7.0 Hz, 1H), 7.72 (d, J = 7.5 Hz, 1H), 7.61 (d, J = 8.2 Hz, 1H), 7.12 (d, J = 7.5 Hz, 1H), 2.39 (s, 3H).

**Example 48**

N-(6-(azetidin-1-yl)-5-chloropyridin-3-yl)-1-(2-carbonyl-1,2-dihydrobenzo[cd]indol-6-yl)-5-(trifluoromethyl)-1H-pyrazole-4-carboxamide

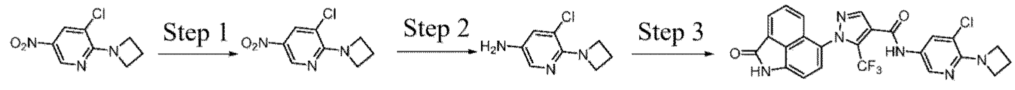


48a 48b 48c 48

Referring to the synthesis method of step 1 in Examples **30,** step 2 in Example **44,** step 11 in Example 1 in sequence, N-(6-(azetidin-1-yl)-5-chloropyridin-3-yl)-1-(2-carbonyl-1,2-dihydrobenzo[cd]indol-6-yl)-5-(trifluoromethyl)-1H-pyrazole-4-carboxamide **48** (5 mg) was obtained through the reaction of 2,3-dichloro-5-nitropyridine **48a** (150 mg, 0.70 mmol), yield: 1.4%.

MS m/z (ESI): 513 [M+1].

1H NMR (400 MHz, DMSO) δ 11.11 (s, 1H), 10.59 (s, 1H), 8.43 (s, 1H), 8.35 (d, J = 2.2 Hz, 1H), 8.14 (d, J = 6.9 Hz, 1H), 8.06 (d, J = 2.2 Hz, 1H), 7.90 (dd, J = 8.3, 7.0 Hz, 1H), 7.69 (d, J = 7.5 Hz, 1H), 7.58 (d, J = 8.3 Hz, 1H), 7.11 (d, J = 7.5 Hz, 1H), 4.13 (t, J = 7.5 Hz, 4H), 2.32 - 2.20 (m, 2H).

## Example 49

*N*-(5-difluoromethyl-6-(2H-1,2,3-triazol-2-yl)-pyridin-3-yl)-1-(2-oxo-1,2-dihydrobenzo[*cd*]indol-6-yl)-5-trifluoromethyl-1*H*-pyrazole-4-carboxamide

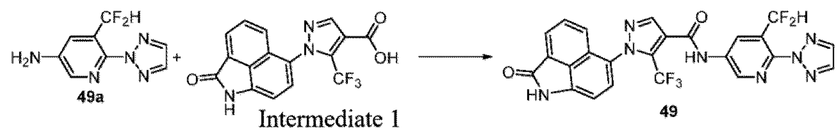

49a Intermediate 1 49

Referring to the synthesis method of the step 11 in Example 1, the title product N-(5-difluoromethyl-6-(2H-1,2,3-triazol-2-yl)-pyridin-3-yl)-1-(2-oxo-1,2-dihydrobenzo[cd]indol-6-yl)-5-trifluoromethyl-1H-pyrazole-4-carboxamide(21 mg) was obtained through 5-difluoromethyl-6-(2H-1,2,3-triazol-2-yl)pyridin-3-amine (21 mg, 0.10 mmol, prepared by the well-known method "WO 2018020474") and intermediate **1** (34 mg, 0.10 mmol), yield: 38.8%.

MS m/z (ESI): 541 [M+1]

$^1$H NMR (400 MHz, DMSO-$d_6$) δ 11.13 (bs, 2H), 9.07 (d, 1H), 8.78 (d, 1H), 8.56 (s, 1H), 8.25 (s, 2H), 8.16 (d, 1H), 7.92 (dd, 1H), 7.74 (d, 1H), 7.62 (d, 1H), 7.40 (t, 1H), 7.13 (t, 1H).

## Example 50

*N*-(4-(2H-1,2,3-triazol-2-yl)-3-(trifluoromethyl)phenyl)-1-(2-oxo-1,2-dihydrobenzo[*cd*]indol-6-yl)-5-(trifluoromethyl)-1*H*-pyrazole-4-carboxamide

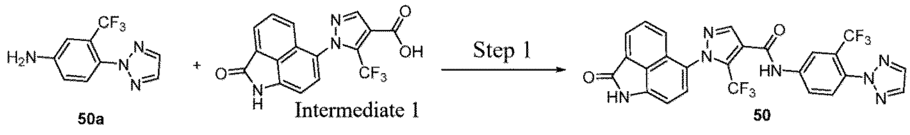


50a Intermediate 1 50

Referring to the synthesis method of step 11 in Example 1, the title product *N*-(4-(2*H*-1,2,3-triazol-2-yl)-3-(trifluoromethyl)phenyl)-1-(2-oxo-1,2-dihydrobenzo[*cd*]indol-6-yl)-5-(trifluoromethyl)-1*H*-pyrazole-4-carboxamide **50** (35 mg) was obtained through intermediate **1** (70 mg, 0.22 mmol) and 4-(2*H*-1,2,3-triazol-2-yl)-3-(trifluoromethyl)aniline **50a** (100 mg, 0.44 mmol, synthesized by the well-known method "Patent WO 2018020474 A1"), yield: 28.5%.

MS m/z(ESI): 558 [M+1]

$^1$H NMR(400 MHz, DMSO-$d_6$)δ 11.12(d, *J* = 4.4 Hz, 2H), 8.54(s, 1H), 8.41(d, *J* = 2.2 Hz, 1H), 8.23(dd, *J* = 8.7, 2.2 Hz, 1H), 8.17 - 8.12(m, 3H), 7.92(dd, *J* = 8.2, 7.1 Hz, 1H), 7.82(d, *J* = 8.8 Hz, 1H), 7.72(d, *J* = 7.5 Hz, 1H), 7.61(d, *J* = 8.3 Hz, 1H), 7.12(d, *J* = 7.5 Hz, 1H).

**Example 51**

*N*-(4-(1*H*-pyrazol-1-yl)-3-(trifluoromethyl)phenyl)-1-(2-carbonyl-1,2-dihydrobenzo[*cd*]indol-6-yl)-5-(trifluoromethyl)-1*H*-pyrazole-4-carboxamide

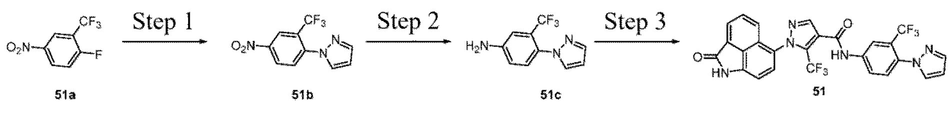

51a 51b 51c 51

Step 1

1-(4-nitro-2-(trifluoromethyl)phenyl)-1*H*-pyrazole

1-fluoro-4-nitro-2-(trifluoromethyl)benzene **51a** (1.00 g, 4.78 mmol), potassium carbonate (661 mg, 4.78 mmol), pyrazole (488 mg, 7.17 mmol) and DMF (3 mL) were added to a large microwave tube, and the mixture was heated to 100°C in a microwave reactor and reacted for 2 h. The reaction liquid was filtered and concentrated. The residue was dissolved in 100 mL of ethyl acetate, and the obtained product was washed with water (20 mL×2), washed with saturated sodium chloride aqueous solution (30 mL×1), dried over anhydrous sodium sulfate, filtered, and concentrated, and the obtained residue was purified by silica gel column chromatography with eluent system C to obtain the title product 1-(4-nitro-2-(trifluoromethyl)phenyl)-1*H*-pyrazole **51b** (1.00 g), yield: 81.3%.
MS m/z (ESI):258 [M+1]
Referring to the synthesis method of step 2 to step 3 in **Example36,** *N*-(4-(1*H*-pyrazol-1-yl)-3-(trifluoromethyl)phenyl)-1-(2-carbonyl-1,2-dihydrobenzo[*cd*]indol-6-yl)-5-(trifluoromethyl)-1*H*-pyrazole-4-carboxamide **51** (29 mg) was obtained through 1-(4-nitro-2-(trifluoromethyl)phenyl)-1*H*-pyrazole **51b** (0.30 g, 1.17 mmol), yield: 32.5%.

MS m/z (ESI):558 [M+1]
$^1$H NMR (400 MHz, DMSO): δ 11.12 (s, 1H), 11.07 (s, 1H), 8.53 (s, 1H), 8.35 (d, *J* = 2.4 Hz, 1H), 8.19-8.12 (m, 2H), 8.02 (d, *J* = 2.4 Hz, 1H), 7.92 (dd, *J* = 8.3, 7.0 Hz, 1H), 7.78-7.69 (m, 2H), 7.63 (dd, *J* = 13.5, 8.5 Hz, 2H), 7.13 (d, *J* = 7.5 Hz, 1H), 6.53 (t, *J* = 2.2 Hz, 1H).

**Example 52**

1-(2-carbonyl-1,2-dihydrobenzo[cd]indol-6-yl)-N-(4-(pyrrolidine-1-carbonyl)-3-(trifluoromethyl)phenyl)-5-(trifluoromethyl)-1H-pyrazole-4-carboxamide

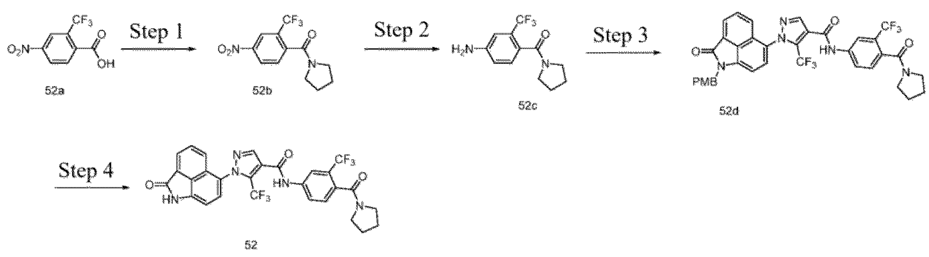

52a 52b 52c 52d 52

Referring to the synthesis method of step 1 in Example 45 and step 2 to step 4 in Example 46, 1-(2-carbonyl-1,2-dihydrobenzo[cd]indol-6-yl)-N-(4-(pyrrolidine-1-carbonyl)-3-(trifluoromethyl)phenyl)-5-(trifluoromethyl)-1H-pyrazole-4-carboxamide (20 mg) was obtained through 4-nitro-2-(trifluoromethyl)benzoic acid **52a** (2 g, 8.50 mmol), yield: 0.4%.
MS m/z (ESI): 588 [M+1].
1H NMR (400 MHz, $CD_3OD$) δ 8.30 (s, 1H), 8.23 (d, J = 2.1 Hz, 1H), 8.15 (d, J = 7.0 Hz, 1H), 8.06 (dd, J = 8.4, 2.2 Hz, 1H), 7.87 (dd, J = 8.3, 7.0 Hz, 1H), 7.66 (dd, J = 9.2, 7.9 Hz, 2H), 7.50 (d, J = 8.3 Hz, 1H), 7.13 (d, J = 7.5 Hz, 1H), 3.60 (t, J = 6.9 Hz, 2H), 3.22 (t, J = 6.7 Hz, 2H), 2.04 - 1.84 (m, 5H).

**Example 53**

*N*-(3-chloro-4-(2*H*-1,2,3-triazol-2-yl)phenyl)-1-(2-oxo-1,2-dihydrobenzo[*cd*]indol-6-yl)-5-(trifluoromethyl)-1*H*-pyrazole-4-carboxamide

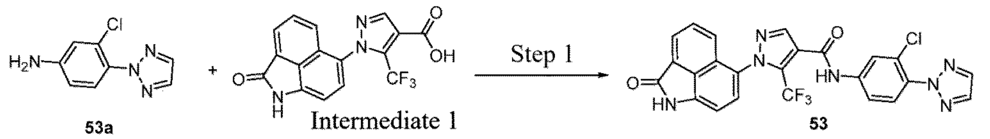


53a Intermediate 1 53

Intermediate **1** (70 mg, 0.22 mmol), 3-chloro-4-(2*H*-1,2,3-triazol-2-yl)aniline **53a** (85 mg, 0.44 mmol, synthesized by the well-known method "Patent WO 2018020474 A1"), pyridine (76 mg, 0.96 mmol) and phosphorus oxychloride (73 mg, 0.48 mmol) were dissolved in 5 mL of dichloromethane and the mixture was reacted under stirring at room temperature for 2 h. Dichloromethane (50 mL) was added to the reaction liquid, and the obtained product was washed with water (50 mL×1) and saturated sodium chloride solution (50 mL×1) in sequence, dried over anhydrous sodium sulfate, filtered, and the filtrate was concentrated under reduced pressure, and the obtained residue was purified by silica gel column chromatography using eluent system A to obtain the title product *N*-(3-chloro-4-(2*H*-1,2,3-triazol-2-yl)phenyl)-1-(2-oxo-1,2-dihydrobenzo[*cd*]indol-6-yl)-5-(trifluoromethyl)-1*H*-pyrazole-4-carboxamide **53** (36 mg), yield: 31.2%.

MS m/z(ESI): 524 [M+1]
$^1$H NMR(400 MHz, DMSO-$d_6$) δ 11.12(s, 1H), 11.00(s, 1H), 8.52(s, 1H), 8.19 - 8.13(m, 4H), 7.95 - 7.84(m, 2H), 7.77 - 7.69(m, 2H), 7.61(d, *J* = 8.3 Hz, 1H), 7.12(d, *J* = 7.5 Hz, 1H).

**Example 54**

*N*-(3-chloro-4-(1*H*-pyrazol-1-yl)phenyl)-1-(2-carbonyl-1,2-dihydrobenzo[*cd*]indol-6-yl)-5-(trifluoromethyl)-1*H*-pyrazole-4-carboxamide

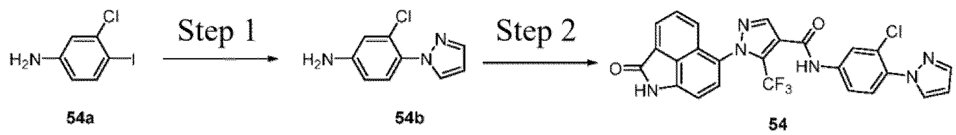


54a 54b 54

Step 1

3-chloro-4-(1*H*-pyrazol-1-yl)aniline

3-chloro-4-iodo-aniline **54a** (1.00 g, 3.95 mmol), pyrazole (564 mg, 8.29 mmol), cesium carbonate (2.44 g, 7.50 mmol), copper iodide (75 mg, 0.39 mmol), (*1R*,*2R*)-(-)-*N*,*N*-dimethyl-1,2-cyclohexanediamine (168 mg, 1.18 mmol) were added to a three-necked flask, nitrogen replacement was performed three times, 10 mL DMF was added, and the system was heated to 140°C and reacted for 3 h. The reaction was cooled to room temperature, filtered, and concentrated. The residue was dissolved in 100 mL of ethyl acetate, and washed with water (20 mL×2) and saturated sodium chloride aqueous solution (30 mL×1) in sequence, dried over anhydrous sodium sulfate, filtered and concentrated. The obtained residue was purified by silica gel column chromatography with eluent system C to obtain the title product 3-chloro-4-(1*H*-pyrazol-1-yl)aniline **54b** (650 mg), yield: 85.1%.
MS m/z (ESI):194 [M+1]

Step 2

*N*-(3-chloro-4-(1*H*-pyrazol-1-yl)phenyl)-1-(2-carbonyl-1,2-dihydrobenzo[*cd*]indol-6-yl)-5-(trifluoromethyl)-1*H*-pyrazole-4-carboxamide

Referring to the synthesis method of step 11 in Example **1,** the title product *N*-(3-chloro-4-(1*H*-pyrazol-1-yl)phenyl)-1-(2-carbonyl-1,2-dihydrobenzo[*cd*]indol-6-yl)-5-(trifluoromethyl)-1*H*-pyrazole-4-carboxamide **54** (15 mg) was obtained through intermediate **1** (30 mg, 0.09 mmol) and 6-(4,5-dimethyloxazol-2-yl)-5-(trifluoromethyl)pyridin-3-amine **54b** (35 mg, 0.18 mmol, synthesized using the well-known method "Patent US20180170909A1"), yield: 31.9%.

MS m/z (ESI):523 [M+1]

$^1$H NMR (400 MHz, DMSO): δ 11.12 (s, 1H), 11.07 (s, 1H), 8.53 (s, 1H), 8.35 (d, *J* = 2.4 Hz, 1H), 8.19-8.12 (m, 2H), 8.02 (d, *J* = 2.4 Hz, 1H), 7.92 (dd, *J* = 8.3, 7.0 Hz, 1H), 7.78-7.69 (m, 2H), 7.63 (dd, **J** = 13.5, 8.5 Hz, 2H), 7.13 (d, *J* = 7.5 Hz, 1H), 6.53 (t, *J* = 2.2 Hz, 1H).

**Example 55**

5-cyclopropyl-1-(2-oxo-1,2-dihydrobenzo[*cd*]indol-6-yl)-*N*-(2-(trifluoromethyl)pyridin-4-yl)-1*H*-pyrazole-4-carboxamide

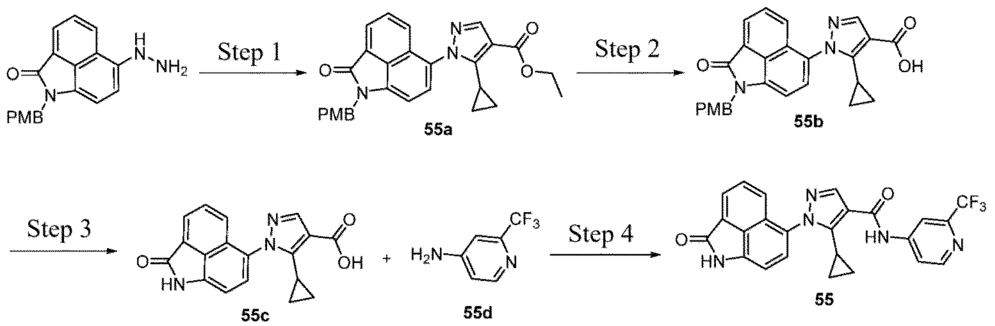


55a 55b 55c 55d 55

Step 1

Ethyl 5-cyclopropyl-1-(1-(4-methoxybenzyl)-2-oxo-1,2-dihydrobenzo[*cd*]indol-6-yl)-1*H*-pyrazole-4-carboxylate

6-hydrazino-1-[(4-methoxybenzyl]benzo[*cd*]indol-2(1*H*)-one (5.10 g, 15.99 mmol), potassium carbonate (4.14 g, 30 mmol) and ethyl 2-(cyclopropylcarbonyl)-3-(dimethylamino) acrylate (6.33 g, 30 mmol) were dissolved in 50 mL of ethanol, and the mixture was reacted under stirring at 80°C for 3 h. The reaction liquid was concentrated under reduced pressure, and the obtained residue was purified by silica gel column chromatography with eluent system A to obtain the title product ethyl 5-cyclopropyl-1-(1-(4-methoxybenzyl)-2-oxo-1,2-dihydrobenzo[*cd*]indol-6-yl)-1H-pyrazole-4-carboxylate **55a** (1.60 g), yield: 21.4%.

MS m/z(ESI): 468 [M+1].

Step 2

5-cyclopropyl-1-(1-(4-methoxybenzyl)-2-oxo-1,2-dihydrobenzo[cd]indol-6-yl)-1H-pyrazole-4-carboxylic acid

Ethyl 5-cyclopropyl-1-(1-(4-methoxybenzyl)-2-oxo-1,2-dihydrobenzo[*cd*]indol-6-yl)-1*H*-pyrazole-4-carboxylate **55a** (1.6 g, 3.42 mmol) and sodium hydroxide (673 mg, 17.11 mmol) were dissolved in 10 mL of tetrahydrofuran and 5 mL of water, and the mixture was reacted under stirring at 60°C for 16 h. 2M hydrochloric acid was added to the reaction liquid to adjust the pH to less than 7, the obtained mixture was extracted with ethyl acetate (50 mL×3), the organic phases were combined, and washed with water (50 mL×1) and saturated sodium chloride solution (50 mL×1) in sequence, dried over anhydrous sodium sulfated, and filtered, and the filtrate was concentrated under reduced pressure to obtain the title product 5-cyclopropyl-1-(1-(4-methoxybenzyl)-2-oxo-1,2-dihydrobenzo[*cd*]indol-6-yl)-1*H*-pyrazole-4-carboxylic acid **55b** (1.45 g), yield: 96.5%.

MS m/z (ESI): 440 [M+1]

Step 3

5-cyclopropyl-1-(2-oxo-1,2-dihydrobenzo[*cd*]indol-6-yl)-1*H*-pyrazole-4-carboxylic acid

5-cyclopropyl-1-(1-(4-methoxybenzyl)-2-oxo-1,2-dihydrobenzo[cd]indol-6-yl)-1*H*-pyrazole-4-carboxylic acid **55b** (1.45 g, 3.30 mmol) was dissolved in 20 mL of trifluoroacetic acid, and the mixture was reacted under stirring at 90°C for 16 h. The reaction liquid was filtered, and the filtrate was concentrated under reduced pressure. The obtained residue was purified by silica gel column chromatography with eluent system A to obtain the title product 5-cyclopropyl-1-(2-oxo-1,2-dihydrobenzo[*cd*]indol-6-yl)-1*H*-pyrazole-4-carboxylic acid **55c** (794 mg), yield: 75.2%. MS m/z (ESI): 320 [M+1]

Step 4

5-cyclopropyl-1-(2-oxo-1,2-dihydrobenzo[*cd*]indol-6-yl)-N-(2-trifluoromethylpyridin-4-yl)-1*H*-pyrazole-4-carboxamide

5-cyclopropyl-1-(2-oxo-1,2-dihydrobenzo[*cd*]indol-6-yl)-1*H*-pyrazole-4-carboxylic acid **55c** (64 mg, 0.20 mmol), 2-trifluoromethyl-4-aminopyridine (31 mg, 0.19 mmol), pyridine (76 mg, 0.96 mmol) and phosphorus oxychloride (73 mg, 0.48 mmol) were dissolved in 5 mL of dichloromethane, and the mixture was reacted under stirring at room temperature for 2 h. Dichloromethane (50 mL) was added to the reaction liquid, and the obtained product was washed with water (50 mL×1) and saturated sodium chloride solution (50 mL×1) in sequence, dried over anhydrous sodium sulfate, filtered, and the filtrate was concentrated under reduced pressure, and the obtained residue was purified by silica gel column chromatography using eluent system A to obtain the title product 5-cyclopropyl-1-(2-oxo-1,2-dihydrobenzo[*cd*]indol-6-yl)-N-(2-trifluoromethylpyridin-4-yl)-1*H*-pyrazole-4-carboxamide **55** (32 mg), yield: 34.4%.

MS m/z (ESI): 464 [M+1].

$^1$H NMR(400 MHz, DMSO-$d_6$)δ 11.05(s, 1H), 10.63(s, 1H), 8.67(d, *J* = 5.5 Hz, 1H), 8.32(s, 1H), 8.29(d, *J* = 1.8 Hz, 1H), 8.13(d, *J* = 6.9 Hz, 1H), 8.01(dd, *J* = 5.6, 1.7 Hz, 1H), 7.91 - 7.84(m, 1H), 7.70(dd, *J* = 24.2, 7.9 Hz, 2H), 7.11(d, *J* = 7.5 Hz, 1H), 2.01(td, *J* = 8.6, 4.3 Hz, 1H), 0.75 - 0.50(m, 4H).

**Example 56**

*N*-(6-(2*H*-1,2,3-triazol-2-yl)-5-(trifluoromethyl)pyridin-3-yl)-5-cyclopropyl-1-(2-oxo-1,2-dihydrobenzo[cd]indol-6-yl)-1*H*-pyrazole-4-carboxamide

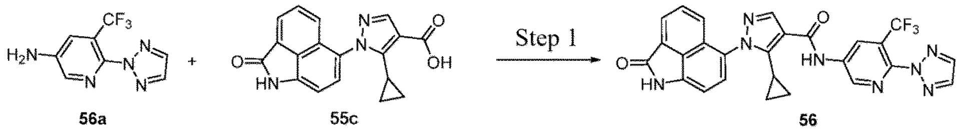


Referring to the synthesis method of the step 11 in Example 1, the title product *N*-(6-(2*H*-1,2,3-triazol-2-yl)-5-(trifluoromethyl)pyridin-3-yl)-5-cyclopropyl-1-(2-oxo-1,2-dihydrobenzo[*cd*]indol-6-yl)-1*H*-pyrazole-4-carboxamide **56** (31 mg) was obtained through **55c** (70 mg, 0.22 mmol) and 6-(2H-1,2,3-triazol-2-yl)-3-amino-5-trifluoromethylpyridine **56a** (100 mg, 0.44 mmol, synthesized by the well-known method "Patent US20180170909A1"), yield 26.6%.

MS m/z(ESI): 531 [M+1]
$^1$H NMR(400 MHz, DMSO-$d_6$)δ 11.05(s, 1H), 10.74(s, 1H), 9.21(d, *J* = 2.4 Hz, 1H), 8.93(d, *J* = 2.4 Hz, 1H), 8.36(s, 1H), 8.21(s, 2H), 8.13(d, *J* = 6.9 Hz, 1H), 7.92 - 7.86(m, 1H), 7.72(dd, *J* = 20.8, 7.9 Hz, 2H), 7.12(d, *J* = 7.5 Hz, 1H), 2.10 - 1.98(m, 1H), 0.77 - 0.67(m, 2H), 0.66 - 0.54(m, 2H).

**Example 57**

N-(6-(1H-pyrazol-1-yl)-5-(trifluoromethyl)pyridin-3-yl)-5-cyclopropyl-1-(2-carbonyl-1,2-dihydrobenzo[cd]indol-6-yl)-1H-pyrazole-4-carboxamide

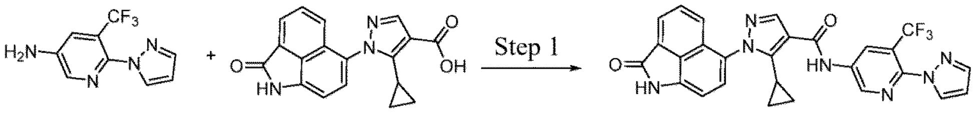


31c 55c 57

Referring to the synthesis method of step 11 in Example 1, N-(6-(1H-pyrazol-1-yl)-5-(trifluoromethyl)pyridin-3-yl)-5-cyclopropyl-1-(2-carbonyl-1,2-dihydrobenzo[cd]indol-6-yl)-1H-pyrazole-4-carboxamide **57** (23 mg) was obtained through 5-cyclopropyl-1-(2-carbonyl-1,2-dihydrobenzo[cd]indol-6-yl)-1H-pyrazole-4-carboxylic acid **55c** (50 mg, 0.16 mmol) and 3-trifluoromethyl-5-amino-2-(1H-pyrazol-1-yl)pyridine **31c** (35 mg, 0.16 mmol), yield: 25.6%. MS m/z (ESI): 530 [M+1].

**Example 58**

N-(6-(1H-pyrazol-1-yl)-5-(difluoromethyl)pyridin-3-yl)-5-cyclopropyl-1-(2-carbonyl-1,2-dihydrobenzo[cd]indol-6-yl)-1H-pyrazole-4-carboxamide

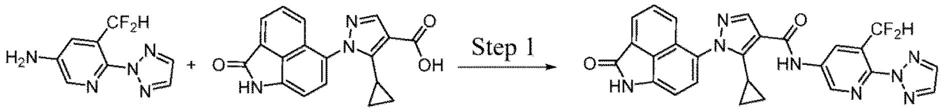


49a 55c 58

Referring to the synthesis method of step 11 in Example 1, N-(6-(1H-pyrazol-1-yl)-5-(difluoromethyl)pyridin-3-yl)-5-cyclopropyl-1-(2-carbonyl-1,2-dihydrobenzo[cd]indol-6-yl)-1H-pyrazole-4-carboxamide **57** (17 mg) was obtained through 5-cyclopropyl-1-(2-carbonyl-1,2-dihydrobenzo[cd]indol-6-yl)-1H-pyrazole-4-carboxylic acid **55c** (50 mg, 0.16 mmol) and 3-difluoromethyl-5-amino-2-(1H-pyrazol-1-yl)pyridine **49a** (34 mg, 0.16 mmol), yield: 20%.

MS m/z (ESI): 513 [M+1].

**Example 59**

N-(5-chloro-6-(2H-1,2,3-triazol-2-yl)pyridin-3-yl)-5-cyclopropyl-1-(2-carbonyl-1,2-dihydrobenzo[cd]indol-6-yl)-1H-pyrazole-4-carboxamide

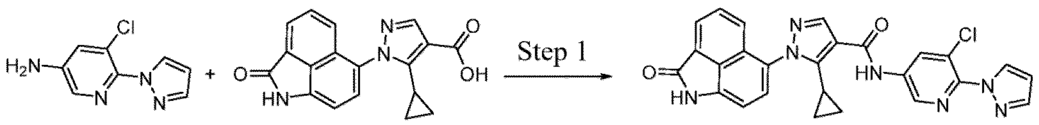


59a 55c 59

Referring to the synthesis method of step 11 in Example **1,** N-(5-chloro-6-(1H-pyrazol-1-yl)pyridin-3-yl)-5-cyclopropyl-1-(2-carbonyl-1,2-dihydrobenzo[cd]indol-6-yl)-1H-pyrazole-4-carboxamide **59** (13 mg) was obtained through the reaction of 5-cyclopropyl-1-(2-carbonyl-1,2-dihydrobenzo[cd]indol-6-yl)-1H-pyrazole-4-carboxylic acid **55c** (50 mg, 0.16 mmol) and 5-chloro-6-(1H-pyrazol-1-yl)pyridin-3-amine **59a** (31 mg, 0.16 mmol), yield: 16.3%.

MS m/z (ESI): 496 [M+1].

**Example 60**

*N*-(5-chloro-6-(2*H*-1,2,3-triazol-2-yl)-pyridin-3-yl)-5-cyclopropyl-1-(2-oxo-1,2-dihydrobenzo[*cd*]indol-6-yl)-1*H*-pyrazole-4-carboxamide

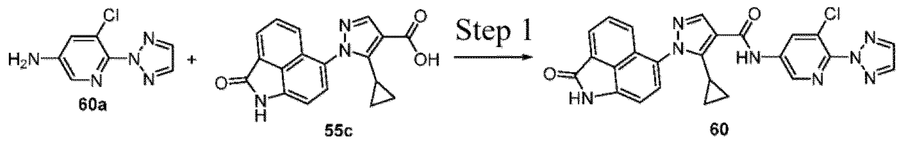


60a 55c 60

Referring to the synthesis method of the step 11 in Example 1, the title product *N*-(5-chloro-6-(2*H*-1,2,3-triazol-2-yl)-pyridin-3-yl)-5-cyclopropyl-1-(2-oxo-1,2-dihydrobenzo[cd]indol-6-yl)-1*H*-pyrazole-4-carboxamide **60** (25 mg) was obtained through **55c** (35 mg, 0.10 mmol) and 6-(2*H*-1,2,3-triazol-2-yl)-3-amino-5-chloropyridine **60a** (20 mg, 0.10 mmol, synthesized by the well-known method "Patent US20180170909A1"), yield 50.3%.

MS m/z (ESI): 497 [M+1]

$^1$H NMR (400 MHz, DMSO-$d_6$) δ 11.04 (s, 1H), 10.61 (s, 1H), 8.88 (d, 1H), 8.70 (d, 1H), 8.33 (s, 1H), 8.18 (s, 2H), 8.13 (d, 1H), 7.88 (dd, 1H), 7.74 (d, 1H), 7.68 (d, 1H), 7.11 (d, 1H), 2.02 (ddd, 1H), 0.77-0.68 (m, 2H), 0.64-0.55 (m, 2H).

## Example 61

N-(4-(2H-1,2,3-triazol-2-yl)-3-(trifluoromethyl)phenyl)-5-cyclopropyl-1-(2-carbonyl-1,2-dihydrobenzo[cd]indol-6-yl)-1H-pyrazole-4-carboxamide

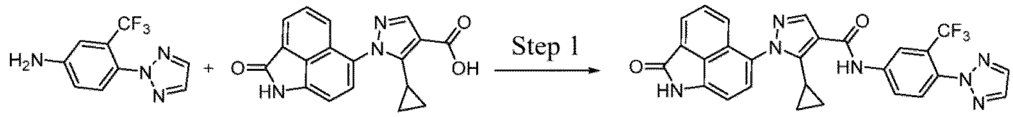


50c 55c 61

Referring to the synthesis method of step 11 in Example 1, N-(4-(2H-1,2,3-triazol-2-yl)-3-(trifluoromethyl)phenyl)-5-cyclopropyl-1-(2-carbonyl-1,2-dihydrobenzo[cd]indol-6-yl)-1H-pyrazole-4-carboxamide **61** (21 mg) was obtained through the reaction of 5-cyclopropyl-1-(2-carbonyl-1,2-dihydrobenzo[cd]indol-6-yl)-1H-pyrazole-4-carboxylic acid **55c** (50 mg, 0.16 mmol) and 3-chloro-5-amino-2-(1H-pyrazol-1-yl)pyridine **50c** (36 mg, 0.16 mmol), yield: 24.8%.

MS m/z (ESI): 530 [M+1].

1H NMR (400 MHz, DMSO) δ 11.04 (s, 1H), 10.50 (s, 1H), 8.44 (d, J = 2.3 Hz, 1H), 8.32 (s, 1H), 8.25 (dd, J = 8.8, 2.4 Hz, 1H), 8.14 (d, J = 13.5 Hz, 3H), 7.88 (dd, J = 8.3, 6.9 Hz, 1H), 7.76 (dd, J = 10.3, 8.6 Hz, 2H), 7.68 (d, J = 7.5 Hz, 1H), 7.11 (d, J = 7.5 Hz, 1H), 2.09 - 1.97 (m, 1H), 0.76 - 0.68 (m, 2H), 0.63 -0.55 (m, 2H).

## Example 62

*N*-(4-(1*H*-pyrazol-1-yl)-3-trifluoromethylphenyl)-5-cyclopropyl-1-(2-oxo-1,2-dihydrobenzo[*cd*]indol-6-yl)-1*H*-pyrazole-4-carboxamide

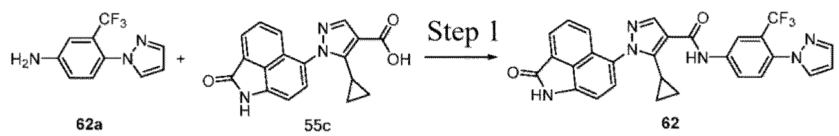


62a 55c 62

Referring to the synthesis method of the step 11 in Example 1, the title product *N*-(4-(1*H*-pyrazol-1-yl)-3-trifluoromethylphenyl)-5-cyclopropyl-1-(2-oxo-1,2-dihydrobenzo[*cd*]indol-6-yl)-1*H*-pyrazole-4-carboxamide **62** (18 mg) was obtained through **55c** (35 mg, 0.10 mmol) and 4-(1*H*-pyrazol-1-yl)- 3-trifluoromethylaniline **62a** (23 mg, 0.10 mmol, synthesized by the well-known method "Patent WO 2006122806"), yield 34.1%.

MS m/z (ESI): 529 [M+1]

**Example 63**

*N*-(3-chloro-4-(2*H*-1,2,3-triazol-2-yl)phenyl)-5-cyclopropyl-1-(2-oxo-1,2-dihydrobenzo[*cd*]indol-6-yl)-1*H*-pyrazole-4-carboxamide

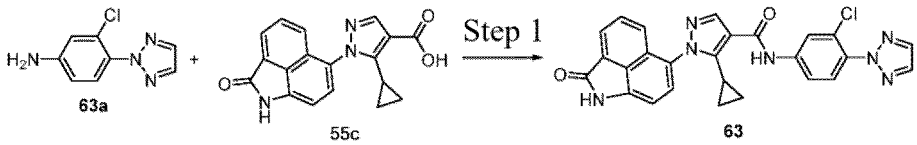


63a 55c 63

Referring to the synthesis method of the step 11 in Example 1, the title product *N*-(3-chloro-4-(2*H*-1,2,3-triazol-2-yl)phenyl)-5-cyclopropyl-1-(2-oxo-1,2-dihydrobenzo[*cd*]indol-6-yl)-1*H*-pyrazole-4-carboxamide **63** (19 mg) was obtained through **55c** (35 mg, 0.10 mmol) and 3-chloro-4-(2*H*-1,2, 3-triazol-2-yl) aniline **63a** (19 mg, 0.10 mmol, synthesized by the well-known method "Patent WO 2018020474A1"), yield 38.3%.

MS m/z (ESI): 496 [M+1]
$^1$H NMR (400 MHz, DMSO-$d_6$) δ 11.03 (s, 1H), 10.35 (s, 1H), 8.28 (s, 1H), 8.20 (d, 1H), 8.14 (s, 2H), 8.12 (d, 1H), 7.92-7.84 (m, 2H), 7.75 (d, 1H), 7.67 (dd, 2H), 7.11 (d, 1H), 2.06-1.95 (m, 1H), 0.75-0.66 (m, 2H), 0.65-0.54 (m, 2H).

**Example 64**

N-(3-chloro-4-(1H-pyrazol-1-yl)phenyl)-5-cyclopropyl-1-(2-carbonyl-1,2-dihydrobenzo[cd]indol-6-yl)-1H-pyrazole-4-carboxamide

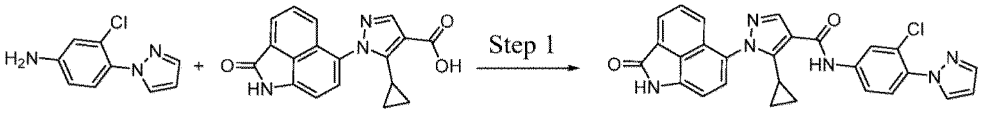


54b 55c 64

Referring to the synthesis method of step 11 in Example **1,** N-(3-chloro-4-(1H-pyrazol-1-yl)phenyl)-5-cyclopropyl-1-(2-carbonyl-1,2-dihydrobenzo[cd]indol-6-yl)-1H-pyrazole-4-carboxamide **64** (13 mg) was obtained through the reaction of 5-cyclopropyl-1-(2-carbonyl-1,2-dihydrobenzo[cd]indol-6-yl)-1H-pyrazole-4-carboxylic acid **55c** (50 mg, 0.16 mmol) and 3-chloro-4-(1H-pyrazol-1-yl)aniline **54b** (31 mg, 0.16 mmol), yield: 16.4%.

MS m/z (ESI): 495 [M+1].

1H NMR (400 MHz, DMSO) δ 11.04 (s, 1H), 9.66 (s, 1H), 8.63 - 8.54 (m, 1H), 8.27 (s, 1H), 8.15 - 8.05 (m, 2H), 7.93 - 7.83 (m, 3H), 7.83 - 7.75 (m, 2H), 7.67 (d, J = 7.5 Hz, 1H), 7.11 (d, J = 7.4 Hz, 1H), 6.62 - 6.54 (m, 1H), 2.04 (tt, J = 8.5, 5.5 Hz, 1H), 0.77 - 0.66 (m, 2H), 0.64 - 0.55 (m, 2H).

**Example 65**

5-difluoromethyl-1-(2-oxo-1,2-dihydrobenzo[*cd*]indol-6-yl)-*N*-(2-trifluoromethylpyridin-4-yl)-1*H*-pyrazole-4-carboxamide

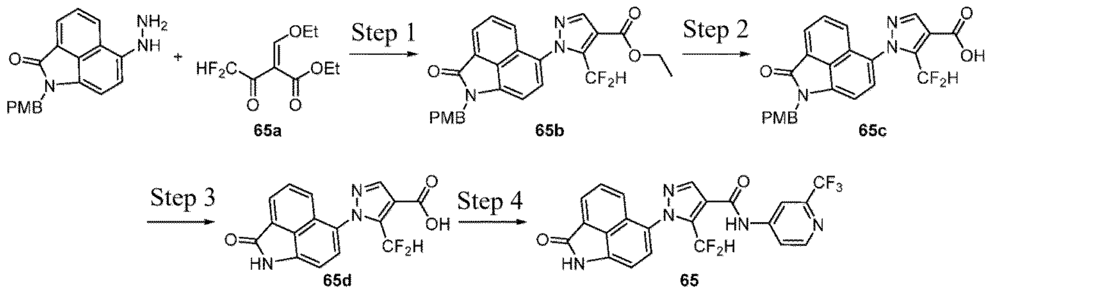


65a 65b 65c 65d 65

Referring to the synthesis method of step 8 to step 11 in Example 1, the title product 5-difluoromethyl-1-(2-oxo-1,2-dihydrobenzo[*cd*]indol-6-yl)-*N*-(2-trifluoromethylpyridin-4-yl)-1*H*-pyrazole-4-carboxamide **65** (19 mg) was obtained through 6-hydrazino-1-[(4-methoxybenzyl]benzo[*cd*]indol-2(1*H*)-one (320 mg, 1 mmol) and ethyl (Z)-2-ethoxymethylene-4,4-bisfluoro-3-oxobutyrate **65a** (444 mg, 2 mmol, synthesized by the well-known method "Patent WO 2019039429A1"), yield 4.0%.

MS m/z (ESI): 474 [M+1]

$^1$H NMR (400 MHz, DMSO-$d_6$) δ 11.10 (s, 1H), 11.02 (s, 1H), 8.72 (d, 1H), 8.62 (s, 1H), 8.32 (d, 1H), 8.13 (d, 1H), 8.03 (d, 1H), 7.87 (dd, 1H), 7.67 (d, 1H), 7.62 (d, 2H), 7.50 (t, 1H).

**Example 66**

*N*-(6-(2*H*-1,2,3-triazol-2-yl)-5-trifluoromethylpyridin-3-yl)-5-difluoromethyl-1-(2-oxo-1,2-dihydrobenzo[*cd*]indol-6-yl)-1*H*-pyrazole-4-carboxamide

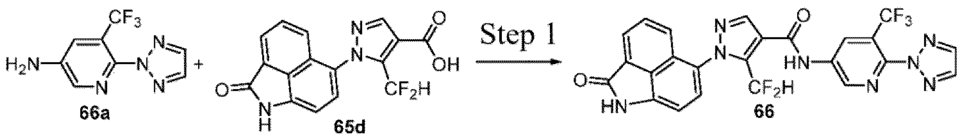


66a 65d 66

Referring to the synthesis method of the step 11 in Example 1, the title product *N*-(6-(2*H*-1,2,3-triazol-2-yl)-5-trifluoromethylpyridin-3-yl)-5-difluoromethyl-1-(2-oxo-1,2-dihydrobenzo[cd]indol-6-yl)-1*H*-pyrazole-4-carboxamide **66** (19 mg) was obtained through **65d** (35 mg, 0.10 mmol) and 3-trifluoromethyl-4-(2*H*-1,2,3-triazol-2-yl)aniline **66a** (23 mg, 0.10 mmol), yield 35.1%.

MS m/z (ESI): 541 [M+1]

$^1$H NMR (400 MHz, DMSO-$d_6$) δ 11.10 (s, 2H), 9.22 (d, 1H), 8.94 (d, 1H), 8.64 (s, 1H), 8.22 (s, 2H), 8.14 (d, 1H), 7.88 (dd, 1H), 7.69 (d, 1H), 7.63 (d, 1H), 7.63 (t, 1H), 7.12 (d, 1H).

**Example 67**

*N*-(6-(2*H*-1,2,3-triazol-2-yl)-5-chloropyridin-3-yl)-5- difluoromethyl-1-(2-oxo-1,2-dihydrobenzo[cd]indol-6-yl)-1*H*-pyrazole-4-carboxamide

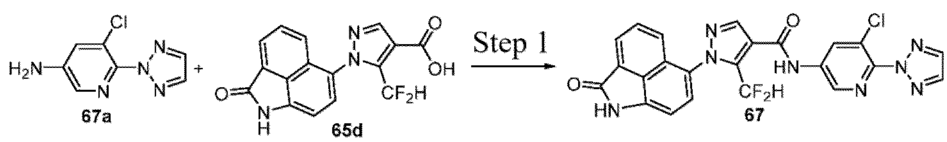


67a 65d 67

Referring to the synthesis method of the step 11 in Example 1, the title product *N*-(6-(2*H*-1,2,3-triazol-2-yl)-5-chloropyridin-3-yl)-5-difluoromethyl-1-(2-oxo-1,2-dihydrobenzo[*cd*]indol-6-yl)-1*H*-pyrazole-4-carboxamide **67** (17 mg)

was obtained through **65d** (35 mg, 0.10 mmol) and 3-chloro-4-(2*H*-1,2,3-triazol-2-yl)aniline **67a** (20 mg, 0.10 mmol), yield 38.3%.

MS m/z (ESI): 507 [M+1]
$^1H$ NMR (400 MHz, DMSO-$d_6$) δ 11.09 (s, 1H), 11.02 (s, 1H), 8.91 (d, 1H), 8.70 (d, 1H), 8.63 (s, 1H), 8.20 (s, 2H), 8.13 (d, 1H), 7.87 (dd, 1H), 7.68 (d, 1H), 7.62 (d, 1H), 7.59 (t, 1H), 7.11 (d, 1H).

**Example 68**

N-(6-(2H-1,2,3-triazol-2-yl)-5-(trifluoromethyl)pyridin-3-yl)-1-(2-carbonyl-1,2-dihydrobenzo[cd]indol-6-yl)-5-(trifluoromethyl)-1H-pyrazole-4-carboxamide

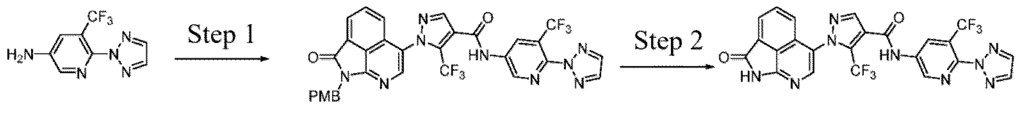


68a 68b 68

Referring to the synthesis method of step 11 and step 12 in Example 3, N-(6-(2H-1,2,3-triazol-2-yl)-5-(trifluoromethyl)pyridin-3-yl)-1-(2-carbonyl-1,2-dihydrobenzo[cd]indol-6-yl)-5-(trifluoromethyl)-1H-pyrazole-4-carboxamide **68** (20 mg) was obtained through 6-(2H-1,2,3-triazol-2-yl)-5-(trifluoromethyl)pyridin-3-amine **68a** (50 mg, 0.22 mmol), yield: 16.4%.

MS m/z (ESI): 560 [M+1].

1H NMR (400 MHz, $CD_3OD$) δ 8.30 (s, 1H), 8.23 (d, J = 2.1 Hz, 1H), 8.15 (d, J = 7.0 Hz, 1H), 8.06 (dd, J = 8.4, 2.2 Hz, 1H), 7.87 (dd, J = 8.3, 7.0 Hz, 1H), 7.66 (dd, J = 9.2, 7.9 Hz, 2H), 7.50 (d, J = 8.3 Hz, 1H), 7.13 (d, J = 7.5 Hz, 1H).

**Example 69**

N-(6-(1H-pyrazol-1-yl)-5-(trifluoromethyl)pyridin-3-yl)-1-(2-carbonyl-1,2-dihydropyrrolo[4,3,2-ij]isoquinolin-6-yl)-5-(trifluoromethyl)-1H-pyrazole-4-carboxamide

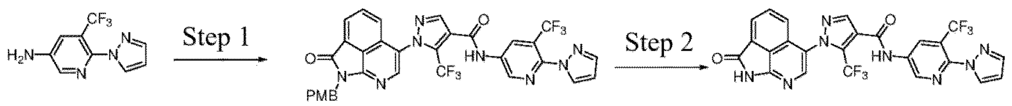


69a 69b 69

Referring to the synthesis method of step 11 and step 12 in Example 3, N-(6-(1H-pyrazol-1-yl)-5-(trifluoromethyl)pyridin-3-yl)-1-(2-carbonyl-1,2-dihydropyrrolo[4,3,2-ij]isoquinolin-6-yl)-5-(trifluoromethyl)-1H-pyrazole-4-carboxamide **69** (20 mg) was obtained through 6-(1H-pyrazol-1-yl)-5-(trifluoromethyl)pyridin-3-amine **69a** (50 mg, 0.22 mmol), yield: 16.4%.

MS m/z (ESI): 559 [M+1].

1H NMR (400 MHz, $CD_3OD$) δ 9.06 (s, 1H), 8.84 (s, 1H), 8.40 (s, 1H), 8.31 (s, 1H), 8.17 (s, 2H), 8.06 (t, J = 7.5 Hz, 1H), 7.78 (s, 1H), 7.72 (d, J = 8.2 Hz, 1H), 6.57 (s, 1H).

**Example 70**

N-(5-chloro-6-(2H-1,2,3-triazol-2-yl)pyridin-3-yl)-1-(2-carbonyl-1,2-dihydropyrrolo[4,3,2-ij]isoquinolin-6-yl)-5-(trifluoromethyl)-1H-pyrazole-4-carboxamide

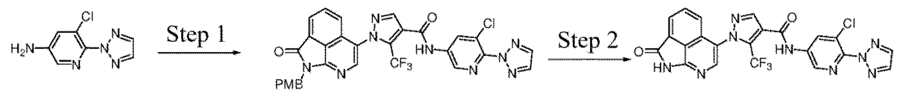


70a 70b 70

Referring to the synthesis method of step 11 and step 12 in Example 3, N-(5-chloro-6-(2H-1,2,3-triazol-2-yl)pyridin-3-yl)-1-(2-carbonyl-1,2-dihydropyrrolo[4,3,2-ij]isoquinolin-6-yl)-5-(trifluoromethyl)-1H-pyrazole-4-carboxamide **70** (20 mg) was obtained through 5-chloro-6-(2H-1,2,3-triazol-2-yl)pyridin-3-amine **70a** (50 mg, 0.26 mmol), yield: 14.9%.

MS m/z (ESI): 526 [M+1].

$^1$H NMR (400 MHz, $CD_3OD$) δ 9.06 (s, 1H), 8.84 (s, 1H), 8.40 (s, 1H), 8.31 (s, 1H), 8.17 (s, 2H), 8.06 (t, J = 7.5 Hz, 1H), 7.78 (s, 1H), 6.57 (s, 1H).

**Example 71**

*N*-(5-chloro-6-(1*H*-pyrazol-1-yl)pyridin-3-yl)-1-(2-carbonyl-1,2-dihydropyrrolo[4,3,2-*ij*]isoquinolin-6-yl)-5-(trifluoromethyl)-1*H*-pyrazole-4-carboxamide

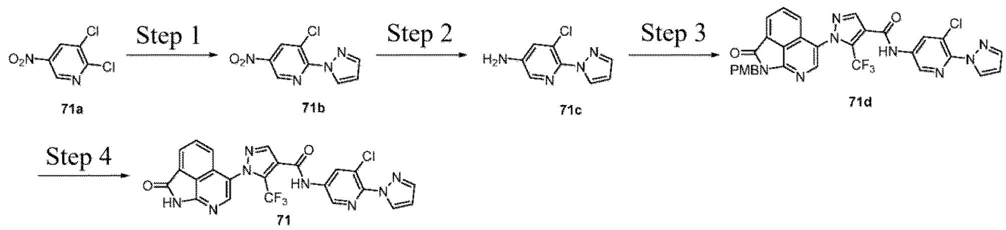


71a 71b 71c 71d 71

Referring to step 1 to step 2 in Example **36,** step 3 to step 4 in Example **73,** *N*-(5-chloro-6-(1*H*-pyrazol-1-yl)pyridin-3-yl)-1-(2-carbonyl-1,2-dihydropyrrolo[*4,3,2-ij*]isoquinolin-6-yl)-5-(trifluoromethyl)-1*H*-pyrazole-4-carboxamide **71** (13 mg) was obtained through 5-nitro-2,3-dichloropyridine **71a** (1.00 g, 5.18 mmol), yield: 0.5%.
MS m/z (ESI):525 [M+1]

**Example 72**

*N*-(3-(difluoromethyl)-4-(2H-1,2,3-triazol-2-yl)phenyl)-1-(2-carbonyl-1,2-dihydropyrrolo[*4,3,2-ij*]isoquinolin-6-yl)-5-(trifluoromethyl)-1*H*-pyrazole-4-carboxamide

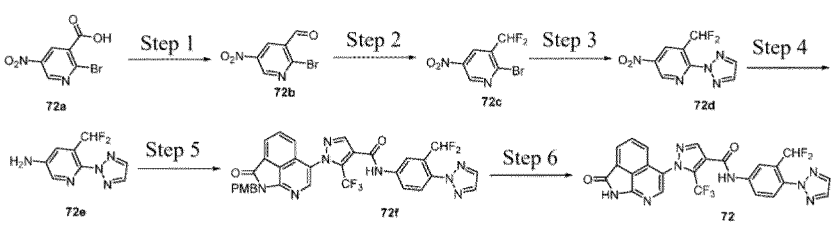


72a 72b 72c 72d 72e 72f 72

Step 1

2-bromo-5-nitronicotinaldehyde

2-bromo-5-nitronicotine acid **72a** (2.00 g, 8.10 mmol) was dissolved in dichloromethane (30 mL), nitrogen replacement was performed three times, and the mixture was cooled to -78°C. Diisobutylaluminum hydride (12.15 mL, 12.15 mmol, 1 M in hexane) was added dropwise to the system. After the addition was completed, the mixture was reacted at -78°C for 2 h. 10 mL of saturated ammonium chloride was added to quench the reaction, the obtained product

was extracted with dichloromethane (50 mL×3), the organic phases were combined, washed with saturated sodium chloride aqueous solution (30 mL×1), dried over anhydrous sodium sulfate, filtered and concentrated. The obtained residue was purified by silica gel column chromatography with eluent system C to obtain the title product 2-bromo-5-nitronicotinaldehyde **72b** (1.50 g), yield: 80.1%.
MS m/z (ESI): 232 [M+1]

Step 2

2-bromo-3-(difluoromethyl)-5-nitropyridine

2-bromo-5-nitronicotinaldehyde **72b** (1.20 g, 5.19 mmol) was dissolved in dichloromethane (20 mL), nitrogen replacement was performed three times, and the mixture was cooled to -78°C.

Diethylamine sulfur trifluoride (1.67 g, 10.39 mmol, 1.4 mL) was added dropwise to the system. After the addition was completed, the system was naturally warmed to 25°C and reacted under stirring for 2 h. Aqueous sodium carbonate solution was added to quench the reaction, the obtained product was extracted with dichloromethane (50 mL×3), the organic phases were combined, washed with saturated sodium chloride aqueous solution (30 mL), dried over anhydrous sodium sulfate, filtered and concentrated. The obtained residue was purified by silica gel column chromatography with eluent system C to obtain the title product 2-bromo-3-(difluoromethyl)-5-nitropyridine **72c** (1.10 g), yield: 83.7%.
MS m/z (ESI): 254 [M+1]

Step 3 to step 6

Referring to the synthesis method of step 1 to step 4 in Example **73,** *N*-(3-(difluoromethyl)-4-(2*H*-1,2,3-triazol-2-yl)phenyl)-1-(2-carbonyl-1,2-dihydropyrrolo[4,3,2-*ij*]isoquinolin-6-yl)-5-(trifluoromethyl)-1*H*-pyrazole-4-carboxamide **72** (11 mg) was obtained through 2-bromo-3-(difluoromethyl)-5-nitropyridine **72c** (1.10 g, 4.35 mmol), yield: 0.5%.
MS m/z (ESI):541 [M+1]

**Example 73**

*N*-(4-(*2H*-1,2,3-triazol-2-yl)-3-(trifluoromethyl)phenyl)-1-(2-carbonyl-1,2-dihydropyrrolo[*4,3,2-ij*]isoquinolin-6-yl)-5-(trifluoromethyl)-1*H*-pyrazole-4-carboxamide

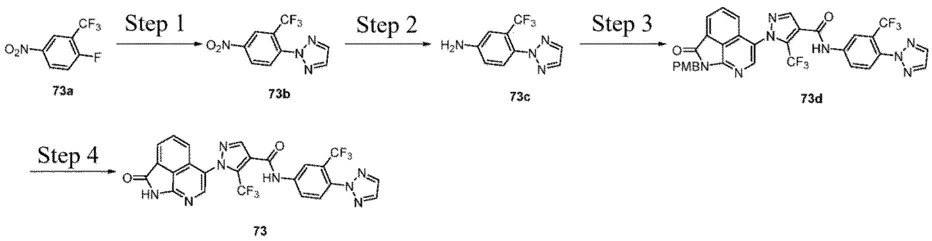


73a 73b 73c 73d

73

Step 1

2-(4-nitro-2-(trifluoromethyl)phenyl)-2*H*-1,2,3-triazole

1-fluoro-4-nitro-2-(trifluoromethyl)benzene **73a** (3.00 g, 14.35 mmol), potassium carbonate (5.95 g, 43.04 mmol), 1,2,3-triazole (1.49 g, 21.52 mmol) and DMF (40 mL) were added to a reaction bottle, nitrogen replacement was performed three times, and the reaction mixture was heated to 100°C for reaction for 2 h. The reaction was cooled to room temperature, filtered, and concentrated. The residue was dissolved in 100 mL of ethyl acetate, washed with water (20 mL × 2), washed with saturated sodium chloride aqueous solution (30 mL), dried over anhydrous sodium sulfate, filtered, and concentrated. The obtained residue was purified by silica gel column chromatography with eluent system C to obtain the title product 2-(4-nitro-2-(trifluoromethyl)phenyl)-2*H*-1,2,3-triazole **73b** (2.20 g), yield: 59.4%.
MS m/z (ESI):259[M+1]

Step 2

4-(2*H*-1,2,3-triazol-2-yl)-3-(trifluoromethyl)aniline

Referring to the synthesis method of step 2 in Example **34,** 4-(2*H*-1,2,3-triazol-2-yl)-3-(trifluoromethyl)aniline **73c** (1.50 g) was obtained through 2-(4-nitro-2-(trifluoromethyl)phenyl)-2*H*-1,2,3-triazole **73b** (2.20 g, 8.52 mmol), yield: 77.1%.
MS m/z (ESI):229 [M+1]

Step 3

*N*-(4-(2*H*-1,2,3-triazol-2-yl)-3-(trifluoromethyl)phenyl)-1-(1-(4-methoxybenzyl)-2-carbonyl-1,2-dihydropyrrolo[*4,3,2-ij*]isoquinolin-6-yl)-5-(trifluoromethyl)-1*H*-pyrazole-4-carboxamide

1-[2-[(4-methoxyphenyl)methyl]-3-carbonyl-2,11-diazatricyclo[6.3.1.04,12]dodecan-1(11),4(12),5,7,9-penten-9-yl]-5-(trifluoromethyl)pyrazole-4-carboxylic acid (80 mg, 0. 17 mmol), 4-(2*H*-1,2,3-triazol-2-yl)-3-(trifluoromethyl)aniline **73c** (77.94 mg, 0.34 mmol) and DMAP (4.17 mg, 0.034 mmol) were dissolved in dichloromethane (5 mL), nitrogen replacement was performed three times, the mixture was cooled to -10°C, and pyridine (68 mg, 0.85 mmol, 69 μL) and phosphorus oxychloride (131 mg, 0.85 mmol, 80 μL) were added. The reaction temperature was raised to 25°C for reaction for 2 h. The reaction was quenched by adding water and concentrated. The obtained mixture was extracted with ethyl acetate (30 mL×3), the organic phases were combined, washed with saturated sodium chloride aqueous solution (30 mL), dried over anhydrous sodium sulfate, filtered and concentrated. The obtained residue was purified by silica gel column chromatography with eluent system C to obtain the title product *N*-(4-(2*H*-1,2,3-triazol-2-yl)-3-(trifluoromethyl)phenyl)-1-(1-(4-methoxybenzyl)-2-carbonyl-1,2-dihydropyrrolo[*4,3,2-ij*]isoquinolin-6-yl)-5-(trifluoromethyl)-1*H*-pyrazole-4-carboxamide **73d** (56 mg), yield: 48.3%.
MS m/z (ESI): 679 [M+1]

Step 4

*N*-(4-(2*H*-1,2,3-triazol-2-yl)-3-(trifluoromethyl)phenyl)-1-(2-carbonyl-1,2-dihydropyrrolo[*4,3,2-ij*]isoquinolin-6-yl)-5-(trifluoromethyl)-1*H*-pyrazole-4-carboxamide

*N*-(4-(2*H*-1,2,3-triazol-2-yl)-3-(trifluoromethyl)phenyl)-1-(1-(4-methoxybenzyl)-2-carbonyl-1,2-dihydropyrrolo[*4,3,2-ij*]isoquinolin-6-yl)-5-(trifluoromethyl)-1*H*-pyrazole-4-carboxamide **73d** (56 mg, 0.082 mmol) was dissolved in acetonitrile (5 mL) and water (1 mL), ceric ammonium nitrate (88 mg, 0.17 mmol) was added, and the system was reacted at 25°C for 1 h. The reaction was quenched by adding water and concentrated. The obtained mixture was extracted with ethyl acetate (20 mL×3), the organic phases were combined, washed with saturated sodium chloride aqueous solution (20 mL), dried over anhydrous sodium sulfate, filtered and concentrated. The obtained residue was purified by silica gel column chromatography with eluent system A to obtain the title product *N*-(4-(2*H*-1,2,3-triazol-2-yl)-3-(trifluoromethyl)phenyl)-1-(2-carbonyl-1,2-dihydropyrrolo[*4,3,2-ij*]isoquinolin-6-yl)-5-(trifluoromethyl)-1*H*-pyrazole-4-carboxamide **73** (18 mg), yield: 39.1%.

MS m/z (ESI): 559 [M+1]
$^1$H NMR (400 MHz, DMSO-d6) δ 11.74 (d, 1H), 11.15 (s, 1H), 8.58 (s, 1H), 8.41 (m, 2H), 8.20 (m, 4H), 8.09 (dd, 1H), 7.83 (d, 1H), 7.68 (d, 1H).

**Example 74**

*N*-(4-(1*H*-pyrazol-1-yl)-3-(trifluoromethyl)phenyl)-1-(2-carbonyl-1,2-dihydropyrrolo[4,3,2-*ij*]isoquinolin-6-yl)-5-(trifluoromethyl)-1*H*-pyrazole-4-carboxamide

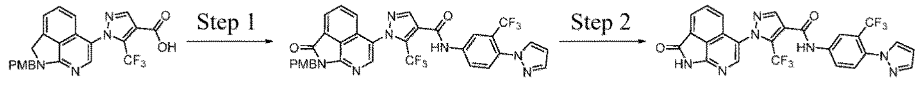


74a 74b 74

Referring to step 1 to step 2 in Example **73,** the title product *N*-(4-(1*H*-pyrazol-1-yl)-3-(trifluoromethyl)phenyl)-1-(2-carbonyl-1,2-dihydropyrrolo[*4,3,2-ij*]isoquinolin-6-yl)-5-(trifluoromethyl)-1*H*-pyrazole-4-carboxamide **74** (15 mg) was obtained through 1-[2-[(4-methoxyphenyl)methyl]-3-carbonyl-2,11-diazatricyclo[6.3.1.04,12]dodecan-1(11),4(12),5,7,9-penten-9-yl]-5-(trifluoromethyl)pyrazole-4-carboxylic acid **74a** (50 mg, 0.11 mmol), yield: 24.5%.

MS m/z (ESI):558 [M+1]

$^1$H NMR (400 MHz, DMSO): δ 11.09 (s, 1H), 8.57 (s, 1H), 8.42 (s, 1H), 8.34 (d, *J* = 2.4 Hz, 1H), 8.20 (d, *J* = 6.9 Hz, 1H), 8.13-8.07 (m, 1H), 8.02 (d, *J* = 2.4 Hz, 1H), 7.75 (d, *J* = 1.9 Hz, 1H), 7.67 (dd, *J* = 14.3, 8.4 Hz, 2H), 6.53 (t, *J* = 2.1 Hz, 1H).

**Example 75**

N-(3-chloro-4-(2H-1,2,3-triazol-2-yl)phenyl)-1-(2-carbonyl-1,2-dihydropyrrolo[4,3,2-ij]isoquinolin-6-yl)-5-(trifluoromethyl)-1H-pyrazole-4-carboxamide

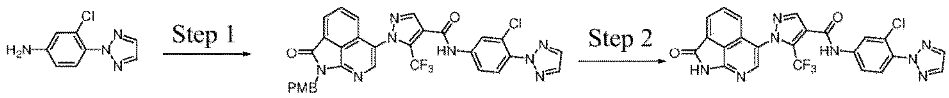


75a 75b 75

Referring to the synthesis method of step 11 and step 12 in Example 3, N-(3-chloro-4-(2H-1,2,3-triazol-2-yl)phenyl)-1-(2-carbonyl-1,2-dihydropyrrolo[4,3,2-ij]isoquinolin-6-yl)-5-(trifluoromethyl)-1H-pyrazole-4-carboxamide **75** (12 mg) was obtained through 3-chloro-4-(2H-1,2,3-triazol-2-yl)aniline **75a** (50 mg, 0.26 mmol), yield: 8.9%.

MS m/z (ESI): 525 [M+1].

1H NMR (400 MHz, $CD_3OD$) δ 8.33 (d, J = 12.5 Hz, 2H), 8.23 - 8.12 (m, 2H), 8.06 (dd, J = 8.2, 7.0 Hz, 1H), 7.99 (s, 2H), 7.81 (dd, J = 8.7, 2.3 Hz, 1H), 7.72 (d, J = 8.2 Hz, 1H), 7.62 (d, J = 8.7 Hz, 1H).

**Example 76**

*N*-(3-chloro-4-(1*H*-pyrazol-1-yl)phenyl)-1-(2-carbonyl-1,2-dihydropyrrolo[4,3,2-*ij*]isoquinolin-6-yl)-5-(trifluoromethyl)-1*H*-pyrazole-4-carboxamide

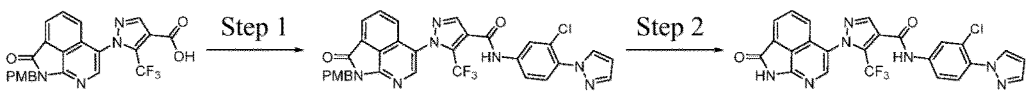


**76a** **76b** **76**

Referring to step 1 to step 2 in Example **73,** the title product *N*-(3-chloro-4-(1*H*-pyrazol-1-yl)phenyl)-1-(2-carbonyl-1,2-dihydropyrrolo[*4,3,2-ij*]isoquinolin-6-yl)-5-(trifluoromethyl)-1*H*-pyrazole-4-carboxamide **76** (13 mg) was obtained through 1-[2-[(4-methoxyphenyl)methyl]-3-carbonyl-2,11-diazatricyclo[6.3.1.04,12]dodecan-1(11),4(12),5,7,9-penten-9-yl]-5-(trifluoromethyl)pyrazole-4-carboxylic acid **76a** (50 mg, 0.11 mmol), yield: 21.2%.
MS m/z (ESI):524 [M+1]

**Example 77**

5-cyclopropyl-1-(2-oxo-1,2-dihydropyrrolo[4,3,2-*ij*]isoquinolin-6-yl)-*N*-(2-(trifluoromethyl)pyridin-4-yl)-1*H*-pyrazole-4-carboxamide

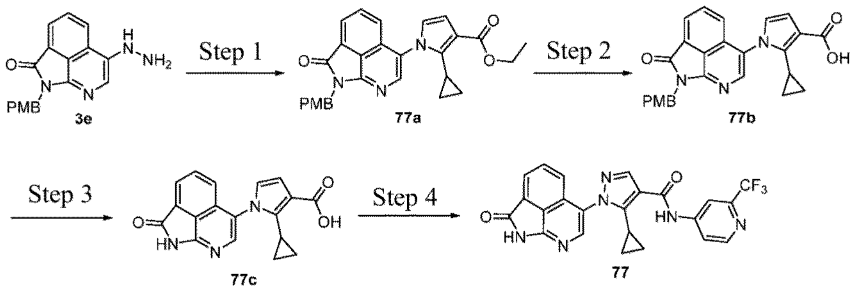


Referring to the synthesis method of step 1 in Example **55,** step 5 and step 6 of the intermediate **1**, and step 11 in Example 1, 5-cyclopropyl-1-(2-oxo-1,2-dihydropyrrolo[*4,3,2-ij*]isoquinolin-6-yl)-*N*-(2-(trifluoromethyl)pyridin-4-yl)-1*H*-pyrazole-4-carboxamide **77** (51 mg) was obtained through 6-hydrazino-1-(4-methoxybenzyl)pyrrolo[*4,3,2-ij*]isoquinolin-2(1H)-one **3e** (400 mg, 1.25 mmol), yield 8.8%.

MS m/z(ESI): 465 [M+1]
$^1$H NMR(400 MHz, DMSO-$d_6$)δ 11.64(s, 1H), 10.64(s, 1H), 8.67(d, *J* = 5.5 Hz, 1H), 8.40(s, 1H), 8.35(s, 1H), 8.28(d, *J* = 1.8 Hz, 1H), 8.17(d, *J* = 7.0 Hz, 1H), 8.08 - 7.98(m, 2H), 7.81(d, *J* = 8.2 Hz, 1H), 2.13 - 2.03(m, 1H), 0.82 - 0.71(m, 2H), 0.62 - 0.53(m, 2H).

### Example 78

*N*-(6-(2*H*-1,2,3-triazol-2-yl)-5-(trifluoromethyl)pyridin-3-yl)-5-cyclopropyl-1-(2-oxo-1,2-dihydropyrrolo[*4,3,2-ij*]isoquinolin-6-yl)-1*H*-pyrazole-4-carboxamide

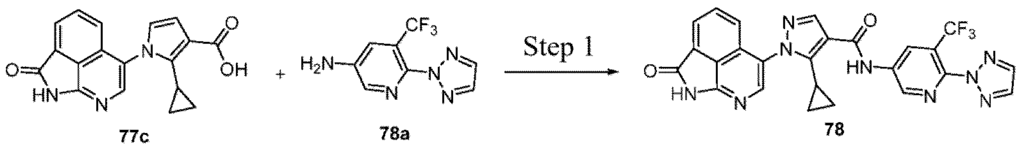


Referring to the synthesis method of the step 11 in Example 1, the title product *N*-(6-(2*H*-1,2,3-triazol-2-yl)-5-(trifluoromethyl)pyridin-3-yl)-5-cyclopropyl-1-(2-oxo-1,2-dihydropyrrolo[4,3,2-ij]isoquinolin-6-yl)-1H-pyrazole-4-carboxamide **78** (41 mg) was obtained through 2-cyclopropyl-1-(2-oxo-1,2-dihydropyrrolo[*4,3,2-ij*]isoquinolin-6-yl)-1*H*-pyrrol-3-carboxylic acid **77c** (70 mg, 0.22 mmol) and 6-(2*H*-1,2,3-triazol-2-yl)-3-amino-5-trifluoromethylpyridine **78a** (100 mg, 0.44 mmol, synthesized by the well-known method "Patent US20180170909A1"), yield 35.2%.

MS m/z(ESI): 531 [M+1]
$^1$H NMR (400 MHz, DMSO) δ 11.64 (s, 1H), 10.76 (s, 1H), 9.21 (d, *J* = 2.3 Hz, 1H), 8.92 (d, *J* = 2.3 Hz, 1H), 8.40 (d, *J* = 8.4 Hz, 2H), 8.21 - 8.15 (m, 3H), 8.08 - 8.02 (m, 1H), 7.82 (d, *J* = 8.2 Hz, 1H), 2.15 - 2.04 (m, 1H), 0.85 - 0.72 (m, 2H), 0.66 - 0.55 (m, 2H).

### Example 79

*N*-(6-(1*H*-pyrazol-1-yl)-5-(trifluoromethyl)pyridin-3-yl)-5-cyclopropyl-1-(2-oxo-1,2-dihydropyrrolo[4,3,2-ij]isoquinolin-6-yl)-1*H*-pyrazole-4-carboxamide

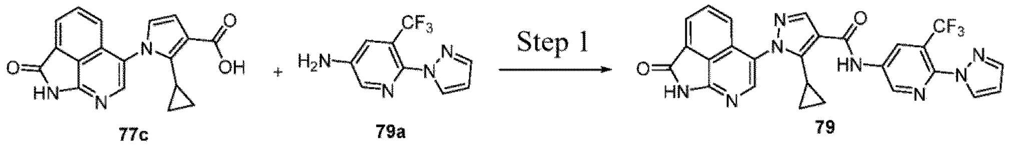

Referring to the synthesis method of step 11 in Example 1, the title product *N*-(6-(1*H*-pyrazol-1-yl)-5-(trifluoromethyl)pyridin-3-yl)-5-cyclopropyl-1-(2-oxo-1,2-dihydropyrrolo[4,3,2-ij]isoquinolin-6-yl)-1*H*-pyrazole-4-carboxamide **79** (35 mg) was obtained through 2-cyclopropyl-1-(2-oxo-1,2-dihydropyrrolo[4,3,2-ij]isoquinolin-6-yl)-1*H*-pyrrol-3-carboxylic acid **77c** (70 mg, 0.22 mmol) and 6-(1*H*-pyrazol-1-yl)-5-(trifluoromethyl)pyridin-3-amino **79a** (100 mg, 0.44 mmol, synthesized by the known method "Patent WO 2018020474 A1"), yield 30.1%.
MS m/z(ESI): 530 [M+1]

**Example 80**

*N*-(5-chloro-6-(2*H*-1,2,3-triazol-2-yl)pyridin-3-yl)-5-cyclopropyl-1-(2-oxo-1,2-dihydropyrrolo[4,3,2-ij]isoquinolin-6-yl)-1*H*-pyrazole-4-carboxamide

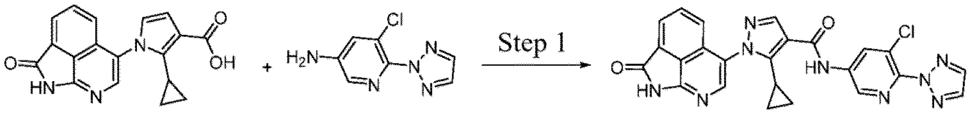

77c 80a 80

Referring to the synthesis method of the step 11 in Example 1, the title product *N*-(5-chloro-6-(2*H*-1,2,3-triazol-2-yl)pyridin-3-yl)-5-cyclopropyl-1-(2-oxo-1,2-dihydropyrrolo[4,3,2-*ij*]isoquinolin-6-yl)-1*H*-pyrazole-4-carboxamide **80** (36 mg) was obtained through 2-cyclopropyl-1-(2-oxo-1,2-dihydropyrrolo[4,3,2-*ij*]isoquinolin-6-yl)-1*H*-pyrrol-3-carboxylic acid **77c** (70 mg, 0.22 mmol) and 6-(2*H*-1,2,3-triazol-2-yl)-3-amino-5-chloropyridine **80a** (86 mg, 0.44 mmol, synthesized by the well known method "Patent US20180170909A1"), yield 32.9%.
MS m/z(ESI): 498 [M+1]

**Example 81**

*N*-(5-chloro-6-(1*H*-pyrazol-1-yl)pyridin-3-yl)-5-cyclopropyl-1-(2-oxo-1,2-dihydropyrrolo[4,3,2-*ij*]isoquinolin-6-yl)-1*H*-pyrazole-4-carboxamide

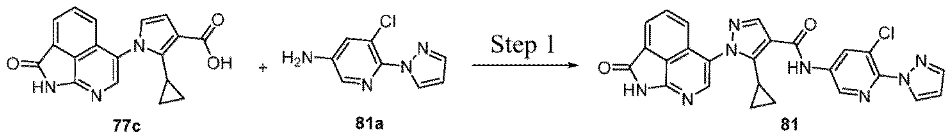

77c 81a 81

Referring to the synthesis method of step 11 in Example 1, the title product *N*-(5-chloro-6-(1*H*-pyrazol-1-yl)pyridin-3-yl)-5-cyclopropyl-1-(2-oxo-1,2-dihydropyrrolo[4,3,2-*ij*]isoquinolin-6-yl)-1H-pyrazole-4-carboxamide **81** (35 mg) was obtained through 2-cyclopropyl-1-(2-oxo-1,2-dihydropyrrolo[4,3,2-ij]isoquinolin-6-yl)-1*H*-pyrrol-3-carboxylic acid **77c** (70 mg, 0.22 mmol) and 5-chloro-6-(1*H*-pyrazol-1-yl)pyridin-3-amine **81a** (85 mg, 0.44 mmol, synthesized by the well-known method "Patent US20180170909 A1"), yield 32.1%.
MS m/z(ESI): 497 [M+1]

**Example 82**

5-cyclopropyl-*N*-(5-(difluoromethyl)-6-(2*H*-1,2,3-triazol-2-yl)pyridin-3-yl)-1-(2-oxo-1,2-dihydropyrrolo[4,3,2-*ij*]isoquinolin-6-yl)-1*H*-pyrazole-4-carboxamide

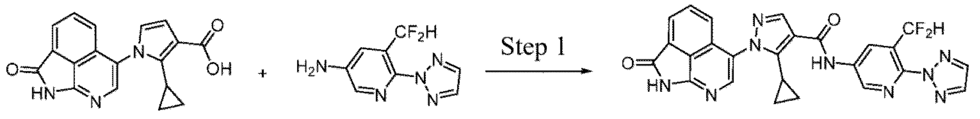


77c 49a 82

Referring to the synthesis method of step 11 in Example 1, the title product 5-cyclopropyl-*N*-(5-(difluoromethyl)-6-(2*H*-1,2,3-triazol-2-yl)pyridin-3-yl)-1-(2-oxo-1,2-dihydropyrrolo[4,3,2-*ij*]isoquinolin-6-yl)-1*H*-pyrazole-4-carboxamide **82** (31 mg) was obtained through 2-cyclopropyl-1-(2-oxo-1,2-dihydropyrrolo[4,3,2-*ij*]isoquinolin-6-yl)-1*H*-pyrrol-3-carboxylic acid **77c** (70 mg, 0.22 mmol) and 5-(difluoromethyl)-6-(2H-1,2,3-triazol-2-yl)pyridin-3-amine **49a** (93 mg, 0.44 mmol), yield 27.5%.
MS m/z(ESI): 514 [M+1]

**Example 83**

*N*-(4-(2*H*-1,2,3-triazol-2-yl)-3-(trifluoromethyl)phenyl)-5- cyclopropyl-1-(2-oxo-1,2-dihydropyrrolo[4,3,2-*ij*]isoquinolin-6-yl)-1*H*-pyrazole-4-carboxamide

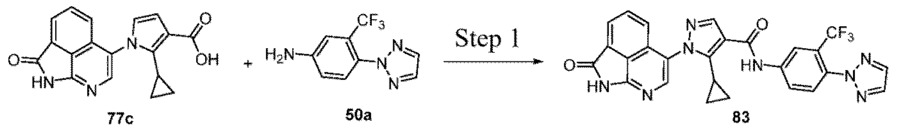


77c 50a 83

Referring to the synthesis method of the step 11 in Example 1, the title product *N*-(4-(2*H*-1,2,3-triazol-2-yl)-3-(trifluoromethyl)phenyl)-5-cyclopropyl-1-(2-oxo-1,2-dihydropyrrolo[4,3,2-*ij*]isoquinolin-6-yl)-1*H*-pyrazole-4-carboxamide **83** (37 mg) was obtained through 2-cyclopropyl-1-(2-oxo-1,2-dihydropyrrolo[4,3,2-*ij*]isoquinolin-6-yl)-1*H*-pyrrol-3-carboxylic acid **77c** (70 mg, 0.22 mmol) and 4-(2*H*-1,2,3-triazol-2-yl)-3-(trifluoromethyl)aniline **50a** (100 mg, 0.44 mmol, synthesized by well known method "Patent WO 2018020474 A1"), yield 31.7%.

MS m/z(ESI): 531 [M+1]
$^1$H NMR (400 MHz, DMSO) δ 11.04 (s, 1H), 10.50 (s, 1H), 8.44 (d, *J* = 2.3 Hz, 1H), 8.32 (s, 1H), 8.25 (dd, *J* = 8.8, 2.4 Hz, 1H), 8.14 (d, *J* = 13.5 Hz, 3H), 7.88 (dd, *J* = 8.3, 6.9 Hz, 1H), 7.76 (dd, J = 10.3, 8.6 Hz, 1H), 7.68 (d, *J* = 7.5 Hz, 1H), 7.11 (d, *J* = 7.5 Hz, 1H), 2.09 - 1.97 (m, 1H), 0.76 - 0.68 (m, 2H), 0.63 -0.55 (m, 2H).

**Example 84**

*N*-(4-(1*H*-pyrazol-1-yl)-3-(trifluoromethyl)phenyl)-5-cyclopropyl-1-(2-oxo-1,2-dihydropyrrolo[4,3,2-*ij*]isoquinolin-6-yl)-1*H*-pyrazole-4-carboxamide

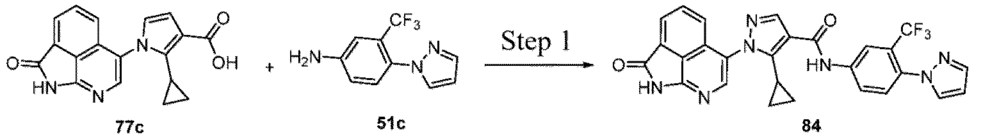


77c 51c 84

Referring to the synthesis method of step 11 in Example 1, the title product *N*-(4-(1*H*-pyrazol-1-yl)-3-(trifluoromethyl)phenyl)-5-cyclopropyl-1-(2-oxo-1,2-dihydropyrrolo[4,3,2-*ij*]isoquinolin-6-yl)-1*H*-pyrazole-4-carboxamide **84** (35 mg) was obtained through 2-cyclopropyl-1-(2-oxo-1,2-dihydropyrrolo[4,3,2-*ij*]isoquinolin-6-yl)-1*H*-pyrrol-3-carboxylic acid **77c** (70 mg, 0.22 mmol) and 4-(1*H*-pyrazol-1-yl)-3-(trifluoromethyl)aniline **51c** (100 mg, 0.44 mmol), yield 30.1%.
MS m/z(ESI): 530 [M+1]

**Example 85**

*N*-(3-chloro-4-(2*H*-1,2,3-triazol-2-yl)phenyl)-5-cyclopropyl-1-(2-oxo-1,2-dihydropyrrolo[4,3,2-*ij*]isoquinolin-6-yl)-1*H*-pyrazole-4-carboxamide

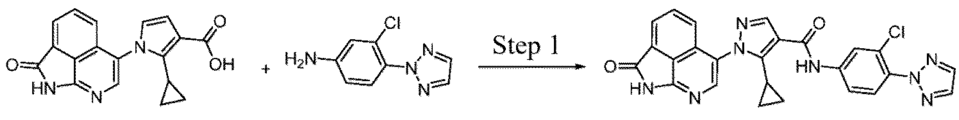


77c 53a 85

Referring to the synthesis method of step 11 in Example 1, the title product *N*-(3-chloro-4-(2*H*-1,2,3-triazol-2-yl)phenyl)-5-cyclopropyl-1-(2-oxo-1,2-dihydropyrrolo[4,3,2-*ij*]isoquinolin-6-yl)-1*H*-pyrazole-4-carboxamide **85** (40 mg) was obtained through 2-cyclopropyl-1-(2-oxo-1,2-dihydropyrrolo[4,3,2-*ij*]isoquinolin-6-yl)-1*H*-pyrrol-3-carboxylic acid **77c** (70 mg, 0.22 mmol) and 3-chloro-4-(2*H*-1,2,3-triazol-2-yl)aniline **53a** (85 mg, 0.44 mmol), yield 34.4%.
MS m/z(ESI): 497 [M+1]

**Example 86**

*N*-(3-chloro-4-(1*H*-pyrazol-1-yl)phenyl)-5-cyclopropyl-1-(2-oxo-1,2-dihydropyrrolo[4,3,2-*ij*]isoquinolin-6-yl)-1*H*-pyrazole-4-carboxamide

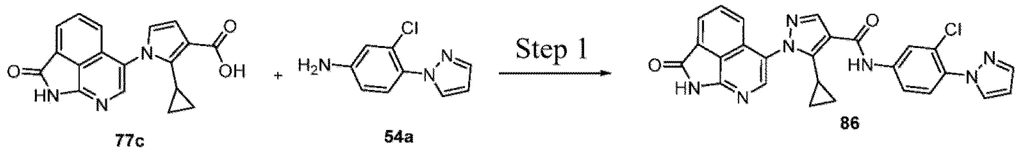


77c 54a 86

Referring to the synthesis method of step 11 in Example 1, the title product *N*-(3-chloro-4-(1*H*-pyrazol-1-yl)phenyl)-5-cyclopropyl-1-(2-oxo-1,2-dihydropyrrolo[4,3,2-*ij*]isoquinolin-6-yl)-1*H*-pyrazole-4-carboxamide **86** (45 mg) was obtained through 2-cyclopropyl-1-(2-oxo-1,2-dihydropyrrolo[4,3,2-*ij*]isoquinolin-6-yl)-1*H*-pyrrol-3-carboxylic acid **77c** (70 mg, 0.22 mmol) and 3-chloro-4-(1*H*-pyrazol-1-yl)aniline **54a** (85 mg, 0.44 mmol), yield 41.4%.

MS m/z(ESI): 496 [M+1]
$^1$H NMR (400 MHz, DMSO) δ 11.04 (s, 1H), 9.66 (s, 1H), 8.63 - 8.54 (m, 1H), 8.27 (s, 1H), 8.15 - 8.05 (m, 2H), 7.93 - 7.83 (m, 2H), 7.83 - 7.75 (m, 2H), 7.67 (d, *J* = 7.5 Hz, 1H), 7.11 (d, *J* = 7.4 Hz, 1H), 6.62 - 6.54 (m, 1H), 2.04 (tt, *J* = 8.5, 5.5 Hz, 1H), 0.77 - 0.66 (m, 2H), 0.64 - 0.55 (m, 2H).

**Example 87** *N*-(6-(2*H*-1,2,3-triazol-2-yl)-5-(trifluoromethyl)pyridin-3-yl)-1-(2-carbonyl-1,2-dihydropyrrolo[2,3,4-de]isoquinolin-6-yl)-5-(trifluoromethyl)-1*H*-pyrazole-4-carboxamide

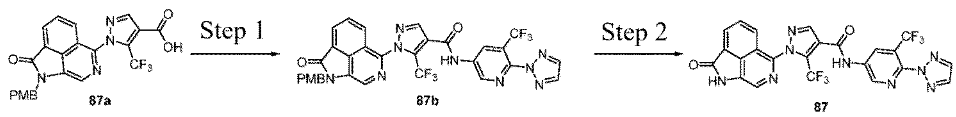


87a 87b 87

Referring to the synthesis method of step 3 to step 4 in Example**73**, the title product *N*-(6-(2*H*-1,2,3-triazol-2-yl)-5-(trifluoromethyl)pyridin-3-yl)-1-(2-carbonyl-1,2-dihydropyrrolo[2,3,4-de]isoquinolin-6-yl)-5-(trifluoromethyl)-1*H*-pyrazole-4-carboxamide **87** (15 mg) was obtained through *N*-(6-(2*H*-1,2,3-triazol-2-yl)-5-(trifluoromethyl)pyridin-3-yl)-1-(1-(4-methoxybenzyl)-2-carbonyl-1,2-dihydropyrrolo[2,3,4-de]isoquinolin-6-yl)-5-(trifluoromethyl)-1*H*-pyrazole-4-carboxamide **87a** (45 mg, 0.096 mmol), yield: 27.9%.

MS m/z (ESI): 560 [M+1]

$^1$H NMR (400 MHz, DMSO): δ 11.47 (s, 1H), 11.27 (s, 1H), 9.18 (d, *J* = 2.4 Hz, 1H), 8.90 (d, *J* = 2.4 Hz, 1H), 8.63 (s, 1H), 8.42 (d, *J* = 6.9 Hz, 1H), 8.21 (d, *J* = 9.9 Hz, 3H), 8.12-8.04 (m, 2H).

**Example 88** 1-(8-isopropyl-2-carbonyl-1,2-dihydrobenzo[cd]indol-6-yl)-5-(trifluoromethyl)-N-(2-(trifluoromethyl)pyridin-4-yl)-1H-pyrazole-4-carboxamide

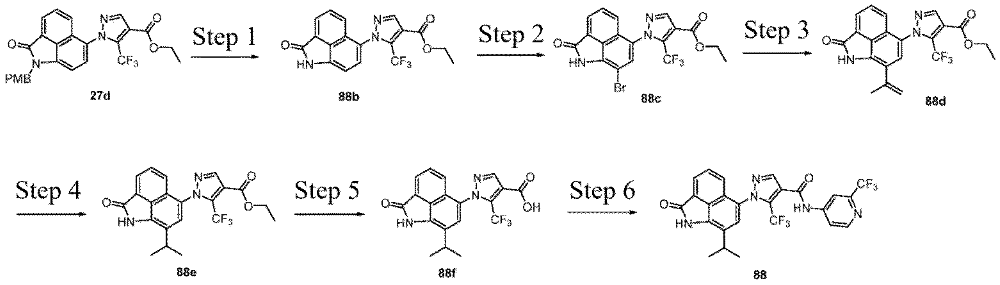


Referring to the synthesis method of step 10 in Example 1, ethyl 1-(2-carbonyl-1,2-dihydrobenzo[cd]indol-6-yl)-5-(trifluoromethyl)-1H-pyrazole-4-carboxylate **88b** (1.49 g) was obtained through the reaction of ethyl 1-(1-(4-methoxybenzyl)-2-carbonyl-1,2-dihydrobenzo[cd]indol-6-yl)-5-(trifluoromethyl)-1H-pyrazole-4-carboxylate **27d** (2 g, 4.04 mmol), yield: 98%.

MS m/z (ESI): 376 [M+1].

Step 2

Ethyl 1-(8-bromo-2-carbonyl-1,2-dihydrobenzo[cd]indol-6-yl)-5-(trifluoromethyl)-1H-pyrazole-4-carboxylate

Ethyl 1-(2-carbonyl-1,2-dihydrobenzo[cd]indol-6-yl)-5-(trifluoromethyl)-1H-pyrazole-4-carboxylate **88b** (200 mg, 0.53 mmol) was dissolved in 5 mL of chloroform, liquid bromine (170 mg, 1.07 mmol) was added dropwise to the reaction liquid under ice bath, and the mixture was reacted under stirring at room temperature for 16 h. The reaction liquid was concentrated under reduced pressure to obtain the title product ethyl 1-(8-bromo-2-carbonyl-1,2-dihydrobenzo[cd]indol-6-yl)-5-(trifluoromethyl)-1H-pyrazole-4-carboxylate **88c** (240 mg), yield: 99.2%.

MS m/z (ESI): 455 [M+1].

Step 3

1-(8-isopropyl-2-carbonyl-1,2-dihydrobenzo[cd]indol-6-yl)-5-(trifluoromethyl)-N-(2-(trifluoromethyl)pyridin-4-yl)-1H-pyrazole-4-carboxamide

Ethyl 1-(8-bromo-2-carbonyl-1,2-dihydrobenzo[cd]indol-6-yl)-5-(trifluoromethyl)-1H- pyrazole-4-carboxylate **88c** (160 mg, 0.35 mmol), isopropenylpinacol borate (89 mg, 0.53 mmol), tetrakistriphenylphosphine palladium (32 mg, 0.03 mmol), potassium phosphate (149.55 mg, 0.70 mmol), 2-di-tert-butylphosphino-2',4',6'-triisopropylbiphenyl (29 mg, 0.07 mmol) were dissolved in 4 mL of dioxane and 2 ml of water, and the mixture was heated under microwave at 100°C and reacted for 2 h under nitrogen protection. The reaction liquid was concentrated under reduced pressure, and the obtained residue was purified by silica gel column chromatography with eluent system A to obtain the title product 1-(8-isopropyl-2-carbonyl-1,2-dihydrobenzo[cd]indol-6-yl)-5-(trifluoromethyl)-N-(2-(trifluoromethyl)pyridin-4-yl)-1H-pyrazole-4-carboxamide **88d** (110 mg), yield: 75.2%.

MS m/z (ESI): 416 [M+1].

Step 4

Ethyl 1-(8-isopropyl-2-carbonyl-1,2-dihydrobenzo[cd]indol-6-yl)-5-(trifluoromethyl)-1H- pyrazole-4-carboxylate

1-(8-isopropyl-2-carbonyl-1,2-dihydrobenzo[cd]indol-6-yl)-5-(trifluoromethyl)-N-(2-(trifluoromethyl)pyridin-4-yl)-1H-pyrazole-4-carboxamide **88d** (110 mg, 0.26 mmol) was dissolved in 5 mL of tetrahydrofuran, 5% palladium on

carbon (56.3 mg, 0.03 mmol) was added under nitrogen protection and the mixture was reacted under stirring at room temperature for 1 h in a hydrogen atmosphere. The reaction liquid was filtered, and the filtrate was concentrated under reduced pressure to obtain the title product ethyl 1-(8-isopropyl-2-carbonyl-1,2-dihydrobenzo[cd]indol-6-yl)-5-(trifluoromethyl)-1H-pyrazole-4-carboxylate **88e** (108 mg), yield: 98.2%.

MS m/z (ESI): 418 [M+1].

Referring to the synthesis method of step 9 and step 11 in Example 1, 1-(8-isopropyl-2-carbonyl-1,2-dihydrobenzo[cd]indol-6-yl)-5-(trifluoromethyl)-N-(2-(trifluoromethyl)pyridin-4-yl)-1H-pyrazole-4-carboxamide **30** (12 mg) was obtained through 1-(8-isopropyl-2-carbonyl-1,2-dihydrobenzo[cd]indol-6-yl)-5-(trifluoromethyl)-1H-pyrazole-4-carboxylate **88e** (108 mg, 0.26 mmol), yield: 8.6%.

MS m/z (ESI): 534 [M+1]
1H NMR (400 MHz, DMSO) δ 11.26 (s, 2H), 8.72 (d, J = 5.5 Hz, 1H), 8.53 (s, 1H), 8.26 (d, J = 2.0 Hz, 1H), 8.13 (d, J = 7.0 Hz, 1H), 7.99 (dd, J = 5.5, 2.0 Hz, 1H), 7.85 (dd, J = 8.3, 7.0 Hz, 1H), 7.71 (s, 1H), 7.59 (d, J = 8.2 Hz, 1H), 3.39 (p, J = 6.8 Hz, 1H), 1.30 (d, J = 6.8 Hz, 6H).

**Example 89**

1-(8-ethyl-2-carbonyl-1,2-dihydrobenzo[cd]indol-6-yl)-5-(trifluoromethyl)-N-(2-(trifluoromethyl)pyridin-4-yl)-1H-pyrazole-4-carboxamide

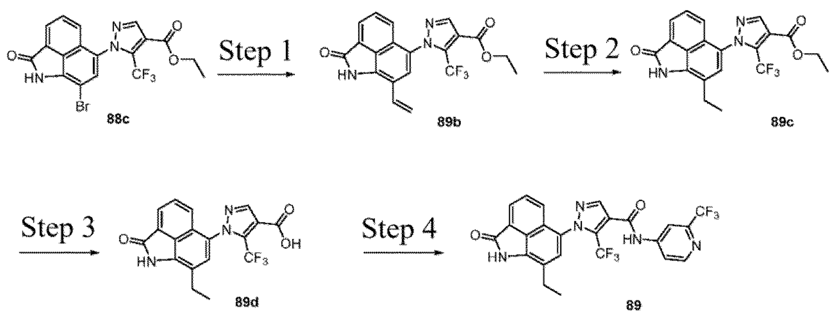

88c 89b 89c 89d 89

Referring to the synthesis method of step 3 to step 4 in Example**88** and step 9 and step 11 in Example 1, 1-(8-ethyl-2-carbonyl-1,2-dihydrobenzo[cd]indol-6-yl)-5-(trifluoromethyl)-N-(2-(trifluoromethyl)pyridin-4-yl)-1H-pyrazole-4-carboxamide **89** (15 mg) was obtained through ethyl 1-(8-bromo-2-carbonyl-1,2-dihydrobenzo[cd]indol-6-yl)-5-(trifluoromethyl)-1H-pyrazole-4-carboxylate **88c** (100 mg, 0.22 mmol), yield: 13.2%.

MS m/z (ESI): 520 [M+1]
1H NMR (400 MHz, DMSO) δ 11.39 (s, 1H), 11.28 (s, 1H), 9.19 (d, J = 2.4 Hz, 1H), 8.91 (d, J = 2.4 Hz, 1H), 8.57 (s, 1H), 8.22 (s, 1H), 8.13 (d, J = 6.9 Hz, 1H), 7.86 (dd, J = 8.2, 7.0 Hz, 1H), 7.68 (s, 1H), 7.59 (d, J = 8.2 Hz, 1H), 2.92 - 2.81 (m, 2H), 1.26 (t, J = 7.5 Hz, 3H).

**Example 90**

1-(8-chloro-2-carbonyl-1,2-dihydrobenzo[cd]indol-6-yl)-5-(trifluoromethyl)-N-(2-(trifluoromethyl)pyridin-4-yl)-1H-pyrazole-4-carboxamide

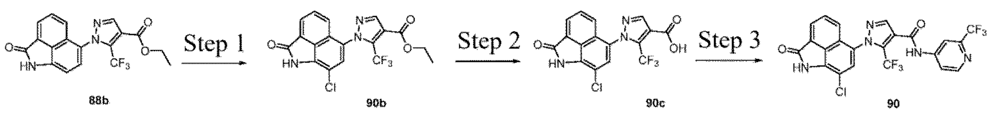

88b 90b 90c 90

Step 1

Ethyl 1-(8-chloro-2-carbonyl-1,2-dihydrobenzo[cd]indol-6-yl)-5-(trifluoromethyl)-1H-pyrazole-4-carboxylate

Ethyl 1-(2-carbonyl-1,2-dihydrobenzo[cd]indol-6-yl)-5-(trifluoromethyl)-1H-pyrazole-4-carboxylate **88b** (300 mg, 0.80 mmol) was dissolved in 4 mL of carboxylic acid, sulfuryl chloride (323 mg, 2.4 mmol) was added under ice bath, and the mixture was heated at 60°C and reacted for 3 h. A small amount of methanol was added to the reaction liquid to quench excess acid chloride, the reaction liquid was concentrated under reduced pressure, and the residue obtained was purified by silica gel column chromatography with eluent system A to obtain the title product ethyl 1-(8-chloro-2-carbonyl-1,2-dihydrobenzo[cd]indol-6-yl)-5-(trifluoromethyl)-1H-pyrazole-4-carboxylate **90b** (320 mg), yield: 97.7%.

MS m/z (ESI): 410 [M+1].

Referring to the synthesis method of step 9 and step 11 in Example 1, 1-(8 -chloro-2-carbonyl-1,2-dihydrobenzo[cd]indol-6-yl)-5-(trifluoromethyl)-N-(2-(trifluoromethyl) pyridin-4-yl)-1H-pyrazole-4-carboxamide **90** (21 mg) was obtained through ethyl 1-(8-chloro-2-carbonyl-1,2-dihydrobenzo[cd]indol-6-yl)-5-(trifluoromethyl)-1H-pyrazole-4-carboxylate **90b** (80 mg, 0.20 mmol), yield: 20.0%.

MS m/z (ESI): 526 [M+1]

1H NMR (400 MHz, DMSO) δ 11.71 (s, 1H), 11.28 (s, 1H), 8.72 (d, J = 5.5 Hz, 1H), 8.57 (s, 1H), 8.26 (d, J = 2.0 Hz, 1H), 8.21 (d, J = 7.0 Hz, 1H), 8.00 (dd, J = 5.5, 2.0 Hz, 1H), 7.97 - 7.88 (m, 2H), 7.60 (d, J = 8.2 Hz, 1H).

**Example 91**

1-(8-methyl-2-carbonyl-1,2-dihydrobenzo[cd]indol-6-yl)-5-(trifluoromethyl)-N-(2-(trifluoromethyl)pyridin-4-yl)-1H-pyrazole-4-carboxamide

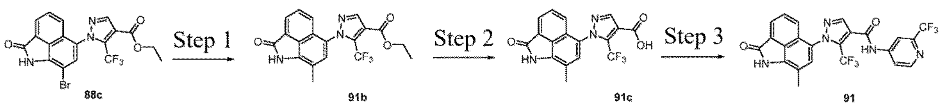


88c 91b 91c 91

Step 1

Ethyl 1-(8-methyl-2-carbonyl-1,2-dihydrobenzo[cd]indol-6-yl)-5-(trifluoromethyl)-1H-pyrazole-4-carboxylate

Ethyl 1-(8-bromo-2-carbonyl-1,2-dihydrobenzo[cd]indol-6-yl)-5-(trifluoromethyl)-1H- pyrazole-4-carboxylate **88c**(180 mg, 0.40 mmol) and tetratrisphenylphosphine palladium (92 mg, 0.08 mmol) were dissolved in 1 mL of tetrahydrofuran, 2 mL of a solution of dimethylzinc in tetrahydrofuran (1 M) was added under ice bath and the reaction mixture was heated at 60 °C and reacted for 16 h. A small amount of methanol was added to the reaction liquid to quench excess dimethylzinc, the reaction liquid was concentrated under reduced pressure, and the residue obtained was purified by silica gel column chromatography with eluent system A to obtain the title product ethyl 1-(8-methyl-2-carbonyl-1,2-dihydrobenzo[cd]indol-6-yl)-5-(trifluoromethyl)-1H-pyrazole-4-carboxylate **91b** (100 mg), yield: 64.8%.

MS m/z (ESI): 390 [M+1].

Referring to the synthesis method of step 9 and step 11 in Example 1, 1-(8-methyl-2-carbonyl-1,2-dihydrobenzo[cd]indol-6-yl)-5-(trifluoromethyl)-N-(2-(trifluoromethyl)pyridin-4-yl)-1H-pyrazole-4-carboxamide **91** (8 mg) was obtained through ethyl 1-(8-methyl-2-carbonyl-1,2-dihydrobenzo[cd]indol-6-yl)-5-(trifluoromethyl)-1H-pyrazole-4-carboxylate **91b** (100 mg, 0.26 mmol), yield: 6.1%.

MS m/z (ESI): 506 [M+1]

1H NMR (400 MHz, DMSO) δ 11.20 (s, 1H), 9.10 (d, J = 2.4 Hz, 1H), 8.84 (d, J = 2.4 Hz, 1H), 8.49 (s, 1H), 8.14 (s, 2H), 8.05 (d, J = 6.9 Hz, 1H), 7.77 (dd, J = 8.3, 7.0 Hz, 1H), 7.56 (s, 1H), 7.49 (d, J = 8.3 Hz, 1H), 2.42 (s, 3H).

**Example 92**

1-(8-methyl-2-carbonyl-1,2-dihydrobenzo[cd]indol-6-yl)-5-(trifluoromethyl)-N-(2-(trifluoromethyl)pyridin-4-yl)-1H-pyrazole-4-carboxamide

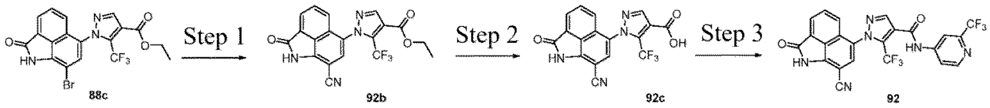


88c 92b 92c 92

Step 1

Ethyl 1-(8-cyano-2-carbonyl-1,2-dihydrobenzo[cd]indol-6-yl)-5-(trifluoromethyl)-1H- pyrazole-4-carboxylate

Ethyl 1-(8-bromo-2-carbonyl-1,2-dihydrobenzo[cd]indol-6-yl)-5-(trifluoromethyl)-1H- pyrazole-4-carboxylate **88c** (100 mg, 0.22 mmol), zinc cyanide (31 mg, 0.26 mmol) and tetratrisphenylphosphine palladium (25 mg, 0.02 mmol) were dissolved in 4 mL of dimethylformamide, and the reaction mixture was heated under microwave at 60°C under nitrogen protection and reacted for 16 h. The reaction liquid was poured into 50 mL of ethyl acetate and the obtained product was washed with saturated sodium chloride aqueous solution (50 mL×2). The organic phase was dried over anhydrous sodium sulfate and concentrated under reduced pressure. The obtained residue was purified by silica gel column chromatography with eluent system A to obtain the title product ethyl 1-(8-cyano-2-carbonyl-1,2-dihydrobenzo[cd]indol-6-yl)-5-(trifluoromethyl)-1H-pyrazole-4-carboxylate **92b** (60 mg), yield: 68.1%.

MS m/z (ESI): 401 [M+1].

Referring to the synthesis method of step 9 and step 11 in Example 1, 1-(8-cyano-2-carbonyl-1,2-dihydrobenzo[cd]indol-6-yl)-5-(trifluoromethyl)-N-(2-(trifluoromethyl)pyridin-4-yl)-1H-pyrazole-4-carboxamide **92** (6 mg) was obtained through ethyl 1-(8-cyano-2-carbonyl-1,2-dihydrobenzo[cd]indol-6-yl)-5-(trifluoromethyl)-1H-pyrazole-4-carboxylate **92b** (60 mg, 0.15 mmol), yield: 8.0%.

MS m/z (ESI): 517 [M+1]
1H NMR (400 MHz, DMSO) δ 12.21 (s, 1H), 11.28 (s, 1H), 8.72 (d, J = 5.5 Hz, 1H), 8.58 (s, 1H), 8.28 - 8.20 (m, 2H), 8.11 (s, 1H), 8.06 - 7.97 (m, 2H), 7.59 (d, J = 8.3 Hz, 1H).

**Example 93**

1-(8-methoxy-2-carbonyl-1,2-dihydrobenzo[cd]indol-6-yl)-5-(trifluoromethyl)-N-(2-(trifluoromethyl)pyridin-4-yl)-1H-pyrazole-4-carboxamide

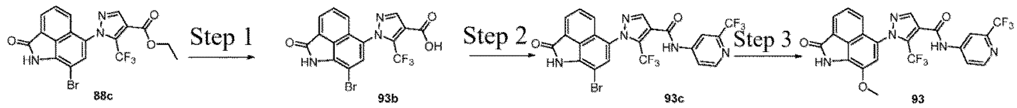


88c 93b 93c 93

Referring to the synthesis method of step 9 and step 11 in Example 1, 1-(8 -bromo-2-carbonyl-1,2-dihydrobenzo[cd]indol-6-yl)-5-(trifluoromethyl)-N-(2-(trifluoromethyl) pyridin-4-yl)-1H-pyrazole-4-carboxamide **93c** (108 mg) was obtained through ethyl 1-(8-bromo-2-carbonyl-1,2-dihydrobenzo[cd]indol-6-yl)-5-(trifluoromethyl)-1H-pyrazole-4-carboxylate **88c** (210 mg, 0.20 mmol), yield: 41.1%.
MS m/z (ESI): 570 [M+1]

Step 3

1-(8-methoxy-2-carbonyl-1,2-dihydrobenzo[cd]indol-6-yl)-5-(trifluoromethyl)-N-(2-(trifluoromethyl)pyridin-4-yl)-1H-pyrazole-4-carboxamide

Ethyl 1-(8-bromo-2-carbonyl-1,2-dihydrobenzo[cd]indol-6-yl)-5-(trifluoromethyl)-1H- pyrazole-4-carboxylate

**93c** (100 mg, 0.18 mmol), sodium methoxide (96 mg, 1.79 mmol) and cuprous chloride (100 mg, 1.01 mmol) were dissolved in 4 mL of dimethylformamide and 2 mL of methanol, and the reaction mixture was heated in a sealed tube at 100°C and reacted for 16 h under nitrogen protection. The reaction liquid was poured into 50 mL of ethyl acetate and the obtained product was washed with saturated sodium chloride aqueous solution (50 mL×2). The organic phase was dried over anhydrous sodium sulfate and concentrated under reduced pressure. The obtained residue was purified by silica gel column chromatography with eluent system B to obtain the title product 1-(8-methoxy-2-carbonyl-1,2-dihydrobenzo[cd]indol-6-yl)-5-(trifluoromethyl)-N-(2-(trifluoromethyl)pyridin-4-yl)-1H-pyrazole-4-carboxamide **93** (6 mg), yield: 6.6%.

MS m/z (ESI): 522 [M+1].

$^1$H NMR (400 MHz, DMSO) δ 11.06 (d, *J* = 76.0 Hz, 2H), 8.71 (d, *J* = 5.5 Hz, 1H), 8.54 (s, 1H), 8.41 (d, *J* = 8.2 Hz, 1H), 8.25 (d, *J* = 2.0 Hz, 1H), 8.20 (d, *J* = 7.0 Hz, 1H), 7.98 (dt, *J* = 8.3, 5.8 Hz, 2H), 7.02 (s, 1H), 4.15 (s, 3H).

**Example 94**

1-(2-imine-1,2-dihydrobenzo[*cd*]indol-6-yl)-5-(trifluoromethyl)-*N*-(2-(trifluoromethyl)pyridin-4-yl)-1*H*-pyrazole-4-carboxamide

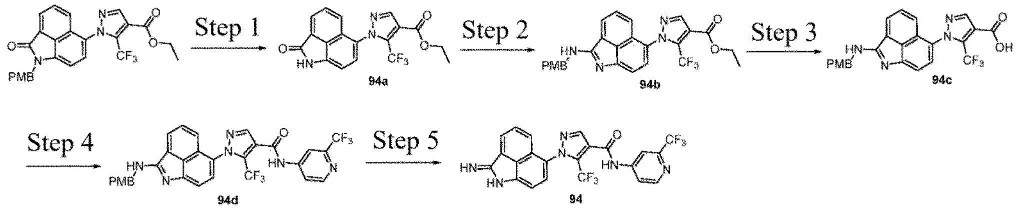


Step 1

Ethyl 1-(2-oxo-1,2-dihydrobenzo[*cd*]indol-6-yl)-5-(trifluoromethyl)-1*H*-pyrazole-4-carboxylate

Ethyl 1-(1-(4-methoxybenzyl)-2-oxo-1,2-dihydrobenzo[*cd*]indol-6-yl)-5-(trifluoromethyl)-1*H*-pyrazole-4-carboxylate (2 g, 4.04 mmol) was dissolved in 10 mL of trifluoroacetic acid, and the mixture was reacted under stirring at 70°C for 16 h. The reaction liquid was concentrated under reduced pressure, and the obtained residue was purified by silica gel column chromatography with eluent system A to obtain the title product ethyl 1-(2-oxo-1,2-dihydrobenzo[*cd*]indol-6-yl)-5-(trifluoromethyl)-1*H*-pyrazole-4-carboxylate **94a** (1.50 g), yield: 99.0%.
MS m/z(ESI): 376 [M+1]

Step 2

Ethyl 1-(2-((4-methoxybenzyl)amino)benzo[*cd*]indol-6-yl)-5-(trifluoromethyl)-1*H*-pyrazole-4-carboxylate

Ethyl 1-(2-oxo-1,2-dihydrobenzo[*cd*]indol-6-yl)-5-(trifluoromethyl)-1*H*-pyrazole-4-carboxylate **94a**(1.50 g, 4 mmol) and 4-methoxybenzylamine (822 mg, 6 mmol) were dissolved in 1 mL of phosphorus oxychloride and 10 mL of toluene, and the mixture was reacted under stirring at 100°C for 1 h. The reaction liquid was concentrated under reduced pressure, and the obtained residue was purified by silica gel column chromatography with eluent system A to obtain the title product ethyl 1-(2-((4-methoxybenzyl)amino)benzo[*cd*]indol-6-yl)-5-(trifluoromethyl)-1*H*-pyrazole-4-carboxylate **94b** (900 mg), yield: 45.5%.
MS m/z(ESI): 495 [M+1]

Referring to the synthesis method of step 9, step 11 to step 10 in Example 1 in sequence, 1-(2-imine-1,2-dihydrobenzo[*cd*]indol-6-yl)-5-(trifluoromethyl)-*N*-(2-(trifluoromethyl)pyridin-4-yl)-1*H*-pyrazole-4-carboxamide **94** (35 mg) was obtained through ethyl 1-(2-((4-methoxybenzyl)amino)benzo[*cd*]indol-6-yl)-5-(trifluoromethyl)-1*H*-pyrazole-4-carboxylate **94b** (300 mg, 0.61 mmol), yield: 11.8%.

MS m/z(ESI): 491 [M+1].

$^1$H NMR(400 MHz, DMSO-$d_6$)δ 11.29(s, 1H), 8.71(d, J = 5.5 Hz, 1H), 8.50(s, 1H), 8.29 - 8.21(m, 2H), 8.17(d, J = 6.9 Hz, 1H), 7.99(dd, J = 5.5, 1.7 Hz, 1H), 7.79 - 7.70(m, 1H), 7.54(d, J = 7.4 Hz, 1H), 7.41(d, J = 8.1 Hz, 1H), 7.06(d, J = 7.4 Hz, 1H), 3.79 - 3.51(m, 1H).

**Example 95**

1-(2-thio-1,2-dihydrobenzo[cd]indol-6-yl)-5-(trifluoromethyl)-N-(2-(trifluoromethyl)pyridin-4-yl)-1H-pyrazole-4-carboxamide

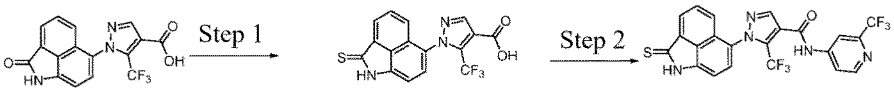


95a 95b 95

Step 1

1-(2-thio-1,2-dihydrobenzo[cd]indol-6-yl)-5-(trifluoromethyl)-1H-pyrazole-4-carboxylic acid

1-(2-carbonyl-1,2-dihydrobenzo[cd]indol-6-yl)-5-(trifluoromethyl)-1H-pyrazole-4-carboxylic acid **95a** (50 mg, 0.14 mmol) and Lawson's reagent (100 mg, 0.25 mmol) were dissolved in 10 mL of toluene and the mixture was reacted at 100°C for 1 h (microwave). The reaction liquid was cooled to room temperature and poured into 10 mL of water. The obtained mixture was filtered, the filtrate was extracted with ethyl acetate (20 mL×3), the organic phases were combined, washed with saturated sodium chloride solution (50 mL×2), dried over anhydrous sodium sulfate, filtered and concentrated to obtain a crude product, and the obtained residue was purified by silica gel column chromatography with eluent system A to obtain the title product 1-(2-thio-1,2-dihydrobenzo[cd]indol-6-yl)-5-(trifluoromethyl)-1H-pyrazole-4-carboxylic acid **95b** (30 mg), yield: 57.3%.
MS m/z (ESI): 364 [M+1]

Referring to the synthesis method of step 11 in Example 1, 1-(2-thio-1,2-dihydrobenzo[cd]indol-6-yl)-5-(trifluoromethyl)-N-(2-(trifluoromethyl)pyridin-4-yl)-1H-pyrazole-4-carboxamide **95** (8 mg) was obtained through 1-(2-thio-1,2-dihydrobenzo[cd]indol-6-yl)-5-(trifluoromethyl)-1H-pyrazole-4-carboxylic acid **95b** (30 mg, 0.083 mmol), yield: 19.1%.

MS m/z (ESI): 508 [M+1].

1H NMR (400 MHz, $CD_3OD$) δ 8.63 (d, J = 5.6 Hz, 1H), 8.35 (s, 1H), 8.24 (dd, J = 4.6, 2.6 Hz, 2H), 7.98 (dd, J = 5.6, 2.1 Hz, 1H), 7.84 (dd, J = 8.3, 7.2 Hz, 1H), 7.67 (dd, J = 10.8, 7.9 Hz, 2H), 7.23 (d, J = 7.6 Hz, 1H).

**Example 96**

1-(1-oxo-2*H*-naphtho[1,8-*cd*]isothiazol-5-yl)-5-(trifluoromethyl)-*N*-(2-(trifluoromethyl)pyridin-4-yl)-1*H*-pyrazole-4-carboxamide

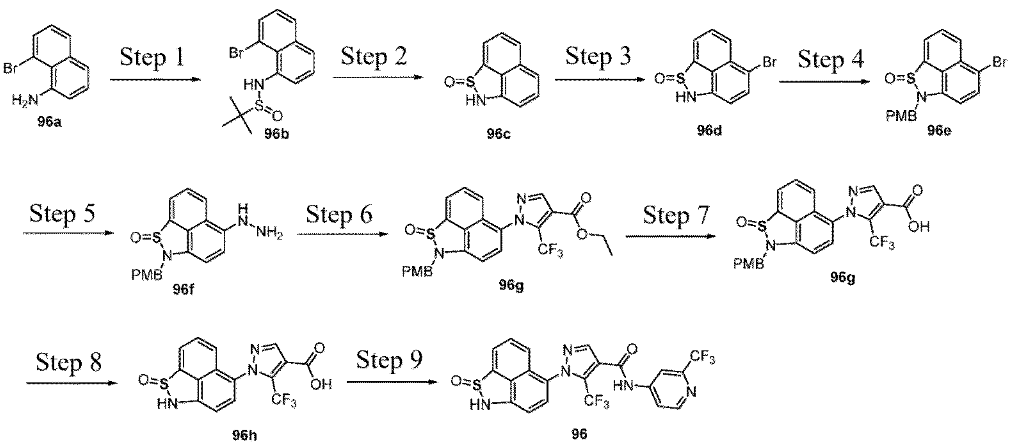


96a 96b 96c 96d 96e 96f 96g 96g 96h 96

Step 1

*N*-(8-bromonaphthalen-1-yl)-tert-butylsulfinamide

8-bromo-1-naphthylamine **96a** (5 g, 22.62 mmol) was dissolved in 50 mL of dichloromethane, and pyridine (3.95 g, 50 mmol) and tert-butylsulfinyl chloride (4.20 g, 30 mmol) were added in sequence and the mixture was reacted under stirring at room temperature for 1 h. The reaction liquid was concentrated under reduced pressure, and the obtained residue was purified by silica gel column chromatography with eluent system A to obtain the title product N-(8-bromonaphthalen-1-yl)-tert-butylsulfinamide **96b** (4 g), yield: 54.4%.
MS m/z(ESI): 326 [M+1]

Step 2

1-oxo-2*H*-naphtho[1,8-*cd*]isothiazole

*N*-(8-bromonaphthalen-1-yl)-tert-butylsulfinamide **96b** (4 g, 12.31 mmol) and azobisisobutyronitrile (2.46 g, 15 mmol) were dissolved in 100 mL of toluene. Tributyltin hydride (4.41 g, 15 mmol) was added dropwise at 90°C, and the mixture was reacted under stirring at 90°C for 2 h. The reaction liquid was concentrated under reduced pressure, and the obtained residue was purified by silica gel column chromatography with eluent system A to obtain the title product 1-oxo-2*H*-naphtho[1,8-*cd*]isothiazole **96c** (1.20 g), yield: 51.6%.
MS m/z(ESI): 190 [M+1]

Referring to the synthesis method of step 1 to step 6 of the intermediate **1** and step 11 of the example **1** in sequence, 1-(1-oxo-2*H*-naphtho[1,8-*cd*]isothiazol-5-yl)-5-(trifluoromethyl)-*N*-(2-(trifluoromethyl) pyridin-4-yl)-1*H*-pyrazole-4-carboxamide **96** (41 mg) was obtained through 1-oxo-2*H*-naphtho[1,8-*cd*]isothiazole **96c** (1.20 g, 6.32 mmol), yield: 1.3%.

MS m/z(ESI): 512 [M+1].

**Example 97**

1-(2-oxo-1,2-dihydropyrrolo[4,3,2-*ij*]isoquinolin-6-yl)-5-trifluoromethyl-*N*-(2-trifluoromethylpyridin-4-yl)-1*H*-pyrazole-4-carboxamide

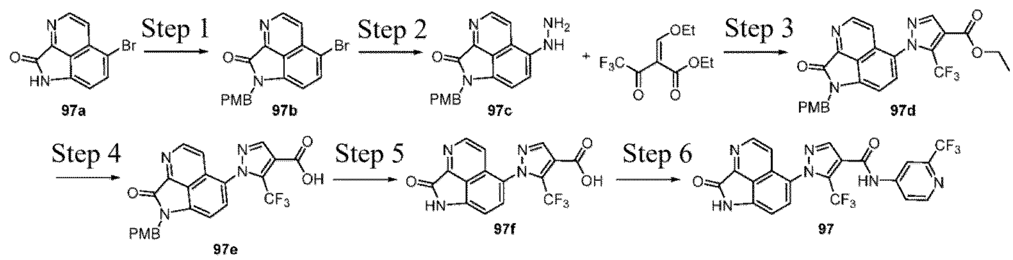


Referring to the synthesis method of step 5 in Example **2** and step 7 to step 11 in Example **1** in sequence, the title product 1-(2-oxo-1,2-dihydropyrrolo[4,3,2-*ij*]isoquinolin-6-yl)-5-trifluoromethyl-*N*-(2-trifluoromethylpyridin-4-yl)-1*H*-pyrazole-4-carboxamide **97** (126 mg) was obtained through 6-bromopyrrolo[2,3,4-*ij*]isoquinolin-2(1*H*)-one **97a** (2.49 g, 10 mmol, prepared by the well known method "Patent WO 2022081928A1"), yield: 2.6%.
MS m/z (ESI): 493 [M+1]

**Example 98**

1-(2-carbonyl-1,2-dihydropyrrolo[4,3,2-de]isoquinolin-6-yl)-5-(trifluoromethyl)-*N*-(2-(trifluoromethyl)pyridin-4-yl)-1*H*-pyrazole-4-carboxamide

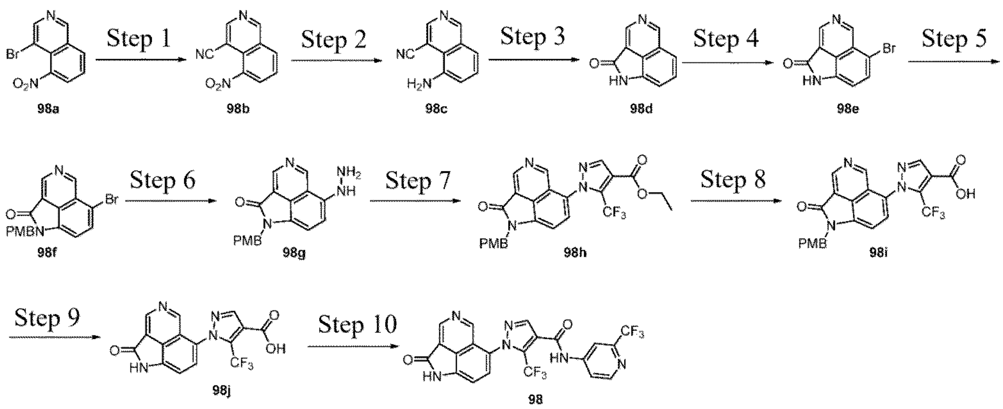


Referring to the synthesis method of step 1 in Example **8,** step 2 in Example **34,** step 4 in Example **8,** step 1 to step 6 of Intermediate **1,** and step 3 in Example **34** in sequence, 1-(2-carbonyl-1,2-dihydropyrrolo[4,3,2-de]isoquinolin-6-yl)-5-(trifluoromethyl)-*N*-(2-(trifluoromethyl)pyridin-4-yl)-1*H*-pyrazole-4-carboxamide **98** (6 mg) was obtained through the reaction of 4-bromo-5-nitro isoquinoline **98a** (10.00 g, 39.68 mmol), yield: 4.3%.

MS m/z (ESI): 493 [M+1]

$^1$H NMR (400 MHz, DMSO): δ 11.36 (s, 1H), 11.17 (s, 1H), 9.66 (s, 1H), 9.32 (s, 1H), 8.37 (d, J = 6.4 Hz, 1H), 8.12 (s, 1H), 7.66 (d, J = 8.4 Hz, 1H), 7.34-7.12 (m, 2H), 6.78 (d, J = 9.2 Hz, 1H).

**Example 99**

1-(2-oxo-1,2-dihydropyrrolo[4,3,2-*de*]quinolin-6-yl)-5-(trifluoromethyl)-*N*-(2-(trifluoromethyl)pyridin-4-yl)-1*H*-pyrazole-4-carboxamide

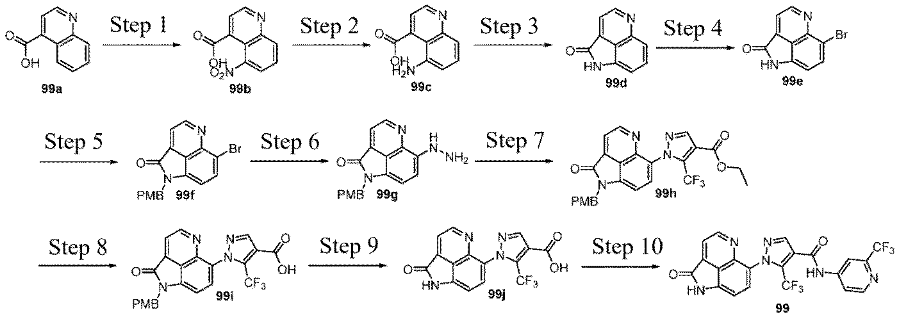


Step 1

5-nitroquinolin-4-carboxylic acid

4-carboxyquinoline **99a** (10 g, 57.80 mmol) was dissolved in 30 mL of concentrated sulfuric acid, 10 mL of concentrated nitric acid was added at 0°C, and the mixture was reacted under stirring at 0°C for 1 h. The reaction liquid was poured into 200 mL of crushed ice, the pH was adjusted to 2 with 50% aqueous sodium hydroxide solution, the solid was collected by filtration, and dried under reduced pressure to obtain the title product 5-nitroquinolin-4-carboxylic acid **99b** (6 g), yield: 47.6%.
MS m/z(ESI): 219 [M+1]

Step 2

5-aminoquinolin-4-carboxylic acid

5-nitroquinolin-4-carboxylic acid **99b** (6 g, 27.52 mmol) was dissolved in 500 mL of methanol. 5% palladium

on carbon (1 g) was added. The operation of exhausting air and introducing hydrogen was repeated three times. The mixture was reacted under stirring at 40°C for 16 h. The reaction liquid was filtered, and the filtrate was concentrated under reduced pressure to obtain the title product 5-aminoquinolin-4-carboxylic acid **99c** (3 g), yield: 58.0%.
MS m/z(ESI): 189 [M+1]

Step 3

2-oxo-1,2-dihydropyrrolo[4,3,2-de]quinoline

5-aminoquinolin-4-carboxylic acid **99c** (3 g, 15.96 mmol) was dissolved in 50 mL of acetic acid. The mixture was reacted under stirring at 100°C for 1 h. The reaction liquid was concentrated under reduced pressure, and the obtained residue was purified by silica gel column chromatography with eluent system A to obtain the title product 2-oxo-1,2-dihydropyrrolo[4,3,2-*de*]quinoline **99d** (600 mg), yield: 22.1%.
MS m/z(ESI): 171 [M+1]

Referring to the synthesis method of step 1 to step 6 of the intermediate **1** and step 11 of the example **1** in sequence, 1-(2-oxo-1,2-dihydropyrrolo[4,3,2-*de*]quinolin-6-yl)-5-(trifluoromethyl)-*N*-(2-(trifluoromethyl) pyridin-4-yl)-1*H*-pyrazole-4-carboxamide **99** (21 mg) was obtained through 2-oxo-1,2-dihydropyrrolo[4,3,2-*de*]quinoline **99d** (600 mg, 3.53 mmol), yield: 1.2%.

MS m/z(ESI): 493 [M+1].

$^1$H NMR (400 MHz, DMSO) δ 11.37 (s, 2H), 9.25 (d, *J* = 4.4 Hz, 1H), 9.19 (d, *J* = 2.1 Hz, 1H), 8.90 (d, *J* = 2.3 Hz, 1H), 8.54 (s, 1H), 8.22 (s, 1H), 8.09 (d, *J* = 4.4 Hz, 1H), 7.99 (d, *J* = 7.5 Hz, 1H), 7.17 (d, *J* = 7.5 Hz, 1H).

**Example 100**

1-(2-oxo-1,2-dihydropyrrolo[4,3,2-*de*]quinazolin-6-yl)-5-trifluoromethyl-*N*-(2-trifluoromethylpyridin-4-yl)-1*H*-pyrazole-4-carboxamide

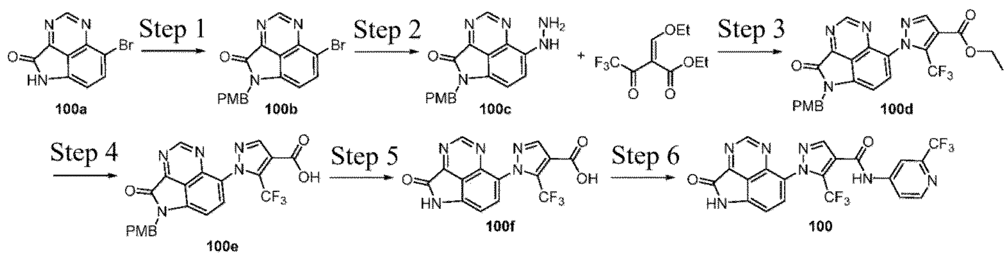

100a 100b 100c 100d 100e 100f 100

Referring to the synthesis method of step 5 in Example **2** and step 7 to step 11 in Example **1** in sequence, the title product 1-(2-oxo-1,2-dihydropyrrolo[4,3,2-*de*]quinazolin-6-yl)-5-trifluoromethyl-*N*-(2-trifluoromethylpyridin-4-yl)-1*H*-pyrazole-4-carboxamide **100** (213 mg) was obtained through 6-bromopyrrolo[4,3,2-*de*]quinazolin-2(1*H*)-one **100a** (2.50 g, 10.00 mmol, prepared by the well known method "Patent WO 2022081928A1"), yield: 4.3%.
MS m/z (ESI): 494 [M+1]

**Example 101**

1-(4-oxo-4,5-dihydropyrrolo[4,3,2-*de*]cinnolin-8-yl)-5-trifluoromethyl-*N*-(2-trifluoromethylpyridin-4-yl)-1*H*-pyrazole-4-carboxamide

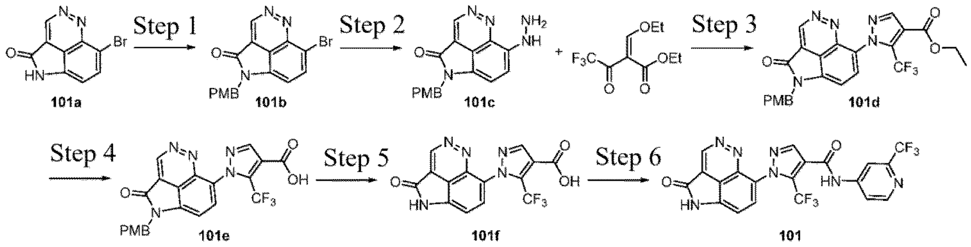

101a 101b 101c 101d 101e 101f 101

Referring to the synthesis method of step 5 in Example **2** and step 7 to step 11 in Example **1** in sequence, the title product 1-(4-oxo-4,5-dihydropyrrolo[4,3,2-*de*]cinnolin-8-yl)-5-trifluoromethyl-*N*-(2-trifluoromethyl pyridin-4-yl)-1*H*-pyrazole-4-carboxamide **101** (263 mg) was obtained through 8-bromopyrrolo[4,3,2-*de*]cinnolin-4(5*H)*-one **101a** (2.50 g, 10 mmol, prepared by the well known method "Patent WO 2022081928A1"), yield: 5.3%.
MS m/z (ESI): 494 [M+1]

**Example 102**

1-(1-methyl-2-carbonyl-1,2-dihydrobenzo[*cd*]indol-6-yl)-5-(trifluoromethyl)-*N*-(2-(trifluoromethyl)pyridin-4-yl)-1*H*-pyrazole-4-carboxamide

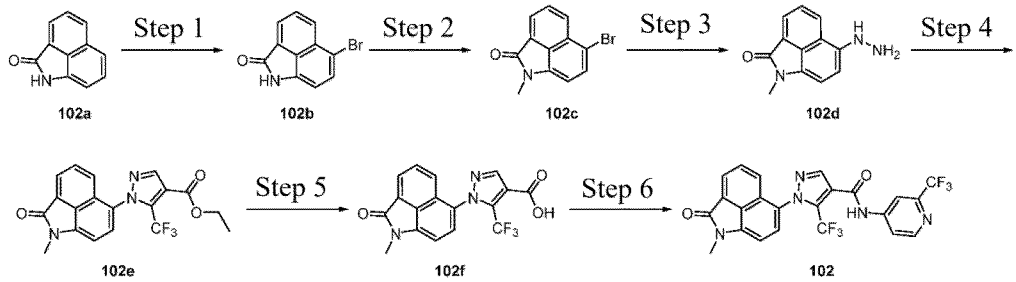

102a 102b 102c 102d 102e 102f 102

Step 1

6-bromobenzo[*cd*]indol-2(1*H*)-one

Referring to the synthesis method of step 1 of intermediate 1, the title product 6-bromobenzo[*cd*]indol-2(1*H*)-one **102b** (6.80 g) was obtained through benzo[*cd*]indol-2(1H)-one **102a** (5.00 g, 29.55 mmol), yield: 93.2%.
MS m/z (ESI): 248 [M+1]

Step 2

6-bromo-1-methylbenzo[*cd*]indol-2(1*H*)-one

6-bromobenzo[*cd*]indol-2(1*H*)-one **102b** (3 g, 12.09 mmol) was dissolved in tetrahydrofuran (30 mL), nitrogen replacement was performed three times, the reaction was cooled to 0°C, sodium hydride (967 mg, 24.19 mmol, 60% purity) was added to the system under nitrogen atmosphere, and the reaction system was stirred at 0°C for 0.5 h, methyl iodide (2.06 g, 14.51 mmol) was added dropwise to the reaction system, and the system was reacted at 0°C for 3 h. Water (5 mL) was added to quench the reaction, the obtained product was extracted with ethyl acetate (100 mL×3), the organic phases were combined, washed with saturated sodium chloride solution (50 mL), dried over anhydrous sodium sulfate, and filtered, and the filtrate was concentrated, and the obtained residue was purified by silica gel column chromatography with eluent system A to obtain the title product 6-bromo-1-methyl benzo[cd]indol-2(1*H*)-one **102c** (2 g), yield: 63.1%.
MS m/z (ESI): 263 [M+1]

Step 3 to step 6

Referring to the synthesis method of step 3 to step 5 of the intermediate 1, and step 3 in Example **34** in sequence, 1-(1-methyl-2-carbonyl-1,2-dihydrobenzo[*cd*]indol-6-yl)-5-(trifluoromethyl)-*N*-(2-(trifluoromethyl)pyridin-4-yl)-1*H*-pyrazole-4-carboxamide **102** (41 mg) was obtained through 6-bromo-1-methylbenzo[*cd*]indol-2(1*H*)-one **102c** (2.00 g, 7.63 mmol), yield: 1.1%.

MS m/z (ESI): 506 [M+1]

$^1$H NMR (400 MHz, DMSO): δ 11.30 (s, 1H), 8.72 (d, *J* = 5.5 Hz, 1H), 8.54 (s, 1H), 8.25 (d, *J* = 1.7 Hz, 1H), 8.19 (d, *J* = 7.0 Hz, 1H), 7.99 (dd, *J* = 5.4, 1.7 Hz, 1H), 7.91 (dd, *J* = 8.2, 7.1 Hz, 1H), 7.81 (d, *J* = 7.5 Hz, 1H), 7.61 (d, *J* = 8.3 Hz, 1H), 7.31 (d, *J* = 7.6 Hz, 1H), 3.44 (s, 3H).

**Example 103**

*N*-(6-(2*H*-1,2,3-triazol-2-yl)-5-trifluoromethylpyridin- 3-yl)-1-(2-oxo-1,2-dihydropyrrolo[4,3,2-*de*]quinazolin-6-yl)-5-trifluoromethyl-1*H*-pyrazole-4-carboxamide

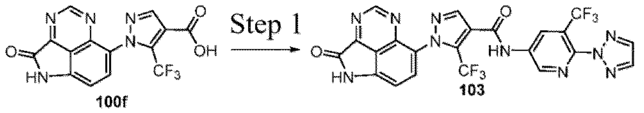


100f 103

Referring to step 11 in example **1,** the title product *N*-(6-(2*H*-1,2,3-triazol-2-yl)-5-trifluoromethylpyridin-3-yl)-1-(2-oxo-1,2-dihydropyrrolo[4,3,2-*de*]quinazolin-6-yl)-5-trifluoromethyl-1*H*-pyrazole-4-carboxamide **103** (23 mg) was obtained through 1-(2-oxo-1,2-dihydropyrrolo[4,3,2-*de*]quinazolin-6-yl)-5-trifluoromethyl-1*H*-pyrazole-4-carboxylic acid **100f** (35 mg, 0.10 mmol), yield: 41.0%.
MS m/z (ESI): 561 [M+1]

**Example 104**

1-(1,2-dihydroacenaphthylen-5-yl)-5-(trifluoromethyl)-N-(2-(trifluoromethyl)pyridin-4-yl)-1H-pyrazole-4-carboxamide

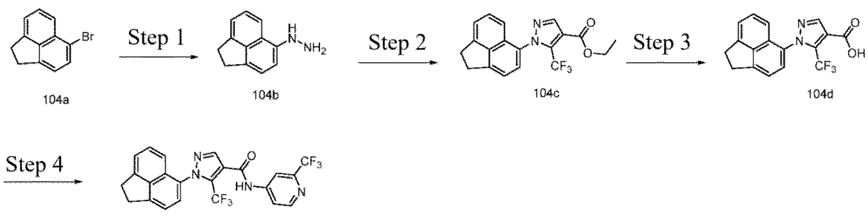


104a 104b 104c 104d 104

Referring to the synthesis method of step 7 to step 9 and step 11 in Example 1, 1-(1,2-dihydroacenaphthylen-5-yl)-5-(trifluoromethyl)-N-(2-(trifluoromethyl)pyridin-4-yl)-1H-pyrazole-4-carboxamide **104** (10 mg) was obtained through 5-bromo-1,2-dihydroacenaphthylene **104a** (2 g, 8.6 mmol), yield: 0.2%.

MS m/z (ESI): 477 [M+1].

1H NMR (400 MHz, DMSO) δ 11.30 (s, 1H), 8.72 (d, J = 5.6 Hz, 1H), 8.53 (s, 1H), 8.26 (d, J = 2.0 Hz, 1H), 8.00 (dd, J = 5.5, 2.0 Hz, 1H), 7.66 (d, J = 7.3 Hz, 1H), 7.58 (t, J = 7.6 Hz, 1H), 7.47 (dd, J = 7.2, 3.7 Hz, 2H), 7.00 (d, J = 8.3 Hz, 1H), 3.48 (s, 4H).

**Example 105**

1-(2-carbonyl-1,2,3,4-tetrahydro-5-oxa-1,2a-diazaacenaphthylen-6-yl)-5-(trifluoromethyl)-N-(2-(trifluoromethyl)pyridin-4-yl)-1H-pyrazole-4-carboxamide

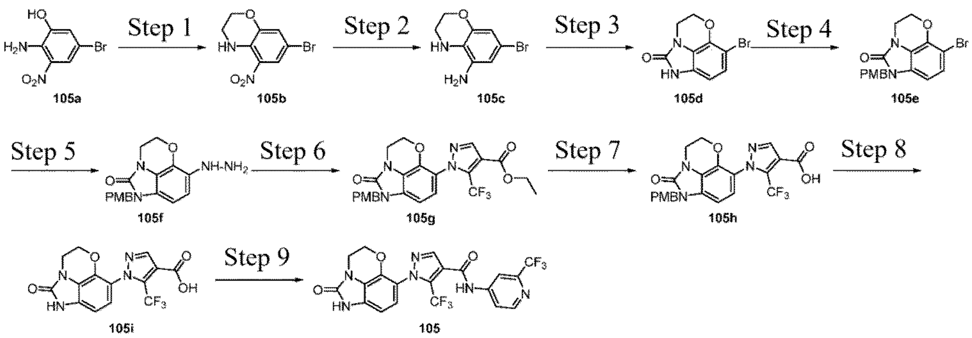


Step 1

7-bromo-5-nitro-3,4-dihydro-2*H*-benzo[*b*][1,4]oxazine

2-amino-5-bromo-3-nitrophenol **105a** (10 g, 42.91 mmol, synthesized by the well known method "Charifson, Paul S.; et al Journal of MedicinalChemistry, 2008, vol. 51, #17, p.5243-5263") and potassium carbonate (18.00 g, 130.43 mmol) were dissolved in DMF (100 mL), nitrogen replacement was performed three times, and dibromoethane (9.62 g, 51.72 mmol) was added to the system, and the system was heated to 100°C and reacted for 5 h. The system was cooled to room temperature, filtered, and concentrated. The residue was dissolved in 200 mL of ethyl acetate, the obtained product was washed with water (50 mL×3) and saturated sodium chloride solution (50 mL×1), dried over anhydrous sodium sulfate, and filtered, the filtrate was concentrated under reduced pressure, and the obtained residue was purified by silica gel column chromatography with eluent system C to obtain the title product 7-bromo-5-nitro-3,4-dihydro-2*H*-benzo[*b*][1,4]oxazine **105b** (5.20 g), yield: 46.8%.
MS m/z (ESI): 260 [M+1]

Step 2

7-bromo-3,4-dihydro-2*H*-benzo[*b*][1,4]oxazin-5-amine

Referring to the synthesis method of step 2 in Example **34,** the title product 7-bromo-3,4-dihydro-2*H*-benzo[*b*][1,4]oxazin-5-amine **105c** (3.81 g) was obtained through 7-bromo-3,4-dihydro-2*H*-benzo[*b*][1,4]oxazin-5-amine **105b**(5.20 g, 20.07 mmol), yield: 82.6%.
MS m/z (ESI): 230 [M+1]

Step 3

6-bromo-3,4-dihydro-5-oxa-1,2a-diazaacenaphthylen-2(1*H*)-one

7-bromo-3,4-dihydro-2*H*-benzo[*b*][1,4]oxazin-5-amine **105c** (3.81 g, 16.63 mmol) was dissolved in tetrahydrofuran (50 mL), nitrogen replacement was performed three times, the obtained mixture was cooled to 0°C in an ice-water bath, and triethylamine (5.05 g, 49.90 mmol) was added dropwise to the system. Triphosgene (2.47 g, 8.32 mmol) was dissolved in tetrahydrofuran (10 mL) and the mixture was added dropwise to the system. After the addition was completed, the obtained mixture was reacted at 0°C for 2 h. Aqueous sodium bicarbonate solution was added to quench the reaction, the obtained product was concentrated, extracted with ethyl acetate (80 mL×3), washed with water (50 mL) and saturated sodium chloride solution (50 mL×1) in sequence, dried over anhydrous sodium sulfate, and filtered, and the filtrate was concentrated under reduced pressure and the obtained residue was purified by silica gel column chromatography with eluent system C to obtain the title product 6-bromo-3,4-dihydro-5-oxa-1,2a-diazaacenaphthylen-2(1*H*)-one **105d** (1.92 g), yield: 45.3%.
MS m/z (ESI):256 [M+1]

Step 4 to step 8

1-(2-carbonyl-1,2,3,4-tetrahydro-5-oxa-1,2a-diazaacenaphthylen-6-yl)-5-(trifluoromethyl)-*N*-(2-(trifluoromethyl)pyridin-4-yl)-1*H*-pyrazole-4-carboxamide

Referring to the synthesis method of step 2 to step 6 of the intermediate **1,** and step 3 in example **34** in sequence, 1-(2-carbonyl-1,2,3,4-tetrahydro-5-oxa-1,2a-diazaacenaphthylen-6-yl)-5-(trifluoromethyl)-*N*-(2-(trifluoromethyl)pyridin-4-yl)-1*H*-pyrazole-4-carboxamide **105** (12 mg) was obtained through 6-bromo-3,4-dihydro-5-oxa-1,2a-diazaacenaphthylen-2(1*H*)-one **105d** (1.80 g, 7.06 mmol), yield: 0.3%.

MS m/z (ESI): 499 [M+1]
$^1$H NMR (400 MHz, DMSO): δ 11.41 (s, 1H), 10.91 (s, 1H), 8.47 (d, *J* = 6.8 Hz, 1H), 8.25 (s, 1H), 7.68-7.43 (m, 2H), 7.16 (d, *J* = 8.8 Hz, 2H), 4.37 (t, *J* = 7.2 Hz, 2H), 3.58 (t, *J* = 6.8 Hz, 2H).

**Example 106**

1-(2-carbonyl-1,2,5,6-tetrahydro-4H-imidazo[*4,5,1-ij*]quinolin-7-yl)-5-(trifluoromethyl)-*N*-(2-(trifluoromethyl)pyridin-4-yl)-1*H*-pyrazole-4-carboxamide

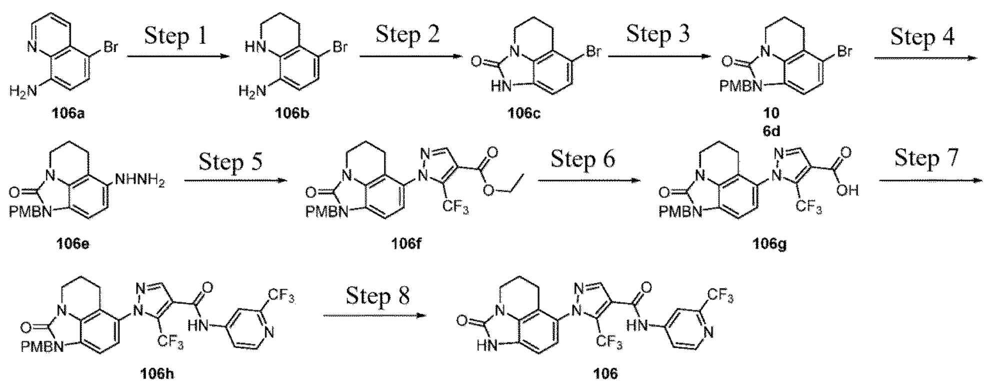


Step 1

5-bromo-1,2,3,4-tetrahydroquinolin-8-amine

5-Bromoquinolin-8-amine **106a** (2.90 g, 13 mmol) was dissolved in acetic acid (30 mL), nitrogen replacement was performed three times, platinum dioxide (295 mg, 1.30 mmol) was added, and hydrogen replacement was performed three times, and the system was reacted in hydrogen atmosphere at 25°C for 24 h. The reaction was quenched by introducing air, filtered, and concentrated. The residue was dissolved in 100 mL of ethyl acetate, the obtained product was washed by adding 1 M sodium hydroxide solution (20 mL), washed with saturated sodium chloride aqueous solution (30 mL), dried over anhydrous sodium sulfate, filtered, and concentrated. The title product 5-bromo-1,2,3,4-tetrahydroquinolin-8-amine **106b** (2.51 g) was obtained, yield: 85.1%.
MS m/z (ESI): 228 [M+1]

Step 2

7-bromo-5,6-dihydro-4*H*-imidazo[*4,5,1-ij*]quinolin-2(1*H*)-one

5-bromo-1,2,3,4-tetrahydroquinolin-8-amine **106b** (1.8 g, 7.93 mmol) was dissolved in tetrahydrofuran (80 mL), and nitrogen replacement was performed three times, the system was cooled to 0°C, triethylamine (2.41 g, 23.78 mmol, 3.3 mL) and triphosgene (1.65 g, 5.55 mmol) were added to the system, and the system was reacted at 0°C for 1 h. 20 mL of water was added to quench the reaction, and the obtained product was concentrated, extracted with ethyl acetate (50 mL×3), washed with saturated sodium chloride solution (50 mL), dried over anhydrous sodium sulfate, and filtered, the filtrate was concentrated, and the obtained residue was purified by silica gel column chromatography with eluent

system C to obtain the title product 7-bromo-5,6-dihydro-4*H*-imidazo[*4,5,1-ij*]quinolin-2(1*H*)-one **106c** (0.82 g), yield: 39.8%. MS m/z (ESI): 254 [M+1]

Step 3 to step 8

Referring to the synthesis method of step 2 to step 5 of intermediate **1,** step 11 to step 12 in Example **87** in sequence, 1-(2-carbonyl-1,2,5,6-tetrahydro-4H-imidazo[*4,5,1-ij*]quinolin-7-yl)-5-(trifluoromethyl)-*N*-(2-(trifluoromethyl)pyridin-4-yl)-1*H*-pyrazole-4-carboxamide **106** (11 mg) was obtained through 7-bromo-5,6-dihydro-4*H*-imidazo[*4,5,1-ij*]quinolin-2(1*H*)-one **106c** (1.65 g, 6.52 mmol), yield: 0.3%.

MS m/z (ESI): 497 [M+1]
$^1$H NMR (400 MHz, DMSO): δ 10.56 (s, 1H), 9.76 (s, 1H), 8.42 (d, J = 8.4 Hz, 1H), 8.21 (s, 1H), 7.62-7.53 (m, 2H), 7.25-7.06 (m, 2H), 3.88 (t, J = 8.0 Hz, 2H), 3.46 (t, J = 6.4 Hz, 2H), 1.85-1.72 (m, 2H).

## Example 107

1-(5-carbonyl-2,3-dihydro-5H-[1,4]oxaazino[2,3,4-ij]quinolin-7-yl)-5-(trifluoromethyl)-N-(2-(trifluoromethyl)pyridin-4-yl)-1H-pyrazole-4-carboxamide

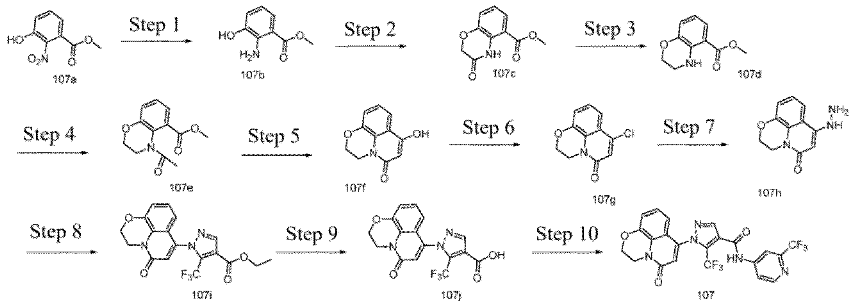


Step 1

Methyl 2-amino-3-hydroxyl benzoate

Methyl 3-hydroxyl-2-nitro benzoate **107a** (5 g, 25.36 mmol) was dissolved in 20 mL of ethanol and 5 mL of water. Ammonium chloride (2 g, 37.39 mmol) and reduced iron powder (2 g, 35.81 mmol) were added and the reaction was carried out at 80°C for 2 h. The reaction liquid was cooled to room temperature and poured into 50 mL of water. The obtained mixture was filtered, the filtrate was extracted with ethyl acetate (50 mL×3), the organic phases were combined, washed with saturated sodium chloride solution (50 mL×2), dried over anhydrous sodium sulfate, filtered and concentrated to obtain a crude product, and the obtained residue was purified by silica gel column chromatography with eluent system A to obtain the title product methyl 2-amino-3-hydroxyl benzoate **107b** (3 g), yield: 70.8%.
MS m/z (ESI): 168 [M+1]

Step 2

Methyl 3-carbonyl-3,4-dihydro-2H-benzo[b][1,4]oxazin-5-carboxylate

Methyl 2-amino-3-hydroxyl benzoate **107b** (3 g, 17.95 mmol) was dissolved in 20 mL of acetonitrile. Triethylamine (2 g, 19.76 mmol) and chloroacetyl chloride (2 g, 17.71 mmol) were added and the reaction was carried out at 20°C for 12 h. The reaction liquid was cooled to room temperature and poured into 50 mL of water. The obtained mixture was filtered, the filtrate was extracted with ethyl acetate (50 mL×3), the organic phases were combined, washed with saturated sodium chloride solution (50 mL×2), dried over anhydrous sodium sulfate, filtered and concentrated to obtain a crude product, and the obtained residue was purified by silica gel column chromatography with eluent system A to obtain the title product methyl 3-carbonyl-3,4-dihydro-2H-benzo[b][1,4]oxazin-5-carboxylate **107c** (1.5 g), yield: 40.3%.
MS m/z (ESI): 208 [M+1]

Step 3

Methyl 3,4-dihydro-2H-benzo[b][1,4]oxazin-5-carboxylate

Methyl 3-carbonyl-3,4-dihydro-2H-benzo[b][1,4]oxazin-5-carboxylate **107c** (1.5 g, 7.24 mmol) was dissolved in 20 mL of tetrahydrofuran. Boron trifluoride diethyl etherate (2 g, 7.00 mmol, 50%) was added at 0°C, and sodium borohydride (500 mg, 13.22 mmol) was added and the reaction was carried out at 0°C for 2 h. The reaction liquid was cooled to room temperature and poured into 50 mL of water. The obtained mixture was filtered, the filtrate was extracted with ethyl acetate (50 mL×3), the organic phases were combined, washed with saturated sodium chloride solution (50 mL×2), dried over anhydrous sodium sulfate, filtered and concentrated to obtain a crude product, and the obtained residue was purified by silica gel column chromatography with eluent system A to obtain the title product methyl 3,4-dihydro-2H-benzo[b][1,4]oxazin-5-carboxylate **107d** (1 g), yield: 71.5%.
MS m/z (ESI): 194 [M+1]

Step 4

4-Acetyl-3,4-dihydro-2H-benzo[b][1,4]oxazin-5-carboxylate methyl

Methyl 3,4-dihydro-2H-benzo[b][1,4]oxazin-5-carboxylate **107d**(1 g, 5.18 mmol) was dissolved in 20 mL of acetonitrile. Acetyl chloride (500 mg, 6.37 mmol) and triethylamine (700 mg, 6.92 mmol) were added, and the reaction was carried out at 20°C for 2 h. The reaction liquid was cooled to room temperature and poured into 50 mL of water. The obtained mixture was filtered, the filtrate was extracted with ethyl acetate (50 mL×3), the organic phases were combined, washed with saturated sodium chloride solution (50 mL×2), dried over anhydrous sodium sulfate, filtered and concentrated to obtain a crude product, and the obtained residue was purified by silica gel column chromatography with eluent system A to obtain the title product methyl 4-acetyl-3,4-dihydro-2H-benzo[b][1,4]oxazin-5-carboxylate **107e** (600 mg), yield: 49.3%.
MS m/z (ESI): 236 [M+1]

Step 5

7-hydroxyl-2,3-dihydro-5H-[1,4]oxaazino[2,3,4-ij]quinolin-5-one

Methyl 4-acetyl-3,4-dihydro-2H-benzo[b][1,4]oxazin-5-carboxylate **107d** (600 mg, 2.55 mmol) was dissolved in 20 mL of tetrahydrofuran. Bis(trismethylsilyl)aminosodium (2 mL, 2N) was added at 0°C, followed by reaction at 0°C for 2 h. The reaction liquid was cooled to room temperature and poured into 50 mL of water. The obtained mixture was filtered, the filtrate was extracted with ethyl acetate (50 mL×3), the organic phases were combined, washed with saturated sodium chloride solution (50 mL×2), dried over anhydrous sodium sulfate, filtered and concentrated to obtain a crude product, and the obtained residue was purified by silica gel column chromatography with eluent system A to obtain the title product 7-hydroxyl-2,3-dihydro-5H-[1,4]oxaazino[2,3,4-ij]quinolin-5-one **107f** (400 mg), yield: 77.1%.
MS m/z (ESI): 204 [M+1]

Step 6

7-chloro-2,3-dihydro-5H-[1,4]oxaazino[2,3,4-ij]quinolin-5-one

7-hydroxyl-2,3-dihydro-5H-[1,4]oxaazino[2,3,4-ij]quinolin-5-one **107f** (400 mg, 1.97 mmol) was dissolved in 20 mL of phosphorus oxychloride. The mixture was reacted at 100°C for 2 h. The reaction liquid was cooled to room temperature and poured into 50 mL of water. The obtained mixture was filtered, the filtrate was extracted with ethyl acetate (50 mL×3), the organic phases were combined, washed with saturated sodium chloride solution (50 mL×2), dried over anhydrous sodium sulfate, filtered and concentrated to obtain a crude product, and the obtained residue was purified by silica gel column chromatography with eluent system A to obtain the title product 7-chloro-2,3-dihydro-5H-[1,4]oxaazino[2,3,4-ij]quinolin-5-one **107g** (250 mg), yield: 57.3%.
MS m/z (ESI): 222 [M+1]

Step 7

7-hydrazino-2,3-dihydro-5H-[1,4]oxaazino[2,3,4-ij]quinolin-5-one

7-chloro-2,3-dihydro-5H-[1,4]oxaazino[2,3,4-ij]quinolin-5-one **107g** (250 mg, 1.13 mmol) was dissolved in 20 mL of hydrazine hydrate. The mixture was reacted at 100°C for 2 h. The reaction liquid was cooled to room temperature and poured into 50 mL of water. The obtained mixture was filtered and concentrated to obtain the title product 7-hydrazino-2,3-dihydro-5H-[1,4]oxaazino[2,3,4-ij]quinolin-5-one **107h** (200 mg), yield: 81.6%.
MS m/z (ESI): 218 [M+1]

Referring to the synthesis method of step 8, step 9 and step 11 in Example 1, 1-(5-carbonyl-2,3-dihydro-5H-[1,4 ]oxaazino[2,3,4-ij]quinolin-7-yl)-5-(trifluoromethyl)-N-(2-(trifluoromethyl)pyridin-4-yl)-1H-pyrazole-4-carboxamide **107** (20 mg) was obtained through 7-hydrazino-2,3-dihydro-5H-[1,4]oxaazino[2,3,4-ij]quinolin-5-one **107h** (200 mg, 0.92 mmol), yield: 4.3%.

MS m/z (ESI): 510 [M+1].

1H NMR (400 MHz, DMSO) δ 8.35 - 8.21 (m, 1H), 8.15 (dd, J = 8.3, 1.2 Hz, 1H), 7.85 (t, J = 8.0 Hz, 1H), 7.72 (t, J = 5.3 Hz, 1H), 7.47 - 7.34 (m, 2H), 7.02 - 6.83 (m, 2H), 4.72 (d, J = 5.3 Hz, 2H), 3.74 (s, 3H).

**Example 108**

1-(3-carbonyl-2,3-dihydronaphtho[1,8-de][1,2]oxazin-7-yl)-5-(trifluoromethyl)-N-(2-(trifluoromethyl)pyridin-4-yl)-1H-pyrazole-4-carboxamide

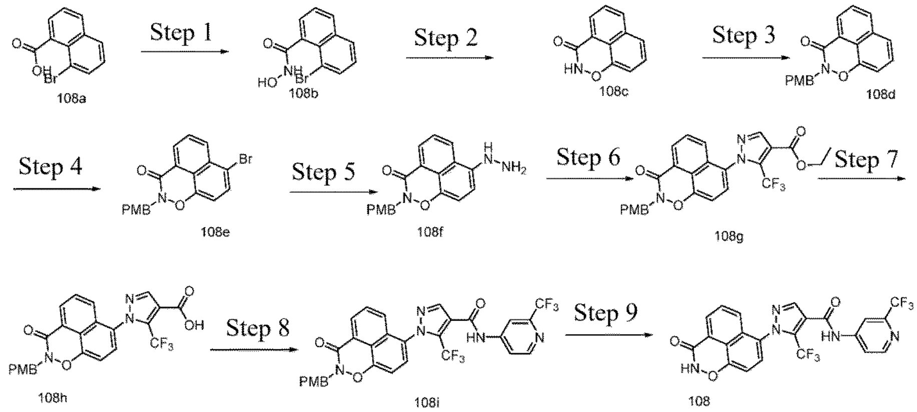


Step 1

8-bromo-N-hydroxyl-1-naphthylamide

8-bromo-1-naphthoic acid **108a** (5 g, 19.91 mmol) was dissolved in 20 mL of dichloromethane. 2-(7-azobenzotriazole)-N,N,N',N'-tetrakismethylurea hexafluorophosphate (10 g, 26.30 mmol) and triethylamine (4 g, 39.53 mmol) were added, and the mixture was reacted at 20°C for 1 h, then hydroxylamine hydrochloride (1.5 g, 21.58 mmol) was added, and the mixture was reacted at 20°C for 1 h. The reaction liquid was cooled to room temperature and poured into 50 mL of water. The obtained mixture was filtered, the filtrate was extracted with ethyl acetate (50 mL×3), the organic phases were combined, washed with saturated sodium chloride solution (50 mL×2), dried over anhydrous sodium sulfate, filtered and concentrated to obtain a crude product, and the obtained residue was purified by silica gel column chromatography with eluent system A to obtain the title product 8-bromo-N-hydroxyl-1-naphthylamide **108b** (2 g), yield: 37.7%.
MS m/z (ESI):266 [M+1]

Step 2

Naphtho[1,8-de][1,2]oxazin-3(2H)-one

8-bromo-N-hydroxyl-1-naphthylamide **108b** (2 g, 7.52 mmol) was dissolved in 20 mL of dioxane. Cesium carbonate (4 g, 12.28 mmol), 4,5-bisbisphenylphosphine-9,9-dimethylxanthene (500 mg, 0.86 mmol) and tris(dibenzylideneacetone)dipalladium were added (500 mg, 0.55 mmol) and then the mixture was reacted at 110°C for 12 h. The reaction liquid was cooled to room temperature and poured into 50 mL of water. The obtained mixture was filtered, the filtrate was extracted with ethyl acetate (50 mL×3), the organic phases were combined, washed with saturated sodium chloride solution (50 mL×2), dried over anhydrous sodium sulfate, filtered and concentrated to obtain a crude product, and the obtained residue was purified by silica gel column chromatography with eluent system A to obtain the title product naphtho[1,8-de][1,2]oxazin-3(2H)-one **108c** (900 mg),yield: 64.7%.
MS m/z (ESI):186 [M+1]

Step 3

2-(4-methoxybenzyl)naphtho[1,8-de][1,2]oxazin-3(2H)-one

Naphtho[1,8-de][1,2]oxazin-3(2H)-one **108c** (900 mg, 4.86 mmol) was dissolved in 20 mL of tetrahydrofuran. Cesium carbonate (4 g, 12.28 mmol) and 4-methoxychlorobenzyl (1 g, 6.38 mmol) were added and the reaction was carried out at 100°C for 12 h. The reaction liquid was cooled to room temperature and poured into 50 mL of water. The obtained mixture was filtered, the filtrate was extracted with ethyl acetate (50 mL×3), the organic phases were combined, washed with saturated sodium chloride solution (50 mL×2), dried over anhydrous sodium sulfate, filtered and concentrated to obtain a crude product, and the obtained residue was purified by silica gel column chromatography with eluent system A to obtain the title product 2-(4-methoxybenzyl)naphtho[1,8-de][1,2]oxazin-3(2H)-one **108d** (1 g), yield: 67.3%.
MS m/z (ESI):306 [M+1]

Referring to the synthesis method of step 6 to step 9 and step 11 to step 12 in Example 3, 1-(3-carbonyl-2,3-dihydronaphtho[1,8-de][1,2]oxazin-7-yl)-5-(trifluoromethyl)-N-(2-(trifluoromethyl)pyridin-4-yl)-1H-pyrazole-4-carboxamide **108** (10 mg) was obtained through 2-(4-methoxybenzyl)naphtho[1,8-de][1,2]oxazin-3(2H)-one **108d** (1 g, 3.28 mmol), yield: 0.6%. MS m/z (ESI): 508 [M+1].

**Example 109**

1-(3-carbonyl-2,3-dihydro-1H-benzo[de]cinnolin-7-yl)-5-(trifluoromethyl)-N-(2-(trifluoromethyl)pyridin-4-yl)-1H-pyrazole-4-carboxamide

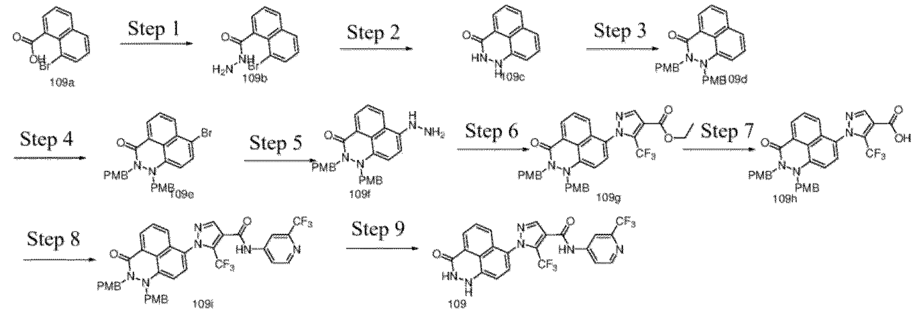


Referring to the synthesis method of Example **108,** 1-(3-carbonyl-2,3-dihydro-1H-benzo[de]cinnolin-7-yl)-5-(trifluoromethyl)-N-(2-(trifluoromethyl)pyridin-4-yl)-1H -pyrazole-4-carboxamide **109** (10 mg) was obtained through 8-bromo-1-naphthoic acid **109a** (5 g, 19.91 mmol), yield: 0.1%.
MS m/z (ESI): 507 [M+1].

## Example 110

1-(1H-naphtho[1,8-de][1,2,3]triazin-7-yl)-5-(trifluoromethyl)-N-(2-(trifluoromethyl)pyridin-4-yl)-1H-pyrazole-4-carboxamide

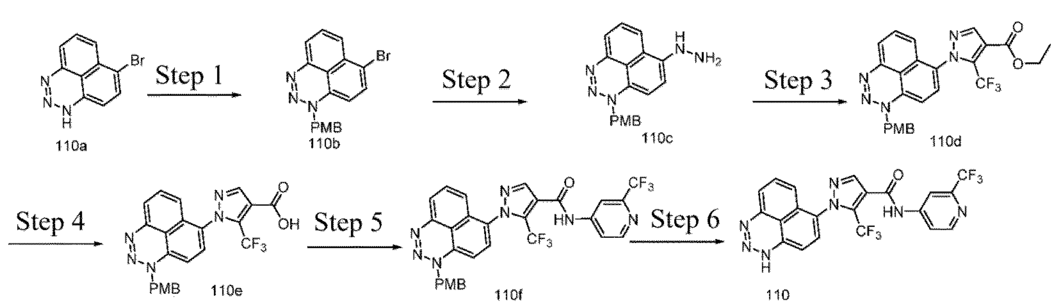


110a 110b 110c 110d 110e 110f 110

The well known method "Patent WO 2022081927A" was used to prepare 7-bromo-1H-naphtho[1,8-de][1,2,3]triazine. Referring to the synthesis method of step 5 to step 9 of Example 108, 1-(1H-naphtho[1,8-de][1,2,3]triazin-7-yl)-5-(trifluoromethyl)-N-(2-(trifluoromethyl)pyridin-4-yl)-1H-pyrazole-4-carboxamide 110 (10 mg) was obtained through 7-bromo-1H-naphtho[1,8-de][1,2,3]triazine **110a** (5 g, 20.15 mmol), yield: 0.1%.

MS m/z (ESI): 492 [M+1].

## Example 111

N-(6-(2H-1,2,3-triazol-2-yl)-5-(trifluoromethyl)pyridin-3-yl)-1-(2-carbonyl-1,2-dihydro-1,2,4-triazacyclopentadieno[cd]inden-5-yl)-5-(trifluoromethyl)-1H-pyrazole-4-carboxamide

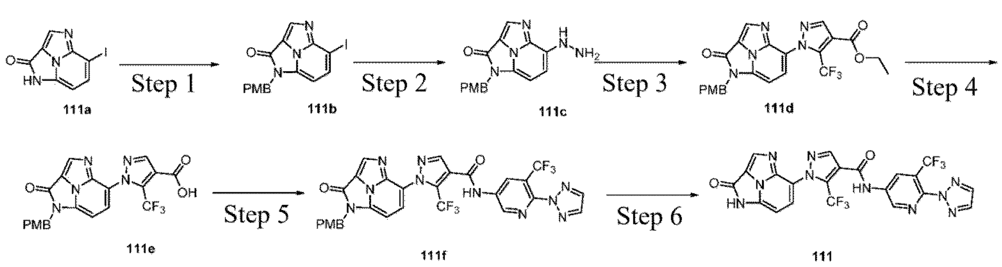


111a 111b 111c 111d 111e 111f 111

Referring to the synthesis method of step 2 of intermediate 1 and step 7 to step 11 in Example 1, N-(6-(2H-1,2,3-triazol-2-yl)-5-(trifluoromethyl)pyridin-3-yl)-1-(2-carbonyl-1,2-dihydro-1,2,4-triazacyclopentadieno [cd]inden-5-yl)-5-(trifluoromethyl)-1H-pyrazole-4-carboxamide **111** (18 mg) was obtained through 5-iodo-1,2a1,4-triazacyclopentadieno[cd]inden-2(1H)-one **111a** (230 mg, 0.81 mmol, prepared by the well known method "Patent WO 2022089406 A1"), yield: 4.0%. MS m/z (ESI): 549 [M+1]

## Example 112

N-(6-(2H-1,2,3-triazol-2-yl)-5-(trifluoromethyl)pyridin-3-yl)-1-(2-carbonyl-1,2-dihydro-1,2a1,3-triazacyclopentadieno[cd]inden-5-yl)-5-(trifluoromethyl)-1H-pyrazole-4-carboxamide

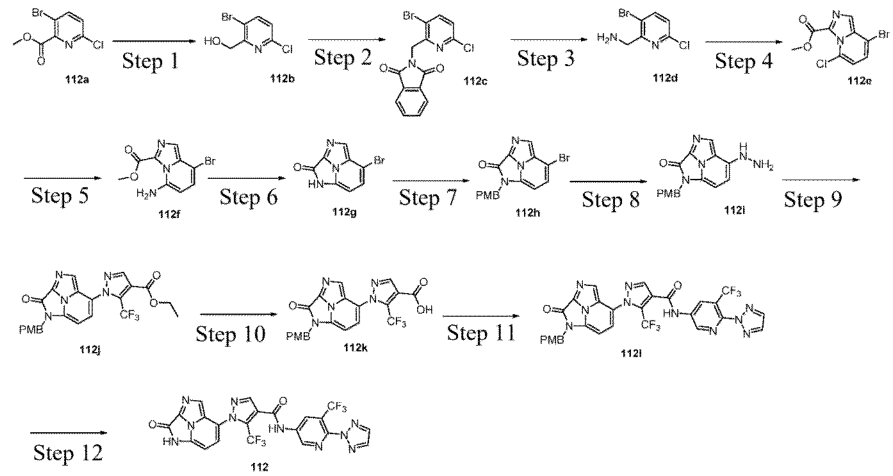


Step 1

(3-bromo-6-chloropyridin-2-yl)methanol

Methyl 3-bromo-6-chloro-pyridin-2-carboxylate **112a** (2 g, 7.98 mmol) was dissolved in 20 ml of methanol, and sodium borohydride (1.51 g, 39.92 mmol) was added slowly in batches under ice bath, the mixture was reacted under stirring at room temperature for 16 h. The reaction liquid was poured into 100 mL of saturated sodium chloride aqueous solution, and the mixture was extracted with dichloromethane (100 mL×3), the organic phases were combined, dried over anhydrous sodium sulfate and concentrated under reduced pressure to obtain the title product (3-bromo-6-chloro-pyridin-2-yl)methanol **112b** (1.60 g), yield: 89.9%.
MS m/z (ESI): 223 [M+1].

Step 2

2-((3-bromo-6-chloropyridin-2-yl)methyl)isodihydroindol-1,3-dione

Methyl 3-bromo-6-chloro-pyridin-2-carboxylate **112b** (1 g, 4.50 mmol) and triphenylphosphine (2.36 g, 8.99 mmol) were dissolved in 20 ml of tetrahydrofuran, phthalimide (727 mg, 4.94 mmol) and diethyl azodicarboxylate (1.72g, 9.89 mmol) were slowly added under ice bath, and the mixture was reacted under stirring at room temperature for 1 h. The reaction liquid was concentrated under reduced pressure, and the obtained residue was purified by silica gel column chromatography with eluent system B to obtain the title product 2-((3-bromo-6-chloropyridin-2-yl)methyl)isodihydroindol-1,3-dione **112c** (1.31 g), yield: 82.3%.
MS m/z (ESI): 352 [M+1].

Step 3

(3-bromo-6-chloropyridin-2-yl)methylamine

2-((3-bromo-6-chloropyridin-2-yl)methyl)isodihydroindol-1,3-dione **112c** (800 mg, 2.28 mmol) was dissolved in 20 mL of ethanol, and hydrazine hydrate (145 mg, 4.55 mmol, 98% purity) was added. The reaction mixture was reacted under stirring at room temperature for 1 h. The reaction liquid was poured into 100 mL of water, ethyl acetate (100 mL×3) was added for extraction. The organic phases were combined and dried over anhydrous sodium sulfate, and distilled under reduced pressure to obtain the title product (3-bromo-6-chloropyridin-2-yl)methylamine **112d** (480 mg), yield: 95.2%.
MS m/z (ESI): 222 [M+1].

Step 4

Methyl 8-bromo-5-chloroimidazo[1,5-a]pyridin-3-carboxylate

(3-bromo-6-chloropyridin-2-yl)methylamine **112d** (400 mg, 1.81 mmol) and ethyl oxalyl monochloride (278 mg,

2.03 mmol) were dissolved in 10 mL of tetrahydrofuran, and triethylamine (206 mg, 2.03 mmol) was added. The reaction mixture was reacted under stirring at room temperature for 6 h. After spinning the reaction liquid to dryness, the residue was dissolved in 6 mL of phosphorus oxychloride, and the reaction was heated at 105°C for 2 h. The reaction liquid was concentrated under reduced pressure, and the obtained residue was purified by silica gel column chromatography with eluent system A to obtain the title product methyl 8-bromo-5-chloroimidazo[1,5-a]pyridin-3-carboxylate **112e** (510 mg), yield: 93.1%.

MS m/z (ESI): 288 [M+1].

Step 5

Methyl 8-bromo-5-aminoimidazo[1,5-a]pyridin-3-carboxylate

Methyl 8-bromo-5-chloroimidazo[1,5-a]pyridin-3-carboxylate **112e** (510 mg, 1.77 mmol) was dissolved in 10 mL of ammoniamethanol (7M) and the mixture was reacted in a sealed tube at 80°C for 16 h. The reaction liquid was concentrated under reduced pressure, and the obtained residue was purified by silica gel column chromatography with eluent system A to obtain the title product methyl 8-bromo-5-aminoimidazo[1,5-a]pyridin-3-carboxylate **112f** (320 mg), yield: 65.1%.

MS m/z (ESI): 270 [M+1].

Step 6

5-bromo-1,2,3-triazacyclopentadieno[cd]inden-2(1H)-one

Methyl 8-bromo-5-aminoimidazo[1,5-a]pyridin-3-carboxylate **112f** (320 mg, 1.19 mmol) was dissolved in 10 mL of acetonitrile, 10 mL of 25% sodium methoxide solution in methanol was added, and the mixture was heated at 70°C and reacted for 16 h. The pH of the reaction liquid was adjusted to acidic with 2M hydrochloric acid, and the obtained residue was purified by silica gel column chromatography with eluent system C to obtain the title product 5-bromo-1,2,3-triazacyclopentadieno[cd]inden-2(1H)-one **112g** (208 mg), yield: 65.6%.

MS m/z (ESI): 238 [M+1].

Referring to the synthesis method of step 2 of intermediate 1 and step 7 to step 11 in Example 1, N-(6-(2H-1,2,3-triazol-2-yl)-5-(trifluoromethyl)pyridin-3-yl)-1-(2-carbonyl-1,2-dihydro-1,2a1,3-triazacyclopentadieno[cd]inden-5-yl)-5-(trifluoromethyl)-1H-pyrazole-4-carboxamide **112** (9 mg) was obtained through 5-bromo-1,2,3-triazacyclopentadieno[cd]inden-2(1H)-one **112g** (208 mg, 0.88 mmol), yield: 1.8%.

MS m/z (ESI): 549 [M+1]

**Example 113**

*N*-(6-(2*H*-1,2,3-triazol-2-yl)-5-(trifluoromethyl)pyridin-3-yl)-1-(2-carbonyl-1,2-dihydro-1,4,4a-triazacyclopentadieno[*cd*]inden-5-yl)-5-(trifluoromethyl)-1*H*-pyrazole-4-carboxamide

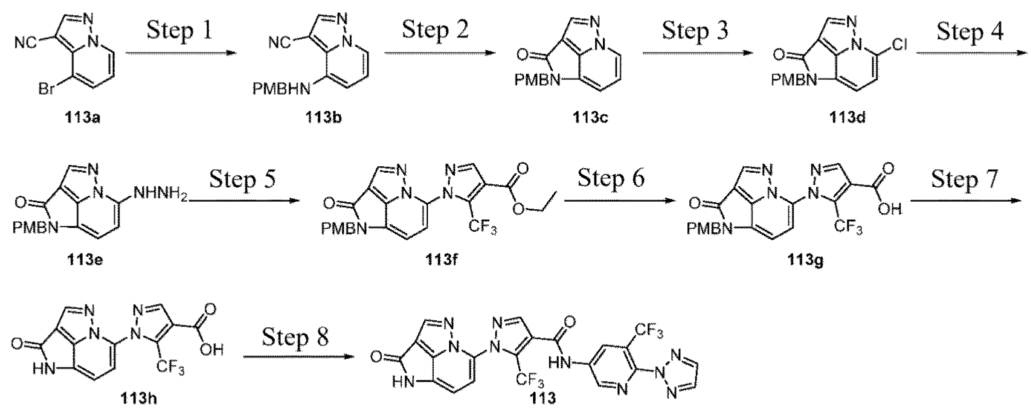


113a 113b 113c 113d 113e 113f 113g 113h 113

Referring to the synthesis method of step 3 to step 4 in Example **8,** step 1 in Example **13,** step 7 in Example **8,** step 4 to step 6 of the intermediate **1,** and step 11 in Example 1 in sequence, N-(6-(2*H*-1,2,3-triazol-2-yl)-5-(trifluoromethyl)pyridin-3-yl)-1-(2-carbonyl-1,2-dihydro-1,4,4a-triazacyclopentadieno[*cd*]inden-5-yl)-5-(trifluoromethyl)-1*H*-pyrazole-4-carboxamide **113** (8 mg) was obtained through the reaction of 4-bromopyrazolo[1,5-a]pyridin-3-carbonitrile **113a** (15 g, 6.78 mmol), yield: 0.2%.

MS m/z (ESI): 549 [M+1]
$^1$H NMR (400 MHz, DMSO): δ 11.10 (s, 1H), 10.82 (s, 1H), 8.26-8.12 (m, 2H), 8.54 (s, 1H), 8.02 (s, 2H), 7.85 (s, 1H), 7.46 (d, *J* = 5.6 Hz, 1H), 6.92 (d, *J* = 7.6 Hz, 1H).

**Example 114**

*N*-(6-(2*H*-1,2,3-triazol-2-yl)-5-trifluoromethylpyridin-3-yl)-1-(4-oxo-4,5-dihydropyrrolo[4,3,2-de]quinazolin-8-yl)-5-trifluoromethyl-1*H*-pyrazole-4-carboxamide

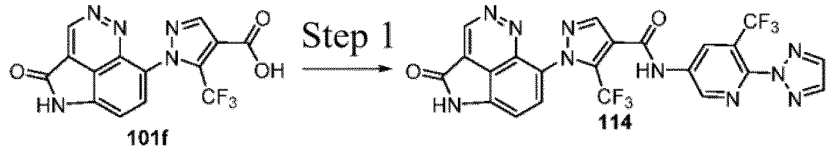


Referring to step 11 in example **1,** the title product *N*-(6-(2*H*-1,2,3-triazol-2-yl)-5-trifluoromethylpyridin-3-yl)-1-(4-oxo-4,5-dihydropyrrolo[4,3,2-*de*]quinazolin-8-yl)-5-trifluoromethyl-1*H*-pyrazole-4-carboxamide **114** (21 mg) was obtained through 1-(4-oxo-4,5-dihydropyrrolo[4,3,2-*de*]quinazolin-8-yl)-5-trifluoromethyl-1*H*-pyrazole-4-carboxylic acid **101f** (35 mg, 0.10 mmol), yield: 37.4%.
MS m/z (ESI): 561 [M+1]

**Example 115**

1-(2-carbonyl-1,2-dihydro-1,2a-diazabenzo[*cd*]azulen-6-yl)-5-(trifluoromethyl)-*N*-(2-(trifluoromethyl)pyridin-4-yl)-1*H*-pyrazole-4-carboxamide

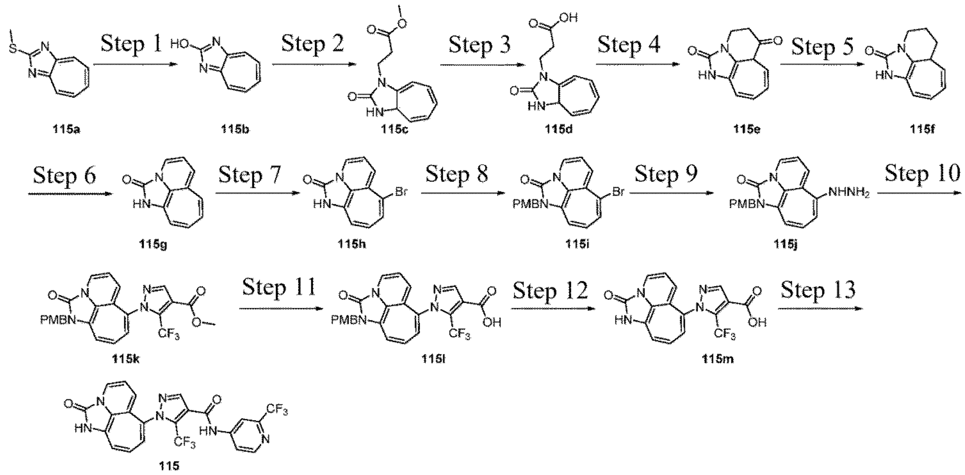


Step 1

Cycloheptatrieno[*d*]imidazol-2-ol

2-(methylthio)cycloheptatrieno[d]imidazole **115a** (10 g, 56.82 mmol, synthesized by the well known method "Journal of the American Chemical Society, 1954, Vol. 76, p. 3352- 3353") was dissolved in concentrated hydrochloric acid (50 mL), the mixture was heated to reflux for 2 h, and cooled to room temperature, ethanol (80 mL) was added,

and the mixture was stirred at room temperature for 1 h. The obtained mixture was filtered, the filter residue was dissolved in water (80 mL), saturated sodium bicarbonate solution was added to adjust the pH to 7-8, the mixture was filtered, the filter cake was washed by adding water (10 mL×2), and dried. The title product cycloheptatrieno[*d*]imidazol-2-ol **115b** (6.51 g) was obtained, yield: 79.8%.
MS m/z (ESI): 147 [M+1]

Step 2

Methyl 3-(2-carbonyl-3,3a-dihydrocycloheptatrieno[*d*]imidazol-1(2*H*)-yl)propionate

Cycloheptatrieno[*d*]imidazol-2-ol **115b** (5 g, 34.21 mmol) was dissolved in DMF (50 mL), potassium carbonate (14.18 g, 102.63 mmol) and methyl bromopropionate (11.43 g, 68.42 mmol) were added, nitrogen replacement was performed three times, and the mixture was heated to 80°C and reacted for 2 h. The obtained product was cooled to room temperature, filtered and concentrated. The obtained residue was purified by silica gel column chromatography with eluent system C to obtain the title product methyl 3-(2-carbonyl-3,3a-dihydrocycloheptatrieno[*d*]imidazol-1(2*H*)-yl)propionate **115c** (7.20 g), yield: 89.8%.
MS m/z (ESI):235 [M+1]

Step 3

3-(2-carbonyl-3,3a-dihydrocycloheptatrieno[*d*]imidazol-1(2*H*)-yl) propionic acid

Referring to the synthesis method of step 5 of intermediate **1**, 3-(2-carbonyl-3,3a-dihydrocycloheptatrieno[*d*]imidazol-1(2*H*)-yl) propionic acid **115d** (6.30 g) was obtained through methyl 3-(2-carbonyl-3,3a-dihydrocycloheptatrieno[*d*]imidazol-1(2*H*)-yl) propionate **115c** (7.20 g, 30.74 mmol), yield: 93.2%.
MS m/z (ESI): 221[M+1]

Step 4

1,3,4,5a-tetrahydro-1,2a-diazabenzo[*cd*]azulen-2,5-dione

3-(2-carbonyl-3,3a-dihydrocycloheptatrieno[*d*]imidazol-1(2*H*)-yl) propionic acid **115d** (6.20 g, 28.15 mmol) was dissolved in dichloromethane (70 mL), nitrogen replacement was performed three times, the system was cooled to 0°C, and thionyl chloride (6.70 g, 56.31 mmol) was added dropwise to the system, after the addition was completed, the mixture was warmed to 25°C and reacted for 2 h. The obtained product was concentrated, the residue was dissolved in methyl chloride (100 mL), aluminum trichloride (11.26 g, 84.45 mmol) was added, and the system was reacted at 40°C for 1 h. Sodium bicarbonate solution was added to quench the reaction, the obtained mixture was extracted with dichloromethane (200 mL×3), washed with saturated sodium chloride solution (100 mL), dried over anhydrous sodium sulfate, and filtered, the filtrate was concentrated, and the obtained residue was purified by silica gel column chromatography with eluent system C to obtain the title product 1,3,4,5a-tetrahydro-1,2a-diazabenzo[cd]azulen-2,5-dione **115e** (3.60 g), yield: 63.2%.
MS m/z (ESI): 203 [M+1]

Step 5

3,4,5,5a-tetrahydro-1,2a-diazabenzo[*cd*]azulen-2(1*H*)-one

1,3,4,5a-tetrahydro-1,2a-diazabenzo[cd]azulen-2,5-dione **115e** (3.50 g, 17.31 mmol) was dissolved in trifluoroacetic acid (40 mL), nitrogen replacement was performed three times, the system was cooled to 0°C, trisethylsilane (2.42 g, 20.77 mmol) was added dropwise to the system, the system was reacted at 0°C for 2 h. The obtained product was concentrated, water (30 mL) was added to the residue, the obtained mixture was extracted with ethyl acetate (80 mL×3), washed with sodium bicarbonate solution (50 mL×2) and saturated sodium chloride solution (50 mL) in sequence, dried over anhydrous sodium sulfated, and filtered, and the filtrate was concentrated. The obtained residue was purified by silica gel column chromatography with eluent system C to obtain the title product 3,4,5,5a-tetrahydro-1,2a-diazabenzo[*cd*]azulen-2(1*H*)-one **115f** (2.81 g), yield: 86.3%.
MS m/z (ESI):189 [M+1]

Step 6

1,2a-diazabenzo[*cd*]azulen-2(1*H*)-one

3,4,5,5a-tetrahydro-1,2a-diazabenzo[*cd*]azulen-2(1*H*)-one **115f** (2.50 g, 13.28 mmol) was dissolved in acetonitrile (40 mL), triethylamine (6.72 g, 66.41 mmol) was added, and the system was reacted at 70°C for 12 h. The obtained product was concentrated, water (20 mL) was added to the residue, the obtained mixture was extracted with ethyl acetate (60 mL×3), washed with saturated sodium chloride solution (50 mL×1), dried over anhydrous sodium sulfate, and filtered, the filtrate was concentrated, and the obtained residue was purified by silica gel column chromatography with eluent system C to obtain the title product 1,2a-diazabenzo[*cd*]azulen-2(1*H*)-one **115g** (1.80 g), yield: 73.6%.
MS m/z (ESI):185 [M+1]

Step 7 to step 13

1-(2-carbonyl-1,2-dihydro-1,2a-diazabenzo[*cd*]azulen-6-yl)-5-(trifluoromethyl)-*N*-(2-(trifluoromethyl)pyridin-4-yl)-1*H*-pyrazole-4-carboxamide

Referring to the synthesis method of step 1 to step 6 of intermediate **1** and step 11 of example **1** in sequence, 1-(2-carbonyl-1,2-dihydro-1,2a-diazabenzo[*cd*]azulen-6-yl)-5-(trifluoromethyl)-*N*-(2-(trifluoromethyl)pyridin-4-yl)-1*H*-pyrazole-4-carboxamide **115** (14 mg) was obtained through 1,2a-diazabenzo[*cd*]azulen-2(1*H*)-one **115g** (7.80 g, 42.35 mmol), yield: 0.07%.

MS m/z (ESI): 507 [M+1]
$^1$H NMR (400 MHz, DMSO): δ 10.21 (s, 1H), 9.15 (d, *J* = 5.6 Hz, 1H), 8.48 (d, *J* = 3.6 Hz, 1H), 8.16 (s, 1H), 7.65 (d, *J* = 7.0 Hz, 1H), 7.51 (s, 1H), 7.36 (s, 1H), 6.92-6.63 (m, 4H), 6.22 (s, 1H).

Example 116

1-(1-oxo-1,2-dihydro-2,9*a*-diazabenzo[*cd*]azulen-5-yl)-5-trifluoromethyl-*N*-(2-trifluoromethylpyridin-4-yl)-1*H*-pyrazole-4-carboxamide

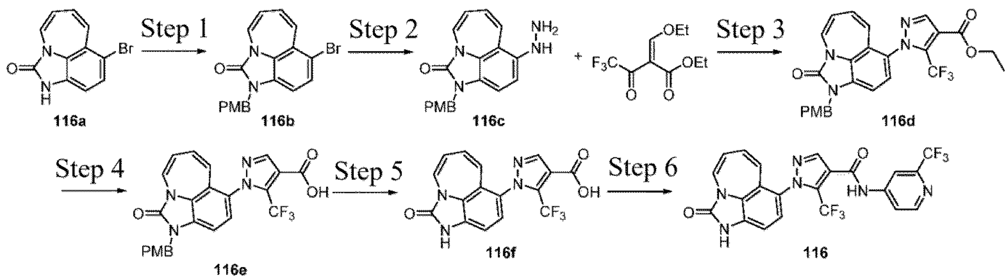


Referring to the synthesis method of step 5 in Example **2** and step 7 to step 11 in Example **1** in sequence, 1-(1-oxo-1,2-dihydro-2,9*a*-diazabenzo[*cd*]azulen-5-yl)-5-trifluoromethyl-*N*-(2-trifluoromethylpyridin-4-yl)-1*H*-pyrazole-4-carboxamide **116** (240 mg) was obtained through 5-bromo-2,9*a*-diazabenzo[*cd*]azulen-1-(2*H*)-one **116a** (2.63 g, 10.00 mmol, prepared by the well known method "Patent WO 2022081928A1"), yield: 4.7%.
MS m/z (ESI): 507 [M+1].

## Biological test and evaluation

The present invention will be further described and explained below in conjunction with test examples, but these examples are not meant to limit the scope of the present invention.

**Test example 1. Determination of the inhibitory effect of compounds of the present invention on MALT1 enzyme activity**

1.1 Experimental purpose:

Ac-LRSR-AMC was used as the substrate to test the $IC_{50}$ of the inhibitory activity of compounds on MALT1 enzyme activity at their Km concentration.

1.2 Experimental instruments and reagents:

Instruments:

| Instrument name | Manufacturer | Specification and model |
|---|---|---|
| Centrifuge | Eppendorf | 5810R |
| Microcentrifuge | DALB SCIENTIFIC CO., LTD. | D1008 |
| Microplate reader | Bio Tek | H1MFD |

Reagents:

| Reagent materials | Manufacturer | Item No. |
|---|---|---|
| Recombinant human MALT1/MLT protein | Abcam | ab271604 |
| Ac-LRSR-AMC | SM biochemicals | ab271604 |

1.3 Test method:

Ac-LRSR-AMC was used as the substrate which was digested by MALT1 to produce products, which produced fluorescence that could be detected at 460 nm. The highest concentration of the compound detected was 1000 nM, 3-fold dilution, a total of 11 concentrations (1000 nM - 0.017 nM). The reaction system is 10 nM FL MALTI protein, 200 mM Ac-LRSR-AMC, 50 mM Tris pH 7.5, 0.6M Citrate, 1mM DTT, 1mM EDTA, 0.05% BSA and 1.5% DMSO. The compound and the enzyme were pre-incubated at room temperature for 50 min. The substrate was then added and the reaction was carried out for 4 h.

1.4 Experimental data processing method:

1. Inhibition rate (%):

The original data (460nm reading) was calculated according to the following formula to obtain the inhibition rate.

Inhibition rate % = [(average value of positive control wells - value of sample wells)/(average value of positive control wells - average value of negative control wells)] × 100,

where the positive control wells are compound-free enzyme reaction wells, and the negative control wells are reaction wells without enzyme.

2. Curve fitting:

Log(inhibitor) vs. response -- Variable slope (four parameters) in GraphPad Prism 6 was used to perform fitting equation analysis on compound concentration and corresponding inhibition rate, the curve was fit and compound $IC_{50}$ value was obtained.

The fitting calculation equation is Y=Bottom +(Top-Bottom)/(1+10^((Log$IC_{50}$-X)*HillSlope))

1.5 Experimental conclusion:

**Table 1: $IC_{50}$ value of the compound's inhibitory activity on MALT1 enzyme activity**

| Example number | MALT1 $IC_{50}$ ($\mu M$) |
|---|---|
| **Example 3** | 0.105 |
| **Example 5** | 0.015 |
| **Example 13** | 0.107 |
| **Example 15** | 0.169 |
| **Example 18** | 0.024 |
| **Example 27** | 0.162 |
| **Example 30** | 0.141 |
| **Example 31** | 0.040 |
| **Example 32** | 0.123 |
| **Example 33** | 0.046 |
| **Example 34** | 0.032 |
| **Example 35** | 0.059 |
| **Example 36** | 0.053 |
| **Example 37** | 0.048 |
| **Example 44** | 0.080 |
| **Example 45** | 0.123 |
| **Example 46** | 0.093 |
| **Example 47** | 0.123 |
| **Example 48** | 0.140 |
| **Example 49** | 0.028 |
| **Example 50** | 0.039 |
| **Example 51** | 0.047 |
| **Example 52** | 0.104 |
| **Example 53** | 0.025 |
| **Example 55** | 0.171 |
| **Example 56** | 0.031 |
| **Example 60** | 0.016 |
| **Example 63** | 0.028 |
| **Example 66** | 0.193 |
| **Example 67** | 0.048 |
| **Example 68** | 0.051 |
| **Example 69** | 0.081 |
| **Example 73** | 0.191 |
| **Example 74** | 0.196 |
| **Example 75** | 0.024 |

(continued)

| Example number | MALT1 $IC_{50}$ ($\mu M$) |
|---|---|
| **Example 78** | 0.035 |
| **Example 83** | 0.050 |
| **Example 87** | 0.084 |
| **Example 89** | 0.106 |
| **JNJ-67856633** | 0.531 |

1.6 Experimental conclusion

The data in the table show that the compounds of the examples of the present invention have good inhibitory activity on MALT1 enzyme activity.

**Test example 2. Determination of the inhibitory effect of compounds of the present invention on the proliferation of TMD8 and OCI-Ly3 cells**

2.1 Experimental purpose:

The compound's inhibitory effect on the proliferation of TMD8 and OCI-Ly3 cells was evaluated through cell proliferation inhibition experiments.

2.2 Experimental instruments and reagents:

Instruments:

| Instrument name | Manufacturer | Specification and model |
|---|---|---|
| Full-function microplate reader | Bio Tek USA | H1MFD |
| Centrifuge | eppendorf | 5702R |
| Cell counter | Life | Countess II |
| $CO_2$ incubator | Thermo | 311 |
| Biosafety cabinet | Shanghai Boxun Industry Co., Ltd | BSC-1300IIA2 |

Reagents:

| Reagent materials | Manufacturer | Item No. |
|---|---|---|
| RPMI 1640 | GIBCO | 22400-089 |
| DMEM | GIBCO | 11995-065 |
| PBS | GIBCO | 10010-023 |
| FBS | GIBCO | 10099-141C |
| Trypsin (TE) | GIBCO | 25200-056 |
| CellTiter-Glo® Luminescent Cell Viability test kit (CTG) | Promega | G7573 |
| 96V bottom plate (Compoud plate) | Axygen | WIPP02280 |
| 96-well plate | Corning | 3610 |

2.3 Experimental method:

The compound's inhibitory effect on the proliferation of TMD8 and OCI-Ly3 cells was detected by the CellTiter-Glo method. The highest concentration of the compound detected was 1000 nM, 3-fold dilution, a total of 9 concentrations (1000 nM - 0.15 nM). After the cells were plated at an appropriate density, compounds were added the next day and incubated for 96 h before detection using the CellTiter-Glo Luminescebt detection kit.

2.4 Experimental data processing method:

1. Inhibition rate (%):

Inhibition rate % = [(average value of positive control wells - value of sample wells)/(average value of positive control wells - average value of negative control wells)] × 100,

where the positive control wells are compound-free wells and the negative control wells are PBS wells.

2. Curve fitting:

Log(inhibitor) vs. response -- Variable slope (four parameters) in GraphPad Prism 6 was used to perform fitting equation analysis on compound concentration and corresponding inhibition rate, the curve was fit and compound $IC_{50}$ value was obtained.

The fitting calculation equation is

$$Y=Bottom +(Top-Bottom)/(1+10^{\wedge}((LogIC_{50}-X)*HillSlope))$$

2.5 Experimental conclusion:

**Table 2: $IC_{50}$ value of the compound's inhibitory activity on the proliferation of OCI-Ly3 cells**

| Example number | Inhibition test of proliferation of OCI-Ly3 cells $IC_{50}$ (μM) |
|---|---|
| **Example 5** | 0.120 |
| **Example 13** | 0.272 |
| **Example 18** | 0.088 |
| **Example 27** | 0.804 |
| **Example 31** | 0.206 |
| **Example 32** | 0.266 |
| **Example 34** | 0.212 |
| **Example 35** | 0.263 |
| **Example 44** | 0.149 |
| **Example 46** | 0.222 |
| **Example 49** | 0.097 |
| **Example 50** | 0.219 |
| **Example 51** | 0.083 |
| **Example 53** | 0.101 |
| **Example 55** | 0.211 |
| **Example 56** | 0.152 |

(continued)

| Example number | Inhibition test of proliferation of OCI-Ly3 cells $IC_{50}$ (μM) |
|---|---|
| **Example 60** | 0.132 |
| **Example 63** | 0.154 |
| **Example 66** | 0.087 |
| **Example 68** | 0.271 |
| **Example 74** | 0.234 |
| **Example 75** | 0.213 |
| **Example 92** | 0.128 |

2.6 Experimental conclusion

The data in the table show that the compounds of the present invention have good inhibitory activity on proliferation of TMD8 and OCI-Ly3 cells.

**Test example 3. Intracellular MALT1 protease activity detection experiment**

3.1 Research purpose:

By detecting the digestion level of the substrate BCL-10 by MALT1 in TMD8 cells, the inhibitory effect of the compound on MALT1 protease activity in the cellular environment was tested.

3.2 Experimental instruments and reagents:

3.2.1 Instruments:

| Instrument name | Manufacturer | Specification and model |
|---|---|---|
| Full-function microplate reader | Bio Tek USA | H1MFD |
| Centrifuge | eppendorf | 5702R |
| Cell counter | Life | Countess II |
| $CO_2$ incubator | Thermo | 311 |
| Biosafety cabinet | Shanghai Boxun Industry Co., Ltd | BSC-1300IIA2 |

3.2.2 Reagents:

| Reagent materials | Manufacturer | Item No. |
|---|---|---|
| RPMI 1640 | GIBCO | 22400-089 |
| Penicillin-Streptomycin | Hyclone | SV30010 |
| PBS | GIBCO | 10010-023 |
| FBS | GIBCO | 10099-141C |
| Recombinant Bcl10 antibody | Abcam | Ab33905 |
| Bcl-10 antibody | Santacruz | Sc-5273HRP |

(continued)

| Reagent materials | Manufacturer | Item No. |
|---|---|---|
| Recombinant human Bcl10 protein | Abcam | ab124575 |
| eBioscience cell stimulation cocktail (500X) | Invitrogen | 00-4970-93 |
| PhosSTOP™ | Roche | 04906837001 |
| 96V bottom plate (PS) | Corning | 3894 |
| Dimethyl sulfoxide | Sigma | D2650 |
| cOmplete Tablets EDTA-free, EASYpack | Roche | 0469312001 |
| Cell Lysis Buffer (10X) | Cell Signaing | 9803 |
| 96-well assay plate | Corning | 3590 |
| Blocker BSA(10%) in PBS | Thermo | 37525 |
| 5×ELISA/ELISPOT diluent | Thermo | 00-4202-56 |
| ELISA wash buffer(20×) | CST | 9801P2 |
| TMB Substrate | CST | 7004P6 |
| STOP Solution | CST | 7002P6 |

3.3 Experimental method:

The ELISA sandwich method was used to detect the content of undigested BCL-10 protein in cells and determine the inhibitory effect of compounds on intracellular MALT1 protease activity. The highest concentration of the compound detected was 10000 nM, 3-fold dilution, a total of 9 concentrations (10000 nM - 1.5 nM). Cells were plated at an appropriate density. Compounds of corresponding concentrations were first added for action for 30 min, and then eBioscience cell stimulation cocktail was added to stimulate the cells for 2 h. Cell lysate was then collected. The cell lysate was added to the assay plate previously coated with recombinant BCL-10 antibody (Abcam, #Ab33905) and incubated at 4°C overnight. The next day, BCL-10 detection antibody (Santacruz, #Sc-5273HRP) was added and incubated for 2 h, TMB substrate was used for color development for about 30 min, then the color development was terminated, and a microplate reader was used for detection.

3.4 Experimental data processing method:

1) Inhibition rate (%):

Inhibition rate % = [(sample well value - average value of negative control wells)/(average value of positive control wells - average value of negative control wells)] × 100,

where the positive control well is a high-concentration reference compound well and the negative control well is DMSO well.

2) Curve fitting: Log(inhibitor) vs. response -- Variable slope (four parameters) in GraphPad Prism 6 was used to perform fitting equation analysis on compound concentration and corresponding inhibition rate, the curve was fit and compound $IC_{50}$ value was obtained.

The fitting calculation equation is

$$Y=Bottom+(Top-Bottom)/(1+10^{\wedge}((LogIC_{50}-X)*HillSlope))$$

3.5 Experimental conclusion:

**Table 3: $IC_{50}$ value of the compound's inhibitory activity on MALT1 protease activity in cells**

| Example number | Bcl10 TMD8 $IC_{50}$ ($\mu$M) |
|---|---|
| **Example 3** | 0.083 |
| **Example 5** | 0.036 |
| **Example 18** | 0.061 |
| **Example 27** | 0.051 |
| **Example 31** | 0.103 |
| **Example 46** | 0.080 |
| **Example 49** | 0.073 |
| **Example 53** | 0.056 |
| **Example 55** | 0.050 |
| **Example 56** | 0.093 |
| **Example 60** | 0.057 |
| **Example 63** | 0.058 |
| **Example 68** | 0.072 |
| **Example 75** | 0.083 |
| **Example 78** | 0.067 |
| **Example 88** | 0.072 |

3.6 Experimental conclusion

The data in the table show that the compounds of the examples of the present invention have good inhibitory activity on MALT1 protease activity.

**Test example 4. Pharmacokinetics determination of oral administration in Balb/C mice**

4.1 Research purpose:

Balb/C mice were used as test animals to study the pharmacokinetic behavior of the compound in the plasma of mice after oral administration at a dose of 30 mg/kg.

4.2 Test scheme

4.2.1 Test drugs:

the example compound of the present invention, made in house.

4.2.2 Test animals:

3 Balb/C mice (3/example), male, from Shanghai Jiesijie Experimental Animal Co., Ltd., animal production license number (SCXK (Shanghai) 2013-0006 N0.311620400001794).

4.2.3 Administration:

3 Balb/C mice, male; p.o. respectively at a dose of 30 mg/kg and an administration volume of 10 mL/kg after the mice were fasted overnight.

4.2.4 Sample collection:

Before and after administration of mice, 0.1 mL of blood was collected from the orbit at 0, 0.5, 1, 2,4, 6, 8 and 24 h, and placed in EDTA-$K_2$ test tubes, which was centrifuged at 6000 rpm at 4°C for 6 min to separate plasma which was stored at -80°C.

4.2.5 Sample processing:

1) 160 uL of acetonitrile was added to 40 uL of plasma sample for precipitation, and same was mixed and centrifuged at 3500 × g for 5 to 20 min.
2) 100 uL of the treated supernatant solution was taken and subjected to LC/MS/MS to analyze the concentrations of the test compounds.

4.2.6 Liquid phase analysis:

• Liquid phase conditions: Shimadzu LC-20AD pump
• Mass spectrometry conditions: AB Sciex API 4000 mass spectrometer
• Chromatographic column: Phenomenex Gemiu 5 um C18 50 × 4.6 mm
• Mobile phase: liquid A is 0.1% aqueous formic acid solution, liquid B is acetonitrile
• flow rate: 0.8 mL/min
• Elution time: 0-4.0 min, the eluent is as follows:

| Time/min | Liquid A | Liquid B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

4.3. Test results and analysis

The main pharmacokinetic parameters were calculated using WinNonlin 8.2. The results of mouse pharmacokinetic experiments are shown in the table below.

**Table 4: Pharmacokinetic parameters of oral administration to mice of the compounds of the present invention**

| Example number (30 mg/kg, P.O.) | Tmax (hr) | Cmax (ng/mL) | AUC 0-t (ng/mL*h) | $AUC_{0-\infty}$ (ng/mL*hr) | $T_{1/2}$ (hr) | MRT (hr) |
|---|---|---|---|---|---|---|
| **Example 3** | 4.0 | 23,733 | 330,088 | 341,390 | 4.7 | 7.4 |
| **Example 5** | 2.0 | 7,177 | 64,062 | 64,102 | 2.1 | 5.2 |
| **Example 13** | 4.0 | 4,380 | 62,663 | 67,797 | 6.2 | 9.4 |
| **Example 18** | 2.0 | 22,933 | 221,792 | 221,855 | 1.9 | 5.4 |
| **Example 27** | 4.0 | 71,267 | 1,130,746 | 1,584,433 | 13.1 | 18.7 |
| **Example 31** | 2.0 | 25,867 | 196,904 | 196,956 | 1.9 | 4.7 |
| **Example 49** | 2.0 | 15,267 | 104,907 | 104,960 | 2.1 | 4.3 |
| **Example 50** | 8.0 | 3,023 | 54,382 | NA | NA | NA |

(continued)

| Example number (30 mg/kg, P.O.) | Tmax (hr) | Cmax (ng/mL) | AUC 0-t (ng/mL*h) | $AUC_{0-\infty}$ (ng/mL*hr) | $T_{1/2}$ (hr) | MRT (hr) |
|---|---|---|---|---|---|---|
| **Example 53** | 8.0 | 14,067 | 256,222 | NA | NA | NA |
| **Example 55** | 4.0 | 28,267 | 400,925 | 471,673 | 8.6 | 12.2 |
| **Example 68** | 0.5 | 2,670 | 10,168 | 10,388 | 1.2 | 2.6 |
| **Example 75** | 2.0 | 1,293 | 12,178 | 12,239 | 3.0 | 5.5 |

5.4 Experimental conclusion

The data in the table show that the compounds of the present invention have good oral pharmacokinetic parameters in mice.

**Test example 6. Permeability test of the compound of the present invention**

6.1 Experimental purpose:

The purpose of this experiment is to test the bidirectional permeability of the compound through the Caco-2 cell model and evaluate whether the compound is transported by efflux transporters.

6.2 Experimental instruments and materials:

Liquid chromatography-mass spectrometer, centrifuge, vortexer, pipette, 96-well test plate (HTS Transwell Plate), acetonitrile solution containing internal standard, Caco-2 cells (ATCC), Hank's balance solution (HBSS), fluorescent yellow (LY), dimethyl sulfoxide (DMSO).

6.3 Experimental steps:

1) Culturing Caco-2 monolayer cells: Caco-2 cells in good condition were selected and plated, the medium was changed every 2 to 3 days, and the cells were cultured for 21 to 28 days to form a dense cell monolayer for testing.
2) Testing the permeability of the example compounds:

a. 108 μL of transport buffer (HBSS containing 10 μM of the example compound) was added to the administration end of A to B.
b. 300 μL of transport buffer (HBSS containing DMSO) was added to the receiving end of A to B.
c. 300 μL of transport buffer (HBSS containing 10 μM example compound) was added to the administration end of A to B.
d. 108 μL of transport buffer (HBSS containing DMSO) was added to the receiving end of A to B.
e. incubation was performed for 90 min.
d. a sample was taken, processed and detected with mass spectrometry.

6.4 Chromatographic conditions:

Instruments: Liquid Chromatography;

Chromatographic column: Waters XSelect HSS T3 2.5 μm (2.1 × 50mm);

Mobile phase: Phase A: 0.1% formic acid in pure water; Phase B: 0.1% formic acid in acetonitrile.

6.5 Mass spectrometry conditions:

Instruments: API6500+ liquid chromatography-mass spectrometer;

The ion source is electrospray ionization source (ESI);

The detection method is positive ion detection;

The scanning method is selected reaction monitoring (MRM).

6.6 Experimental results

**Table 6: Experimental test of permeability of the example compounds**

| Example number | $P_{app}$ ($10^{-6}$ cm·s$^{-1}$) | | $P_{app}$(B-A)/ $P_{app}$(A-B) |
|---|---|---|---|
| | A-B | B-A | |
| **Example 3** | 1.57 | 10.24 | 6.52 |
| **Example 5** | 1.00 | 7.03 | 7.09 |
| **Example 13** | 7.63 | 19.08 | 2.50 |
| **Example 27** | 4.21 | 11.52 | 2.74 |
| **Example 53** | 1.58 | 4.68 | 2.95 |
| **Example 55** | 2.09 | 4.08 | 1.97 |

6.7 Experimental conclusion

The data in the table show that the compounds of the embodiments of the present invention have good permeability and have significantly improved efflux properties.

**Test example 7. Tumor inhibition experiment of compounds of the present invention on OCI-LY3 cell subcutaneous xenograft tumor NOG mouse model of human diffuse large B-cell lymphoma**

7.1 Experimental purpose:

The efficacy of test drugs was evaluated in OCI-LY3 mouse subcutaneous tumor model of human diffuse large B-cell lymphoma

7.2 Experimental instruments and reagents:

7.2.1 Instruments:

1. Precision balance (SECURA225D-1CN, Sartorius)

2. Electronic balance (HZ2002A, Changzhou Keyuan Electronic Instrument Co., Ltd)

3. Biological safety cabinet (BSC1300L-II-A2, SHINVA)

4. Vernier caliper (CD-6"ASX(0-150)mm, Mitutoyo)

5. Biological safety cabinet (BSC-1604IIA2, AIRTECH)

6. Centrifuge (5910R, eppendorf)

7. $CO_2$ incubator (HERAcell-240i, Thermo)

7.2.2 Reagents:

1. Carboxymethylcellulose sodium (CMC-Na) (C5678, SIGMA)

2. Kolliphor HS 15 (polyethylene glycol 12 hydroxystearate) (42966, SIGMA)

3. RPMI1640 (22400-089, Gibco)

4. FBS (35-081-CV, Corning)

5. DPBS (21-031-CVC, Corning)

6. Matrigel Matrix (354234, Corning)

7. OCI-LY3 cells (ACC-761, DSMZ)

7.3 Experimental operations and data processing:

1. Animal: NOG mice, female, 6-8 weeks old, weighing 18-22 grams, a total of 65 mice were needed. Provided by Beijing Vital River Laboratory Animal Technology Co., Ltd. or other qualified suppliers.
2. Cell: Human diffuse large B-cell lymphoma OCI-LY3 cells were cultured in vitro as monolayers and conventional digestion treatment with trypsin-EDTA was carried out twice a week for passage. When the cell confluence was 80%-90% and the number reached the required number, cells were collected, counted, and inoculated.
3. Grouping for vaccination: Cells (5×10^6 cells+ Matrigel/0.2 ml) were inoculated subcutaneously into each mouse. The growth of animals and transplanted tumors were observed regularly. When the tumor grew to a volume of about 150-200 $mm^3$, the tumor volume of each mouse was calculated, and the mice were divided into random groups according to tumor volume and body weight. The calculation formula of tumor volume was $V = 0.5a \times b^2$, wherein a and b represented the long and short diameters of the tumor, respectively.
4. Administration: According to the grouping results, the test drug was started to be administered (administration method: oral administration; Administration volume: 10 mL/kg;
Administration frequency: 2 times/day; Administration cycle: 28 days; Vehicle: 10%solutol HS15-0.25%CMC-Na). Tumors were measured and weighed twice a week after starting administration of test drugs.
5. Animals were euthanized after the experiment.
6. Data processing: The tumor inhibitory efficacy of the compound was evaluated by TGI (%) or relative tumor proliferation rate T/C (%). TGI (%) reflects the tumor growth inhibition rate. Calculation of TGI (%): TGI (%) = [(1-(average tumor volume at the end of administration in a treatment group - average tumor volume at the beginning of administration in the treatment group))/ (average tumor volume at the end of administration in the solvent control group - average tumor volume at the beginning of administration in the solvent control group)] × 100%. Relative tumor proliferation rate T/C (%): The calculation formulas are as follows: T/C % = TRTV / CRTV × 100 % (TRTV: RTV of treatment group; CRTV: RTV of negative control group). The relative tumor volume (RTV) was calculated based on the results of tumor measurement. The calculation formula is $RTV = V_t/V_0$, where $V_0$ is the average tumor volume measured at grouping for administration (i.e. $d_0$), $V_t$ is the average tumor volume at a certain measurement, and TRTV and CRTV take the data on the same day.

After the experiment, the tumor weight was measured, and the T/Cweight percentage was calculated. Tweight and Cweight represented the tumor weight of the administration group and the vehicle control group, respectively.

7.4 Experimental results: **Table 7 Inhibitory experiment on tumor by the example compounds**

| Grouping (30 mg, BID × 4 weeks) | Weight | Tumor volume ($mm^3$, Mean ± SD) | ΔT/ΔC(%) | TGI (%) |
|---|---|---|---|---|
| | Day 28 | Day 28 | Day 28 | Day 28 |
| Example 18 | 21.03 ± 0.42 | 635 ± 46 (1274 ± 82) | 43.82 | 62.73 |
| Example 27 | 20.34 ± 0.78 | 230 ± 50 (1274 ± 82) | 19.83 | 93.04 |
| Example 53 | 20.06 ± 1.08 | 519 ± 89 (1274 ± 82) | 41.76 | 67.23 |
| Example 55 | 21.15 ± 0.72 | 287 ± 25 (1274 ± 82) | 22.76 | 87.99 |
| Remarks: The data in parentheses indicate the tumor volume corresponding to the vehicle group (i.e., the control group) at the corresponding time in this example. | | | | |

7.5 Experimental conclusion:

The experimental results in the table show that the example compounds of the present invention have good anti-tumor effects and low toxicity, and there is no significant change in the body weight of animals in each group.

## Claims

**1.** A compound represented by general formula (I), or a stereoisomer or pharmaceutically acceptable salt thereof:

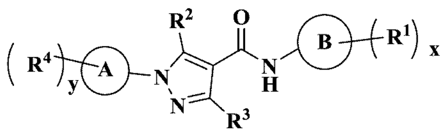

(I)

wherein:

ring A is selected from cycloalkyl, heterocyclyl, aryl or heteroaryl, wherein the cycloalkyl, heterocyclyl, aryl or heteroaryl can be optionally further substituted;
ring B is selected from cycloalkyl, heterocyclyl, aryl or heteroaryl, wherein the cycloalkyl, heterocyclyl, aryl or heteroaryl can be optionally further substituted;
$R^1$ is independently selected from hydrogen, deuterium, halogen, amino, hydroxyl, cyano, nitro, alkyl, alkenyl, alkynyl, deuterated alkyl, haloalkyl, alkoxy, haloalkoxy, hydroxyalkyl, cycloalkyl, cycloalkoxy, heterocyclyl, aryl, heteroaryl, $-CR_{cc}=CR_{dd}(CH_2)_nR_{aa}$, $-CR_{cc}=CR_{dd}(CH_2)_nNR_{aa}R_{bb}$, $-CR_{cc}=CR_{dd}(CH_2)_nNR_{ee}C(O)R_{aa}$, $-CR_{cc}=CR_{dd}(CH_2)_nNR_{ee}C(O)NR_{aa}R_{bb}$, $-O(CH_2)_nR_{cc}$, $-OC(R_{aa}R_{bb})_n(CH_2)_mR_{cc}$, $-NR_{ee}(CH_2)_nR_{cc}$, $-(CH_2)_n-$, $-(CH_2)_nR_{cc}$, $-(CH_2)_nOR_{cc}$, $-(CH_2)_nSR_{cc}$, $-(CH_2)_nC(O)R_{cc}$, $-(CH_2)_nC(=NR_{ee})R_{cc}$, $-(CH_2)_nC(O)OR_{cc}$, $-(CH_2)_nS(O)_mR_{cc}$, $-(CH_2)_nNR_{aa}R_{bb}$, $-(CH_2)_nC(O)NR_{aa}R_{bb}$, $-(CH_2)_nNR_{ee}C(O)R_{cc}$, $-(CH_2)_nN=S(O)R_{cc}R_{ee}$ or $-(CH_2)_nNR_{ee}S(O)_mR_{cc}$, wherein the alkyl, alkenyl, alkynyl, deuterated alkyl, haloalkyl, alkoxy, haloalkoxy, hydroxyalkyl, cycloalkyl, heterocyclyl, aryl and heteroaryl can be optionally further substituted;
preferably, $R^1$ is independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cycloalkyl, cycloalkoxy, heterocyclyl, aryl, heteroaryl, $-CR_{cc}=CR_{dd}(CH_2)_nR_{aa}$, $-CR_{cc}=CR_{dd}(CH_2)_nNR_{aa}R_{bb}$, $-CR_{cc}=CR_{dd}(CH_2)_nNR_{ee}C(O)R_{aa}$, $-CR_{cc}=CR_{dd}(CH_2)_nNR_{ee}C(O)NR_{aa}R_{bb}$, $-O(CH_2)_nR_{cc}$, $-OC(R_{aa}R_{bb})_n(CH_2)_mR_{cc}$, $-NR_{ee}(CH_2)_nR_{cc}$, $-(CH_2)_n-$, $-(CH_2)_nR_{cc}$, $-(CH_2)_nOR_{cc}$, $-(CH_2)_nSR_{cc}$, $-(CH_2)_nC(O)R_{cc}$, $-(CH_2)_nC(=NR_{ee})R_{cc}$, $-(CH_2)_nC(O)OR_{cc}$, $-(CH_2)_nS(O)_mR_{cc}$, $-(CH_2)_nNR_{aa}R_{bb}$, $-(CH_2)_nC(O)NR_{aa}R_{bb}$, $-(CH_2)_nNR_{ee}C(O)R_{cc}$ or $-(CH_2)_nNR_{ee}S(O)_mR_{cc}$, wherein the alkyl, alkenyl, alkynyl, deuteroalkyl, haloalkyl, alkoxy, haloalkoxy, hydroxyalkyl, cycloalkyl, heterocyclyl, aryl and heteroaryl can be optionally further substituted,
$R^2$ is selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cycloalkyl, cycloalkoxy, heterocyclyl, aryl or heteroaryl,

wherein the alkyl, alkenyl, alkynyl, deuteroalkyl, haloalkyl, alkoxy, haloalkoxy, hydroxyalkyl, cycloalkyl, heterocyclyl, aryl and heteroaryl can be optionally further substituted;

$R^3$ is selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cycloalkyl, cycloalkoxy, heterocyclyl, aryl or heteroaryl, wherein the alkyl, alkenyl, alkynyl, deuteroalkyl, haloalkyl, alkoxy, haloalkoxy, hydroxyalkyl, cycloalkyl, heterocyclyl, aryl and heteroaryl can be optionally further substituted;

$R^4$ is independently selected from hydrogen, deuterium, halogen, amino, hydroxyl, cyano, nitro, alkyl, alkenyl, alkynyl, oxo, thio, deuteroalkyl, haloalkyl, alkoxy, haloalkoxy, hydroxyalkyl, cyano-substituted alkyl, cycloalkyl, heterocyclyl, aryl, heteroaryl, $-CR_{33}=CR_{44}(CH_2)_pR_{11}$, $-CR_{33}=CR_{44}(CH_2)_pNR_{11}R_{22}$, $-CR_{33}=CR_{44}(CH_2)_pNR_{55}C(O)R_{11}$, $-CR_{33}=CR_{44}(CH_2)_pNR_{55}C(O)NR_{11}R_{22}$, $-O(CH_2)_pR_{33}$, $-OC(R_{11}R_{22})_q(CH_2)_pR_{33}$, $-NR_{55}(CH_2)_pR_{33}$, $-(CH_2)_p-$, $-(CH_2)_pR_{33}$, $-(CH_2)_pOR_{33}$, $-(CH_2)_pSR_{33}$, $-(CH_2)_pC(O)R_{33}$, $-(CH_2)_pC(=NR_{55})R_{33}$, $-(CH_2)_pC(O)OR_{33}$, $-(CH_2)_pS(O)_qR_{33}$, $-(CH_2)_pNR_{11}R_{22}$, $-(CH_2)_pC(O)NR_{11}R_{22}$, $-(CH_2)_pNR_{55}C(O)R_{33}$ or $-(CH_2)_pNR_{55}S(O)_qR_{33}$, wherein the alkyl, alkenyl, alkynyl, deuteroalkyl, haloalkyl, alkoxy, haloalkoxy, hydroxyalkyl, cyano-substituted alkyl, cycloalkyl, heterocyclyl, aryl and heteroaryl can be optionally further substituted,

alternatively, any two adjacent or non-adjacent $R^4$ and adjacent atoms are connected to form cycloalkyl, heterocyclyl, aryl or heteroaryl, wherein the cycloalkyl, heterocyclyl, aryl or heteroaryl can be optionally further substituted;

$R_{aa}$ and $R_{bb}$ are each independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl and heteroaryl can be optionally further substituted,

alternatively, $R_{aa}$ and $R_{bb}$ together with adjacent atoms form cycloalkyl, heterocyclyl, aryl or heteroaryl, wherein the cycloalkyl, heterocyclyl, aryl and heteroaryl can be optionally further substituted;

$R_{cc}$, $R_{dd}$ and $R_{ee}$ are each independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl and heteroaryl can be optionally further substituted;

$R_{11}$ and $R_{22}$ are each independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl and heteroaryl optionally can be further substituted,

alternatively, $R_{11}$ and $R_{22}$ together with adjacent atoms form cycloalkyl, heterocyclyl, aryl or heteroaryl, wherein the cycloalkyl, heterocyclyl, aryl and heteroaryl optionally can be further substituted;

$R_{33}$, $R_{44}$ and $R_{55}$ are each independently selected from hydrogen, deuterium, halogen, amino, hydroxyl, cyano, nitro, alkyl, alkenyl, alkynyl, deuteroalkyl, haloalkyl, alkoxy, haloalkoxy, hydroxyalkyl, cyano-substituted alkyl, cycloalkyl, heterocyclyl, aryl or heteroaryl, wherein the amino, alkyl, alkenyl, alkynyl, deuteroalkyl, haloalkyl, alkoxy, haloalkoxy, hydroxyalkyl, cyano-substituted alkyl, cycloalkyl, heterocyclyl, aryl and heteroaryl optionally can be further substituted;

m is 0, 1, 2 or 3;
n is 0, 1, 2 or 3;
p is 0, 1, 2 or 3;
q is 0, 1, 2 or 3;
x is 0, 1, 2, 3 or 4; and
y is an integer from 0 to 6.

**2.** The compound or the stereoisomer or pharmaceutically acceptable salt thereof of claim 1,

wherein, ring A is

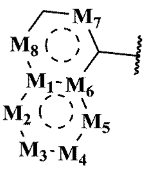

,

wherein, $M_1$ is selected from -N- or -C-, $M_2$ is selected from -C(O)-, -C(S)-, -C(NH)-, -NH-, -N-, - $CH_2$-, -CH-, -O- or -S-, and $M_3$ is selected from -N-, -NH-, -CH-, -$CH_2$-, -C(O)-, -C(S)- or -C(NH)-; $M_4$ is selected from -CH-, -$CH_2$- or -N-, $M_6$ is selected from -N-, -CH- or -CH-, $M_5$ or $M_7$ is -O-, -N-, -CH-, -CH=CH-, -$CH_2$- or absent, and $M_8$ is selected from -N-, -NH-, -O-, -S- or -CH-,
preferably, $M_1$ is selected from -N- or -C-, $M_2$ is selected from -C(O)-, -C(S)-, -C(NH)-, -NH-, -N-, - $CH_2$-, -CH-, -O- or -S-, and $M_3$ is selected from -N-, -NH-, -CH-, -C(O)-, -C(S)- or -C(NH)-; $M_4$ is selected from -CH-, -$CH_2$- or -N-, $M_6$ is selected from -N- or -CH-, $M_5$ or $M_7$ is -CH-, -$CH_2$- or absent, and $M_8$ is selected from -NH-, -O-, -S- or -CH-;
optionally, ring A is further substituted with 1 to 3 $R^4$, and any two $R^4$ and adjacent atoms form $C_{3-8}$ cycloalkyl, 3- to 8-membered heterocyclyl containing 1 to 2 atom(s) selected from oxygen, sulfur or nitrogen, $C_{6-10}$ aryl or 3- to 8-membered heteroaryl containing 1 to 2 atom(s) selected from oxygen, sulfur or nitrogen,
preferably, ring A is further substituted with 1 to 3 $R^4$, and any two $R^4$ and adjacent atoms form $C_{3-8}$ cycloalkyl, 3- to 8-membered heterocyclyl containing 1 to 2 atom(s) selected from oxygen or nitrogen, $C_{6-10}$ aryl or 3- to 8-membered heteroaryl containing 1 to 2 atom(s) selected from oxygen or nitrogen;
preferably,
ring A is

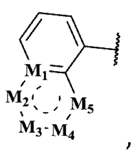

,

$M_1$ is selected from -N- or -C-, $M_2$ is selected from -C(O)-, -C(S)-, -C(NH)-, - NH-, -N-, -$CH_2$-, -CH-, - O- or -S-, $M_3$ is selected from -N-, -NH-, -CH -, -$CH_2$-, -C(O)-, -C(S)- or -C(NH)-; $M_4$ is selected from -CH-, -$CH_2$- or -N-, and $M_5$ is -O-, -N-, -CH-, -$CH_2$- or absent,
preferably, $M_1$ is selected from -N- or -C-, $M_2$ is selected from -C(O)-, -C(S)-, -C(NH)-, -NH-, -N-, - $CH_2$-, -CH-, -O- or -S-, and $M_3$ is selected from -N-, -NH-, -CH-, -C(O)-, -C(S)- or -C(NH)-; $M_4$ is selected from -CH-, -$CH_2$- or -N-, and $M_5$ is -CH-, -$CH_2$- or absent,
alternatively, ring A is

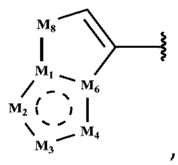

,

$M_1$ is selected from -N- or -C-, $M_2$ is selected from -C(O)-, -C(S)-, -C(NH)-, - NH-, -N- or -CH-, $M_3$ is selected from -CH- or -N-, $M_4$ is selected from -NH- or -CH-, $M_6$ is selected from -NH- or -CH-, and $M_8$ is selected from -S- or -CH-,
optionally, ring A is further substituted with 1 to 2 $R^4$, and any two $R^4$ and adjacent atoms form $C_{3-6}$ cycloalkyl or 3- to 6- membered heterocyclyl containing 1 to 2 members selected from oxygen or nitrogen atoms;
more preferably,
ring A is

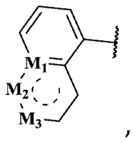

,

$M_1$ is selected from -N- or -C-, $M_2$ is selected from -C(O)-, -C(S)-, -C(NH)-, - NH-, -N-, $-CH_2-$, -CH-, - O- or -S-, $M_3$ is selected from -N-, -NH-, -CH -, -C(O)-, -C(S)- or -C(NH)-;

optionally, ring A is further substituted with 1 to 2 $R^4$, and any two $R^4$ and adjacent atoms form $C_{3-6}$ cycloalkyl or 3- to 6-membered heterocyclyl containing 1 to 2 members selected from oxygen or nitrogen atoms.

**3.** The compound, or the stereoisomer or pharmaceutically acceptable salt thereof of claim 2, wherein, ring A is selected from the following groups:

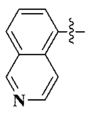, 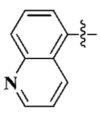, 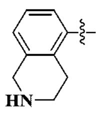, 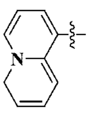, 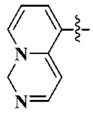, 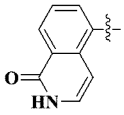, 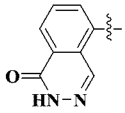, 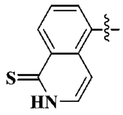, 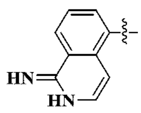,

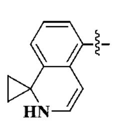, 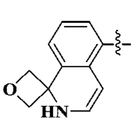, 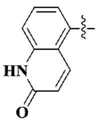, 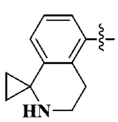, 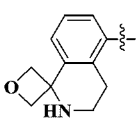, 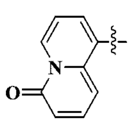, 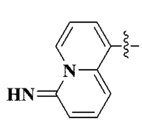, 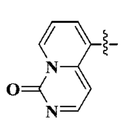,

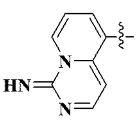, 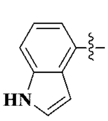, 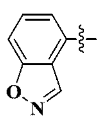, 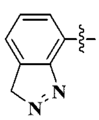, 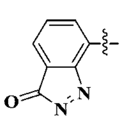, 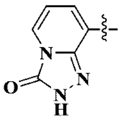, 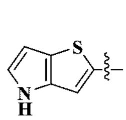, 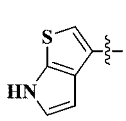,

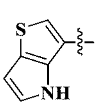, 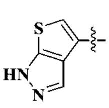, 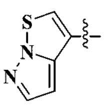, 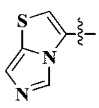, 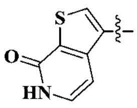, 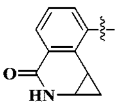, 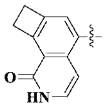, 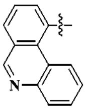, 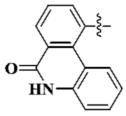,

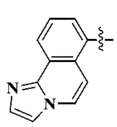, 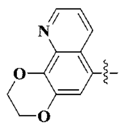, 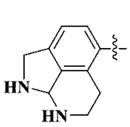, 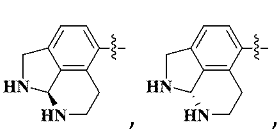, 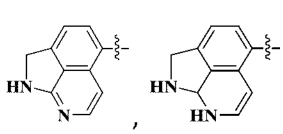

or

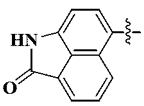.

**4.** The compound, or the stereoisomer or pharmaceutically acceptable salt thereof of any one of claims 1 to 3, wherein, $R^2$ is selected from hydrogen, deuterium, halogen, amino, hydroxyl, cyano, nitro, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12- membered heterocyclyl, $C_{6-12}$ aryl or 5- to 12-membered heteroaryl, wherein the amino, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-6}$ alkyl, $C_{2-6}$ alkenyl, $C_{2-6}$ alkynyl, oxo, thio, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$alkyl, $C_{3-12}$ cycloalkyl, 3- to 12- membered heterocyclyl, $C_{6-12}$ aryl and 5- to 12-membered heteroaryl;

preferably, selected from hydrogen, deuterium, halogen, $C_{1-3}$ alkyl, $C_{1-3}$ deuteroalkyl, $C_{1-3}$ haloalkyl, $C_{1-3}$ alkoxy, $C_{1-3}$ hydroxyalkyl, 3- to 12- membered heterocyclyl or 5- to 12-membered heteroaryl, wherein the $C_{1-3}$ alkyl, $C_{1-3}$ alkoxy, $C_{1-3}$ hydroxyalkyl, 3- to 12- membered heterocyclyl or 5- to 12-membered heteroaryl, which are optionally further substituted with one or more substituents selected from deuterium, halogen, amino, nitro,

hydroxyl, cyano, oxo, $C_{1-3}$ alkyl, $C_{1-3}$ deuteroalkyl, $C_{1-3}$ haloalkyl, $C_{1-3}$ alkoxy, $C_{1-3}$ hydroxyalkyl, $C_{1-3}$ haloalkoxy, $C_{2-6}$ alkenyl, $C_{2-6}$ alkynyl, $C_{3-8}$ cycloalkyl, 3- to 12- membered heterocyclyl, $C_{6-12}$ aryl and 5- to 12-membered heteroaryl;

and further preferably, hydrogen, deuterium, fluorine, chlorine, bromine, methyl, ethyl, trifluoromethyl or cyclopropyl.

**5.** The compound, or the stereoisomer or pharmaceutically acceptable salt thereof according to any one of claims 1 to 4, wherein, $R^3$ is selected from hydrogen, deuterium, halogen, amino, hydroxyl, cyano, nitro, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$alkyl, $C_{3-12}$ cycloalkyl, 3- to 12-membered heterocyclyl, $C_{6-12}$ aryl or 5- to 12-membered heteroaryl, wherein the amino, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-6}$ alkyl, $C_{2-6}$ alkenyl, $C_{2-6}$ alkynyl, oxo, thio, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12- membered heterocyclyl, $C_{6-12}$ aryl and 5- to 12-membered heteroaryl,

preferably, selected from hydrogen, deuterium, halogen, $C_{1-3}$ alkyl, $C_{1-3}$ deuteroalkyl, $C_{1-3}$ haloalkyl, $C_{1-3}$ alkoxy, $C_{1-3}$ hydroxyalkyl, 3- to 12- membered heterocyclyl or 5- to 12-membered heteroaryl, wherein the $C_{1-3}$ alkyl, $C_{1-3}$ alkoxy, $C_{1-3}$ hydroxyalkyl, 3- to 12- membered heterocyclyl or 5- to 12-membered heteroaryl, which are optionally further substituted with one or more substituents selected from deuterium, halogen, amino, nitro, hydroxyl, cyano, oxo, $C_{1-3}$ alkyl, $C_{1-3}$ deuteroalkyl, $C_{1-3}$ haloalkyl, $C_{1-3}$ alkoxy, $C_{1-3}$ hydroxyalkyl, $C_{1-3}$ haloalkoxy, $C_{2-6}$ alkenyl, $C_{2-6}$ alkynyl, $C_{3-8}$ cycloalkyl, 3- to 12- membered heterocyclyl, $C_{6-12}$ aryl and 5- to 12-membered heteroaryl;

and further preferably, hydrogen, deuterium, fluorine, chlorine, bromine, methyl, ethyl or trifluoromethyl.

**6.** The compound, or the stereoisomer or pharmaceutically acceptable salt thereof of any one of claims 1 to 5, wherein, the compound is further as represented by general formula (I-A):

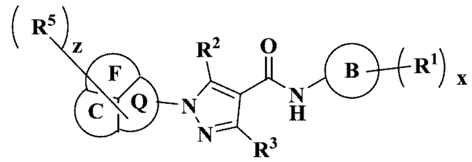

( I-A )

wherein:

ring B is selected from $C_{3-12}$ cycloalkyl, 3- to 14-membered heterocyclyl, $C_{6-14}$ aryl or 5- to 14-membered heteroaryl;

preferably, ring B is selected from $C_{6-12}$ aryl or 5- to 14- membered heteroaryl containing 1 to 3 nitrogen atoms, oxygen atoms and/or sulfur atoms,

more preferably, ring B is selected from phenyl, 5- to 7-membered nitrogen-containing heteroaryl, benzo 5- to 7-membered nitrogen-containing heteroaryl, 5- to 7-membered nitrogen-containing heteroaryl fused phenyl, 5- to 7-membered heteroaryl fused 5- to 7-membered heteroaryl or tricyclic heteroaryl,

more preferably, ring B is selected from phenyl, pyridyl or pyridazinyl;

ring C is selected from $C_{3-6}$ cycloalkyl, 3- to 8-membered heterocyclyl or 5- to 8-membered heteroaryl,

preferably $C_{5-6}$ cycloalkyl or 5- to 6-membered heterocyclyl,

more preferably, cyclopentyl, cyclohexyl, 5-membered heterocyclyl containing 1 to 3 nitrogen atoms, oxygen atoms and/or sulfur atoms, or 6-membered heterocyclyl containing 1 to 3 nitrogen atoms, oxygen atoms and/or sulfur atoms;

ring F is selected from $C_{3-6}$ cycloalkyl, 3- to 8-membered heterocyclyl, $C_{6-12}$ aryl or 5- to 12-membered heteroaryl, preferably $C_{5-6}$ cycloalkyl, 5- to 6-membered heterocyclyl, $C_{6-8}$aryl or 5- to 8-membered heteroaryl;

ring Q is selected from $C_{3-6}$ cycloalkyl, 3- to 8-membered heterocyclyl, $C_{6-12}$ aryl or 5- to 12-membered heteroaryl, preferably $C_{5-6}$ cycloalkyl, 5- to 6-membered heterocyclyl, $C_{6-8}$aryl or 5- to 8-membered heteroaryl;

$R^1$ is independently selected from hydrogen, deuterium, halogen, amino, hydroxyl, cyano, nitro,

$C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$alkyl, $C_{3-12}$ cycloalkyl, 3- to 12- membered heterocyclyl, $C_{6-12}$ aryl, 5- to 12-membered heteroaryl, $-CR_{cc}=CR_{dd}(CH_2)_nR_{aa}$, $-CR_{cc}=CR_{dd}(CH_2)_nNR_{aa}R_{bb}$, $-CR_{cc}=CR_{dd}(CH_2)_nNR_{ee}C(O)R_{aa}$, $-CR_{cc}=CR_{dd}(CH_2)_nNR_{ee}C(O)NR_{aa}R_{bb}$, $-O(CH_2)_nR_{cc}$, $-OC(R_{aa}R_{bb})_n(CH_2)_mR_{cc}$, $-NR_{ee}(CH_2)_nR_{cc}$, $-(CH_2)_n-$, $-(CH_2)_nR_{cc}$, $-(CH_2)_nOR_{cc}$, $-(CH_2)_nSR_{cc}$, $-(CH_2)_nC(O)R_{cc}$, $-(CH_2)_nC(=NR_{ee})R_{cc}$, $-(CH_2)_nC(O)OR_{cc}$, $-(CH_2)_nS(O)_mR_{cc}$, $-(CH_2)_nNR_{aa}R_{bb}$, $-(CH_2)_nC(O)NR_{aa}R_{bb}$, $-(CH_2)_nNR_{ee}C(O)R_{cc}$, $-(CH_2)_nN=S(O)R_{cc}R_{ee}$ or $-(CH_2)_nNR_{ee}S(O)_mR_{cc}$, wherein the amino, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$alkyl, $C_{3-12}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-6}$ alkyl, $C_{2-6}$ alkenyl, $C_{2-6}$ alkynyl, oxo, thio, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$alkyl, $C_{3-12}$ cycloalkyl, 3- to 12- membered heterocyclyl, $C_{6-12}$ aryl and 5- to 12-membered heteroaryl;

$R^2$ is selected from hydrogen, deuterium, halogen, amino, hydroxyl, cyano, nitro, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, $C_{3-8}$ cycloalkyl, 3- to 14-membered heterocyclyl, $C_{6-14}$ aryl or 5- to 14-membered heteroaryl;

$R^3$ is selected from hydrogen, deuterium, halogen, amino, hydroxyl, cyano, nitro, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, $C_{3-8}$ cycloalkyl, 3- to 14-membered heterocyclyl, $C_{6-14}$ aryl or 5- to 14-membered heteroaryl;

$R^5$ is selected from hydrogen, deuterium, halogen, amino, hydroxyl, cyano, nitro, $C_{1-6}$ alkyl, $C_{2-6}$ alkenyl, $C_{2-6}$ alkynyl, oxo, thio, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-8}$ cycloalkyl, 3- to 14-membered heterocyclyl, $C_{6-14}$ aryl, 5- to 14-membered heteroaryl, $-(CH_2)_{n2}C(O)R_a$, $-(CH_2)_{n2}C(O)OR_a$, $-(CH_2)_{n2}OR_a$, $-(CH_2)_{n2}R_a$ or $-(CH_2)_{n2}S(O)_{m2}R_a$, wherein the amino, $C_{1-6}$alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$alkyl, $C_{3-8}$ cycloalkyl, 3- to 14-membered heterocyclyl, $C_{6-14}$ aryl and 5- to 14-membered heteroaryl are optionally substituted with one or more substituents selected from deuterium, halogen, amino, hydroxyl, cyano, nitro, $C_{1-6}$ alkyl, $C_{2-6}$ alkenyl, $C_{2-6}$ alkynyl, oxo, thio, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$alkyl, $C_{3-8}$ cycloalkyl, 3- to 14-membered heterocyclyl, $C_{6-14}$ aryl and 5- to 14-membered heteroaryl,

preferably, $R^5$ is selected from hydrogen, deuterium, halogen, amino, hydroxyl, cyano, nitro, $C_{1-3}$ alkyl, $C_{2-4}$ alkenyl, $C_{2-4}$ alkynyl, oxo, thio, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl, 5- to 12-membered heteroaryl, $-(CH_2)_{n2}C(O)R_a$, $-(CH_2)_{n2}C(O)OR_a$, $-(CH_2)_{n2}OR_a$, $-(CH_2)_{n2}R_a$ or $-(CH_2)_{n2}S(O)_{m2}R_a$, wherein the amino, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-3}$ alkyl, $C_{2-4}$ alkenyl, $C_{2-4}$ alkynyl, oxo, thio, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl and 5- to 12-membered heteroaryl;

$R_a$ is selected from hydrogen, deuterium, halogen, amino, hydroxyl, cyano, nitro, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl or 5- to 12-membered heteroaryl, wherein the amino, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-3}$ alkyl, $C_{2-4}$ alkenyl, $C_{2-4}$ alkynyl, oxo, thio, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl and 5- to 12-membered heteroaryl;

$R_{aa}$ and $R_{bb}$ are each independently selected from hydrogen, deuterium, halogen, amino, hydroxyl, cyano, nitro, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12-membered heterocyclyl, $C_{6-12}$ aryl or 5- to 12-membered heteroaryl, which are optionally substituted with one or more substituents selected from deuterium, halogen, amino, hydroxyl, cyano, nitro, $C_{1-6}$ alkyl, $C_{3-12}$ cycloalkyl, $-O(CH_2)_{n1}R_{c1}$, $-OC(R_{a1}R_{b1})_{m1}(CH_2)_{n1}R_{c1}$, $-NR_{a1}(CH_2)_{n1}R_{c1}$, $-(CH_2)_m-$, $-(CH_2)_{n1}R_{c1}$, $-(CH_2)_{n1}OR_{c1}$, $-(CH_2)_{n1}SR_{c1}$, $-(CH_2)_{n1}C(O)R_{c1}$, $-(CH_2)_{n1}C(O)OR_{c1}$, $-(CH_2)_{n1}S(O)_{m1}R_{c1}$, $N-(CH_2)_{n1}NR_{a1}R_{b1}$, $-(CH_2)_{n1}C(O)NR_{a1}R_{b1}$, $-(CH_2)_{n1}NR_{a1}C(O)R_{c1}$ and $-(CH_2)_{n1}NR_{a1}S(O)_{m1}R_{c1}$,

alternatively, $R_{aa}$ and $R_{bb}$ together with adjacent atoms form $C_{3-12}$ cycloalkyl, 3- to 12- membered heterocyclyl, $C_{6-12}$ aryl or 5- to 12-membered heteroaryl, wherein the $C_{3-12}$ cycloalkyl, 3- to 12-membered heterocyclyl, $C_{6-12}$

aryl and 5- to 12-membered heteroaryl are optionally substituted with one or more substituents selected from deuterium, halogen, amino, hydroxyl, cyano, nitro, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12- membered heterocyclyl, $C_{6-12}$ aryl, 5- to 12-membered heteroaryl, $-O(CH_2)_{n1}R_{c1}$, $-OC(R_{a1}R_{b1})_{n1}(CH_2)_{m1}R_{c1}$, $-NR_{a1}(CH_2)_{n1}R_{c1}$, - $(CH_2)_{n1}$-, $-(CH_2)_{n1}R_{c1}$, $-(CH_2)_{n1}OR_{c1}$, $-(CH2)_{n1}SR_{c1}$, $-(CH_2)_{n1}C(O)R_{c1}$, $-(CH_2)_{n1}C(O)OR_{c1}$, $-(CH_2)_{n1}S(O)_mR_{c1}$, $N-(CH_2)_{n1}NR_{a1}R_{b1}$, $-(CH_2)_{n1}C(O)NR_{a1}R_{b1}$, $-(CH_2)_{n1}NR_{a1}C(O)R_{c1}$ and $-(CH_2)_{n1}NR_{a1}S(O)_{m1}R_{c1}$;

$R_{cc}$ is selected from hydrogen, deuterium, halogen, amino, hydroxyl, cyano, nitro, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12- membered heterocyclyl, $C_{6-12}$ aryl or 5- to 12-membered heteroaryl, which are optionally substituted with one or more substituents selected from deuterium, halogen, amino, hydroxyl, cyano, nitro, $C_{1-6}$ alkyl, $C_{3-12}$ cycloalkyl, - $O(CH_2)_{n1}R_{c1}$, $-OC(R_{a1}R_{b1})_{n1}(CH_2)_mR_{c1}$, $-NR_{a1}(CH_2)_{n1}R_{c1}$, $-(CH_2)_{n1}$-, $-(CH_2)_{n1}R_{c1}$, $-(CH_2)_{n1}OR_{c1}$, - $(CH_2)_{n1}SR_{c1}$, $-(CH_2)_{n1}C(O)R_{c1}$, $-(CH_2)_{n1}C(O)OR_{c1}$, $-(CH_2)_{n1}S(O)_mR_{c1}$, $N-(CH_2)_{n1}NR_{a1}R_{b1}$, - $(CH_2)_{n1}C(O)NR_{a1}R_{b1}$, $-(CH_2)_{n1}NR_{a1}C(O)R_{c1}$ and $-(CH_2)_{n1}NR_{a1}S(O)_{m1}R_{c1}$;

$R_{a1}$, $R_{b1}$ and $R_{c1}$ are each independently selected from hydrogen, deuterium, halogen, amino, hydroxyl, cyano, nitro, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12-membered heterocyclyl, $C_{6-12}$ aryl or 5- to 12-membered heteroaryl, which are optionally substituted with one or more substituents selected from deuterium, halogen, amino, hydroxyl, cyano, nitro, $C_{1-6}$ alkyl and $C_{3-12}$ cycloalkyl;

m is 0, 1, 2 or 3;

m1 is 0, 1, 2 or 3;

m2 is selected from 1 or 2;

n is 0, 1, 2 or 3;

n1 is 0, 1, 2 or 3;

n2 is selected from 0, 1, 2 or 3;

x is selected from 0, 1, 2, 3 or 4; and

z is selected from 0, 1, 2 or 3.

**7.** The compound, or the stereoisomer or pharmaceutically acceptable salt thereof of any one of claims 1 to 6, wherein, the compound is further as represented by general formula (III):

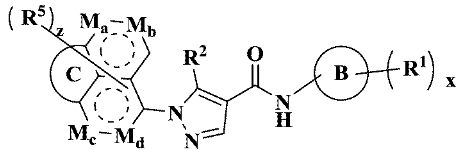

( III )

wherein:

$M_a$, $M_b$, $M_c$ or $M_d$ are each independently selected from -N-, -NH- or -CH-;

ring C is selected from phenyl, $C_{3-6}$ cycloalkyl, 5- to 7-membered heterocyclyl or 5- to 7-membered heteroaryl; preferably, 5-membered heterocyclyl containing 1 to 3 nitrogen atoms and/or 1 to 2 oxygen atoms, 6-membered heterocyclyl containing 1 to 3 nitrogen atoms and/or 1 to 2 oxygen atoms, 5-membered heteroaryl containing 1 to 3 nitrogen atoms or 6-membered heteroaryl containing 1 to 3 nitrogen atoms;

$R^2$ is selected from hydrogen, deuterium, halogen, amino, hydroxyl, cyano, nitro, $C_{1-3}$ alkyl, $C_{2-6}$ alkenyl, $C_{2-6}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, $C_{3-6}$ cycloalkyl, 3- to 12- membered heterocyclyl, $C_{6-12}$ aryl or 5- to 12-membered heteroaryl;

$R^5$ is selected from hydrogen, deuterium, halogen, amino, hydroxyl, cyano, nitro, $C_{1-3}$ alkyl, $C_{2-6}$ alkenyl, $C_{2-6}$ alkynyl, oxo, thio, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl or 5- to 12-membered heteroaryl, wherein the amino, $C_{1-3}$ alkyl, $C_{2-6}$ alkenyl, $C_{2-6}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$alkyl, $C_{3-6}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-3}$ alkyl, $C_{2-6}$ alkenyl, $C_{2-6}$ alkynyl, oxo, thio, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl and 5- to 12-membered heteroaryl;

$R^5$ is preferably selected from hydrogen, deuterium, fluorine, chlorine, bromine, amino, hydroxyl, cyano, nitro, methyl, ethyl, propyl, isopropyl, oxo, methoxy, ethoxy, difluoromethyl, trifluoromethyl, difluoromethoxy, trifluoromethoxy, cyclopropyl or cyclobutyl;
z is selected from 0, 1, 2 or 3.

**8.** The compound, or the stereoisomer or pharmaceutically acceptable salt thereof of any one of claims 1 to 7, wherein, ring B is selected from $C_{3-12}$ cycloalkyl, 3- to 14-membered heterocyclyl, $C_{6-14}$ aryl or 5- to 14-membered heteroaryl;

preferably, ring B is selected from $C_{6-12}$ aryl or 5- to 14-membered heteroaryl containing 1 to 3 nitrogen atoms, 1 oxygen atom and/or 1 sulfur atom;
more preferably, ring B is selected from phenyl, 5- to 7-membered nitrogen-containing heteroaryl, benzo 5- to 7-membered nitrogen-containing heteroaryl, 5- to 7-membered nitrogen-containing heteroaryl fused phenyl, 5- to 7-membered heteroaryl fused 5- to 7-membered heteroaryl or tricyclic heteroaryl;
more preferably, ring B is selected from phenyl, pyridyl or pyridazinyl.

**9.** The compound, or the stereoisomer or pharmaceutically acceptable salt thereof of any one of claims 1 to 8, wherein, the compound is further as represented by general formula (IV) or (V):

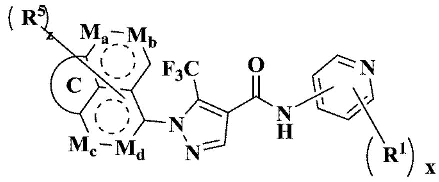

(IV)

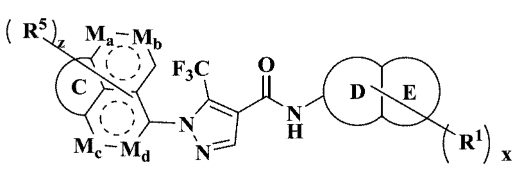

(V)

ring D is selected from phenyl, 5-membered heteroaryl or 6-membered heteroaryl;
preferably, phenyl, 5-membered heteroaryl containing 1 to 3 nitrogen atoms or 6-membered heteroaryl containing 1 to 3 nitrogen atoms;
more preferably, phenyl, pyridyl, pyrimidinyl or pyridazinyl;
ring E is selected from phenyl, $C_{3-6}$ cycloalkyl, 5- to 7-membered heterocyclyl or 5- to 7-membered heteroaryl;
preferably, phenyl, $C_{5-6}$ cycloalkyl, 5-membered heterocyclyl containing 1 to 3 nitrogen atoms and/or 1 to 2 oxygen atoms, 6-membered heterocyclyl containing 1 to 3 nitrogen atoms and/or 1 to 2 oxygen atoms, 5-membered heteroaryl containing 1 to 3 nitrogen atoms or 6-membered heteroaryl containing 1 to 3 nitrogen atoms;
and more preferably, phenyl, cyclopentyl, cyclohexyl, pyrrolyl, pyrazolyl, imidazolyl, triazolyl, pyridyl, pyrimidinyl, pyridazinyl or pyrazinyl.

**10.** The compound, or the stereoisomer or pharmaceutically acceptable salt thereof of any one of claims 1 to 9, wherein, the compound is further as represented by general formula (IV-A) or (V-A):

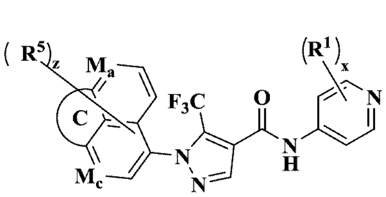

(IV-A)

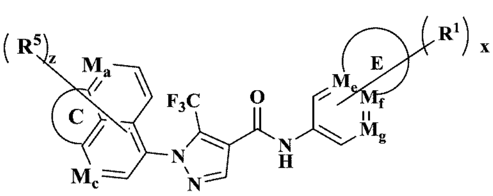

(V-A)

wherein:
$M_a$, $M_c$, $M_e$, $M_f$ or $M_g$ are each independently selected from -N- or -CH-.

**11.** The compound, or the stereoisomer or pharmaceutically acceptable salt thereof of any one of claims 1 to 9, wherein, the compound is further as represented by general formula (IV-B):

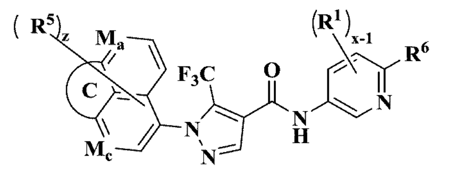

( IV-B )

wherein:

$M_a$ is selected from -N- or -CH-;
$M_c$ is selected from -N- or -CH-;
$R_6$ is selected from 3- to 7-membered heterocyclyl or 5- to 7-membered heteroaryl, wherein the 3- to 7-membered heterocyclyl and 5- to 7-membered heteroaryl are optionally further substituted with one or more substituents selected from deuterium, halogen, amino, hydroxyl, cyano, nitro, $C_{1-6}$ alkyl, $C_{2-6}$ alkenyl, $C_{2-6}$ alkynyl, oxo, thio, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12-membered heterocyclyl, $C_{6-12}$ aryl and 5- to 12-membered heteroaryl,
preferably, $R_6$ is selected from 4-membered heterocyclyl, 5-membered heterocyclyl, 6-membered heterocyclyl or 5-membered heteroaryl, wherein the 4-membered heterocyclyl, 5-membered heterocyclyl, 6-membered heterocyclyl and 5-membered heteroaryl are optionally further substituted with one or more substituents selected from deuterium, halogen, amino, hydroxyl, cyano, nitro, $C_{1-3}$ alkyl, $C_{2-4}$ alkenyl, $C_{2-4}$ alkynyl, oxo, thio, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 8-membered heterocyclyl, $C_{6-10}$ aryl and 5- to 8-membered heteroaryl,
and more preferably, $R_6$ is selected from

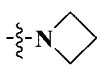 , 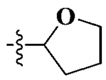 , 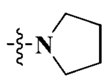 , 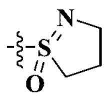 , 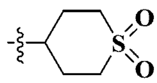 ,

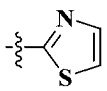 , 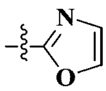 , 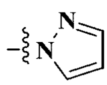 , 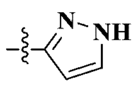 , 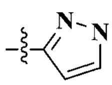 , 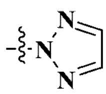 , 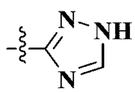 , 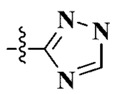 ,

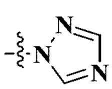 or 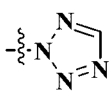 ,

optionally further substituted with one or more substituents selected from deuterium, fluorine, chlorine, bromine, amino, hydroxyl, cyano, nitro, methyl, ethyl, oxo, thio, difluoromethyl, trifluoromethyl, methoxy, ethoxy and trifluoromethoxy.

**12.** The compound, or the stereoisomer or pharmaceutically acceptable salt thereof of any one of claims 1 to 8, wherein, the compound is further as represented by general formula (IV-C):

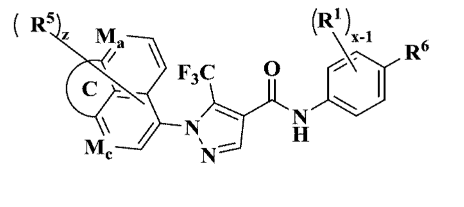

( IV-C )

wherein:

$M_a$ is selected from -N- or -CH-;

$M_c$ is selected from -N- or -CH-;

$R_6$ is selected from 3- to 7-membered heterocyclyl or 5- to 7-membered heteroaryl, wherein the 3- to 7-membered heterocyclyl and 5- to 7-membered heteroaryl are optionally further substituted with one or more substituents selected from deuterium, halogen, amino, hydroxyl, cyano, nitro, $C_{1-6}$ alkyl, $C_{2-6}$ alkenyl, $C_{2-6}$ alkynyl, oxo, thio, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12-membered heterocyclyl, $C_{6-12}$ aryl and 5- to 12-membered heteroaryl,

preferably, $R_6$ is selected from 4-membered heterocyclyl, 5-membered heterocyclyl, 6-membered heterocyclyl or 5-membered heteroaryl, wherein the 4-membered heterocyclyl, 5-membered heterocyclyl, 6-membered heterocyclyl and 5-membered heteroaryl are optionally further substituted with one or more substituents selected from deuterium, halogen, amino, hydroxyl, cyano, nitro, $C_{1-3}$ alkyl, $C_{2-4}$ alkenyl, $C_{2-4}$ alkynyl, oxo, thio, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 8-membered heterocyclyl, $C_{6-10}$ aryl and 5- to 8-membered heteroaryl,

and more preferably, $R_6$ is selected from

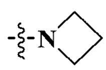, 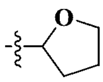, 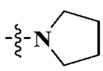, 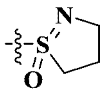, 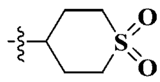,

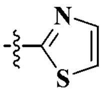, 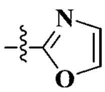, 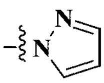, 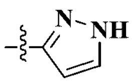, 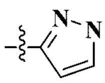, 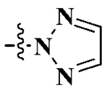, 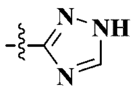, 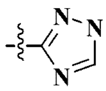, 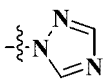

or

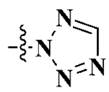,

optionally further substituted with one or more substituents selected from deuterium, fluorine, chlorine, bromine, amino, hydroxyl, cyano, nitro, methyl, ethyl, oxo, thio, difluoromethyl, trifluoromethyl, methoxy, ethoxy and trifluoromethoxy.

**13.** The compound, or the stereoisomer or pharmaceutically acceptable salt thereof of any one of claims 6 to 12, wherein, ring C is selected from

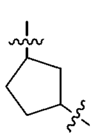, 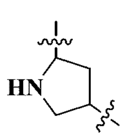, 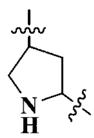, 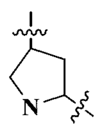,

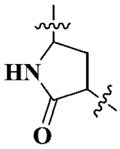, 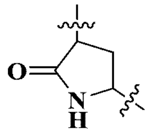, 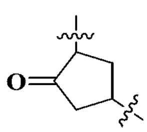, 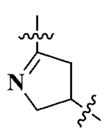, 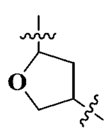, 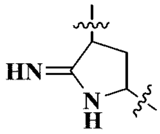, 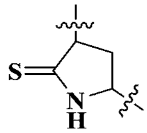,

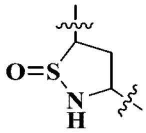, 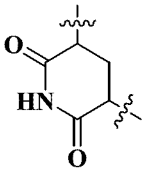, 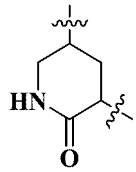, 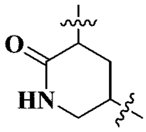, 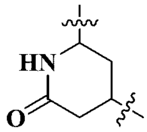, 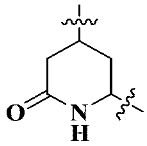, 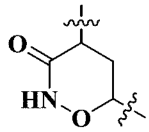,

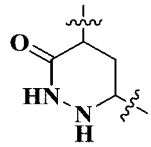 or 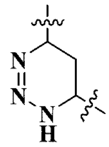,

and preferably, ring C is selected from

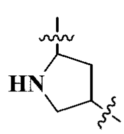, 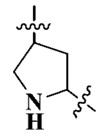, 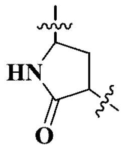, 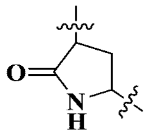,

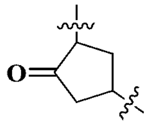, 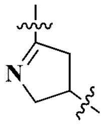, 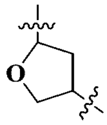, 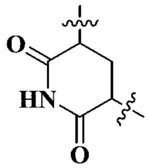, 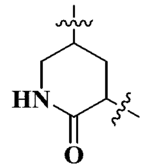, 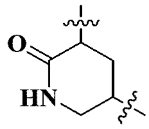, 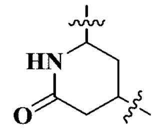 or

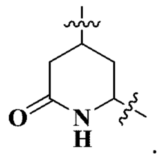.

**14.** The compound, or the stereoisomer or pharmaceutically acceptable salt thereof of any one of claims 1 to 13, wherein, the compound is further represented by general formula (VI):

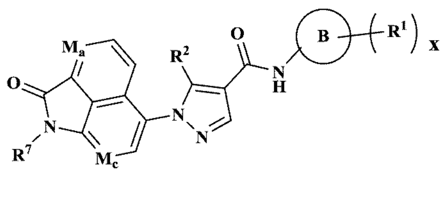

(VI)

$R^7$ is selected from hydrogen, deuterium, halogen, amino, hydroxyl, cyano, nitro, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$alkyl, $C_{3-6}$ cycloalkyl, 3- to 12- membered heterocyclyl, $C_{6-12}$ aryl, 5- to 12-membered heteroaryl, $-(CH_2)_{n2}C(O)R_a$, $-(CH_2)_{n2}C(O)OR_a$, $-(CH_2)_{n2}OR_a$, $-(CH_2)_{n2}R_a$ or - $(CH_2)_{n2}S(O)_{m2}R_a$, wherein the amino, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$alkyl, $C_{3-6}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-3}$ alkyl, $C_{2-4}$ alkenyl, $C_{2-4}$ alkynyl, oxo, thio, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered

heterocyclyl, $C_{6-12}$ aryl and 5- to 12-membered heteroaryl;

$R^7$ is preferably selected from hydrogen, deuterium, methyl, ethyl, propyl, isopropyl, methoxy, ethoxy, difluoromethyl, trifluoromethyl, difluoromethoxy, trifluoromethoxy, cyclopropyl, cyclobutyl, benzyl or p-methoxybenzyl;

$R_a$ is selected from hydrogen, deuterium, halogen, amino, hydroxyl, cyano, nitro, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl or 5- to 12-membered heteroaryl, wherein the amino, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-3}$ alkyl, $C_{2-4}$ alkenyl, $C_{2-4}$ alkynyl, oxo, thio, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12-membered heterocyclyl, $C_{6-12}$ aryl and 5- to 12-membered heteroaryl;

n2 is selected from 0, 1, 2 or 3;

and m2 is selected from 1 or 2.

**15.** The compound, or the stereoisomer or pharmaceutically acceptable salt thereof of any one of claims 1 to 14, wherein, the compound is further represented by general formula (VI-A), (VI-B), (VI-C), (VI-D), (VI-E), (VI-F) or (V!-G):

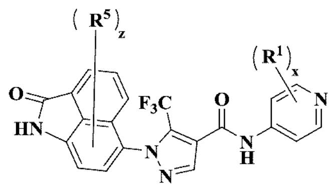

( VI-A)

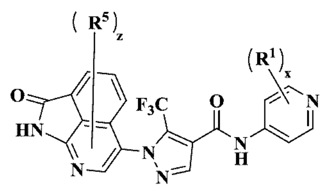

( VI-B)

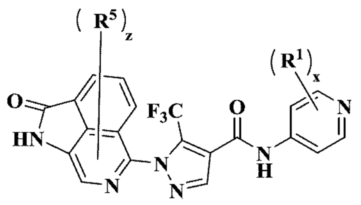

( VI-C)

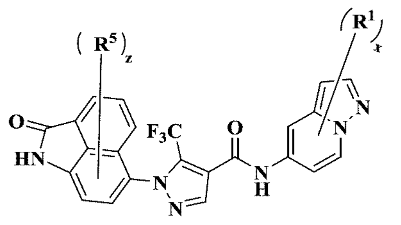

( VI-D)

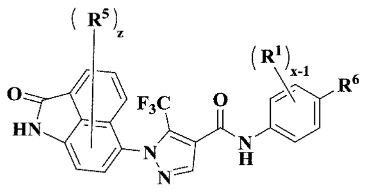

( VI-E)

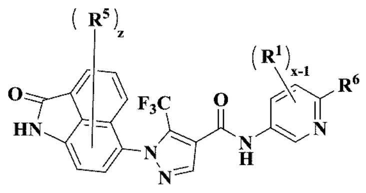

( VI-F)

or

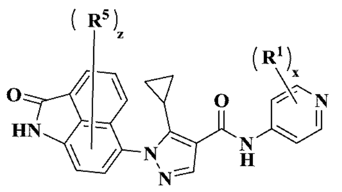

( VI-G) .

**16.** The compound, or the stereoisomer or pharmaceutically acceptable salt thereof of any one of claims 1 to 15, wherein,

$R^1$ is independently selected from hydrogen, deuterium, halogen, amino, hydroxyl, cyano, nitro, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12- membered heterocyclyl, $C_{6-12}$ aryl, 5- to 12-membered heteroaryl, $-CR_{cc}=CR_{dd}(CH_2)_nR_{aa}$, $- CR_{cc}=CR_{dd}(CH_2)_nNR_{aa}R_{bb}$, $-CR_{cc}=CR_{dd}(CH_2)_nNR_{ee}C(O)R_{aa}$, $-CR_{cc}=CR_{dd}(CH_2)_nN-R_{ee}C(O)NR_{aa}R_{bb}$, $- O(CH_2)_nR_{cc}$, $-OC(R_{aa}R_{bb})_n(CH_2)_mR_{cc}$, $-NR_{ee}(CH_2)_nR_{cc}$, $-(CH_2)_n-$, $-(CH_2)_nR_{cc}$, $-(CH_2)_nOR_{cc}$, $-(CH_2)_nSR_{cc}$, $- (CH_2)_nC(O)R_{cc}$, $-(CH_2)_nC(=NR_{ee})R_{cc}$, $-(CH_2)_nC(O)OR_{cc}$, $-(CH_2)_nS(O)_mR_{cc}$, $-(CH_2)_nNR_{aa}R_{bb}$, $-(CH_2)_nC(O)NR_{aa}R_{bb}$, $-(CH_2)_nNR_{ee}C(O)R_{cc}$, $-(CH_2)_nN=S(O)R_{cc}R_{ee}$ or$-(CH_2)_nNR_{ee}S(O)_mR_{cc}$, wherein the amino, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-6}$ alkyl, $C_{2-6}$ alkenyl, $C_{2-6}$ alkynyl, oxo, thio, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12- membered heterocyclyl, $C_{6-12}$ aryl and 5- to 12-membered heteroaryl,

$R^1$ is independently preferably selected from hydrogen, deuterium, halogen, amino, hydroxyl, cyano, nitro, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12- membered heterocyclyl, $C_{6-12}$ aryl, 5- to 12-membered heteroaryl, $-CR_{cc}=CR_{dd}(CH_2)_nR_{aa}$, $- CR_{cc}=CR_{dd}(CH_2)_nNR_{aa}R_{bb}$, $-CR_{cc}=CR_{dd}(CH_2)_nNR_{ee}C(O)R_{aa}$, $-CR_{cc}=CR_{dd}(CH_2)_nNR_{ee}C(O)NR_{aa}R_{bb}$, $- O(CH_2)_nR_{cc}$, $-OC(R_{aa}R_{bb})_n(CH2)_mR_{cc}$, $-NR_{ee}(CH_2)_nR_{cc}$, $-(CH_2)_n-$, $-(CH_2)_nR_{cc}$, $-(CH_2)_nOR_{cc}$, $-(CH_2)_nSR_{cc}$, $- (CH_2)_nC(O)R_{cc}$, $-(CH_2)_nC(=NR_{ee})R_{cc}$, $-(CH_2)_nC(O)OR_{cc}$, $-(CH_2)_nS(O)_mR_{cc}$, $-(CH_2)_nNR_{aa}R_{bb}$, $- (CH_2)_nC(O)NR_{aa}R_{bb}$, $-(CH_2)_nNR_{ee}C(O)R_{cc}$ or $-(CH_2)_nNR_{ee}S(O)_mR_{cc}$, wherein the amino, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-6}$ alkyl, $C_{2-6}$ alkenyl, $C_{2-6}$ alkynyl, oxo, thio, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12- membered heterocyclyl, $C_{6-12}$ aryl and 5- to 12-membered heteroaryl,

more preferably, $R^1$ is selected from hydrogen, deuterium, halogen, amino, hydroxyl, cyano, nitro, $C_{1-3}$ alkyl, $C_{2-6}$ alkenyl, $C_{2-6}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl, 5- to 12-membered heteroaryl, $-O(CH_2)_nR_{cc}$, $-OC(R_{aa}R_{bb})_n(CH2)_mR_{cc}$, $-(CH_2)_n-$, $-(CH_2)_nR_{cc}$ or $-(CH_2)_nOR_{cc}$, wherein the amino, $C_{1-3}$ alkyl, $C_{2-6}$ alkenyl, $C_{2-6}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-3}$ alkyl, $C_{2-6}$ alkenyl, $C_{2-6}$ alkynyl, oxo, thio, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12-membered heterocyclyl, $C_{6-12}$ aryl and 5- to 12-membered heteroaryl;

$R_{aa}$ and $R_{bb}$ are each independently selected from hydrogen, deuterium, halogen, amino, hydroxyl, cyano, nitro, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12-membered heterocyclyl, $C_{6-12}$ aryl or 5- to 12-membered heteroaryl, which are optionally substituted with one or more substituents selected from deuterium, halogen, amino, hydroxyl, cyano, nitro, $C_{1-6}$ alkyl, $C_{3-12}$ cycloalkyl, $-O(CH_2)_{n1}R_{c1}$, $-OC(R_{a1}R_{b1})_{m1}(CH_2)_{n1}R_{c1}$, $-NR_{a1}(CH_2)_{n1}R_{c1}$, $- (CH_2)_{n1}-$, $-(CH_2)_{n1}R_{c1}$, $-(CH_2)_{n1}OR_{c1}$, $-(CH_2)_{n1}SR_{c1}$, $-(CH_2)_{n1}C(O)R_{c1}$, $-(CH_2)_{n1}C(O)OR_{c1}$, $-(CH_2)_{n1}S(O)_{m1}R_{c1}$, $-(CH_2)_{n1}NR_{a1}R_{b1}$, $-(CH_2)_{n1}C(O)NR_{a1}R_{b1}$, $-(CH_2)_{n1}NR_{a1}C(O)R_{c1}$ and $-(CH_2)_{n1}NR_{a1}S(O)_{m1}R_{c1}$,

alternatively, $R_{aa}$ and $R_{bb}$ together with adjacent atoms form $C_{3-12}$ cycloalkyl, 3- to 12- membered heterocyclyl, $C_{6-12}$ aryl or 5- to 12-membered heteroaryl, wherein the $C_{3-12}$ cycloalkyl, 3- to 12-membered heterocyclyl, $C_{6-12}$ aryl and 5- to 12-membered heteroaryl are optionally substituted with one or more substituents selected from deuterium, halogen, amino, hydroxyl, cyano, nitro, $C_{1-6}$alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$alkyl, $C_{3-12}$ cycloalkyl, 3- to 12- membered heterocyclyl, $C_{6-12}$ aryl, 5- to 12-membered heteroaryl, $-O(CH_2)_{n1}R_{c1}$, $-OC(R_{a1}R_{b1})_{n1}(CH_2)_mR_{c1}$, $-NR_{a1}(CH_2)_{n1}R_{c1}$, $- (CH_2)_{n1}-$, $-(CH_2)_{n1}R_{c1}$, $-(CH_2)_{n1}OR_{c1}$, $-(CH_2)_{n1}SR_{c1}$, $-(CH_2)_{n1}C(O)R_{c1}$, $-(CH_2)_{n1}C(O)OR_{c1}$, $-(CH_2)_{n1}S(O)_mR_{c1}$, $N-(CH_2)_{n1}NR_{a1}R_{b1}$, $-(CH_2)_{n1}C(O)NR_{a1}R_{b1}$, $-(CH_2)_{n1}NR_{a1}C(O)R_{c1}$ and $-(CH_2)_{n1}NR_{a1}S(O)_{m1}R_{c1}$;

$R_{cc}$ is selected from hydrogen, deuterium, halogen, amino, hydroxyl, cyano, nitro, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12- membered heterocyclyl, $C_{6-12}$ aryl or 5- to 12-membered heteroaryl, which are optionally substituted with one or more substituents selected from deuterium, halogen, amino, hydroxyl, cyano,

nitro, $C_{1-6}$ alkyl, $C_{3-12}$ cycloalkyl, $-O(CH_2)_{n1}R_{c1}$, $-OC(R_{a1}R_{b1})_{n1}(CH_2)_mR_{c1}$, $-NR_{a1}(CH_2)_{n1}R_{c1}$, $-(CH_2)_{n1}$-, $-(CH_2)_{n1}R_{c1}$, $-(CH_2)_{n1}OR_{c1}$, $-(CH_2)_{n1}SR_{c1}$, $-(CH_2)_{n1}C(O)R_{c1}$, $-(CH_2)_{n1}C(O)OR_{c1}$, $-(CH_2)_{n1}S(O)_mR_{c1}$, $N-(CH_2)_{n1}NR_{a1}R_{b1}$, $-(CH_2)_{n1}C(O)NR_{a1}R_{b1}$, $-(CH_2)_{n1}NR_{a1}C(O)R_{c1}$ and $-(CH_2)_{n1}NR_{a1}S(O)_{m1}R_{c1}$;

$R_{a1}$, $R_{b1}$ and $R_{c1}$ are each independently selected from hydrogen, deuterium, halogen, amino, hydroxyl, cyano, nitro, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ alkoxy, $C_{1-6}$ haloalkoxy, $C_{1-6}$ hydroxyalkyl, cyano-substituted $C_{1-6}$ alkyl, $C_{3-12}$ cycloalkyl, 3- to 12-membered heterocyclyl, $C_{6-12}$ aryl or 5- to 12-membered heteroaryl, which are optionally substituted with one or more substituents selected from deuterium, halogen, amino, hydroxyl, cyano, nitro, $C_{1-6}$ alkyl and $C_{3-12}$ cycloalkyl;

$m_1$ is 0, 1, 2 or 3; and

$n_1$ is 0, 1, 2 or 3.

**17.** The compound, or the stereoisomer or pharmaceutically acceptable salt thereof of any one of claims 1 to 16, wherein, $R^1$ is selected from hydrogen, deuterium, fluorine, chlorine, bromine, amino, hydroxyl, cyano, nitro, methyl, ethyl, propyl, isopropyl, oxo, methoxy, ethoxy, difluoromethyl, trifluoromethyl, difluoromethoxy, trifluoromethoxy, cyclopropyl, cyclobutyl, oxetanyl, azetidinyl, triazolyl, oxazolyl, isoxazolyl, pyrazolyl, pyrrolyl or thiazolyl, wherein the cyclopropyl, cyclobutyl, oxetanyl, azetidinyl, triazolyl, oxazolyl, isoxazolyl, pyrazolyl, pyrrolyl and thiazolyl are optionally substituted with one or more substituents selected from deuterium, fluorine, chlorine, bromine, amino, hydroxyl, cyano, nitro, $C_{1-3}$ alkyl, oxo, thio, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl and cyano-substituted $C_{1-3}$ alkyl.

**18.** The compound, the stereoisomer or pharmaceutically acceptable salt thereof of any one of claims 1 to 17, wherein, the compound has the following structure:

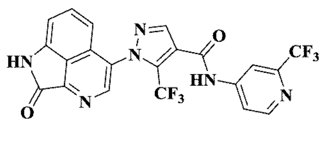 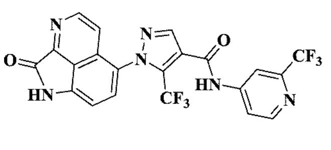 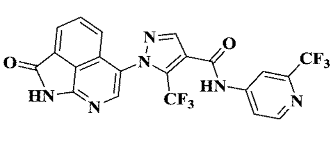

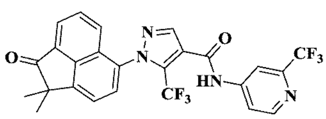 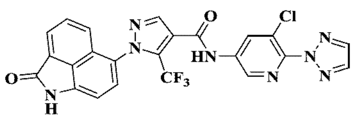 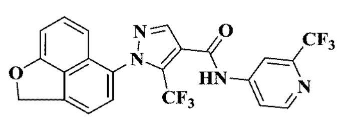

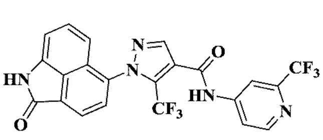 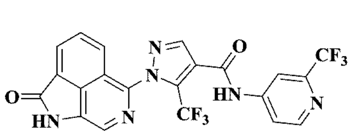 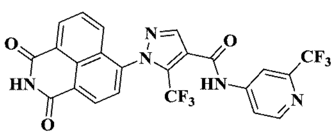

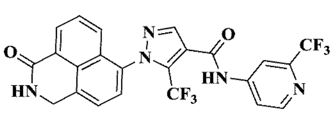 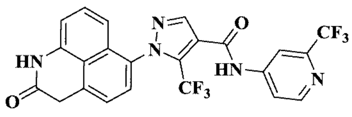 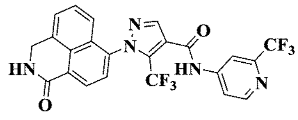

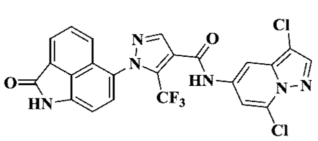 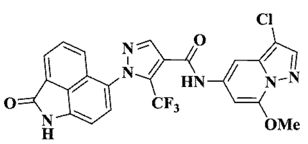 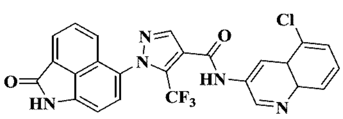

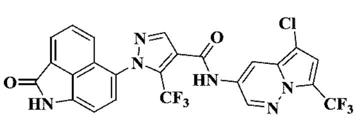 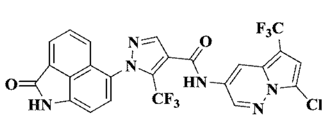 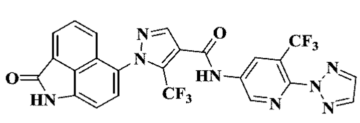

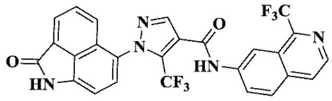 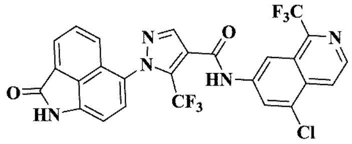 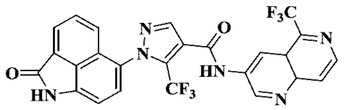

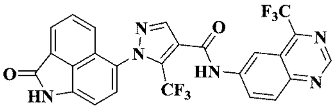 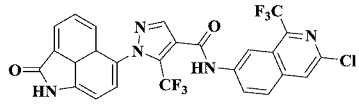 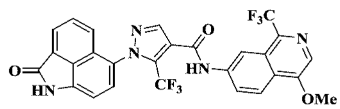

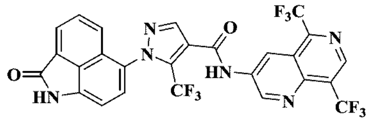 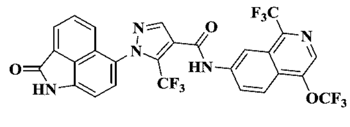 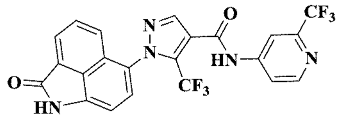

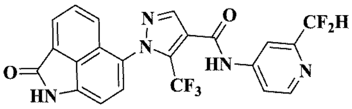 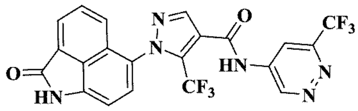 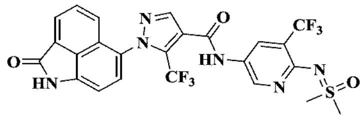

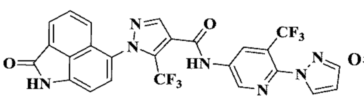 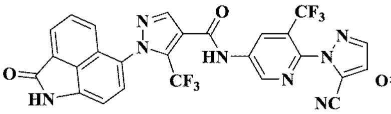 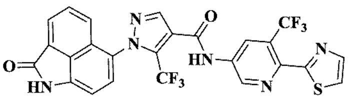

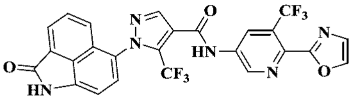 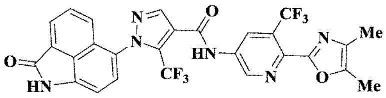 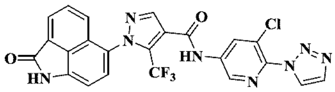

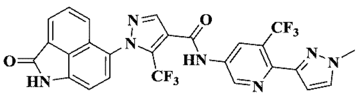 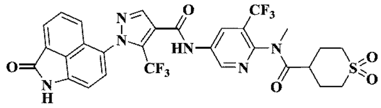 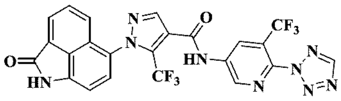

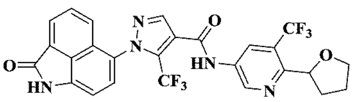 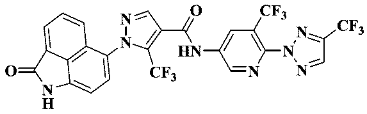 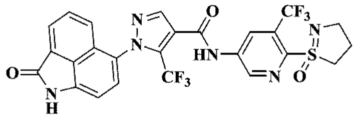

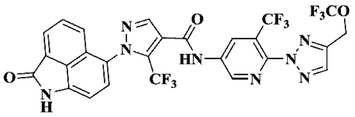 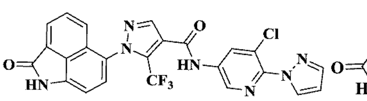 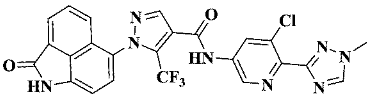

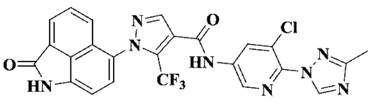 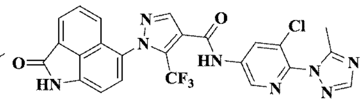 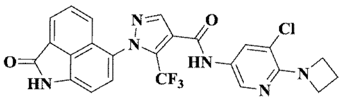

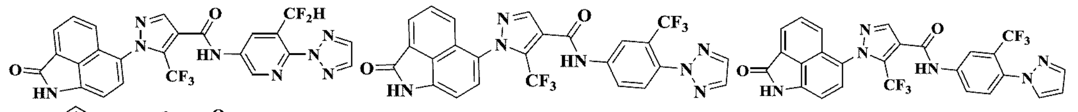

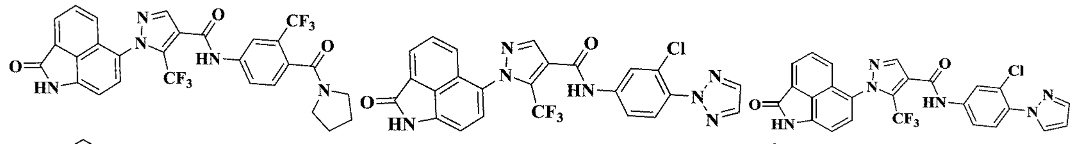

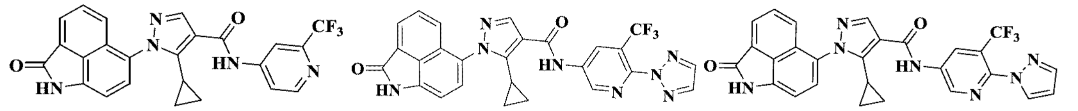

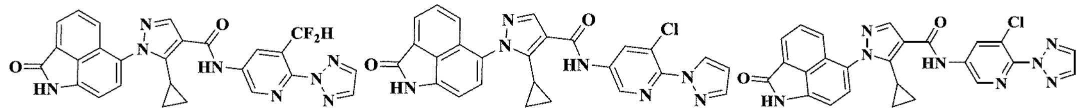

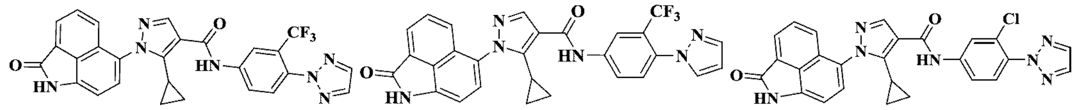

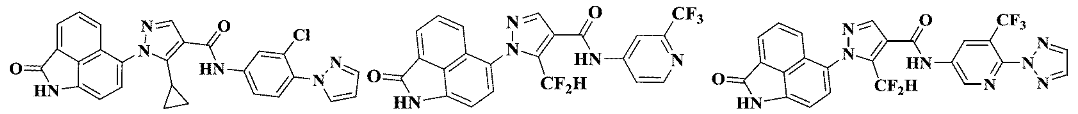

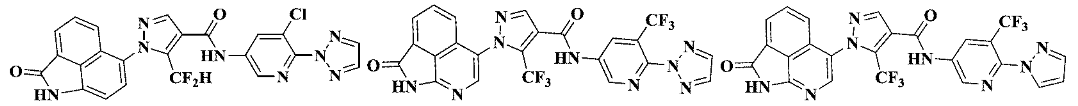

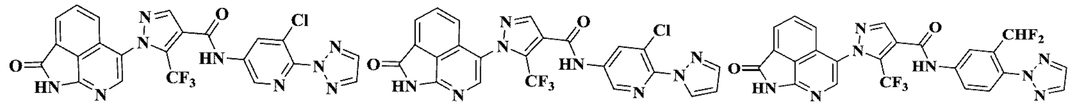

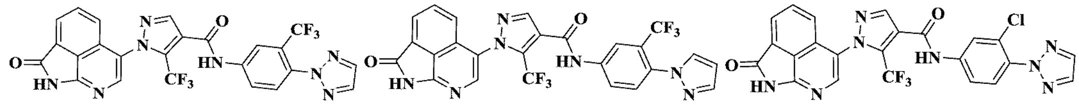

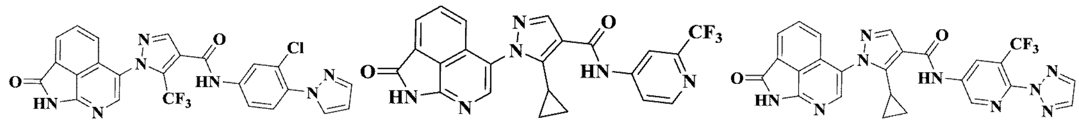

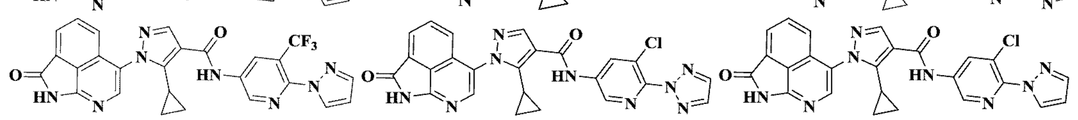

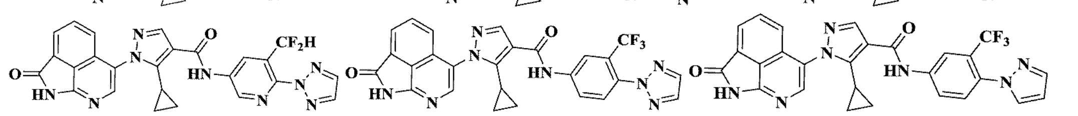

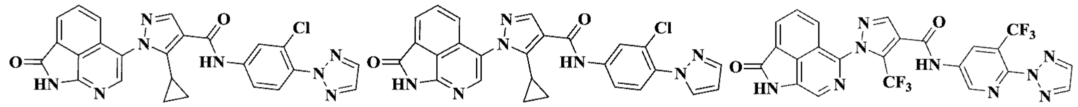

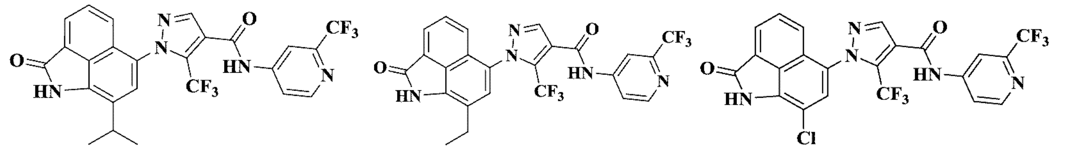

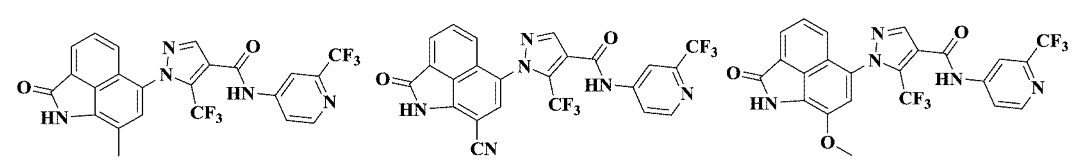

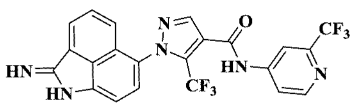 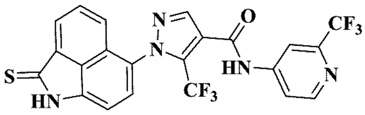 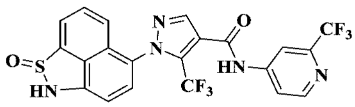

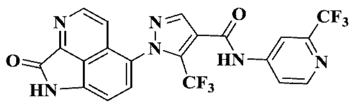 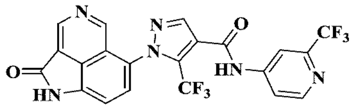 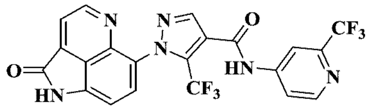

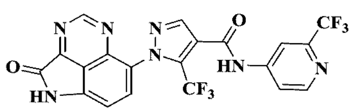 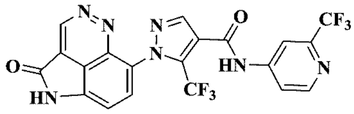 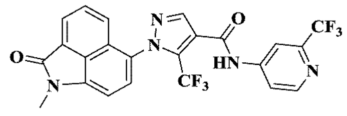

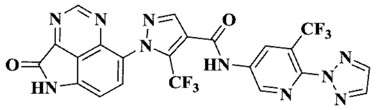 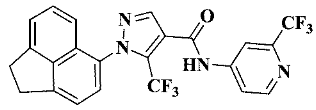 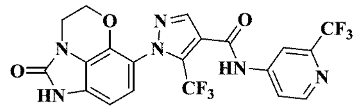

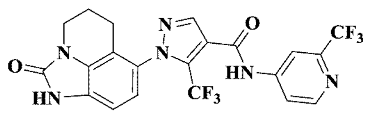 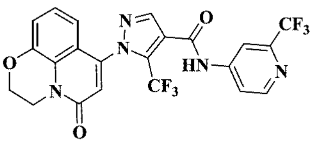 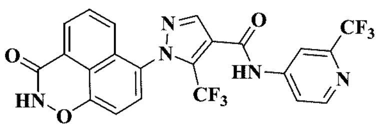

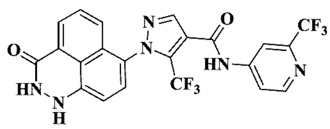 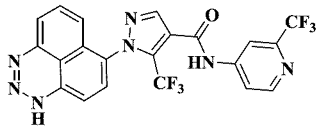 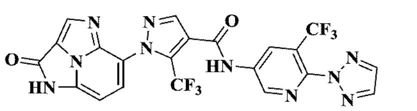

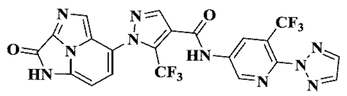 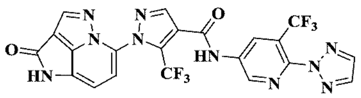 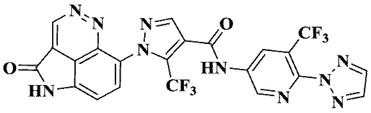

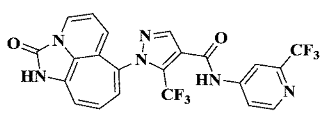 or 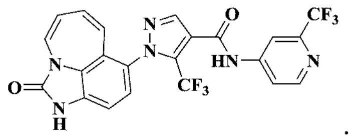 .

**19.** A compound represented by general formula (X-A), a stereoisomer or pharmaceutically acceptable salt thereof:

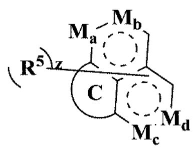

( X-A )

wherein,
$M_a$, $M_b$, $M_c$, $M_d$, $R^5$, ring C and z are as defined in any of the preceding claims.

**20.** A compound represented by general formula (X-B), a stereoisomer or pharmaceutically acceptable salt thereof:

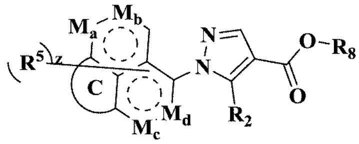

( X-B )

wherein,

$R^8$ is selected from hydrogen, deuterium, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl or 5- to 12-membered heteroaryl, wherein the $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl and 5- to 12-membered heteroaryl are optionally substituted with one or more substituents selected from deuterium, halogen, amino, hydroxyl, cyano, nitro, $C_{1-3}$ alkyl, $C_{2-4}$ alkenyl, $C_{2-4}$ alkynyl, oxo, thio, $C_{1-3}$ deuteroalkyl, $C_{1-3}$ haloalkyl, $C_{1-3}$ alkoxy, $C_{1-3}$ haloalkoxy, $C_{1-3}$ hydroxyalkyl, cyano-substituted $C_{1-3}$ alkyl, $C_{3-6}$ cycloalkyl, 3- to 12- membered heterocyclyl, $C_{6-12}$ aryl and 5- to 12-membered heteroaryl,

preferably, $R^8$ is selected from hydrogen, deuterium, or $C_{1-3}$ alkyl;

$M_a$, $M_b$, $M_c$, $M_d$, $R^2$, $R^5$, ring C and z are as defined in any of the preceding claims.

**21.** A method for preparing the compound of general formula (X-B), the stereoisomer or pharmaceutically acceptable salt thereof, comprising the following steps:

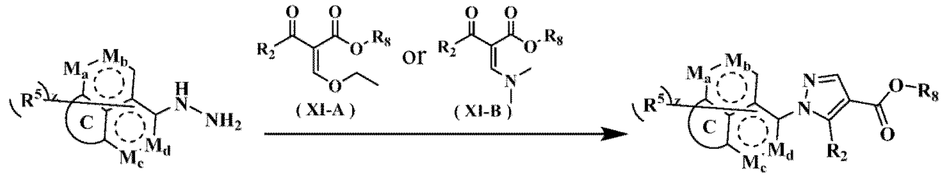
( XI-A ) or ( XI-B )

( X-E ) ( X-B )

reacting the compound of general formula (X-E) with

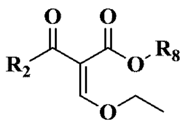

( XI-A )

or

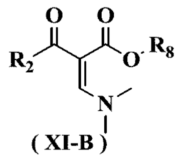

( XI-B )

to obtain the compound of general formula (X-B);

$M_a$, $M_b$, $M_c$, $M_d$, $R^2$, $R^5$, $R^8$, ring C and z are as defined in any of the preceding claims.

**22.** A method for preparing the compound, the stereoisomer or pharmaceutically acceptable salt thereof of claim 7, comprising the following steps:

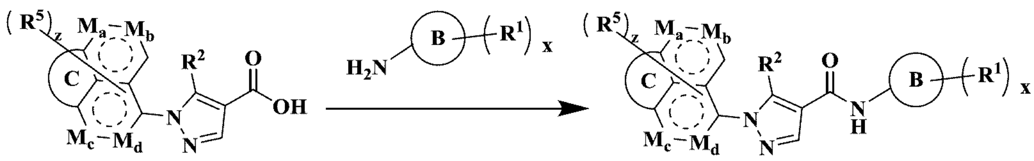

( X-F ) ( III )

subjecting the compound of general formula (X-F) to amide condensation in the presence of a condensing agent to obtain a compound of general formula (III);

the condensing agent is selected from phosphorus oxychloride, thionyl chloride, oxalyl chloride, diisopropyl azodicarboxylate, diethyl azodicarbonate, carbodiimide hydrochloride, N,N'-diisopropylcarbodiimide, dicyclohexylcarbodiimide, HATU, HBTU, HOBT, TCFH, PyBop or PyClock;

and $M_a$, $M_b$, $M_c$, $M_d$, $R^2$, $R^5$, $R^1$, ring B, ring C, x and z are as defined in any of the preceding claims.

**23.** A pharmaceutical composition comprising a therapeutically effective dose of the compound, the stereoisomer or pharmaceutically acceptable salt thereof of any one of claims 1 to 18, and one or more pharmaceutically acceptable carriers, diluents or excipients.

**24.** Use of the compound, the stereoisomer or pharmaceutically acceptable salt thereof of any one of claims 1 to 18 or the pharmaceutical composition of claim 23 in the preparation of an MALT1 inhibitor drug.

**25.** Use of the compound, the stereoisomer or pharmaceutically acceptable salt thereof of any one of claims 1 to 18 or the pharmaceutical composition of claim 23 in the preparation of a drug for the treatment of a cancer or an autoimmune disease; wherein the cancer or the autoimmune disease is selected from B-cell lymphoma, non-Hodgkin lymphoma, mantle cell lymphoma, follicular lymphoma, mucosa-associated lymphoid tissue lymphoma, chronic lymphocytic leukemia, rheumatoid arthritis, psoriatic arthritis, psoriasis, ulcerative colitis, Crohn's disease, systemic lupus erythematosus, asthma and chronic obstructive pulmonary disease.

**INTERNATIONAL SEARCH REPORT**

International application No.
**PCT/CN2022/143665**

**A. CLASSIFICATION OF SUBJECT MATTER**

C07D401/14(2006.01)i;C07D417/14(2006.01)i;C07D487/04(2006.01)i;A61K31/4725(2006.01)i;A61P35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

REGISTRY(STN), CAPLUS(STN), CNKI, DWPI: 上海翰森生物医药科技有限公司, 江苏豪森药业集团有限公司, 吡唑, 酰胺, 苯并吡啶, 喹啉, 异喹啉, 哒嗪, MALT1, 抑制剂, 免疫, 炎症, 炎性, 肿瘤, 癌, pyrazole, carboxamide, inhibitor?, structural formula search

**C. DOCUMENTS CONSIDERED TO BE RELEVANT**

| Category* | Citation of document, with indication, where appropriate, of the relevant passages | Relevant to claim No. |
|---|---|---|
| X | CN 110214136 A (JANSSEN PHARMACEUTICA NV) 06 September 2019 (2019-09-06) claims 1 and 29-32, and description, pp. 22-99, table 1 | 1-5, 8, 16-17, 23-25 |
| X | CN 112585128 A (JANSSEN PHARMACEUTICA NV) 30 March 2021 (2021-03-30) claims 1 and 26-30, and description, pp. 16-48, table 1 | 1, 8, 16-17, 23-25 |
| X | CN 112601747 A (JANSSEN PHARMACEUTICA NV) 02 April 2021 (2021-04-02) claims 1 and 14-18, and description, pp. 6-12, table 1 | 1-2, 4-5, 8, 16-17, 23-25 |
| X | CN 113473971 A (JANSSEN PHARMACEUTICA NV) 01 October 2021 (2021-10-01) claims 1, 20-21 and 28-30, and description p. 2, paragraph [0014] | 1-5, 8, 16-17, 23-25 |
| X | CN 112125885 A (CHINA PHARMACEUTICAL UNIVERSITY) 25 December 2020 (2020-12-25) claim 6 | 19 |
| X | CN 109665987 A (HENAN UNIVERSITY) 23 April 2019 (2019-04-23) description, p. 2, paragraph 8 and p. 3, paragraphs 9-12 | 19 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 March 2023** | **22 March 2023** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**

International application No.
**PCT/CN2022/143665**

**C. DOCUMENTS CONSIDERED TO BE RELEVANT**

| Category* | Citation of document, with indication, where appropriate, of the relevant passages | Relevant to claim No. |
|---|---|---|
| PX | CN 114907358 A (SHANGHAI HANSOH BIOMEDICAL CO., LTD. et al.) 16 August 2022 (2022-08-16)<br>claims 1 and 13, and description, pp. 43-46 | 8, 23 |
| PX | WO 2022185097 A1 (JANSSEN PHARMACEUTICA NV.) 09 September 2022 (2022-09-09)<br>claims 1, 21-22 and 41 | 3, 23-25 |
| PX | WO 2022262855 A1 (TUOJIE BIOTECH (SHANGHAI) CO., LTD.) 22 December 2022 (2022-12-22)<br>claims 1 and 14-16, and description, pp. 40-42, 45-47, 49-50, 52-79 and 83-84 | 3, 23-25 |
| PX | WO 2022081928 A1 (C4 THERAPEUTICS, INC.) 21 April 2022 (2022-04-21)<br>description, p. 396, Scheme5, p. 397, Scheme7, p. 421, embodiment 7, pp. 425-426m embodiments 8-9, and pp. 428-431, embodiments 10-13 | 19 |
| A | CN 110214136 A (JANSSEN PHARMACEUTICA NV) 06 September 2019 (2019-09-06)<br>claims 1 and 29-32, and description, pp. 22-99, table 1 | 6-7, 9-15, 18, 20-22 |

Form PCT/ISA/210 (second sheet) (July 2022)

**INTERNATIONAL SEARCH REPORT**
**Information on patent family members**

International application No.
**PCT/CN2022/143665**

| Patent document cited in search report | Publication date (day/month/year) | Patent family member(s) | Publication date (day/month/year) |
|---|---|---|---|
| CN 110214136 A | 06 September 2019 | JP 2020514267 A | 21 May 2020 |
| | | JP 7138641 B2 | 16 September 2022 |
| | | WO 2018119036 A1 | 28 June 2018 |
| | | IL 267343 A | 29 August 2019 |
| | | US 2018170909 A1 | 21 June 2018 |
| | | US 10954214 B2 | 23 March 2021 |
| | | UY 37537 A | 29 June 2018 |
| | | BR 112019012355 A2 | 26 November 2019 |
| | | CA 3048027 A1 | 28 June 2018 |
| | | AU 2017382185 A1 | 13 June 2019 |
| | | AU 2017382185 B2 | 02 December 2021 |
| | | AU 2017382185 C9 | 04 August 2022 |
| | | AU 2017382185 C1 | 11 August 2022 |
| | | TW 201835067 A | 01 October 2018 |
| | | AU 2022201352 A1 | 24 March 2022 |
| | | EP 3558969 A1 | 30 October 2019 |
| | | US 2022315557 A1 | 06 October 2022 |
| | | MA 47125 A | 28 April 2021 |
| | | EA 201991503 A1 | 29 November 2019 |
| | | MX 2019007540 A | 24 October 2019 |
| | | PE 20191755 A1 | 12 December 2019 |
| | | PH 12019501212 A1 | 16 December 2019 |
| | | AR 110421 A1 | 27 March 2019 |
| | | CO 2019006622 A2 | 28 June 2019 |
| | | CL 2019001709 A1 | 04 October 2019 |
| | | KR 20190093669 A | 09 August 2019 |
| | | TN 2019000192 A1 | 05 October 2020 |
| CN 112585128 A | 30 March 2021 | BR 112020025844 A2 | 23 March 2021 |
| | | EA 202190055 A1 | 23 April 2021 |
| | | US 2019381019 A1 | 19 December 2019 |
| | | US 11040031 B2 | 22 June 2021 |
| | | CA 3104053 A1 | 26 December 2019 |
| | | KR 20210024548 A | 05 March 2021 |
| | | EP 3807266 A1 | 21 April 2021 |
| | | AU 2019289222 A1 | 07 January 2021 |
| | | WO 2019243964 A1 | 26 December 2019 |
| | | JP 2021527654 A | 14 October 2021 |
| | | PH 12020552207 A1 | 28 June 2021 |
| | | IL 279453 A | 31 January 2021 |
| | | IL 279453 B | 01 June 2022 |
| CN 112601747 A | 02 April 2021 | KR 20210024002 A | 04 March 2021 |
| | | PH 12020552206 A1 | 28 June 2021 |
| | | CA 3104055 A1 | 26 December 2019 |
| | | IL 279452 A | 31 January 2021 |
| | | IL 279452 B | 01 June 2022 |
| | | WO 2019243965 A1 | 26 December 2019 |
| | | JP 2021528401 A | 21 October 2021 |
| | | EA 202092962 A1 | 06 September 2021 |
| | | BR 112020025814 A2 | 06 April 2021 |
| | | US 2019381012 A1 | 19 December 2019 |

Form PCT/ISA/210 (patent family annex) (July 2022)

**INTERNATIONAL SEARCH REPORT**
**Information on patent family members**

International application No.
**PCT/CN2022/143665**

| Patent document cited in search report | Publication date (day/month/year) | Patent family member(s) | Publication date (day/month/year) |
|---|---|---|---|
| | | US 10888550 B2 | 12 January 2021 |
| | | AU 2019289223 A1 | 07 January 2021 |
| | | EP 3810609 A1 | 28 April 2021 |
| CN 113473971 A | 01 October 2021 | CA 3129356 A1 | 27 August 2020 |
| | | TW 202045159 A | 16 December 2020 |
| | | WO 2020169738 A1 | 27 August 2020 |
| | | JP 2022523371 A | 22 April 2022 |
| | | EP 3927324 A1 | 29 December 2021 |
| | | PE 20212323 A1 | 14 December 2021 |
| | | CR 20210480 A | 10 November 2021 |
| | | SG 11202109102 PA | 29 September 2021 |
| | | IL 285674 A | 31 October 2021 |
| | | MA 55015 A | 29 December 2021 |
| | | EA 202192322 A1 | 03 December 2021 |
| | | US 2022175760 A1 | 09 June 2022 |
| | | KR 20210132099 A | 03 November 2021 |
| | | ECSP 21067816 A | 30 December 2021 |
| | | UY 38593 A | 31 August 2020 |
| | | AU 2020225342 A1 | 19 August 2021 |
| CN 112125885 A | 25 December 2020 | None | |
| CN 109665987 A | 23 April 2019 | None | |
| CN 114907358 A | 16 August 2022 | None | |
| WO 2022185097 A1 | 09 September 2022 | None | |
| WO 2022262855 A1 | 22 December 2022 | None | |
| WO 2022081928 A1 | 21 April 2022 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018119036 A
- WO 2019243964 A
- WO 2019243965 A
- WO 2020208222 A
- WO 2015181747 A
- WO 2017081641 A
- WO 2018020474 A
- WO 2018226150 A
- WO 2020111087 A
- WO 2014074815 A
- WO 2018165385 A
- WO 2018085247 A
- WO 2017057695 A
- WO 2018021520 A
- WO 2018159650 A
- WO 2004002992 A
- US 20160240789 A1
- US 6667303 B1
- US 20180170909 A1
- US 20140107096 A
- US 20200347052 A
- US 2020102311 A1
- WO 2018020474 A1
- WO 2006122806 A
- WO 2019039429 A1
- WO 2022081928 A1
- WO 2022081927 A
- WO 2022089406 A1


### Non-patent literature cited in the description


- **WANG, HAO et al.** Chemical Communications. Cambridge, 2014, vol. 50, 13485-13488
- *Chemistry -A European Journal,* 2021, vol. 27 (63), 15716-15721
- *Tetrahedron,* 2005, vol. 61 (21), 4983-4987
- **CHARIFSON, PAUL S. et al.** *Journal of Medicinal-Chemistry,* 2008, vol. 51 (17), 5243-5263
- *Journal of the American Chemical Society,* 1954, vol. 76, 3352-3353